# EUROPEAN PATENT SPECIFICATION

(11) **EP 2 635 704 B1**
(45) Date of publication and mention of the grant of the patent: **19.04.2017**
(21) Application number: 11785527.0
(22) Date of filing: 01.11.2011
(51) Int. Cl.: C12Q 1/68, G06F 19/18

(54) **PREDICTING PROGRESSION TO ADVANCED AGE-RELATED MACULAR DEGENERATION USING A POLYGENIC SCORE**
VORHERSAGE DER PROGRESSION VON FORTGESCHRITTENER ALTERSBEDINGTER MAKULADEGENERATION MIT EINER POLYGENEN WERTUNG
PRÉDICTION DE PROGRESSION VERS UNE DÉGÉNÉRESCENCE MACULAIRE LIÉE À L'ÂGE DE STADE AVANCÉ À L'AIDE DE SCORE POLYGÉNIQUE

(30) Priority: 01.11.2010 US 409039 P; 09.09.2011 US 201161573602 P
(43) Date of publication of application: 11.09.2013
(60) Divisional application: 16193134.0
(73) Proprietor: F. Hoffmann-La Roche AG, 4070 Basel (CH)
(72) Inventor: BEHRENS, Timothy, W., South San Francisco, CA 94080 (US); GRAHAM, Robert, R., South San Francisco, CA 94080 (US)
(74) Representative: Brodbeck, Michel
(86) International application number: PCT/US2011/058829
(87) International publication number: WO 2012/061421

(56) References cited:
- WO-A1-2009/146204
- WO-A2-2005/086770
- WO-A2-2010/075519
- WO-A2-2010/085542
- NAJAF AMIN ET AL: "Genetic Scoring Analysis: a way forward in Genome Wide Association Studies?", EUROPEAN JOURNAL OF EPIDEMIOLOGY, KLUWER ACADEMIC PUBLISHERS, DO, vol. 24, no. 10, 2 September 2009 (2009-09-02), pages 585-587, XP019748010, ISSN: 1573-7284, DOI: 10.1007/S10654-009-9387-Y
- SHAUN M. PURCELL ET AL: "Common polygenic variation contributes to risk of schizophrenia and bipolar disorder", NATURE, 1 July 2009 (2009-07-01), XP55014697, ISSN: 0028-0836, DOI: 10.1038/nature08185
- A DEMIRKAN ET AL: "Genetic risk profiles for depression and anxiety in adult and elderly cohorts", MOLECULAR PSYCHIATRY, vol. 16, no. 7, 23 June 2010 (2010-06-23), pages 773-783, XP55014698, ISSN: 1359-4184, DOI: 10.1038/mp.2010.65
- KATHIRESAN S ET AL: "Polymorphisms Associated With Cholesterol and Risk of Cardiovascular Events", JOURNAL OF VASCULAR SURGERY, C.V. MOSBY CO., ST. LOUIS, MO, US, vol. 47, no. 6, 1 June 2008 (2008-06-01), page 1372, XP022689697, ISSN: 0741-5214, DOI: 10.1016/J.JVS.2008.04.037 [retrieved on 2008-05-30]
- W. CHEN ET AL: "Genetic variants near TIMP3 and high-density lipoprotein-associated loci influence susceptibility to age-related macular degeneration", PROCEEDINGS OF THE NATIONAL ACADEMY OF SCIENCES, vol. 107, no. 16, 20 April 2010 (2010-04-20), pages 7401-7406, XP55014711, ISSN: 0027-8424, DOI: 10.1073/pnas.0912702107
- DATABASE MEDLINE [Online] US NATIONAL LIBRARY OF MEDICINE (NLM), BETHESDA, MD, US; May 2010 (2010-05), LEVEZIEL NICOLAS ET AL: "[Genetic factors associated with age-related macular degeneration].", XP002665682, Database accession no. NLM20510150 & MÉDECINE SCIENCES : M/S MAY 2010 LNKD- PUBMED:20510150, vol. 26, no. 5, May 2010 (2010-05), pages 509-515, ISSN: 0767-0974

## Description

### Field of the Invention

The present invention concerns methods for identifying individuals with intermediate age-related macular degeneration (AMD) at greater risk of progression to advanced AMD, using a polygenic score calculated based on the results of genome-wide gene association studies, using thousands of single-nucleotide polymorphisms (SNPs).

### Background of the Invention

### Age-Related Macular Degeneration (AMD)

AMD is age-related degeneration of the macula, which is the leading cause of irreversible visual dysfunction in individuals over the age of 60. Two types of AMD exist, non-exudative (dry) and exudative (wet) AMD. The dry, or nonexudative, form involves atrophic and hypertrophic changes in the retinal pigment epithelium (RPE) underlying the central retina (macula) as well as deposits (drusen) on the RPE. Patients with nonexudative AMD can progress to the wet, or exudative, form of AMD. As the disease progresses, drusen formed initially grow in size and number. In advanced stages of AMD abnormal blood vessels called choroidal neovascular membranes (CNVMs) develop under the retina, leak fluid and blood, and ultimately cause a blinding disciform scar in and under the retina. Nonexudative AMD, which is usually a precursor of exudative AMD, is more common.

### Genomwide association studies

Parallel with sequencing the human genome, an international effort was undertaken with the goal to develop a haplotype map of the human genome, the HapMap, which describes the common patterns of human DNA sequence variation. The HapMap project started in 2002, and its results have been make freely available to the public through periodic releases. In addition, rapid improvements in genotyping techniques and analysis have enabled genomwide association studies on large populations to identify genetic variations with significant population frequences. This, in turn, allowed the investigation of polygenic diseases and traits. Since then, genomwide association studies have identified numerous genetic loci in which common genetic variants, reproducibly associated with polygenic traits, occur. See, e.g. Altshuler et al., Science (2008), 322:881-8 (genetic mapping in human disease); Mohkle et al., Hum Mol Genet (2008) 17:R102-R108 (common genetic variations associated with metabolic and cardiovasular diseases); Lettre et al., Hum Mol Genet (2008):17-R116-R121 (common genetic variations associated with autoimmune diseases); Purcell et al., Nature (2009) 460(7256):748-52 (common genetic variations associated with risk of schizophrenia and bipolar disorder); and Wei et al., PLoS Genet. (2009) 5(10):e1000678. Epub 2009 Oct 9 (Type 1 diabetes).

Johanna M. Seddon, M.D., Sc.M., of Tufts-New England Medical Center, Boston, and colleagues assessed whether certain genetic variants have prognostic importance for progression to advanced AMD and related visual loss, and reported their findings in Seddon et al., JAMA (2007) 297:1793-1800. The study included 1,466 white participants in the Age-Related Eye Disease Study (AREDS), a U.S. multicenter clinical trial conducted from 1990 to 2001 with an average follow-up time of 6.3 years. During the study, 281 participants progressed to advanced AMD in one or both eyes, which included: geographic atrophy (results in thinning and discoloration of the retina), exudative disease (the escape of fluid, cells, and cellular debris from blood vessels), or AMD causing visual loss. Based on genotyping analysis, common polymorphisms in the genes CFH and LOC387715 were identified as being independently related to AMD progression from early stages of AMD (drusen and pigment alterations) to advanced forms of AMD (geographic atrophy or neovascular AMD), which cause visual impairment or blindness. The researchers found that the genetic polymorphisms, CFH Y402H and LOC387715 A69S, were associated with progression to more advanced AMD, with the risk of progression being 2.6 times higher for CFH and 4.1 times higher for LOC387715 risk genotypes after controlling for other factors associated with AMD. The probability of progression was 48 percent for the highest-risk genotype vs. 5 percent for the low-risk genotypes. The presence of all adverse factors (both risk genotypes, smoking, and body mass index 25 or greater) increased risk 19-fold. Smoking and high body mass index increased odds of progression within each risk genotype.

The same group reported results of a later study investigatig the joint effects of genetic, ocular, and environmental variables and predictive models for prevalence and incidence of AMD. (Seddon et al., Investigative Ophthalmology & Visual Science (2009) 50:2044-53. The authors found independent association of six genetic variants (CFH Y402H; CFH rs1410996; LOC387715 A69S (ARMS2); C2 E318D; CFB; C3 R102G) with both prevalence and incidence of advanced AMD. According to the authors, all of these variants except CFB were significantly related to progression to advanced AMD, after controlling for baseline AMD grade and other factors.

Amin et al., European Journal of Epidemiology (2009) 24:585-587; Purcell et al., Nature (2009) 460:748-752; Kathiresan eta al., Journal of Vascular Surgery (2008) 47:1372; and WO2005/086770 (2005) all separately disclose the use of polygenic score for determining a predisposition for multifactorial diseases (schizophrenia and bipolar disorder: Purcell et al., Nature (2009) 460:748-752; cholesterol and risk of cardiovascular events: Kathiresan et al., Journal of Vascular Surgery (2008) 47:1372). They do not relate to advanced age-related macular degeneration (AMD).

WO2009/146204 (2009) discloses the association between rs13117504 and AMD progression. In claim 3 also other polymorphic markers are disclosed.

WO2010/085542; WO2010/075519 and Chen et al., Proceedings of the National Academy of Sciences (2010) 107:7401-7406 all separately disclose the association between polymorphic markers and AMD but do not calculate a polygenic score.

Leveziel et al., Database Medline (2010) Accession Number NLM20510150 discloses that AMD is a multifactorial and polygenic disease and discloses polymorphic markers associated with AMD.

It is established that both genetic, demographic (e.g. age, gender) and environmental (e.g. smoking) factors contribute to the development and progression of AMD, where genetic factors including single nucleotide polymorphisms (SNPs), copy number variants (CNVs) and apigeneic variants, associated with DNA methylation or histone modification. However, the relative contributions of these factors, including contribution of each class of genetic variation to disease risk or progression is as of yet unknown. Accordingly, there is a need for better understanding and tools to predict the likelihood of developing AMD or, for patients already diagnosed with AMD, the risk that their condition will progress.

### Summary of the Invention

The present invention is based, at least in part on the recognition that thousands of common genetic variants with modest effect sizes contribute to the progression of intermediate AMD to advanced AMD, and in aggregate, a polygenic score can explain and predict the risk of progression from intermediate AMD to advanced AMD.

In one aspect, the invention concerns a method for assessing a human subject's risk for developing advanced age-related macular degeneration (AMD), comprising determining in a
biological sample from the subject the presence or absence of risk alleles of common allelic variants associated with AMD at a plurality of independent loci.

In a particular embodiment, determination of the presence or absence of risk alleles is followed by calculating the p olygenic score for the subject, wherein a high polygenic score indicates a higher risk for developing advanced AMD.

The allelic frequency is determined at at least 10,000 independent loci.

In another embodiment, the subject has been diagnosed with early stage AMD.

In yet another embodiment, the subject has been diagnosed with intermediate AMD.

In a further embodiment, the method further comprises assessing one or more aspects of the subject's personal history, such as, for example, one or more of age, ethnicity, body mass index, alcohol consumption history, smoking history, exercise history, diet, family history of AMD or other age-related ocular condition, including the age of the relative at the time of their diagnosis, and a personal history of treatment of AMD.

In a still further embodiment, determining the presence of absence of risk allelec is achieved by amplification of nucleic acid from said sample.

In various embodiments, amplification may comprise PCR, amplification may be located on a chip, where primers for amplification are specific for alleles of the common genetic variants tested.

In a particular embodiment, the amplification comprises: (i) admixing an amplification primer or amplification primer pair with a nucleic acid template isolated from the biological sample, wherein the primer or primer pair is complementary or partially complementary to a region proximal to or including the polymorphism, and is capable of initiating nucleic acid polymerization by a polymerase on the nucleic acid template; and, b) extending the primer or primer pair in a DNA polymerization reaction comprising a polymerase and the template nucleic acid to generate the amplicon.

The amplicon may, for example, be detected by a process that includes one or more of: hybridizing the amplicon to an array, digesting the amplicon with a restriction enzyme, or real-time PCR analysis.

In another embodiment, the amplification comprises performing a polymerase chain reaction (PCR), reverse transcriptase PCR (RT-PCR), or ligase chain reaction (LCR) using nucleic acid isolated from the organism or biological sample as a template in the PCR, RT-PCR, or LCR.

In yet another embodiment, the method mey further comprises cleaving amplified nucleic acid.

A further embodiment, the biological sample is derived from a bodily fluid, such as saliva or blood.

In other embodiments, the method further comprises the step of making a decision on the timing and/or frequency of AMD diagnostic testing for the subject and/or on the timing and/or frequency of AMD treatment for the subject.

In a further embodiment, the method further comprises the step of subjecting the subject identified as having an increased risk of developing advanced AMD to AMD treatment, where the treatment may, for example, comprise administration of a medicament selected from the group consisting of anti-factor D antibodies, anti-VEGF antibodies, CRIg, and CRIg-Ig fusion.

In a still further embodiment, the method comprises determination of the presence or absence of risk alleles for all single nucleotide polymorphisms set forth in Table S1, and the polygenic score is calculated based on such determination.

In another embodiment, the method further comprises the step of recording the results of said determination on a computer readable medium.

In yet another embodiment, the results are communicated to the subject or the subject's physician and/or are recorded in the form of a report.

In another aspect, the invention concerns a report comprising the results of the methods herein.

### Brief Description of the Drawings

The file of this patent contains at least one drawing executed in color. Copies of this patent or patent publication with color drawing(s) will be provided by the Office upon request and payment of the necessary fee
Figure 1: Known AMD risk genes power to predict progression.
Figure 2: Polygenic score identifies individuals a higher risk of progression to advanced AMD.
Figure 3: Polygenic score identifies individuals at higher risk of progression to advanced AMD independent of baseline clinical score.

Table S1: List of 16,617 SNPs. CHR = chromosome; SNP = SNP ID; BP = physical position (base-pairs); A1= first (minor) allele code; F_A - allele 1 frequency in cases; F_U: allele frequency in control cases; A2 = second (major) allele code; CHISQ = CHI Square Value; P = p value (significance value of case/control association test); OR = Odds Ratio for the association to AMD risk. In some cases the minor allele is associated with risk (OR >1) and in some cases the major allele is associated with AMD risk (OR<1).

### Detailed Description of the Invention

### I. Definitions

When trade names are used herein, applicants intend to independently include the trade name product formulation, the generic drug, and the active pharmaceutical ingredient(s) of the trade name product.

Unless stated otherwise, the following terms and phrases as used herein are intended to have the following meanings:
The term "complement-associated eye condition" is used in the broadest sense and includes all eye conditions the pathology of which involves complement, including the classical and the alternative pathways, and in particular the alternative pathway of complement. Complement-associated eye conditions include, without limitation, macular degenerative diseases, such as all stages of age-related macular degeneration (AMD), including dry and wet (non-exudative and exudative) forms, choroidal neovascularization (CNV), uveitis, diabetic and other ischemia-related retinopathies, and other intraocular neovascular diseases, such as diabetic macular edema, pathological myopia, von Hippel-Lindau disease, histoplasmosis of the eye, Central Retinal Vein Occlusion (CRVO), corneal neovascularization, and retinal neovascularization. A preferred group of complement-associated eye conditions includes age-related macular degeneration (AMD), including non-exudative (wet) and exudative (dry or atrophic) AMD, choroidal neovascularization (CNV), diabetic retinopathy (DR), and endophthalmitis.

The term "age-related macular degeneration" or "AMD" is used herein to encompass all stages of AMD, including Category 2 (early stage), Category 3 (intermediate) and Category 4 (advanced) AMD.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In treatment of complement-associated eye conditions, such as AMD, a therapeutic agent may directly beneficially alter the magnitude, severity, progression, or symptoms of the disease, or render the disease more susceptible to treatment by other therapeutic agents.

The "pathology" of a disease, such as a complement-associated eye condition, including AMD, includes all phenomena that compromise the well-being of the patient. This includes, without limitation, morphological correlates with various stages of the disease, such as the presence, number and size of drusen in one or both eyes, accumulating basal laminar deposits (BLamD) and basal linear deposits (BLinD), pigmentary changes, geographic atrhophy (GA) and retinal pigment epithelium (RPE) changes, a break-down of light-sensitive cells and supporting tissue in the central retinal area (advanced dry form), or abnormal and fragile blood vessels under the retina (wet form); physiological changes, such as impaired vision, partial or complete loss of vision.

The term "mammal" as used herein refers to any animal classified as a mammal, including, without limitation, humans, higher primates, domestic and farm animals, and zoo, sports or pet animals such horses, pigs, cattle, dogs, cats and ferrets, etc. In a preferred embodiment of the invention, the mammal is a human or another higher primate.

Administration "in combination with" one or more further therapeutic agents includes simultaneous (concurrent) and consecutive administration in any order.

A "phenotype" is a trait or collection of traits that is/are observable in an individual or population. The trait can be quantitative (a quantitative trait, or QTL) or qualitative. For example, susceptibility to AMD is a phenotype that can be monitored according to the methods, compositions, kits and systems herein.

An "AMD susceptibility phenotype" is a phenotype that displays a predisposition towards developing AMD in an individual. A phenotype that displays a predisposition for AMD, can, for example, show a higher likelihood that the AMD will develop in an individual with the phenotype than in members of a relevant general population under a given set of environmental conditions (diet, physical activity regime, geographic location, etc.).

"Ethnicity" may be based on self-identification (self-reported), but we preferably is based on the use of the genome-wide SNP data to determine how related samples are, and comparison of the samples to reference populations from the Human HapMap project to assign ethnicity. The populations included in the HapMap are Yoruba in Ibadan, Nigeria (abbreviation: YRI); Japanese in Tokyo, Japan (abbreviation: JPT); Han Chinese in Beijing, China (abbreviation: CHB); and CEPH (Centre d'Etude du Polymorphisme Humain) (Utah residents with ancestry from northern and western Europe) (abbreviation: CEU). The principal components approaches use genotype data to estimate axes of variation that can be interpreted as describing continuous ancestral heterogeneity within a group of individuals. These axes of variation are defined as the top eigenvectors of a covariance matrix between individuals in the study population. Then, the association between genotypes and phenotypes can be adjusted for the association attributable to ancestry along each axis. Typically samples that are significant outliers (relative to the population of interest) are excluded from the analysis to control for population stratification. Specifically, genotypes from across the genome are used to calculate eigenvectors (a form of principal components analysis, PCA), samples are then analyzed based on the primary eigenvectors. Extreme outliers (sigma > 6) are removed, and association results are corrected using the first 5 eigenvectors as covariates. See also, Price, A.L. et al. Principal components analysis corrects for stratification in genome-wide association studies. Nat. Genet. 38, 904-909 (2006), and the Example.

A "polymorphism" is a locus that is variable; that is, within a population, the nucleotide sequence at a polymorphism has more than one version or allele.

The term "allele" refers to one of two or more different nucleotide sequences that occur or are encoded at a specific locus, or two or more different polypeptide sequences encoded by such a locus. For example, a first allele can occur on one chromosome, while a second allele occurs on a second homologous chromosome, e.g., as occurs for different chromosomes of a heterozygous individual, or between different homozygous or heterozygous individuals in a population. One example of a polymorphism is a "single nucleotide polymorphism" (SNP), which is a polymorphism at a single nucleotide position in a genome (the nucleotide at the specified position varies between individuals or populations).

An allele "positively" correlates with a trait when it is linked to it and when presence of the allele is an indictor that the trait or trait form will occur in an individual comprising the allele. An allele negatively correlates with a trait when it is linked to it and when presence of the allele is an indicator that a trait or trait form will not occur in an individual comprising the allele.

A marker polymorphism or allele is "correlated" or "associated" with a specified phenotype (e.g. AMD susceptibility, etc.) when it can be statistically linked (positively or negatively) to the phenotype. That is, the specified polymorphism occurs more commonly in a case population (e.g., AMD patients) than in a control population (e.g., individuals that do not have breast cancer). This correlation is often inferred as being causal in nature, but it need not be--simple genetic linkage to (association with) a locus for a trait that underlies the phenotype is sufficient for correlation/association to occur.

A "favorable allele" is an allele at a particular locus that positively correlates with a desirable phenotype, e.g., resistance to AMD, e.g., an allele that negatively correlates with predisposition to AMD. A favorable allele of a linked marker is a marker allele that segregates with the favorable allele. A favorable allelic form of a chromosome segment is a chromosome segment that includes a nucleotide sequence that positively correlates with the desired phenotype, or that negatively correlates with the unfavorable phenotype at one or more genetic loci physically located on the chromosome segment.

An "unfavorable allele" is an allele at a particular locus that negatively correlates with a desirable phenotype, or that correlates positively with an undesirable phenotype, e.g., positive correlation to breast cancer susceptibility. An unfavorable allele of a linked marker is a marker allele that segregates with the unfavorable allele. An unfavorable allelic form of a chromosome segment is a chromosome segment that includes a nucleotide sequence that negatively correlates with the desired phenotype, or positively correlates with the undesirable phenotype at one or more genetic loci physically located on the chromosome segment.

A "risk allele"is an allele that positively correlates with the risk of developing a disease or condition, such as AMD, i.e. indicates that an individual has an increased likelihood to develop AMD, or, progress to a more advanced stage of AMD.

The "polygenic score" is used to define an individuals's risk of developing a disease or progressing to a more advanced stage of a disease, based on a large number, typically thousands, of common genetic variants each of which might have modest individual effect sizes contribute to the disease or its progression, but in aggregate have significant predicting value. In the present case, the polygenic score used to predict the likelihood that a patient will progress to advanced AMD using common single nucleotide polymorphisms (SNPs) associated with AMD. The log of the odds ratio (OR) from every variant reaching a P<0.1 in the discovery dataset is used to calculate the polygenic score. Specifically, for each of the 10,617 variants used in the score, the log of the Odds Ratio is multiplied times the number of reference alleles (0, 1 or 2) carried by the individual. The resulting sum is divided by the number of variants tested in each individual, resulting the final polygenic score. According to the present invention, "high polygenic score" is used to refer to a polygenic score >.0001, "low polygenic score" is used to refer to a polygenic score <.0001, and polygenic scores between these two thresholds are defined as "medium polygenic scores."

"Allele frequency" refers to the frequency (proportion or percentage) at which an allele is present at a locus within an individual, within a line, or within a population of lines. For example, for an allele "A," diploid individuals of genotype "AA," "Aa," or "aa" may have allele frequencies of 2, 1, or 0, respectively. One can estimate the allele frequency within a line or population (e.g., cases or controls) by averaging the allele frequencies of a sample of individuals from that line or population. Similarly, one can calculate the allele frequency within a population of lines by averaging the allele frequencies of lines that make up the population.

An individual is "homozygous" if the individual has only one type of allele at a given locus (e.g., a diploid individual has a copy of the same allele at a locus for each of two homologous chromosomes). An individual is "heterozygous" if more than one allele type is present at a given locus (e.g., a diploid individual with one copy each of two different alleles). The term "homogeneity" indicates that members of a group have the same genotype at one or more specific loci. In contrast, the term "heterogeneity" is used to indicate that individuals within the group differ in genotype at one or more specific loci.

A "locus" is a chromosomal position or region. For example, a polymorphic locus is a position or region where a polymorphic nucleic acid, trait determinant, gene or marker is located. In a further example, a "gene locus" is a specific chromosome location (region) in the genome of a species where a specific gene can be found. Similarly, the term "quantitative trait locus" or "QTL" refers to a locus with at least two alleles that differentially affect the expression or alter the variation of a quantitative or continuous phenotypic trait in at least one genetic background, e.g., in at least one population or progeny.

A "marker," "molecular marker" or "marker nucleic acid" refers to a nucleotide sequence or encoded product thereof (e.g., a protein) used as a point of reference when identifying a locus or a linked locus. A marker can be derived from genomic nucleotide sequence or from expressed nucleotide sequences (e.g., from an RNA, nRNA, mRNA, a cDNA, etc.), or from an encoded polypeptide. The term also refers to nucleic acid sequences complementary to or flanking the marker sequences, such as nucleic acids used as probes or primer pairs capable of amplifying the marker sequence.

A "marker probe" is a nucleic acid sequence or molecule that can be used to identify the presence of a marker locus, e.g., a nucleic acid probe that is complementary to a marker locus sequence. Nucleic acids are "complementary" when they specifically hybridize in solution, e.g., according to Watson-Crick base pairing rules.

A "marker locus" is a locus that can be used to track the presence of a second linked locus, e.g., a linked or correlated locus that encodes or contributes to the population variation of a phenotypic trait. For example, a marker locus can be used to monitor segregation of alleles at a locus, such as a QTL, that are genetically or physically linked to the marker locus. Thus, a "marker allele," alternatively an "allele of a marker locus" is one of a plurality of polymorphic nucleotide sequences found at a marker locus in a population that is polymorphic for the marker locus. In one aspect, the present invention provides marker loci correlating with a phenotype of interest, e.g., a phenotype increasing the likelihood that an individual with intermediate AMD will progress to advanced AMD. Markers corresponding to genetic polymorphisms between members of a population can be detected by methods well-established in the art. These include, e.g., PCR-based sequence specific amplification methods, detection of restriction fragment length polymorphisms (RFLP), detection of isozyme markers, detection of allele specific hybridization (ASH), detection of single nucleotide extension, detection of amplified variable sequences of the genome, detection of self-sustained sequence replication, detection of simple sequence repeats (SSRs), detection of single nucleotide polymorphisms (SNPs), or detection of amplified fragment length polymorphisms (AFLPs).

A "genotype" is the genetic constitution of an individual (or group of individuals) at one or more genetic loci. Genotype is defined by the allele(s) of one or more known loci of the individual, typically, the compilation of alleles inherited from its parents. A "haplotype" is the genotype of an individual at a plurality of genetic loci on a single DNA strand. Typically, the genetic loci described by a haplotype are physically and genetically linked, i.e., on the same chromosome strand.

A "set" of markers or probes refers to a collection or group of markers or probes, or the data derived therefrom, used for a common purpose, e.g., identifying an individual with a specified phenotype (e.g., AMD susceptibility, or susceptibility to develop advanced AMD). Frequently, data corresponding to the markers or probes, or derived from their use, is stored in an electronic medium. While each of the members of a set possess utility with respect to the specified purpose, individual markers selected from the set as well as subsets including some, but not all of the markers, are also effective in achieving the specified purpose.

A "computer readable medium" is an information storage medium that can be accessed by a computer using an available or custom interface. Examples include memory (e.g., ROM or RAM, flash memory, etc.), optical storage media (e.g., CD-ROM), magnetic storage media (e.g., computer hard drives, floppy disks, etc.), punch cards, and many others that are available and know to those skilled in the art. Information can be transmitted between a system of interest and the computer, or to or from the computer to or from the computer readable medium for storage or access of stored information. This transmission can be an electrical transmission, or can be made by other available methods, such as an IR link, a wireless connection, or the like.

The terms "factor D" and "complement factor D" are used interchangeably, and refer to native sequence and variant factor D polypeptides.

A "native sequence" factor D, is a polypeptide having the same amino acid sequence as a factor D polypeptide derived from nature, regardless of its mode of preparation. Thus, native sequence factor D can be isolated from nature or can be produced by recombinant and/or synthetic means. In addition to a mature factor D protein, such as a mature human factor D protein, the term "native sequence factor D", specifically encompasses naturally-occurring precursor forms of factor D (e.g., an inactive preprotein, which is proteolytically cleaved to produce the active form), naturally-occurring variant forms (e.g., alternatively spliced forms) and naturally-occurring allelic variants of factor D, as well as structural conformational variants of factor D molecules having the same amino acid sequence as a factor D polypeptide derived from nature. factor D polypeptides of non-human animals, including higher primates and non-human mammals, are specifically included within this definition.

"factor D variant" or "complement factor D variant" means an active factor D polypeptide as defined below having at least about 80% amino acid sequence identity to a native sequence factor D polypeptide. Ordinarily, a factor D variant will have at least about 80% amino acid sequence identity, or at least about 85% amino acid sequence identity, or at least about 90% amino acid sequence identity, or at least about 95% amino acid sequence identity, or at least about 98% amino acid sequence identity, or at least about 99% amino acid sequence identity with the mature factor D polypeptide. Preferably, the highest degree of sequence identity occurs within the active site of factor D.

The "active site" of factor D is defined by His-57, Asp-102, and Ser-195 (chymotrypsinogen numbering) in the human factor D sequence. factor D has Asp189 (chymotrypsin numbering) at the bottom of the primary specificity pocket and cleaves an Arg peptide bond. The catalytic triad consists of His-57, Asp-102 and Ser-195. Asp-102 and His57 display atypical conformations compared with other serine proteases (Narayana et al., J. Mol. Biol. 235 (1994), 695-708). A unique sal bridge is observed between Asp189 and Arg218 at the bottom of the S1 pocket which elevated loop 214-218 and generated a deep and narrow 1 pocket (Jinget al., J. Mol. Biol. 282 (1998) 1061-1081). This loop and several other residues around the active site were shown by mutational analysis to be the key structural determinants of the factor D esterolytic activity (Kim et al., J. Biol. Chem. 270 (1995) 24399-24405). Based on these results, it was proposed that factor D may undergo a conformational change upon binding C3b-bound factor B, resulting in the expression of proteolytic activity (Volanakis and Narayana, Protein Sci. 5 (1996) 553-564).

The term "VEGF" or "VEGF" as used herein refers to the 165-amino acid human vascular endothelial cell growth factor and related 121-, 189-, and 206-amino acid human vascular endothelial cell growth factors, as described by Leung et al. Science, 246:1306 (1989), and Houck et al. Mol. Endocrin., 5:1806 (1991), together with the naturally occurring allelic and processed forms thereof. The term "VEGF" also refers to VEGFs from non-human species such as mouse, rat or primate. Sometimes the VEGF from a specific species are indicated by terms such as hVEGF for human VEGF, mVEGF for murine VEGF, and etc. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "VEGF.sub.165." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

The term "VEGF variant" as used herein refers to a VEGF polypeptide which includes one or more amino acid mutations in the native VEGF sequence. Optionally, the one or more amino acid mutations include amino acid substitution(s). For purposes of shorthand designation of VEGF variants described herein, it is noted that numbers refer to the amino acid residue position along the amino acid sequence of the putative native VEGF (provided in Leung et al., supra and Houck et al., supra.).

"Percent (%) amino acid sequence identity" is defined as the percentage of amino acid residues in a candidate sequence that are identical with the amino acid residues in a reference factor D sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. Sequence identity is then calculated relative to the longer sequence, i.e. even if a shorter sequence shows 100% sequence identity with a portion of a longer sequence, the overall sequence identity will be less than 100%.

"Percent (%) nucleic acid sequence identity" is defined as the percentage of nucleotides in a candidate sequence that are identical with the nucleotides in a reference factor D-encoding sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. Sequence identity is then calculated relative to the longer sequence, i.e. even if a shorter sequence shows 100% sequence identity with a portion of a longer sequence, the overall sequence identity will be less than 100%.

An "isolated" nucleic acid molecule is a nucleic acid molecule that is identified and separated from at least one contaminant nucleic acid molecule with which it is ordinarily associated in the natural source of the nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes nucleic acid molecules contained in cells that ordinarily express an encoded polypeptide where, for example, the nucleic acid molecule is in a chromosomal location different from that of natural cells.

An "isolated" factor D polypeptide-encoding nucleic acid molecule is a nucleic acid molecule that is identified and separated from at least one contaminant nucleic acid molecule with which it is ordinarily associated in the natural source of the factor D-encoding nucleic acid. An isolated factor D polypeptide-encoding nucleic acid molecule is other than in the form or setting in which it is found in nature. Isolated factor D polypeptide-encoding nucleic acid molecules therefore are distinguished from the encoding nucleic acid molecule(s) as they exists in natural cells. However, an isolated factor D-encoding nucleic acid molecule includes factor D-encoding nucleic acid molecules contained in cells that ordinarily express factor D where, for example, the nucleic acid molecule is in a chromosomal location different from that of natural cells.

The term "antagonist" is used in the broadest sense, and includes any molecule that is capable of neutralizing, blocking, partially or fully inhibiting, abrogating, reducing or interfering with a factor D biological activity. factor D antagonists include, without limitation, anti-factor D antibodies and antigen-binding fragments thereof, other binding polypeptides, peptides, and non-peptide small molecules, that bind to factor D and are capable of neutralizing, blocking, partially or fully inhibiting, abrogating, reducing or interfering with factor D activities, such as the ability to factor D to participate in the pathology of a complement-associated eye condition, in particular AMD.

A "small molecule" is defined herein to have a molecular weight below about 600, preferably below about 1000 daltons.

"Active" or "activity" or "biological activity" in the context of a factor D antagonist is the ability the antagonize (partially or fully inhibit) a biological activity of factor D. A preferred biological activity of a factor D antagonist is the ability to achieve a measurable improvement in the state, e.g. pathology, of a factor D-associated disease or condition, such as, for example, a complement-associated eye condition, in particular AMD. The activity can be determined in *in vitro* or *in vivo* tests, including binding assays, using a relevant animal model, or human clinical trials.

The term "antibody" is used in the broadest sense and specifically covers, without limitation, single monoclonal antibodies (including agonist, antagonist, and neutralizing antibodies) and antibody compositions with polyepitopic specificity. The term "monoclonal antibody" as used herein refers to an antibody obtained from a population of substantially homogeneous antibodies, i.e., the individual antibodies comprising the population are identical except for possible naturally-occurring mutations that may be present in minor amounts.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al. (1975) Nature 256:495, or may be made by recombinant DNA methods (see, e.g., U.S. Pat. No. 4,816,567). The "monoclonal antibodies" may also be isolated from phage antibody libraries using the techniques described in Clackson et al. (1991) Nature 352:624-628 and Marks et al. (1991) J. Mol. Biol. 222:581-597, for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al. (1984) Proc. Natl. Acad. Sci. USA 81:6851-6855).

"Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al. (1986) Nature 321:522-525; Riechmann et al. (1988) Nature 332:323-329; and Presta (1992) Curr. Op. Struct. Biol. 2:593-596.

A "species-dependent antibody" is one which has a stronger binding affinity for an antigen from a first mammalian species than it has for a homologue of that antigen from a second mammalian species. Normally, the species-dependent antibody "binds specifically" to a human antigen (i.e. has a binding affinity (K_{d}) value of no more than about 1.times.10⁻⁷ M, preferably no more than about 1 x 10⁻⁸ M and most preferably no more than about 1 x 10⁻⁹ M) but has a binding affinity for a homologue of the antigen from a second nonhuman mammalian species which is at least about 50 fold, or at least about 500 fold, or at least about 1000 fold, weaker than its binding affinity for the human antigen. The species-dependent antibody can be any of the various types of antibodies as defined above, but preferably is a humanized or human antibody.

As used herein, "antibody mutant" or "antibody variant" refers to an amino acid sequence variant of the antibody wherein one or more of the amino acid residues of the antibody have been modified. Such mutants necessarily have less than 100% sequence identity or similarity with the reference antibody. In a preferred embodiment, the antibody mutant will have an amino acid sequence having at least 75% amino acid sequence identity or similarity with the amino acid sequence of either the heavy or light chain variable domain of the reference antibody, more preferably at least 80%, more preferably at least 85%, more preferably at least 90%, and most preferably at least 95%. Identity or similarity with respect to this sequence is defined herein as the percentage of amino acid residues in the candidate sequence that are identical (i.e same residue) or similar (i.e. amino acid residue from the same group based on common side-chain properties) with the reference antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

As used herein, "antibody variable domain" refers to the portions of the light and heavy chains of antibody molecules that include amino acid sequences of Complementarity Determining Regions (CDRs; ie., CDR1, CDR2, and CDR3), and Framework Regions (FRs). V_{H} refers to the variable domain of the heavy chain. V_{L} refers to the variable domain of the light chain. According to the methods used in this invention, the amino acid positions assigned to CDRs and FRs may be defined according to Kabat (Sequences of Proteins of Immunological Interest (National Institutes of Health, Bethesda, Md., 1987 and 1991)). Amino acid numbering of antibodies or antigen binding fragments is also according to that of Kabat.

As used herein, the term "Complementarity Determining Regions (CDRs; ie., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (i.e. about residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, Md. (1991)) and/or those residues from a "hypervariable loop" (i.e. about residues 26-32 (L1), 50-52 (L2) and 91-96 (L3) in the light chain variable domain and 26-32 (H1), 53-55 (H2) and 96-101 (H3) in the heavy chain variable domain; Chothia and Lesk (1987) J. Mol. Biol. 196:901-917). In some instances, a complementarity determining region can include amino acids from both a CDR region defined according to Kabat and a hypervariable loop. For example, the CDRH1 of the heavy chain of antibody 4D5 includes amino acids 26 to 35.

"Framework regions" (hereinafter FR) are those variable domain residues other than the CDR residues. Each variable domain typically has four FRs identified as FR1, FR2, FR3 and FR4. If the CDRs are defined according to Kabat, the light chain FR residues are positioned at about residues 1-23 (LCFR1), 35-49 (LCFR2), 57-88 (LCFR3), and 98-107 (LCFR4) and the heavy chain FR residues are positioned about at residues 1-30 (HCFR1), 36-49 (HCFR2), 66-94 (HCFR3), and 103-113 (HCFR4) in the heavy chain residues. If the CDRs comprise amino acid residues from hypervariable loops, the light chain FR residues are positioned about at residues 1-25 (LCFR1), 33-49 (LCFR2), 53-90 (LCFR3), and 97-107 (LCFR4) in the light chain and the heavy chain FR residues are positioned about at residues 1-25 (HCFR1), 33-52 (HCFR2), 56-95 (HCFR3), and 102-113 (HCFR4) in the heavy chain residues. In some instances, when the CDR comprises amino acids from both a CDR as defined by Kabat and those of a hypervariable loop, the FR residues will be adjusted accordingly. For example, when CDRH1 includes amino acids H26-H35, the heavy chain FR1 residues are at positions 1-25 and the FR2 residues are at positions 36-49.

As used herein, "codon set" refers to a set of different nucleotide triplet sequences used to encode desired variant amino acids. A set of oligonucleotides can be synthesized, for example, by solid phase synthesis, including sequences that represent all possible combinations of nucleotide triplets provided by the codon set and that will encode the desired group of amino acids. A standard form of codon designation is that of the IUB code, which is known in the art and described herein. A codon set typically is represented by 3 capital letters in italics, eg. NNK, NNS, XYZ, DVK and the like. A "non-random codon set", as used herein, thus refers to a codon set that encodes select amino acids that fulfill partially, preferably completely, the criteria for amino acid selection as described herein. Synthesis of oligonucleotides with selected nucleotide "degeneracy" at certain positions is well known in that art, for example the TRIM approach (Knappek et al. (1999) J. Mol. Biol. 296:57-86); Garrard & Henner (1993) Gene 128:103). Such sets of oligonucleotides having certain codon sets can be synthesized using commercial nucleic acid synthesizers (available from, for example, Applied Biosystems, Foster City, Calif.), or can be obtained commercially (for example, from Life Technologies, Rockville, Md.). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can, but does not necessarily, include restriction enzyme sites useful for, for example, cloning purposes.

The term "antibody fragment" is used herein in the broadest sense and includes, without limitation, Fab, Fab', F(ab')₂, scFv, (scFv)₂, dAb, and complementarity determining region (CDR) fragments, linear antibodies, single-chain antibody molecules, minibodies, diabodies, and multispecific antibodies formed from antibody fragments.

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the V_{H}-V_{L} dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

The "Fab" fragment contains a variable and constant domain of the light chain and a variable domain and the first constant domain (CH1) of the heavy chain. F(ab')₂ antibody fragments comprise a pair of Fab fragments which are generally covalently linked near their carboxy termini by hinge cysteines between them. Other chemical couplings of antibody fragments are also known in the art.

"Single-chain Fv" or "scFv" antibody fragments comprise the V_{H} and V_{L} domains of antibody, wherein these domains are present in a single polypeptide chain. Generally the Fv polypeptide further comprises a polypeptide linker between the V_{H} and V_{L} domains, which enables the scFv to form the desired structure for antigen binding. For a review of scFv, see Pluckthun in The Pharmacology of Monoclonal Antibodies, Vol 113, Rosenburg and Moore eds. Springer-Verlag, New York, pp. 269-315 (1994).

The term "diabodies" refers to small antibody fragments with two antigen-binding sites, which fragments comprise a heavy chain variable domain (V.sub.H) connected to a light chain variable domain (V_{L}) in the same polypeptide chain (V_{H} and V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al. (1993) Proc. Natl. Acad. Sci. USA 90:6444-6448.

The expression "linear antibodies" refers to the antibodies described in Zapata et al. (1995 Protein Eng, 8(10):1057-1062). Briefly, these antibodies comprise a pair of tandem Fd segments (V_{H}-C_{H1}-V_{H}-C_{H1}) which, together with complementary light chain polypeptides, form a pair of antigen binding regions. Linear antibodies can be bispecific or monospecific.

### II. Detailed Description

### Age-Related Macular Regeneration (AMD)

Age-Related Macular Degeneration (AMD) is a slowly progressive degenerative disease that culminates in loss of central vision. Depending on the seriousness of the disease, AMD can be classified into four categories, which have the characteristics listing in the following Table 1.

**Table 1**

| **Category 1** | **Category 2** | **Category 3** | **Category 4** |
|---|---|---|---|
| *No AMD* | *Early Stage AMD* | *Intermediate AMD* | *Advanced AMD* |
| A few small or no drusen | Several small drusen or a few medium-sized drusen in one or both eyes | Many medium-sized drusen or one or more large drusen in one or both eyes | In one eye only, either a break-down of light-sensitive cells and supporting tissue in the central retinal area (advanced dry form), or abnormal and fragile blood vessels under the retina (wet form) |
| AREDS category 1: | AREDS categoryy 2: | AREDS category 3: | AREDS category 4: |
| both eyes are essentially free of abnormalities | mild changes in the worst eye, including multiple small drusen, nonextensive intermediate drusen and/or pigment | in the worst eye at lest one large drusen of at least 125-µm diameterm extensive intermediate drusen, and/or noncentral geographic atrophy | in one eye, advanced AMD, either neovascular or central geographic atrophy, or visual loww due to AMD regardless or phenotype, or |
| | | | in both eyes |
| | abnormalities | | |

Only 18% of patients with intermediate AMD (Category 3) will progress to advanced AMD (Category 4) over 5 years. Identifying individuals at a greater risk of progression would enable clinical trials to test novel AMD therapies and provide insight into pathogenic pathways.

It is known that polymorphosism in Complement Factor H, Complement Factor I, Complement C2, HtrA1 serine peptidase, Complement C3 are associated with AMD. Muations in CFH can activate complement, which in turn may lead to AMD/CNV. It has been reported that complement factor H (CFH) polymorphism accounts for 50% of the attributable risk of AMD (Klein et al., Science 308:385-9 (2005)). A common halpotype in CFH (HF1/CFH) has been found to predispose individuals to age-related macular degeneration (Hageman et al., Proc. Natl. Acad. Sci. USA, 102(2):7227-7232 (2005)). AMD has been segregated as an autosomaldominant trait, with the disease locus mapping to chromosome 1q25-q31 between markers D1S466 and D1S413, with a maximum lod score of about 3.20 (Klein et al., Arch Opthalmol. 116(8):1082-9 (1998); Majewski et al., Am. J. Hum. Genet. 73(3):540-50 (2003); Seddon et al., Am. J. Hum. Genet. 73(4):780-90 (2003); Weeks et al., Am. J. Ophthalmol. 132(5):682-92 (2001); Iyengar et al., Am. J. Hum. Genet. 74(1):20-39 (2004)); chromosome 2q3/2q32 between markers D12S1391 and D2S1384, with a maximum lode score of 2.32/2.03 (Seddon et al., supra); 3p13, between markers D12S1300 and D12S1763, with a maximum lode score of 2.19 (Majewski et al., supra; Schick et al., Am. J Hum. Genet. 72(6):1412-24 (2003)); 6q14 between markers D6S1056 and DS249 with a maximum lode score of 3.59/3.17 (Kniazeva et al., Am. J. Ophthalmol. 130(2):197-202 (2000)); 9q33, at marker D9S934, with a maximum lode score of 2.06 (Mejwski et al., supra); 10q26 at th marker D10S1230, with a maximum lode score of 3.06 (Majewski et al., supra; Iyengar et al., supra; Kenealy et al., Mol. Vis. 10:57-61 (2004); 17q25 at marker D17S928, maximum lode score of 3.16 (Weeks et al., supra); and 22q12 at marker D22S1045, maximum lode score of 2.0 (Seddon et al., supra). Accordingly, genetic screening is an important part of identifying patients who are particularly good candidates for preventative treatment, including prevention of the progression of the disease into a more severe form.

### Methods of Genotyping

The invention involves detection and analysis of a large number of common genetic variants (e.g. SNPs) which can be used to calculate a polygenic score suitable for identifying individuals at a greater risk of progression to advanced AMD. Detection methods for detecting relevant alleles include a variety of methods well known in the art, e.g., gene amplification technologies. For example, detection can include amplifying the polymorphism or a sequence associated therewith and detecting the resulting amplicon. This can include admixing an amplification primer or amplification primer pair with a nucleic acid template isolated from the organism or biological sample (e.g., comprising the SNP or other polymorphism), where the primer or primer pair is complementary or partially complementary to at least a portion of the target gene, or to a sequence proximal thereto. Amplification can be performed by DNA polymerization reaction (such as PCR, RT-PCR) comprising a polymerase and the template nucleic acid to generate the amplicon. The amplicon is detected by any available detection method, e.g., sequencing, hybridizing the amplicon to an array (or affixing the amplicon to an array and hybridizing probes to it), digesting the amplicon with a restriction enzyme (e.g., RFLP), real-time PCR analysis, single nucleotide extension, allele-specific hybridization, or the like. Genotyping can also be performed by other known techniques, such as using primer mass extension and MALDI-TOF mass spectrum (MS) analysis, such as the MassEXTEND methodology of Sequenom, San Diago, CA.

### Polygenic Score to Predict Progression to Advanced AMD

The known AMD risk alleles have limited power to predict progession of AMD, such as progression from intermediate AMD to advanced AMD, individually or in aggregate. Therefore, we have first created a polygenic score in AMD by analyzing the results of a genome-wide association study in 1,100 advanced AMD cases, 8,300 controls and 610,000 SNPs, and creating a reank ordered list of all independent SNPs below P<0.1 threshold. We then tested the hypothesis that a polygenic score consisting of thousands of common variants could be predictive of progression of intermediate AMD to advanced AMD, and found that polygenic score effectively identifies indiciduals at higher risk of progression to advanced ADM. Following a genome-wide association study, a rank-ordered list of all independent SNPs below a P value threshold (such as P<0.1, P<0.05, P<0.001) is created. The score for each individual is the number of risk variants carried, weighted for the effect size (Odds Ratio). In the next stap, performance of polygenic score to predict progression to advanced AMD is assessed.

Our results, discussed in the Example below, show that individuals having intermediate AMD with a high polygenic score have an an about 2.3 fold higher risk of progression to advanced AMD in 2 years, and about 2.6 fold higher risk of progression to advanced AMD in 5 years. A polygenic score significantly improves our ability to predict progression compared to the known AMD risk loci, when used individually or in combination. Accordingly, the polygenic score is a useful tool to identify such patients for early intervention, and also to test candidate agents that might be effective in slowing down or inhibiting the progression to advanced AMD in the most vulnerable patient population.

The present invention provides enhanced early detection options to identify patients that are at the greatest risk for developing advanced AMD, making it possible, in some cases, to prevent development, or at least slowing down the progress, of AMD, e.g., by taking early preventative action, treating the patients with any existing treatment option, changes in the patient's lifestyle, including diet, exercise, etc.). In addition, the polygenic score determined in accordance with the present invention can also assist in providing an indication of how likely it is that a patient will respond to any particular therapy for the treatment of AMD, including experimental therapies. Accordingly, the present invention also enables the identification of a patient population for testing treatment options for preventing or slowing down the progression of an earlier stage of AMD to advanced AMD.

### Treatment of AMD

Complement inhibitors useful to treat AMD include, for example, factor D antagonists and factor H antagonists, and inhibitors that block the action of properdin, factor B, factor Ba, factor Bb, C2, C2a, C3a, C5, C5a, C5b, C6, C7, C8, C9, or C5b-9. Complement inhibitors for the treatment of AMD are disclosed, for example, in U.S. Patent Publication Nos. 20090181017 and 20090214538. factor D antibodies useful to inhibit complement activation and treat complement-associated diseases, including AMD are also disclosed in U.S. Patent Nos. 6,956,107; 7,112,327; and 7,527,970.

AMD can also be treated by anti-VEGF antibodies, which are disclosed, for example, in U.S. Patent No. 7,758,859. In June 2006 the FDA approved Lucentis^{®} (ranibizumab) for treating the more advanced or "wet" form of macular degeneration. Other treatment options include, without limitation, Macugen^{®} (pegaptanib sodium), administered through injections into the eye, with treatments required every six weeks.

For experimental treatment options see, for example, STIgMA (CRIg) or STIgMA-(CRIg)-Ig fusion molecules (see, e.g. U.S. Patent No. 7,419,663); IGF-1 antagonists (see, e.g. U.S. Patent No. 7,432,244);

All publications (including patents and patent applications) cited herein are hereby incorporated in their entirety by reference.

Further details of the invention are provided in the following non-limiting example.

### EXAMPLE

### Predicting Progression to Advanced Age-Related Macular Degeneration Using a Polygenic Score

### METHODS

### Study Samples, Ascertainment and Genotyping

*AMD Cases.* There are 4 AMD case collections used in the study: AREDS (Age-Related Eye Disease Study founded by the National Eye Institute), DAWN, UCSD study and OSHU. We chose 564 samples from the Age-Related Eye Disease Study (AREDS). The inclusion criterion was based on the final AMD status (AMDS TAT) of the patients (6 = Large Drusen, 11 =CNV, 12=CGA, 13= both CNV and CGA) were used as cases in our analysis. We also included 352 AMD CNV cases from the DAWN study. The DAWN study is a genetic sub-study which is a collection of samples from three Phase II/III Lucentis clinical trials (FOCUS, MARINA, and ANCHOR). Another 142 samples were recruited from the UCSD AMD study. Finally, 42 CNV cases from a Lucentis IST preformed at OSHU were included as additional cases.

*AMD controls.* Controls in our analysis come from 4 separate collections. We included 441 samples from the AREDS study with final AMD status ranging from 1 to 5 (1=Control, 2= Control Questionable 1, 3=Control Questionable 2, 4=Control Questionable 3, 5=Control Questionable 4). A total of 1861 control subjects from the New York Cancer Project were collected and then genotyped on the basis of self-described ancestral origin, sex and age. In addition, genotype data from 1722 control samples (all self-described North-Americans of European descent) were obtained from the publicly available iControlDB database (www.illumina.com /pages.ilmn?ID=231). An additional 2277 prostate cancer cases and controls an 2287 breast cancer cases and controls from the Cancer Genetic Markers of Susceptibility Project (CGEMS) (http://cgems.cancer.gov/data/) were included after obtaining pemission.

After performing quality control (QC) on each sample collection separately, all sample collections were pooled together and further quality control was performed.

Table S1 describes the number of individuals in each collection, genotyping array and number of SNPs samples were genotyped on.

### Quality Control

Before merging sample collections, we preformed quality control in each sample collection independently. We removed low-quality SNPs (call rate < 50%) and individual samples with call rates of less than 95 %.

### Sample Quality Control.

We excluded samples with > 5% missing genotypes, one sample from each of the cryptic related or unexpected duplicate pairs (identified using identity by descent measures calculated using PLINK), population outliers (samples with values >5 s.d. away from the mean for the first 10 eigenvectors) identified using eigenstrat, and samples with mismatch between reported gender and that determined based on the genotype data.

### SNP Quality Control

After pooling all samples we performed the following SNP QC. We removed SNPs with call rate < 95%. SNPs with differential missingness between cases and controls (P< 1x10-4) were excluded from the final dataset. In addition we tested each SNP for Hardy-Weinberg equilibrium and SNPs that did not pass P<1x10-4 in controls were excluded.

### Population Stratification Analysis

For each cohort, we used ancestry-informative markers to correct for possible population stratification. A subset of 5,486 uncorrelated ancestry-informative markers that passed stringent quality control criteria were used to infer the top ten principal components of genetic variation using EIGENSTRAT (Price, A.L. et al. Principal components analysis corrects for stratification in genome-wide association studies. Nat. Genet. 38, 904-909 (2006)).. Outliers were removed from each sample set (defined as s.d. > 6). To correct for the case-control stratification, we applied the correction of the Cochran-Armitage test statistic incorporated in EIGENSTRAT.

### Association Analysis

We performed logistic regression on AMD status for each SNP using principal components as covariates. We included in our model principal components that showed association with AMD case/control status.

### Creating a Polygenic Score in Target Samples

We selected SNPs with MAF > 2% in the pooled samples and a genotyping call rate > 99%. Since a lot of the remaining SNPs are in strong LD with each other, we pruned the SNPs in order to have an independent set of SNPs. We used the ***--indep-pairwise*** command in PLINK with a threshold r2=.25 with a 200-SNP sliding window and 20-SNP overlap between adjacent windows.

In each analysis, we formed independent discovery and target samples. In each of the scenarios described above, we computed association statistics for each SNP in the discovery sample using logistic regression with principal components as covariates. We created a P-value rank-ordered list for the pruned list of SNPs. We created subsets of SNPs based on different P-value thresholds (P<.0001, P<.001, P<.01, P<.05, P<.10, P<.20, P<.30, P<.40, P<.50, P<1.00). For each SNP subset, we used a reference allele and the log of the odds ratio (OR) from the discovery dataset to create a polygenic score in the second indepenedent target dataset. The score is the average sum across SNPs of the number of reference alleles (0, 1 or 2) at that SNP multiplied by the log OR for that SNP. We proceeded to test the hypothesis that the polygenic score is a predictor of disease or disease progression.

### RESULTS AND DISCUSSION

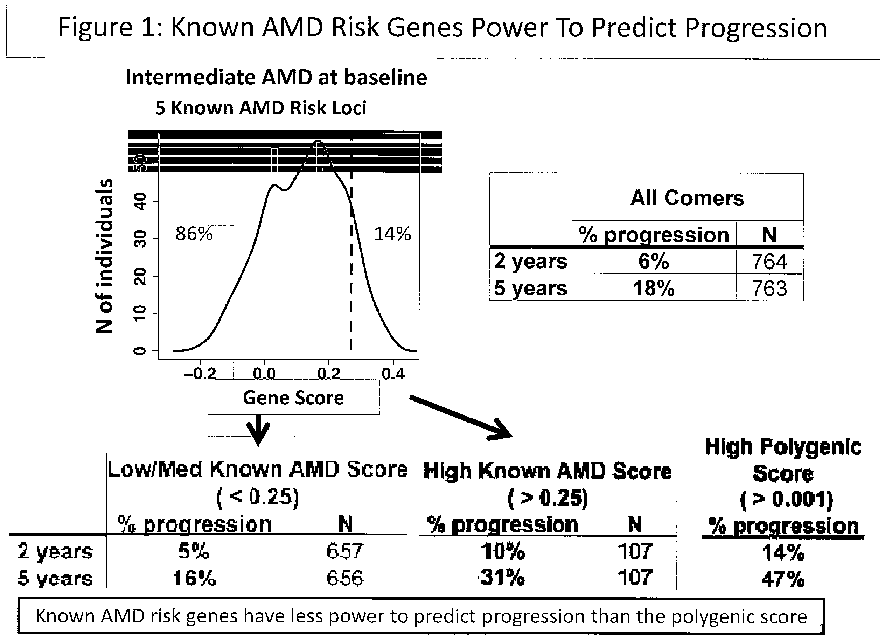

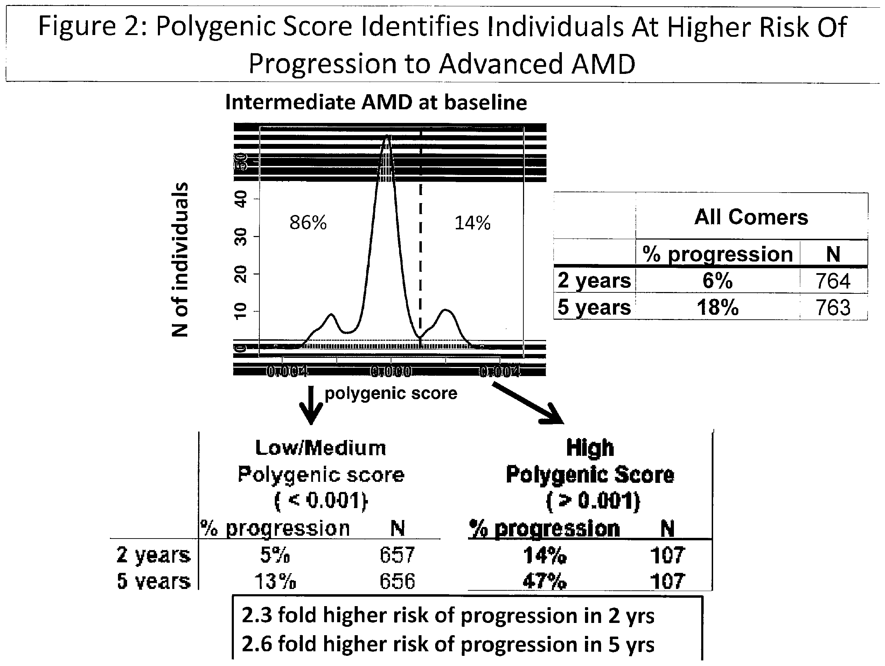

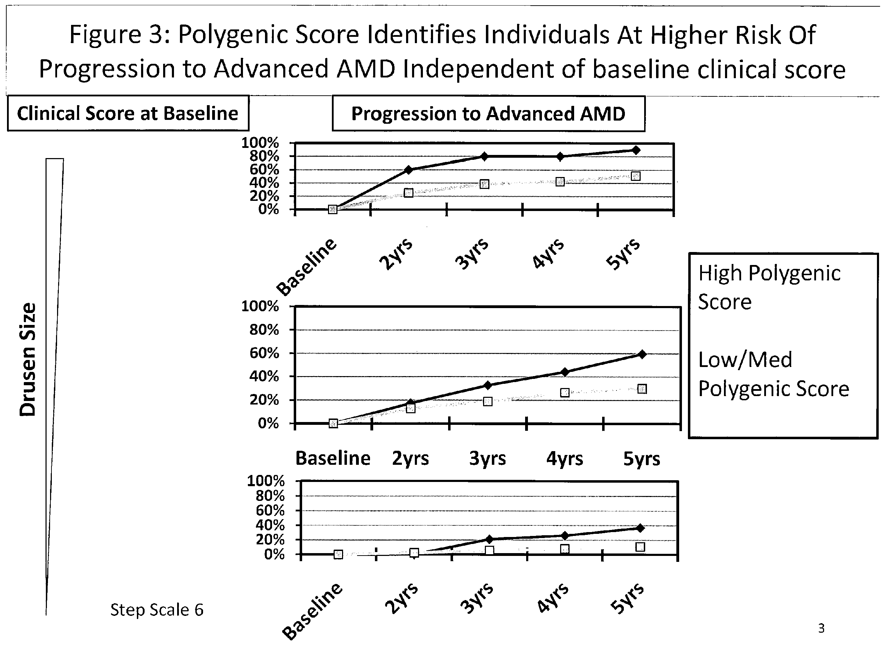

We first confirmed the ability of 7 known SNPs at 5 known loci associated with AMD: complement factor H (rs10737680 and rs1329424); complement factor I (rs2285714); complement C2 (rs429608 and rs9380272)'HTRA1 (rs3793917); and complement C3 (rs2230199) (see Table 1 of Chen et al., PNAS 107(16):7401-7406 (2010)) to enrich for progression to advanced AMD in 764 individuals with Intermediate AMD (category 3) from the Age-Related Eye Disease natural history study. Using a composite score of the 7 known AMD risk alleles we identified a population (14% of the intermediate AMD population) with a progression rate of 31% at 5 yrs, a 1.6 fold increase over the unselected population. We next tested the hypothesis that a polygenic score consisting of thousands of common variants could be predictive of progression to advanced AMD. We conducted a genome-wide association study on 925 advanced AMD cases and 7,863 healthy controls of European descent. We created a polygenic score composed of 10,616 independent loci with p-value <0.10 from the genome-wide association scan. For each of the 764 individuals with Intermediate AMD (category 3), a polygenic score was calculated as the average sum of the number of risk alleles (0, 1 or 2) at each SNP weighted by the log odds ratio for that SNP. Individuals with high polygenic score (14% of the intermediate AMD population) have a 47% risk of progression at 5 yrs compared to only 13 % risk for the rest of the intermediate AMD population. The results are shown in Figures 1-3. This represents a 2.6 fold increase over the unselected population, and a significant improvement in predictive power to a score composed of 7 confirmed AMD loci. Our results demonstrate that thousands of common variants can be predictive of AMD progression, and suggests that hundreds of AMD risk loci of modest individual effects contribute to the heritability of AMD.

This application includes a table entitled "Table S1." Table S1 was submitted in landscape orientation with the filing of this application. Table S1 provides a list of 16,617 SNPs. CHR = chromosome; SNP = SNP ID; BP = physical position (base-pairs); A1= first (minor) allele code; F_A - allele 1 frequency in cases; F_U: allele frequency in control cases; A2 = second (major) allele cod; CHISQ = CHI Square Value; P = p value (significance value of case/control association test); OR = Odds Ratio for the association to AMD risk. In some cases the minor allele is associated with risk (OR >1) and in some cases the major allele is associated with AMD risk (OR<1).

The results of this polygenic score analysis can be further refined and supplemented by analyzing additional genome-wide association study (GWAS) data, which are publicly available or are generated in future GWAS studies. Further refinement of the analysis can also be achieved by further analysis of the existing or future data sets, for example by comparative analysis of the choroidal neovascularization (CNV) vs. GA involving the center of the macula (CGA) data. There are also other methodologies available for determining polygenic scored, such as, for example, Support Vector Machine (SMV) algorithms.

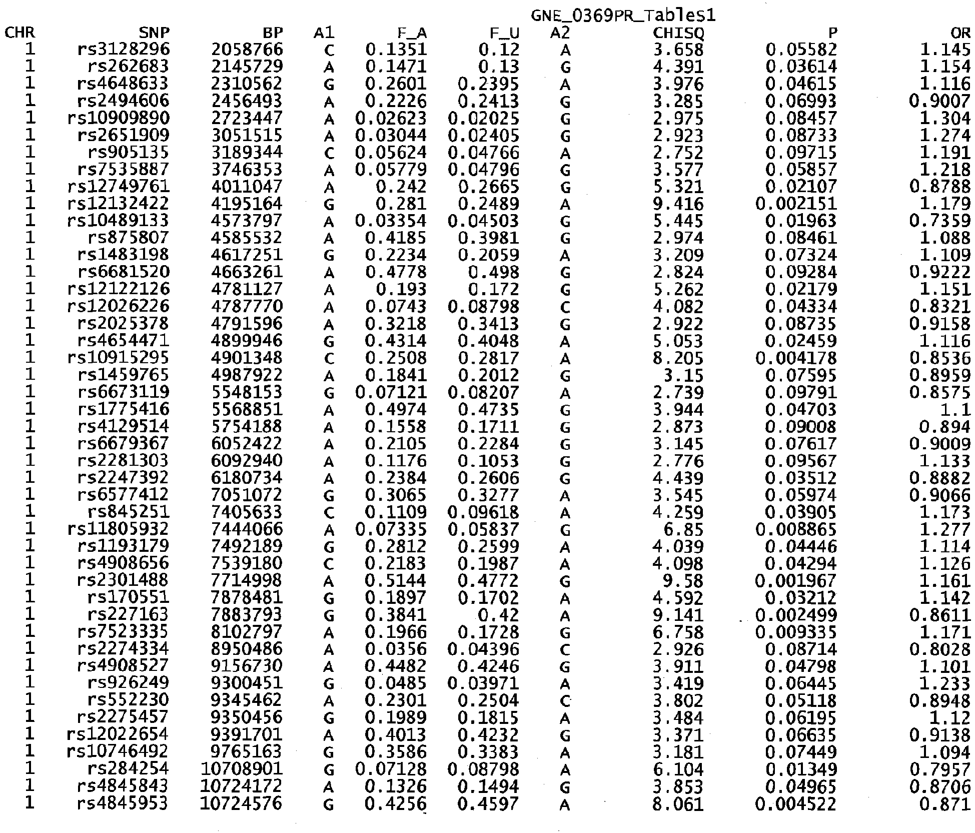

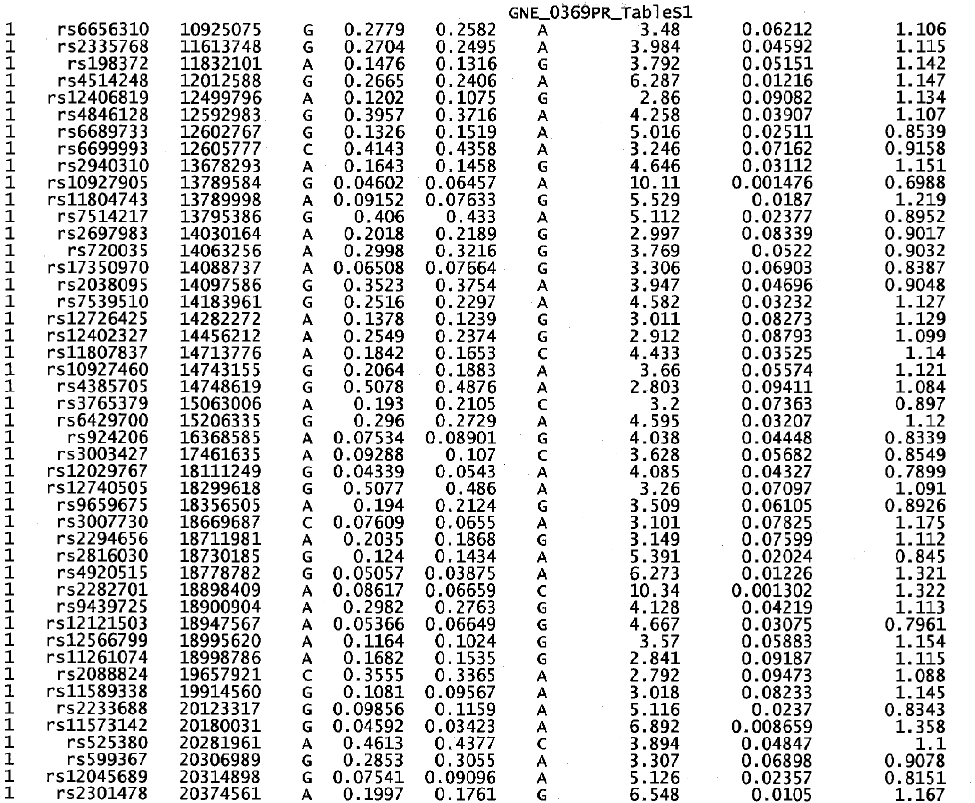

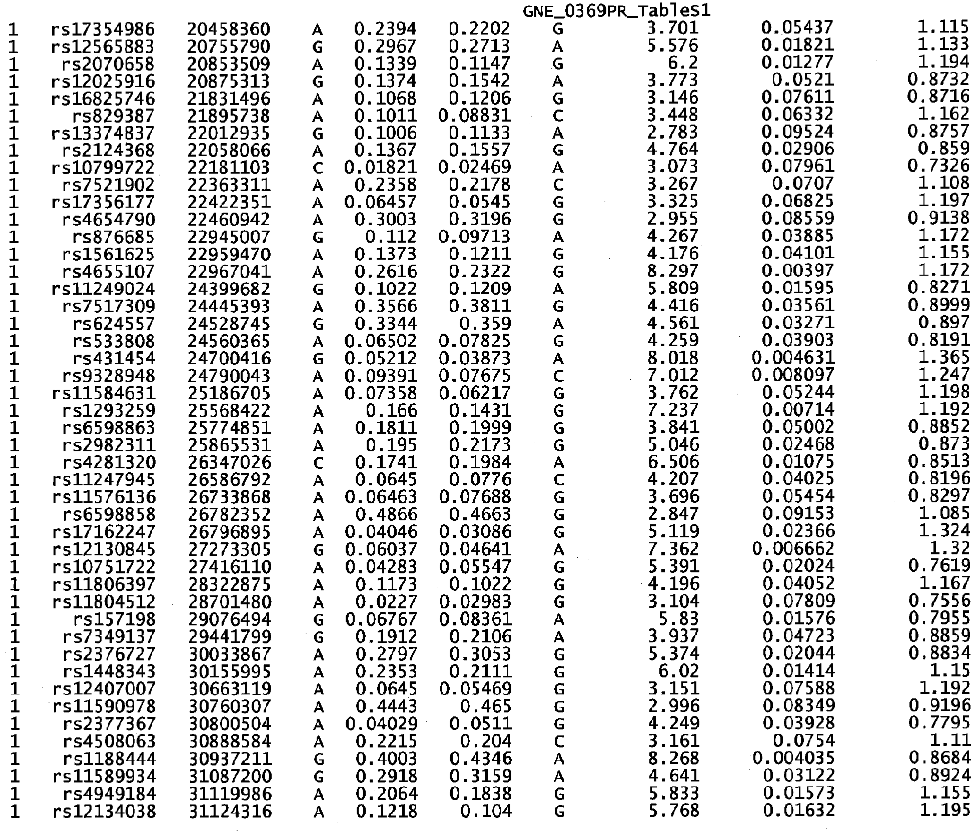

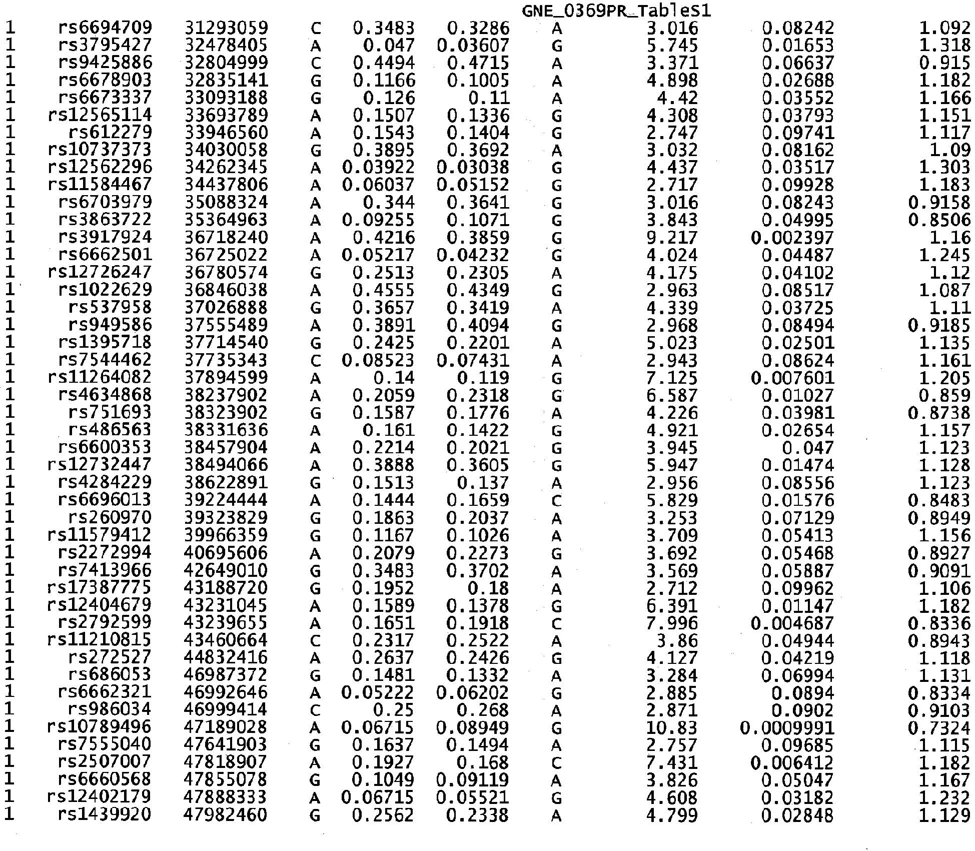

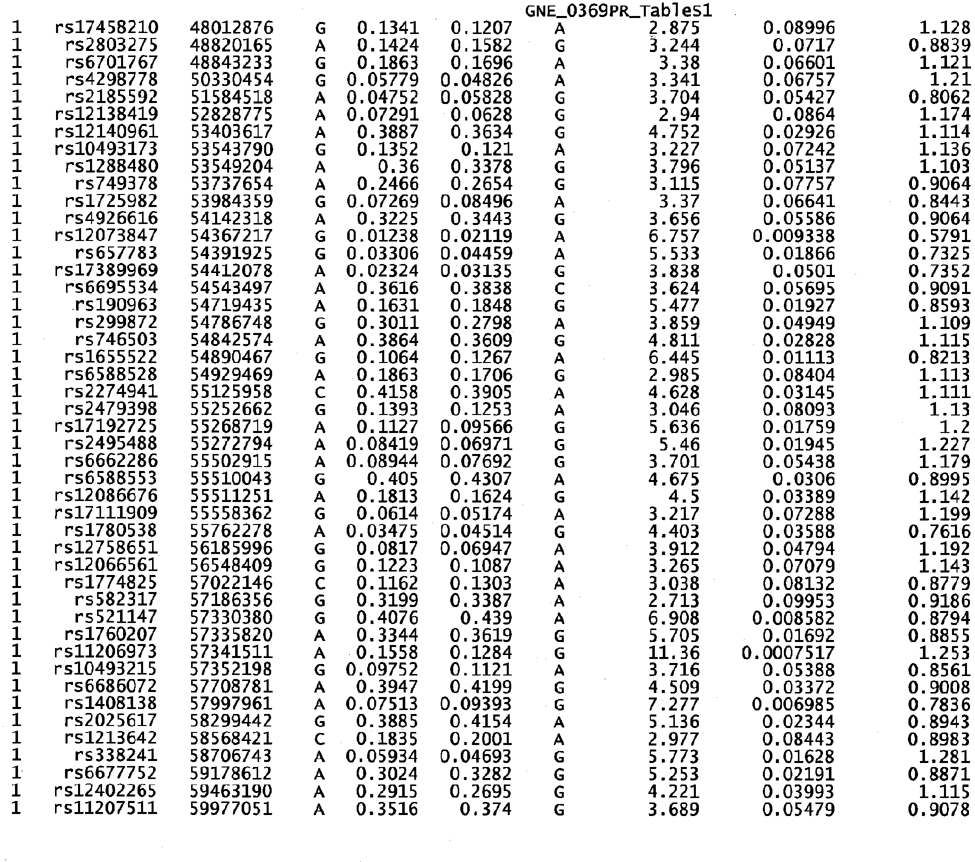

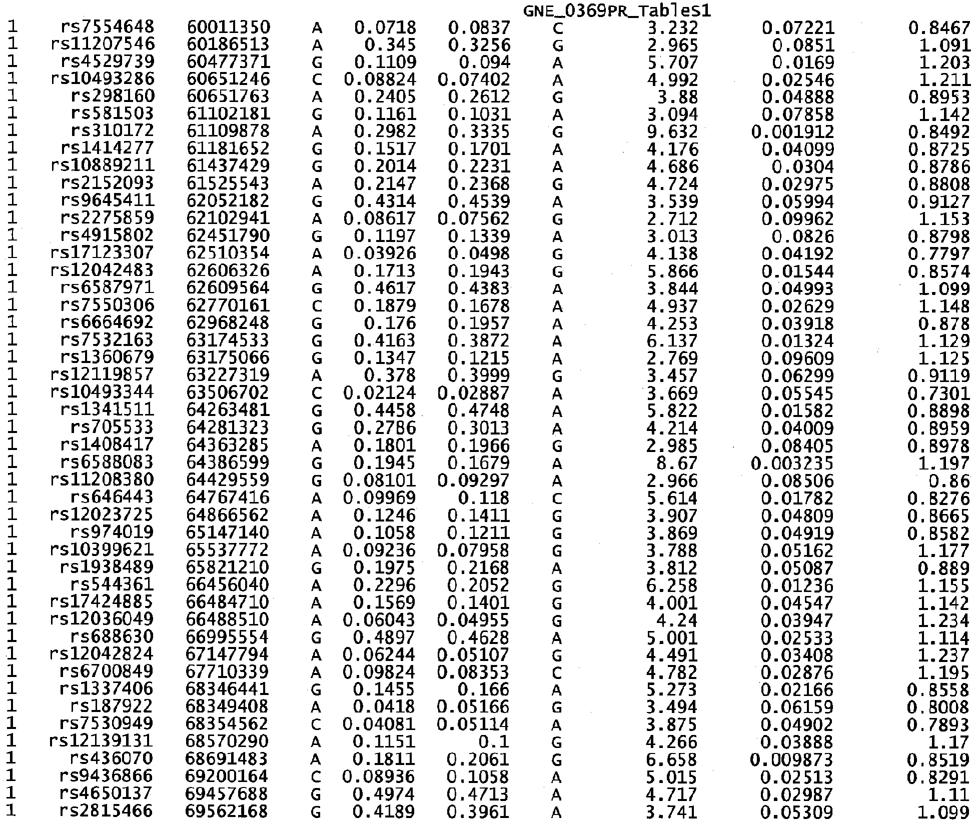

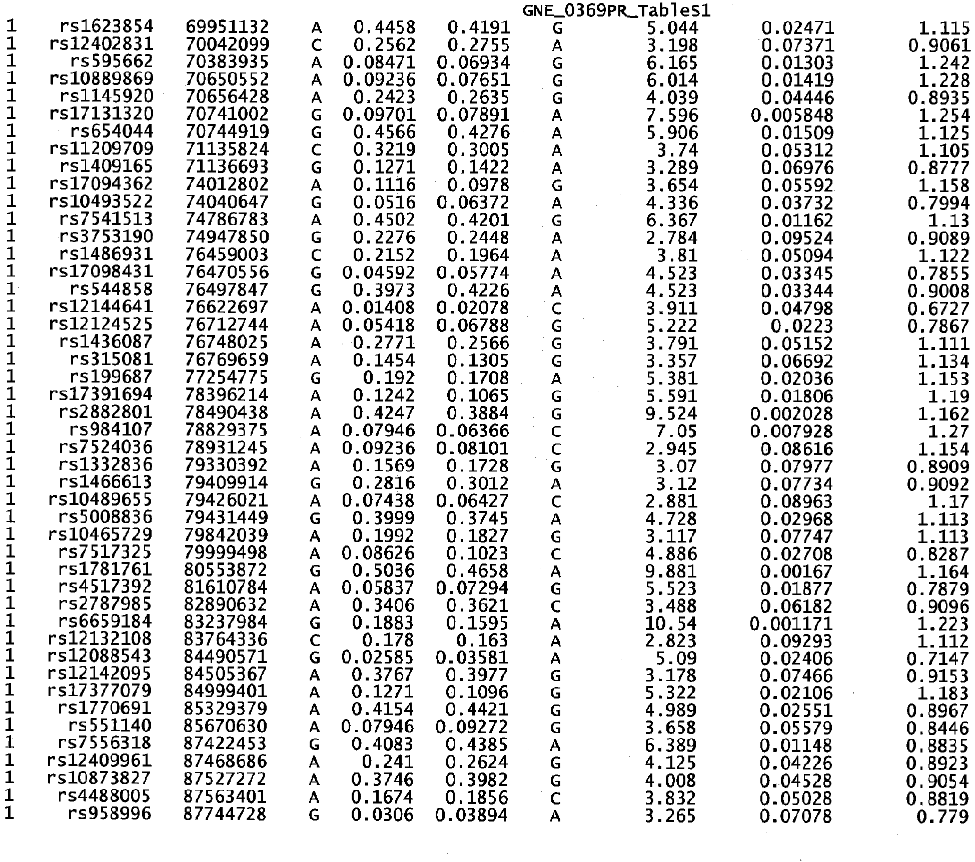

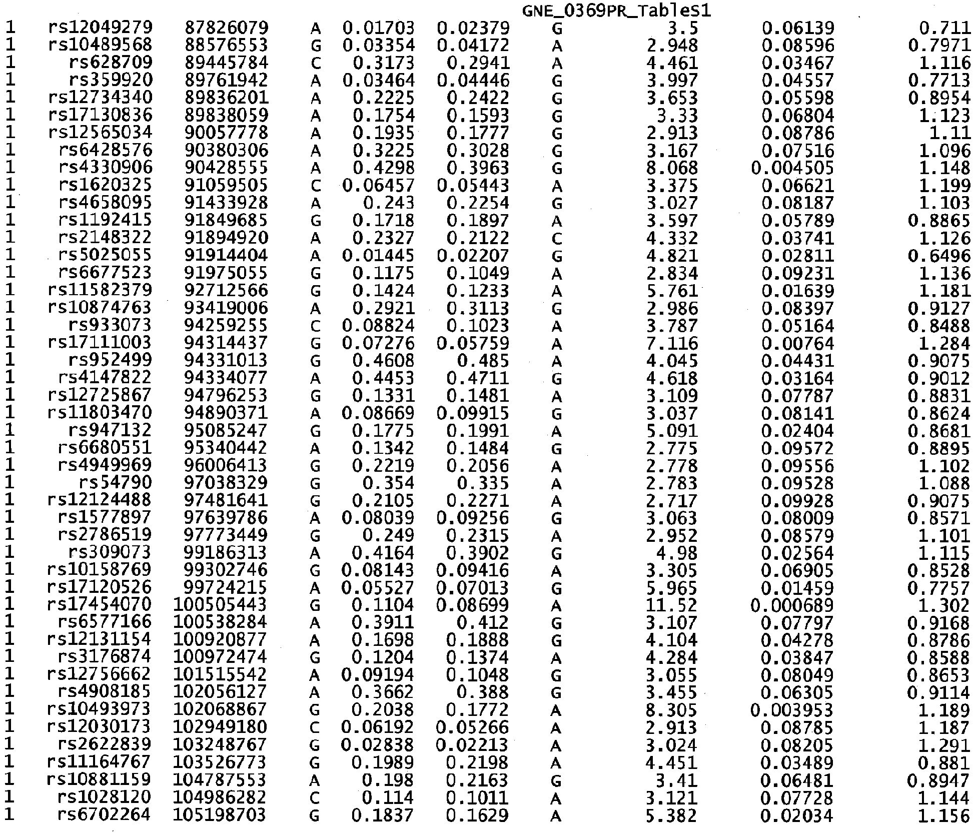

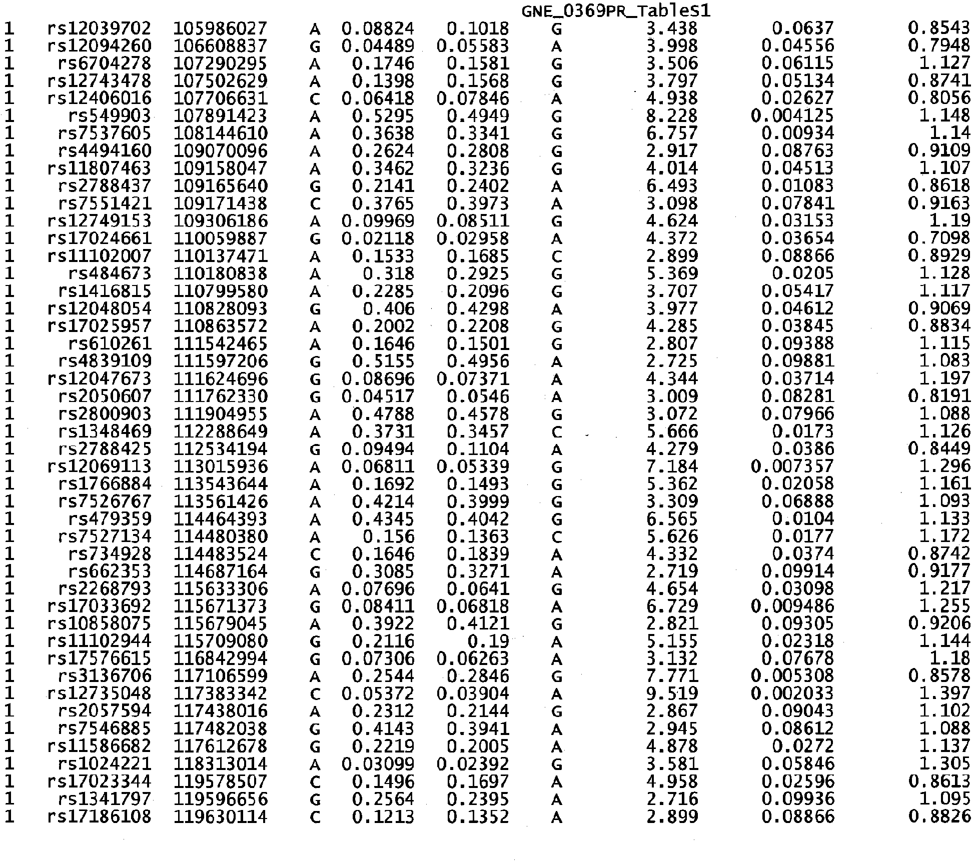

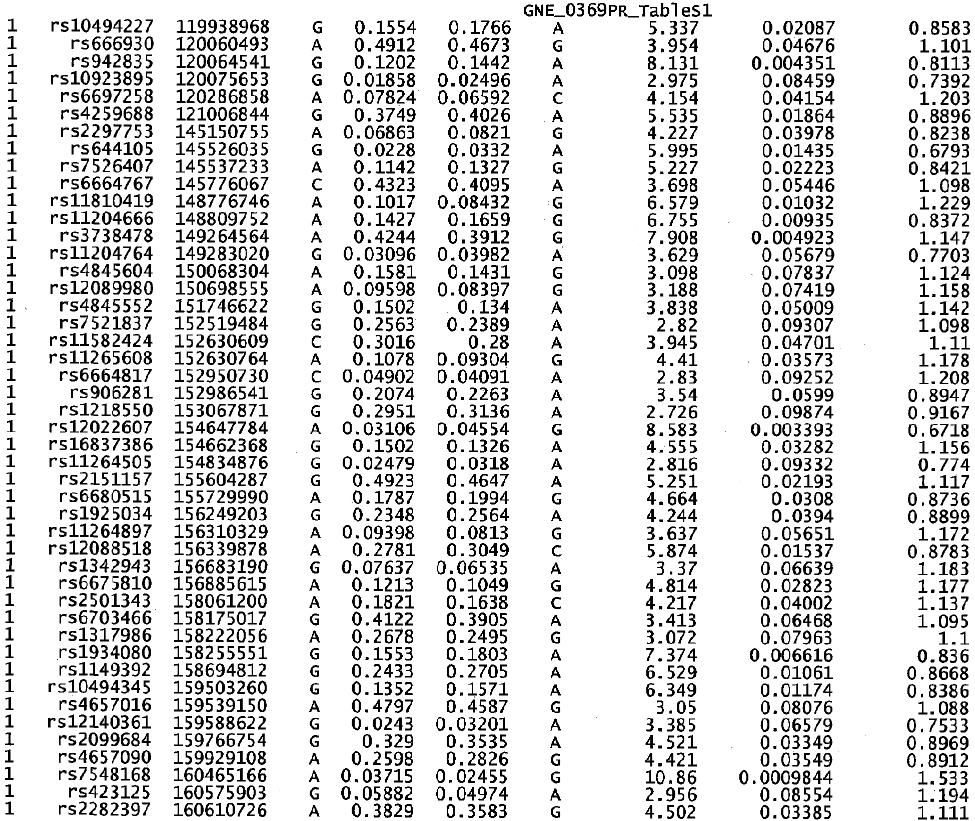

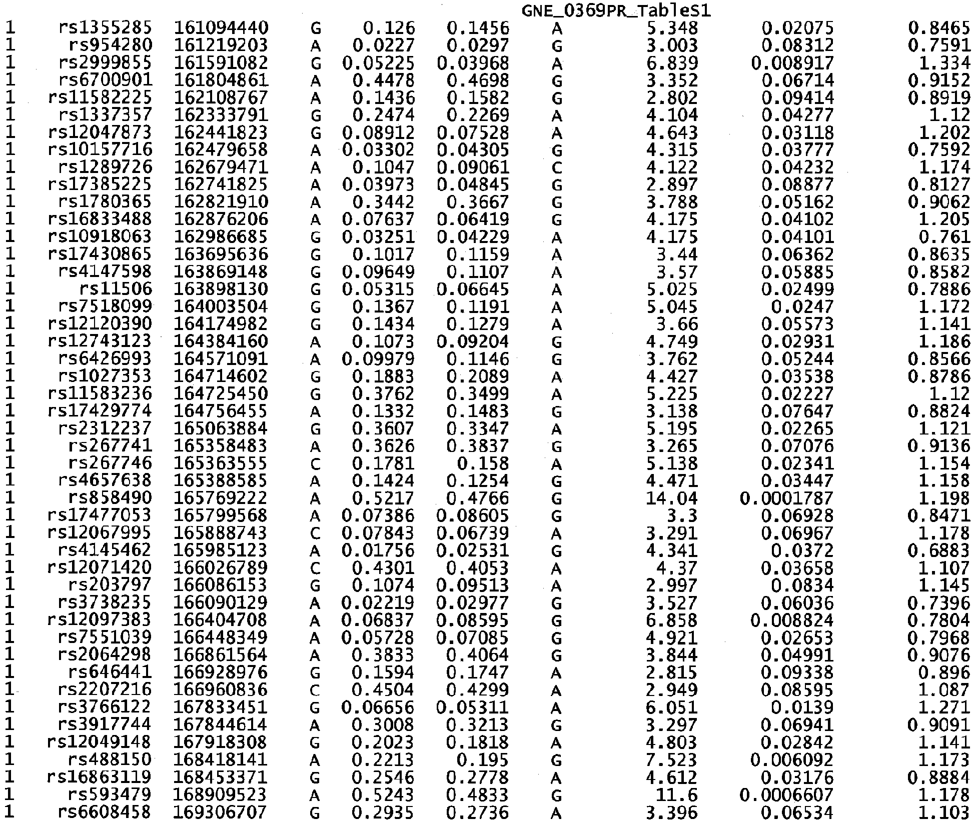

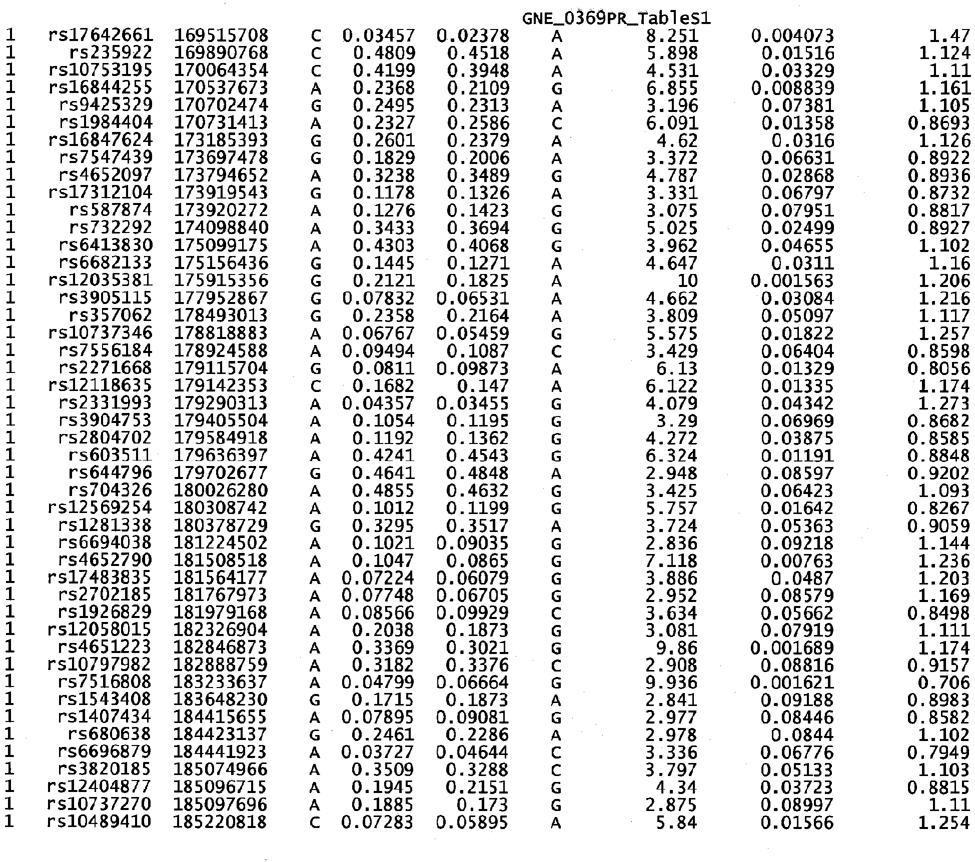

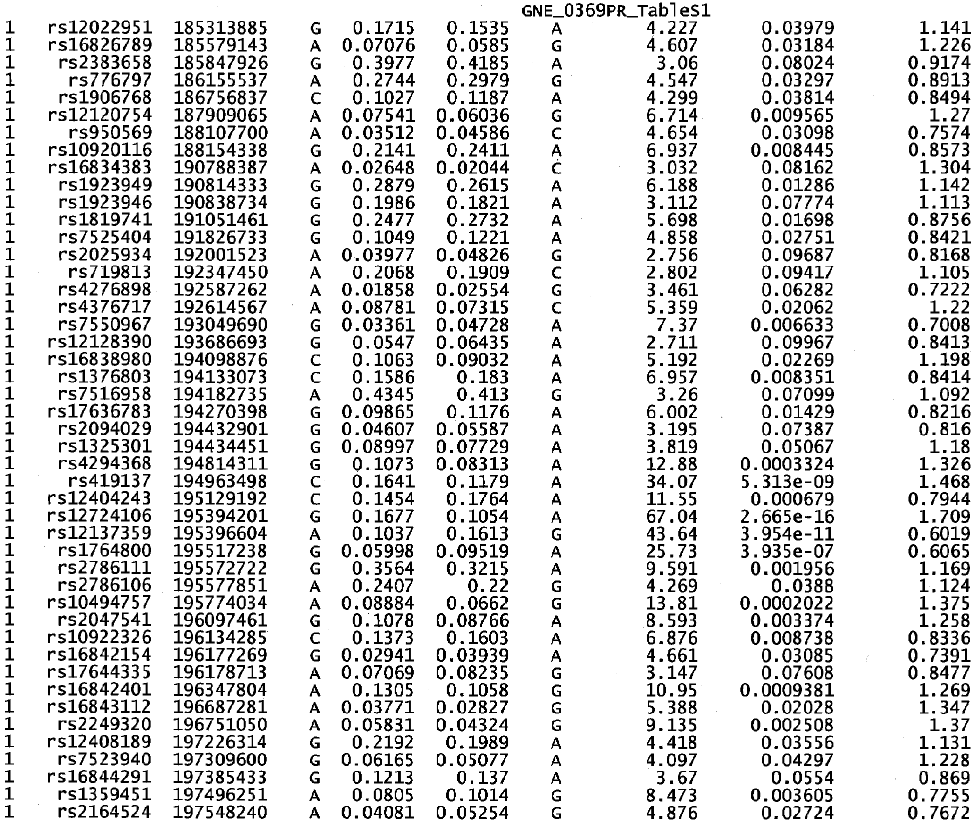

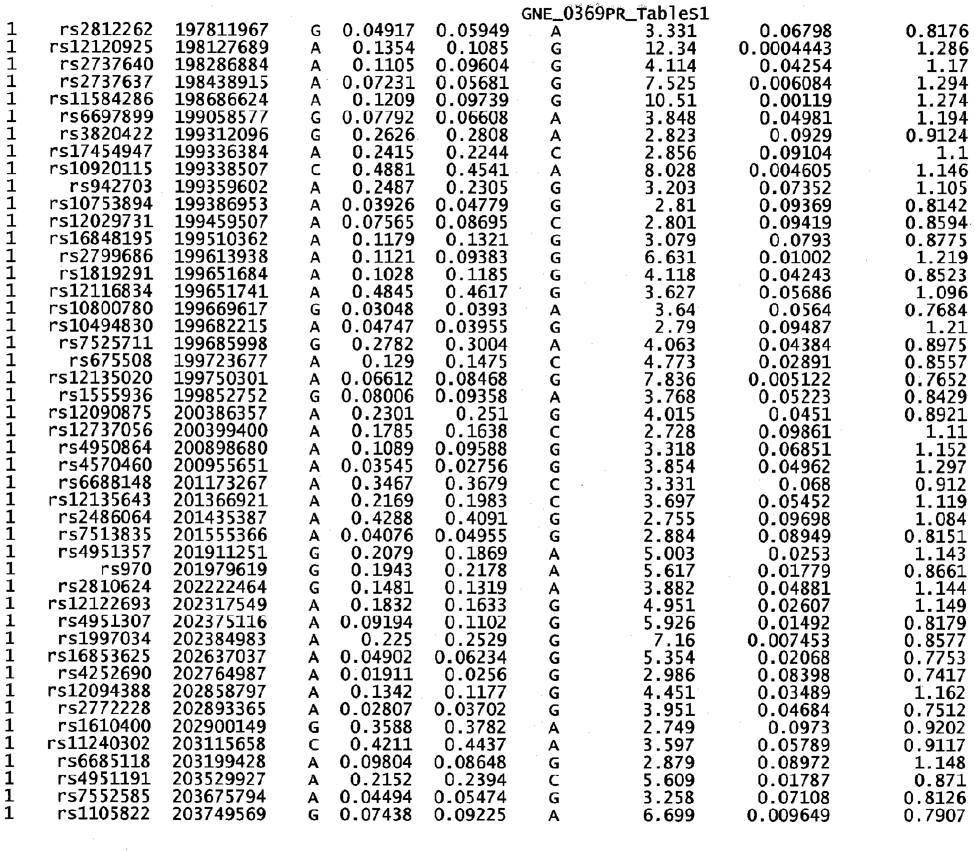

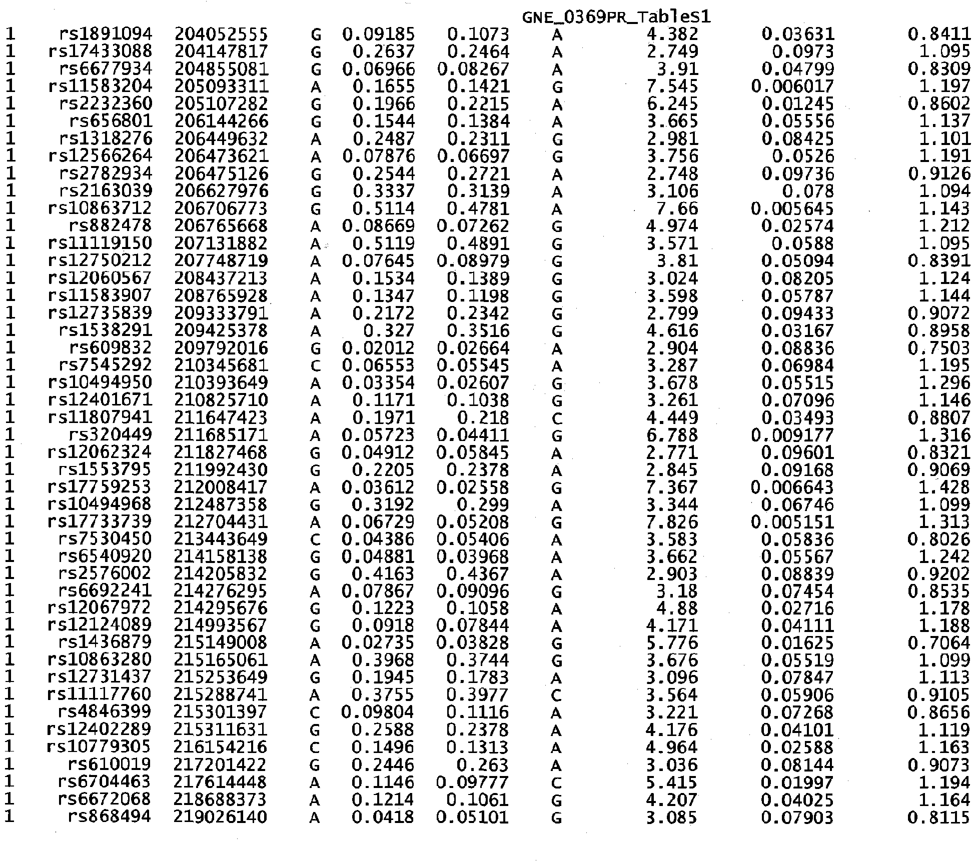

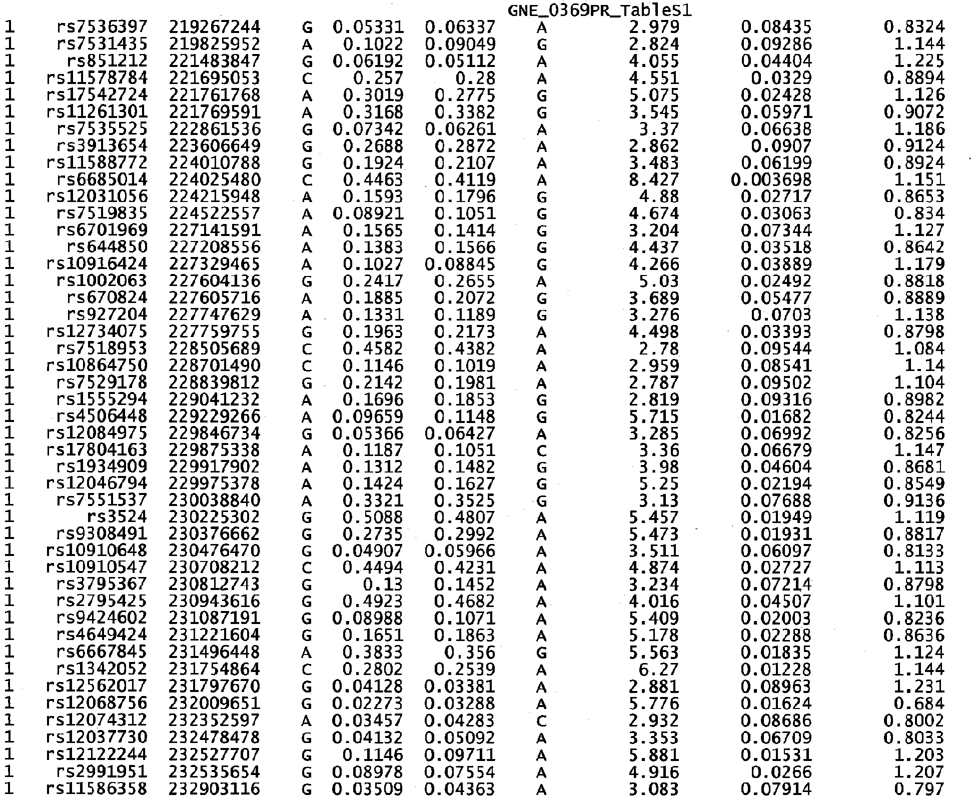

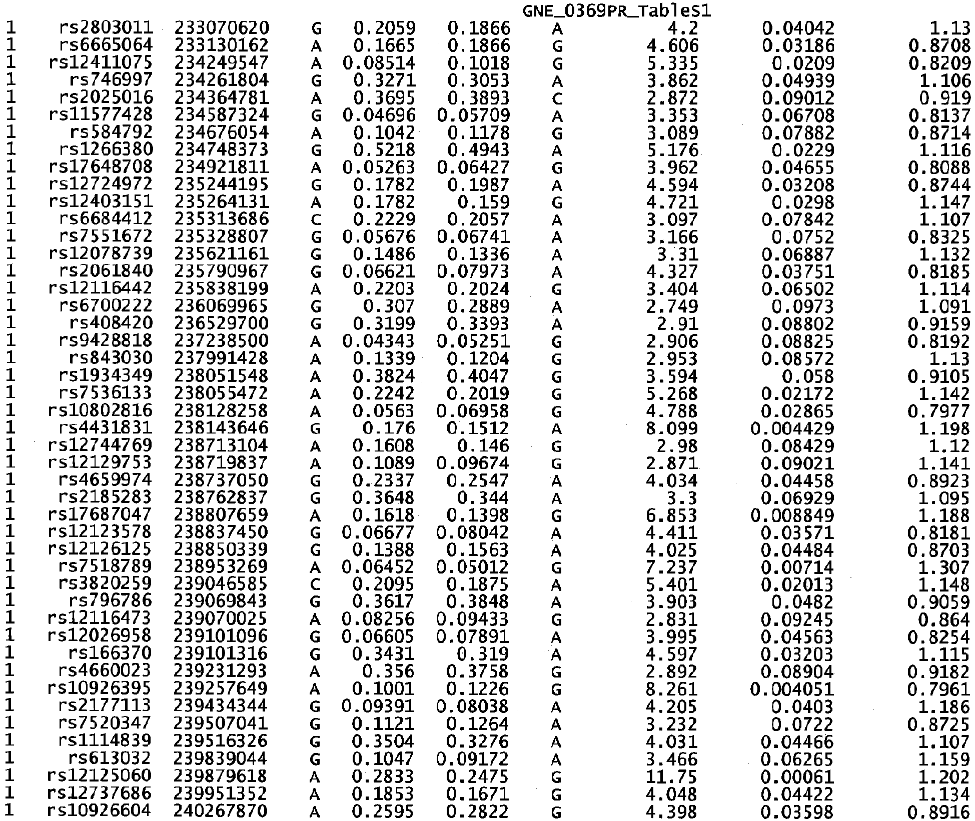

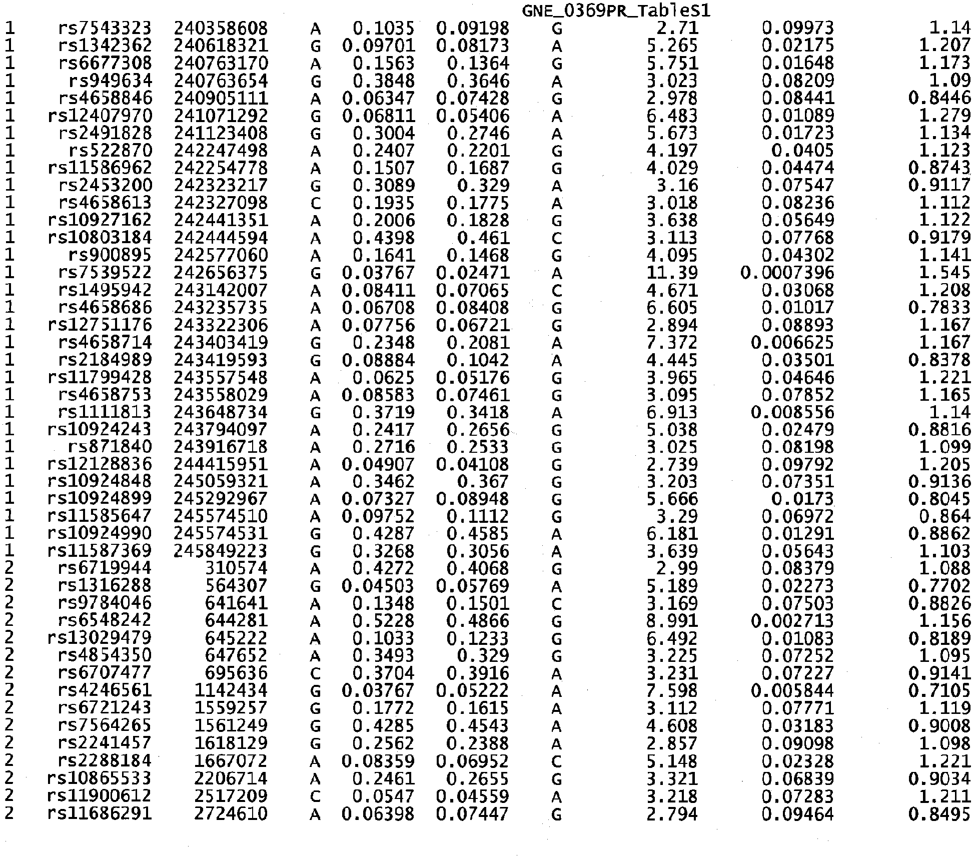

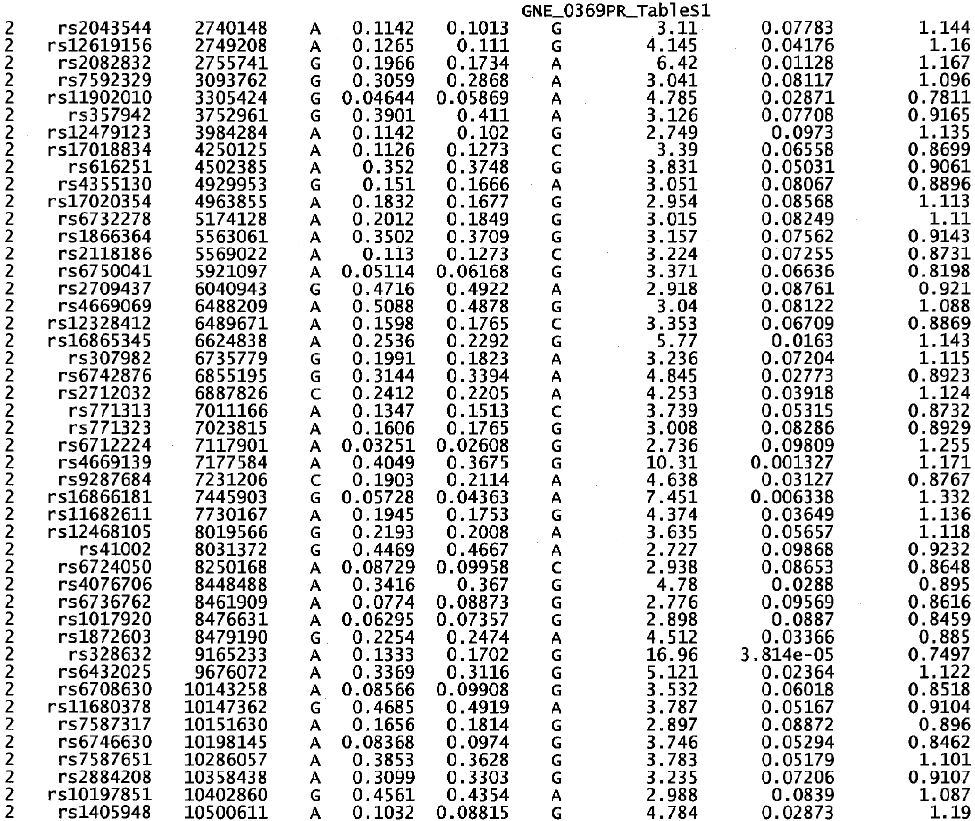

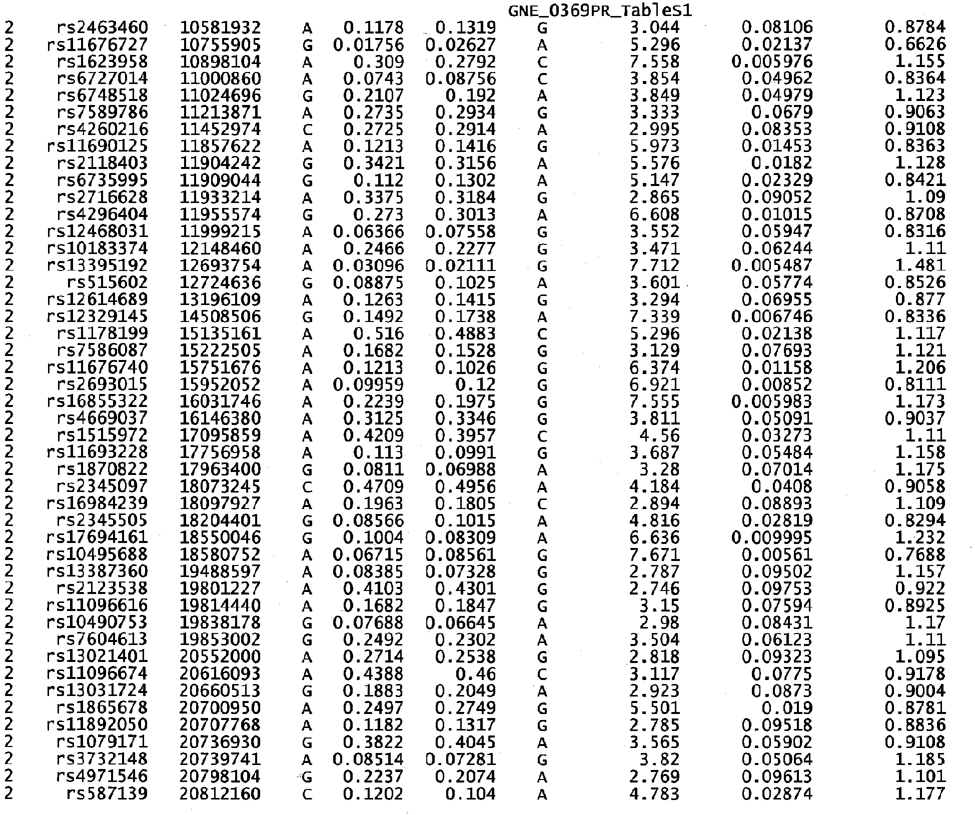

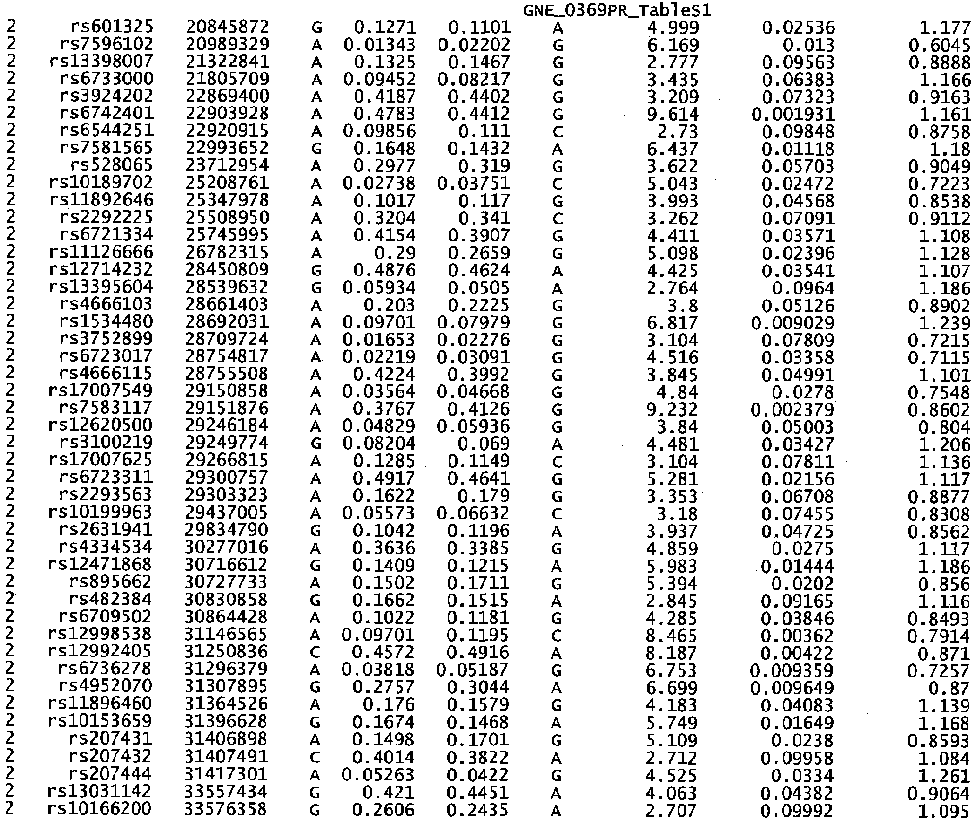

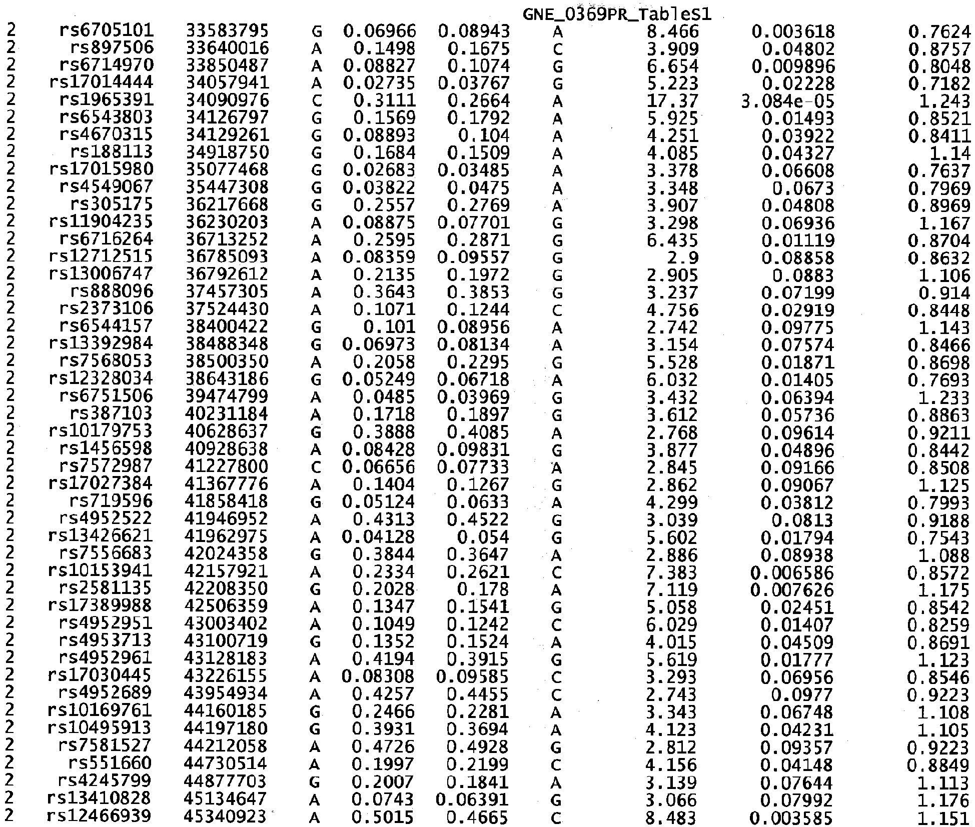

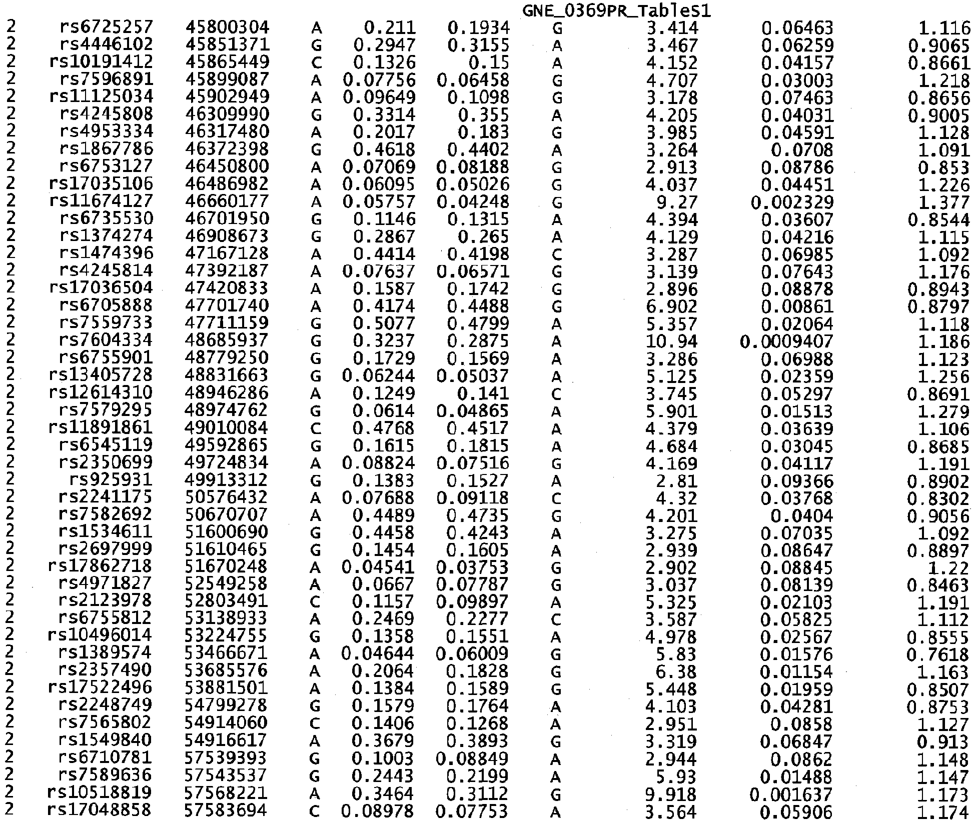

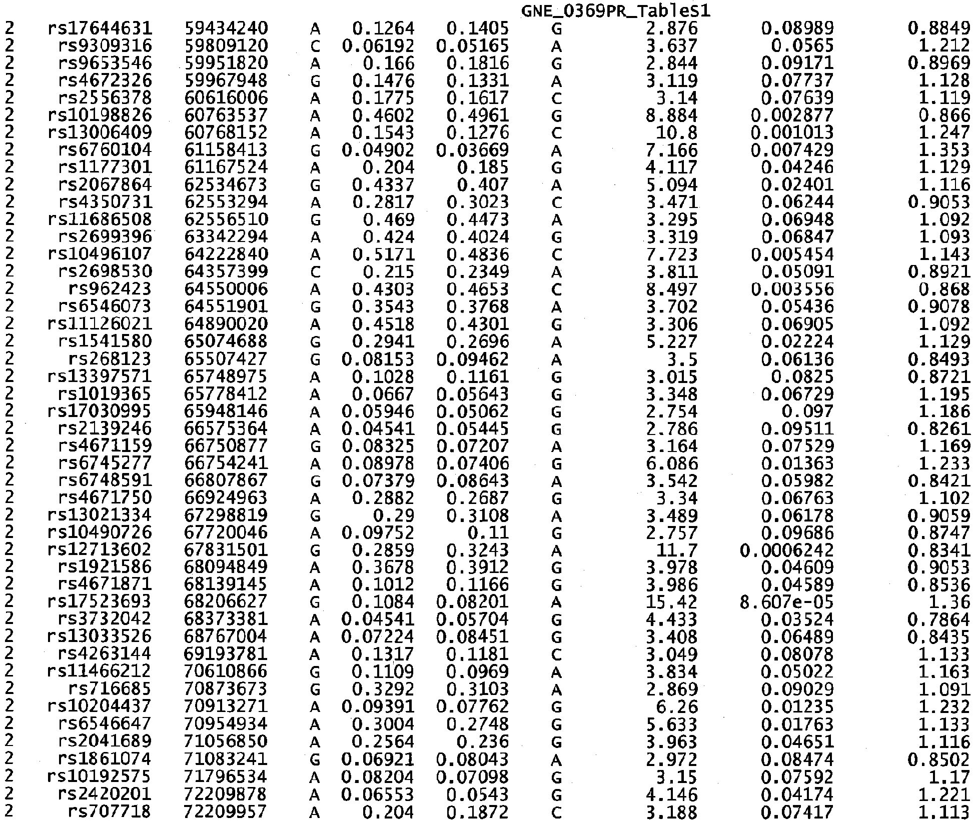

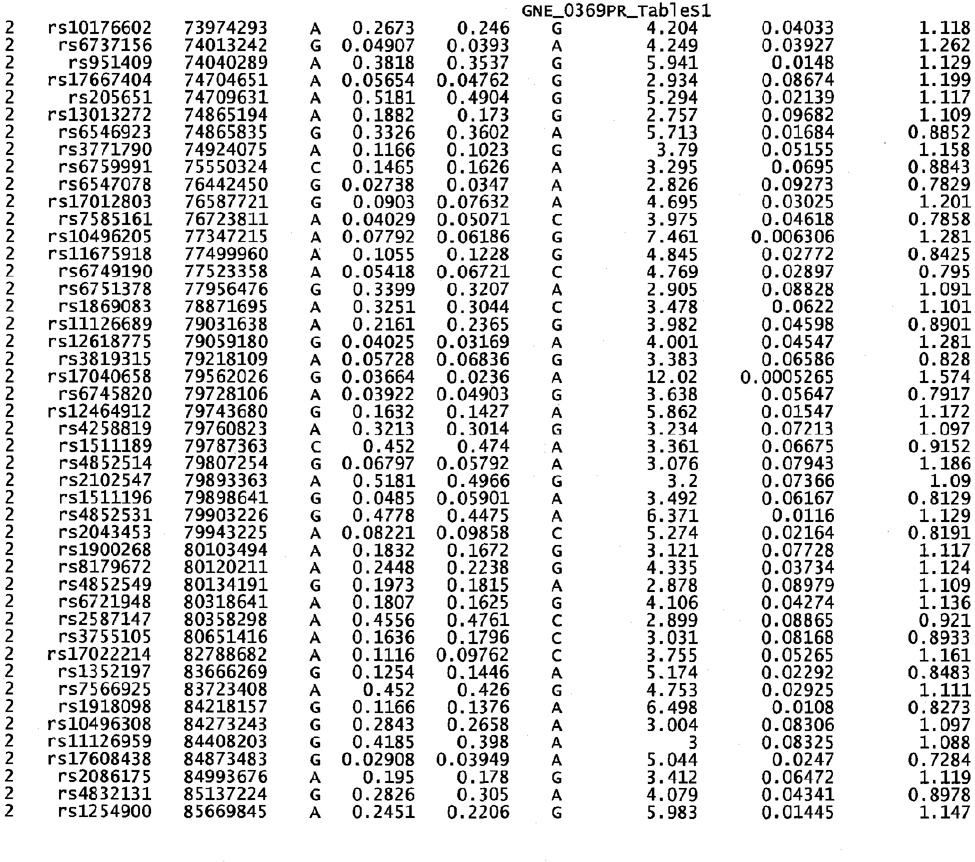

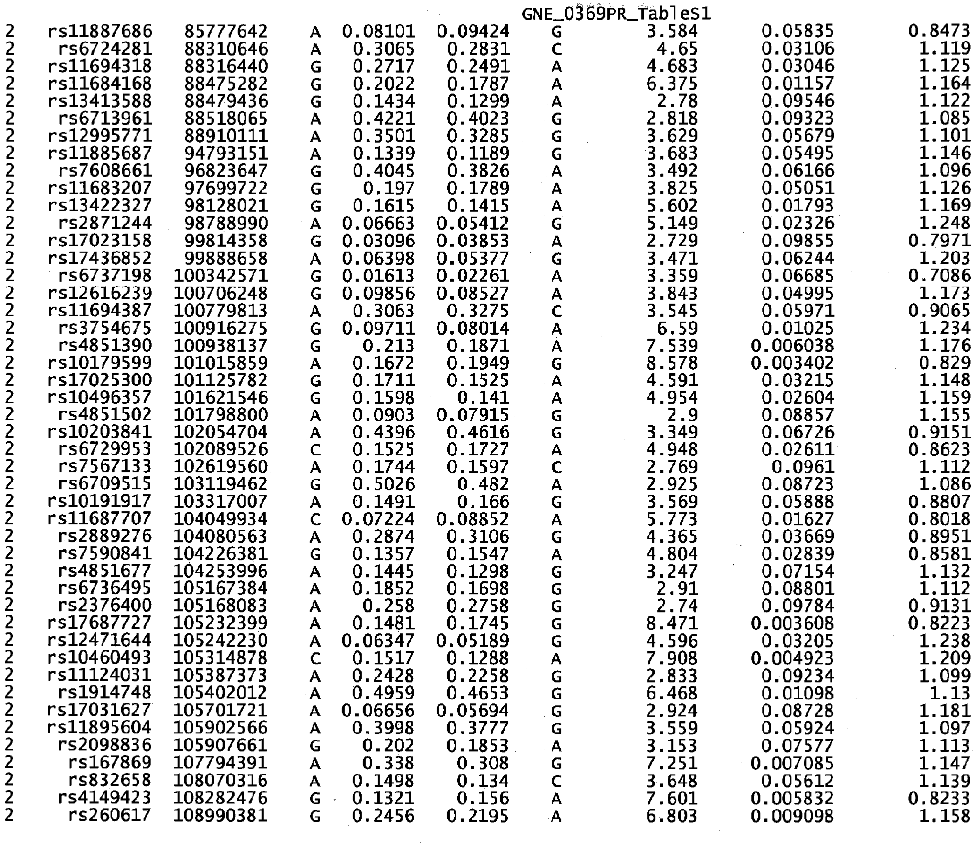

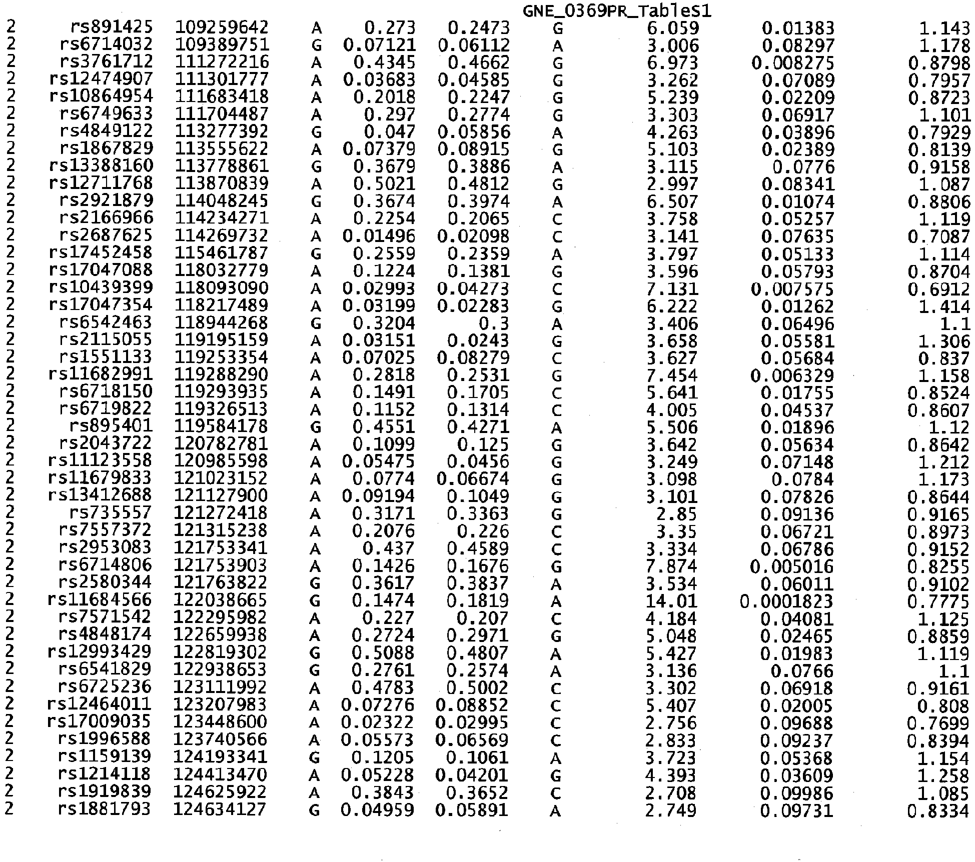

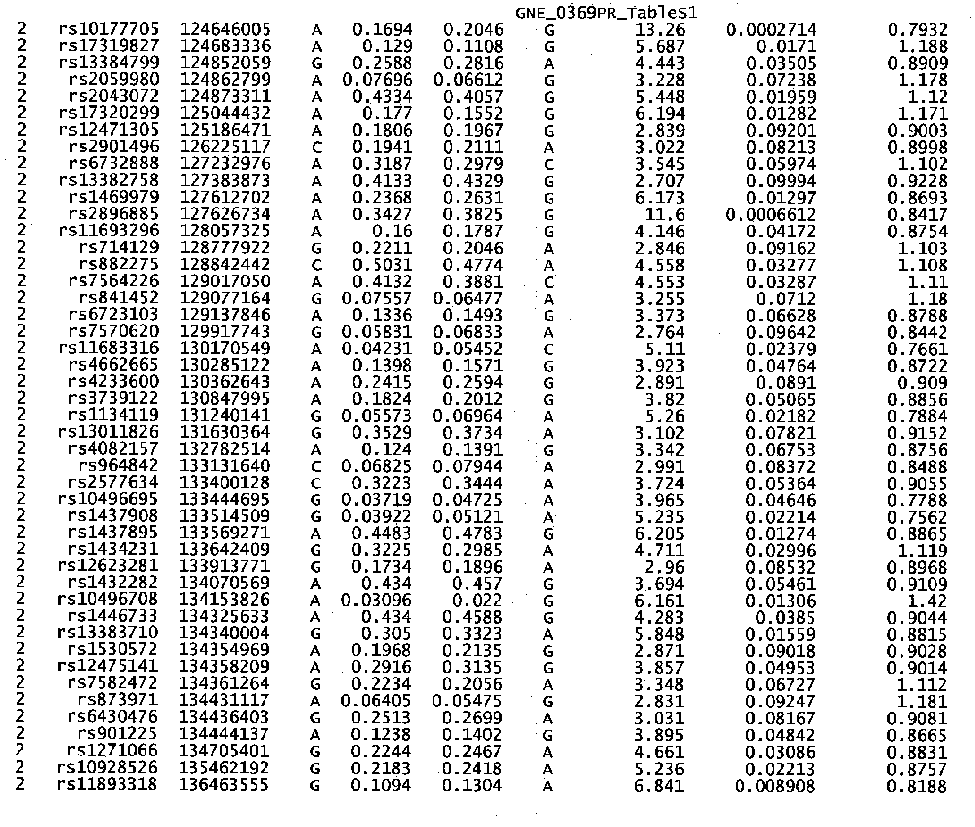

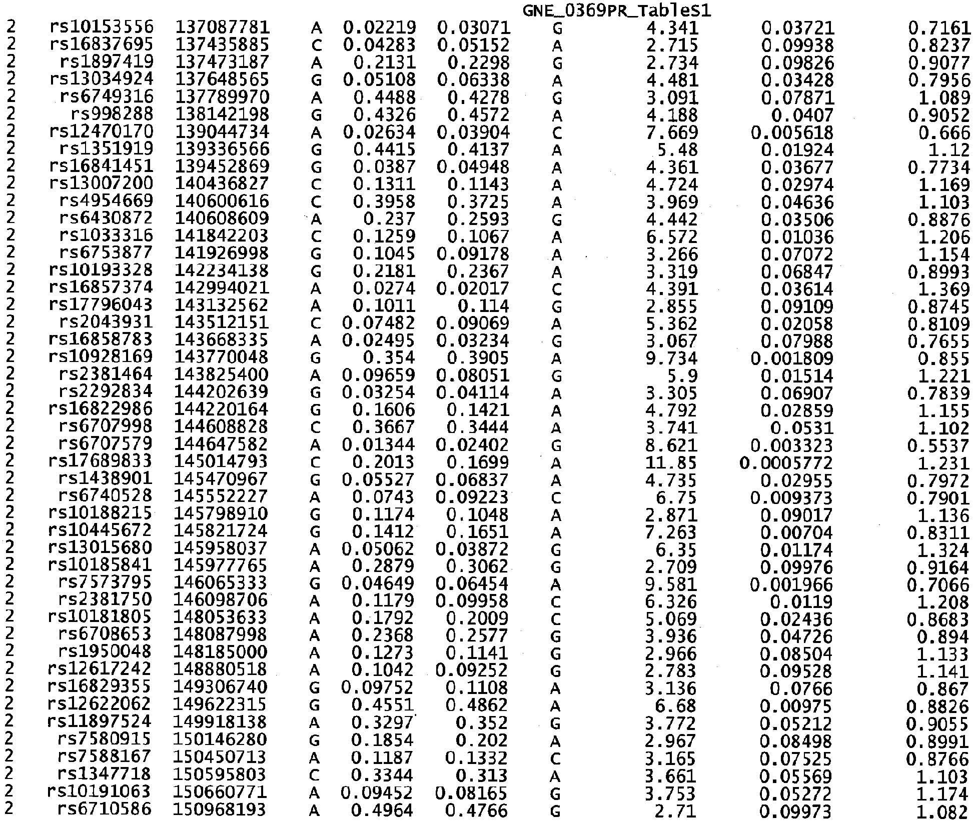

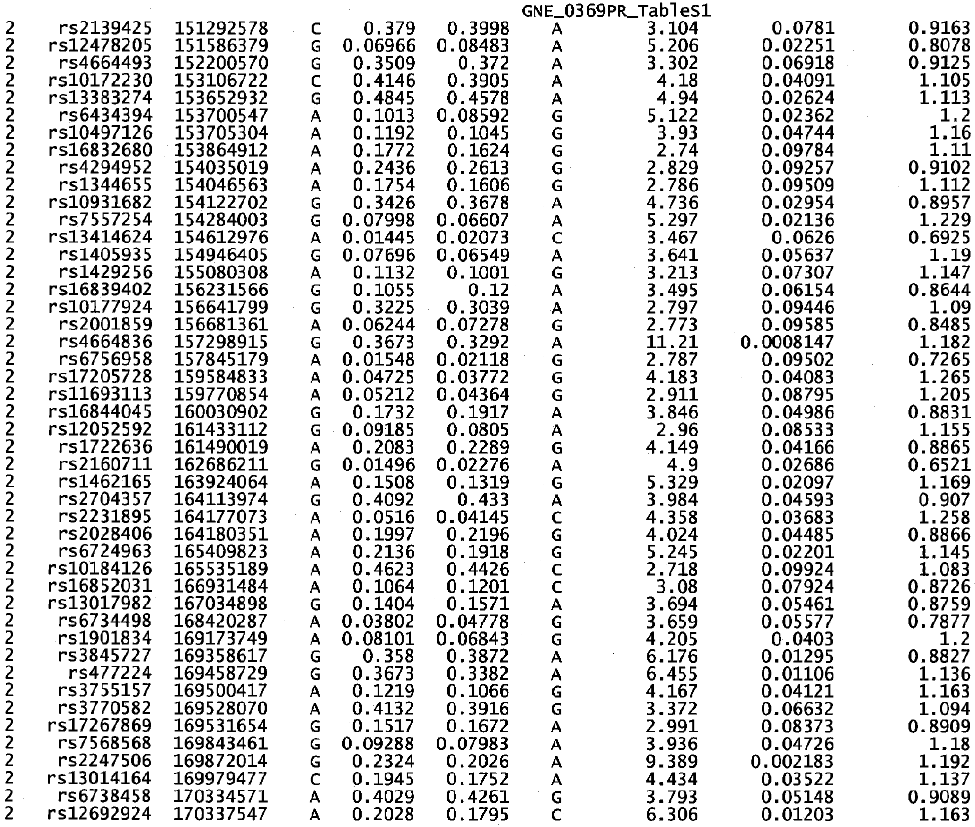

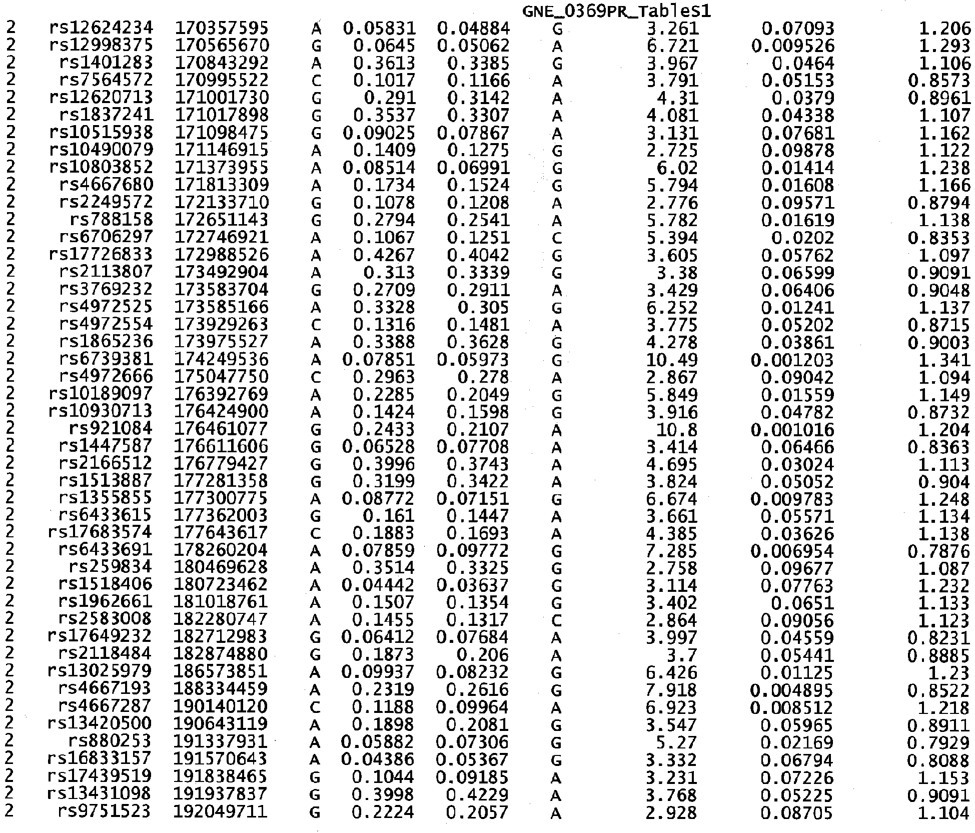

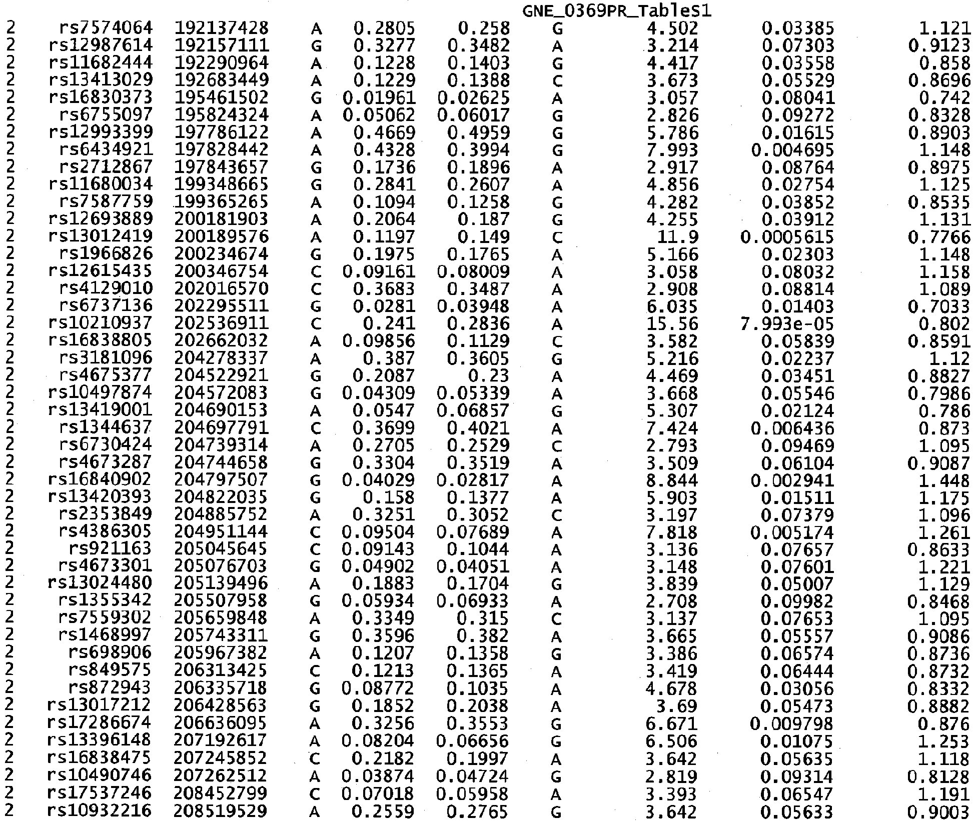

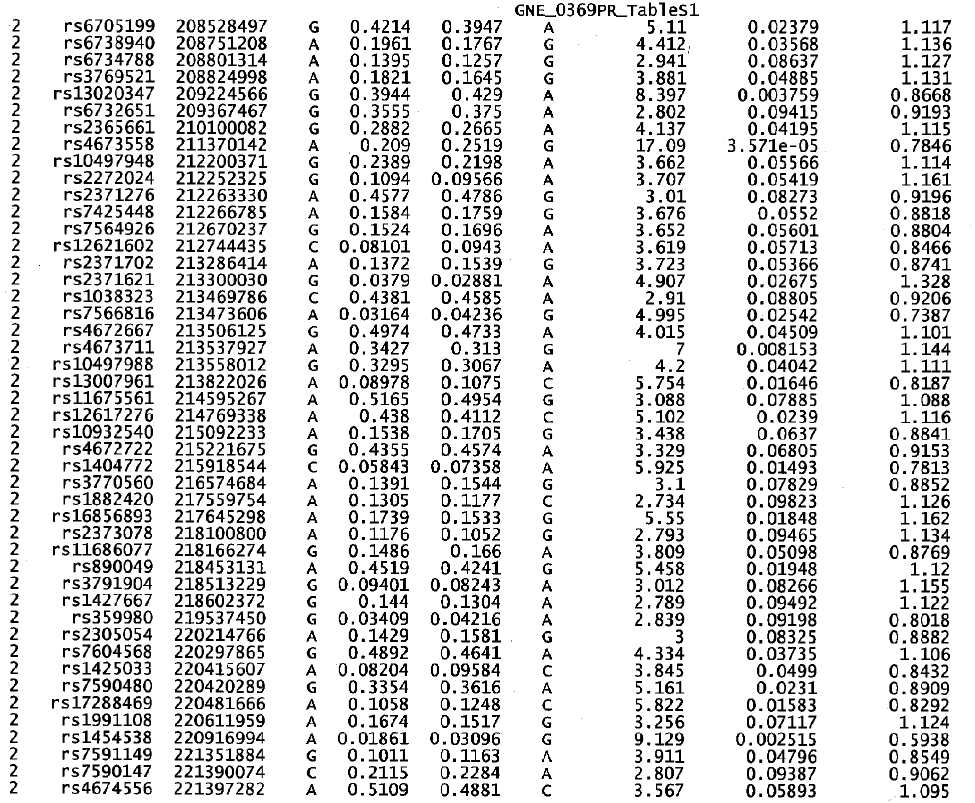

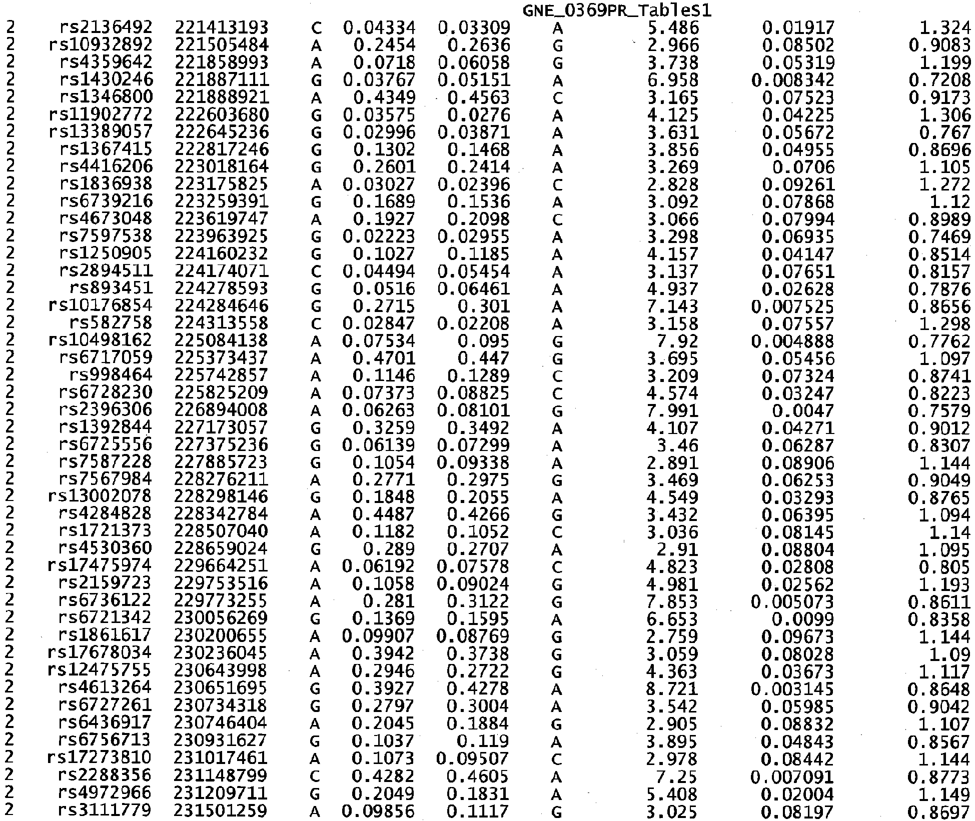

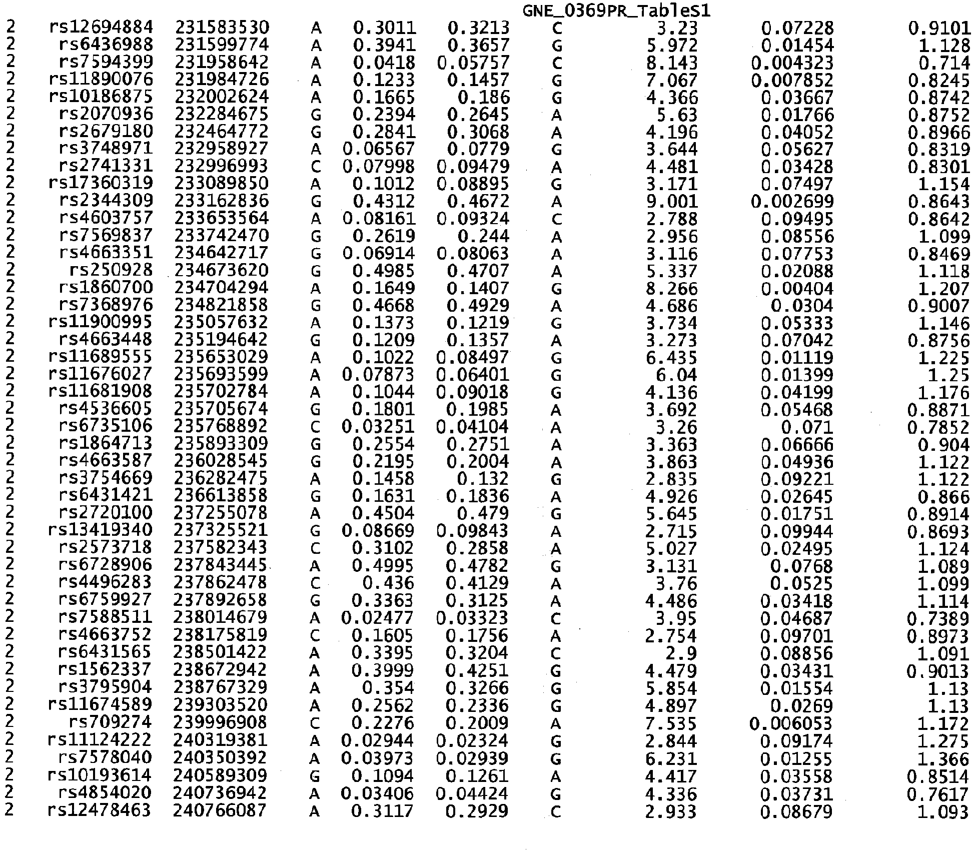

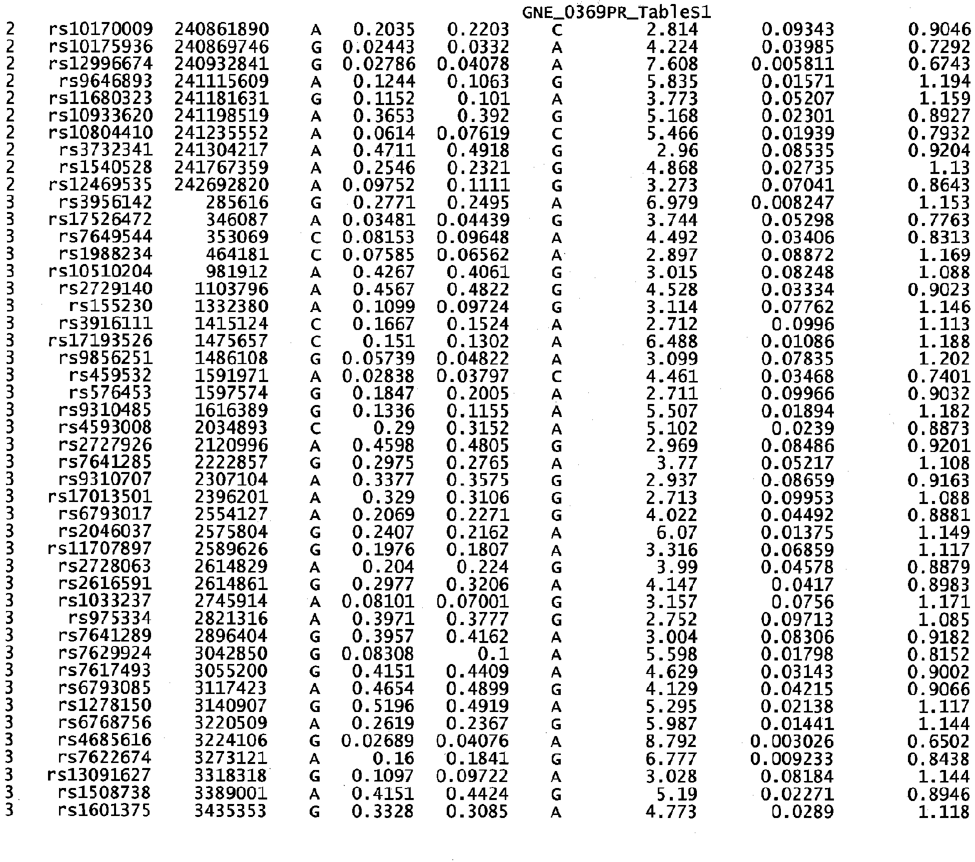

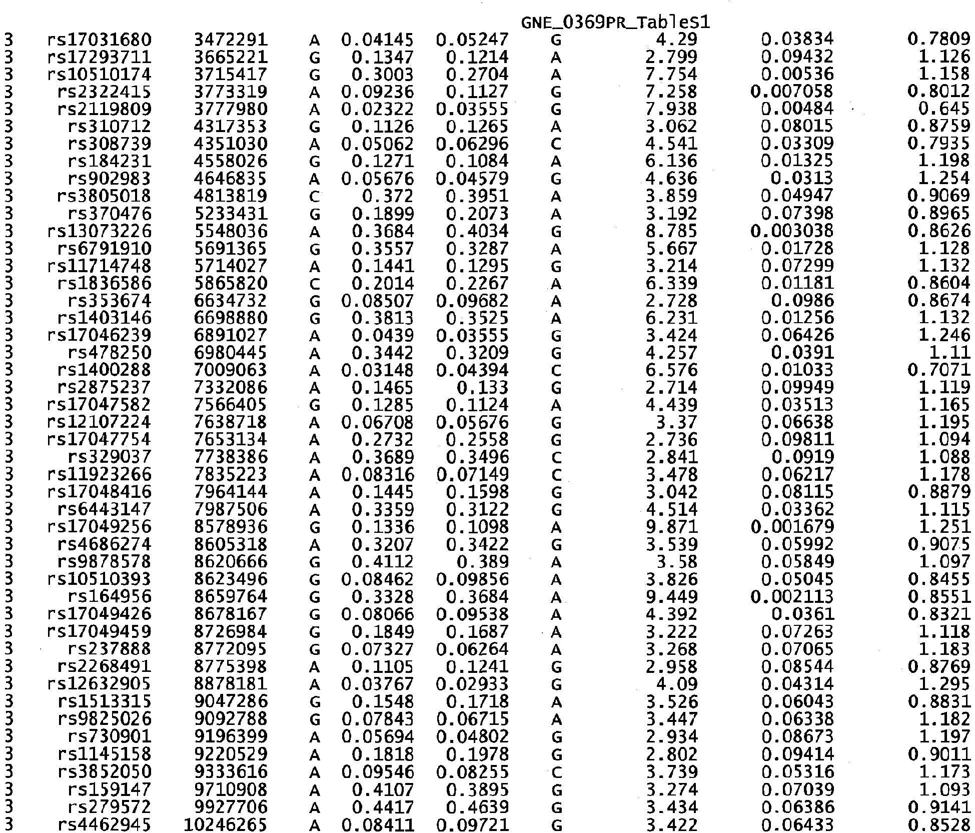

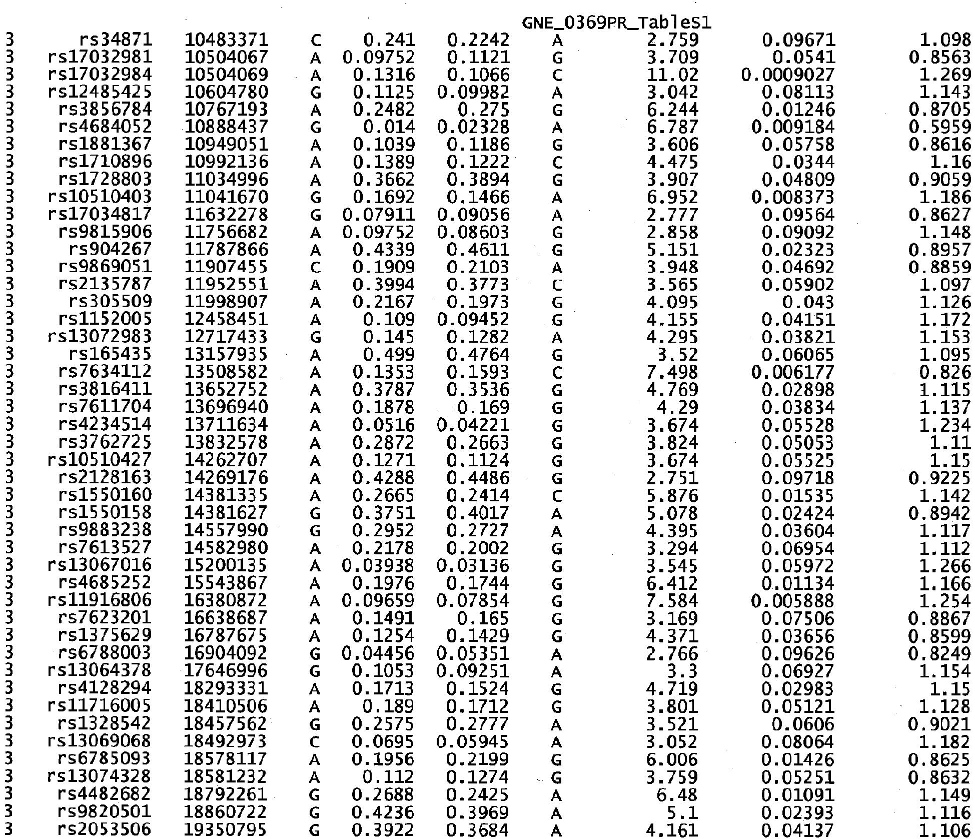

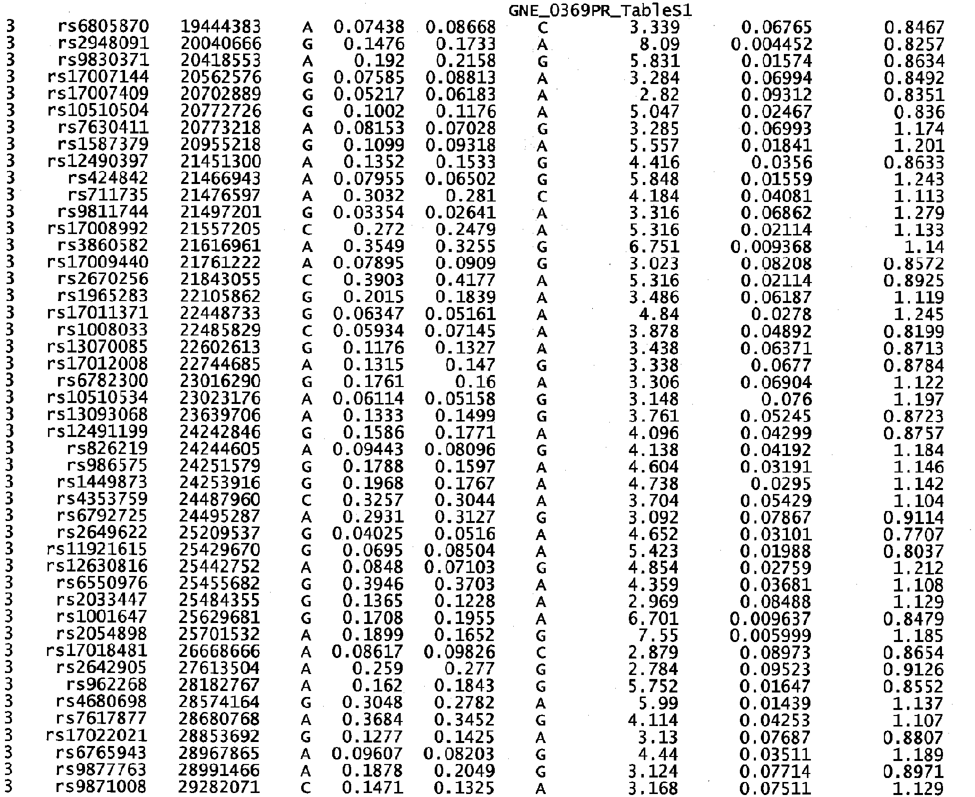

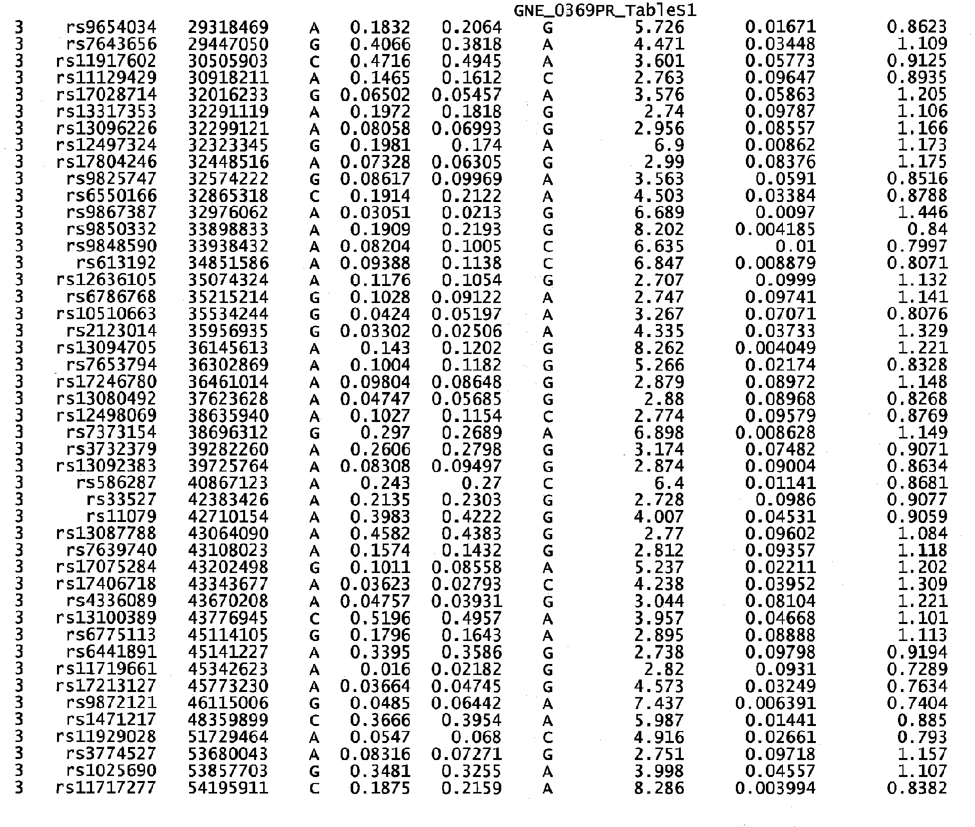

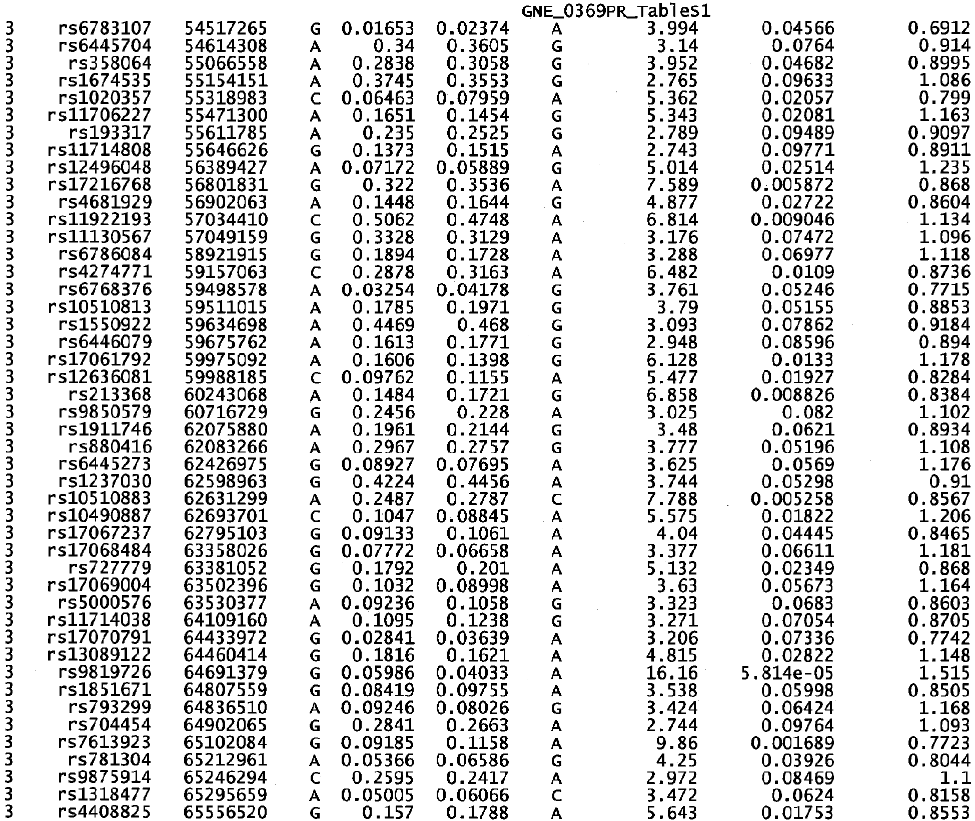

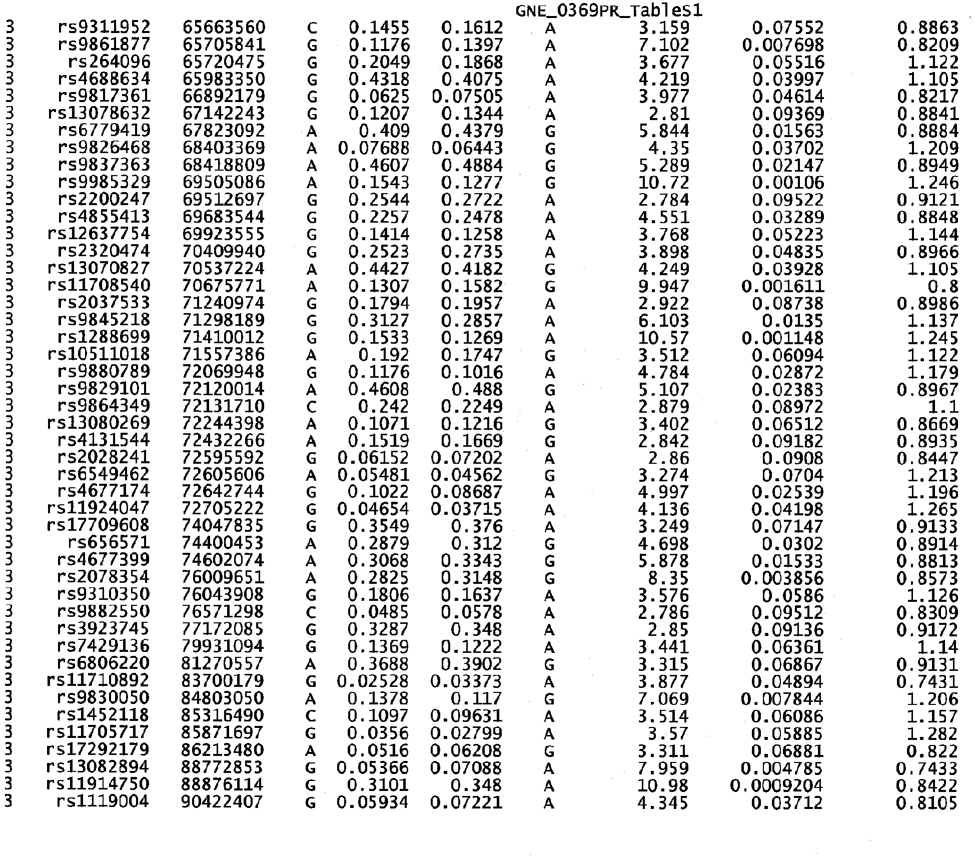

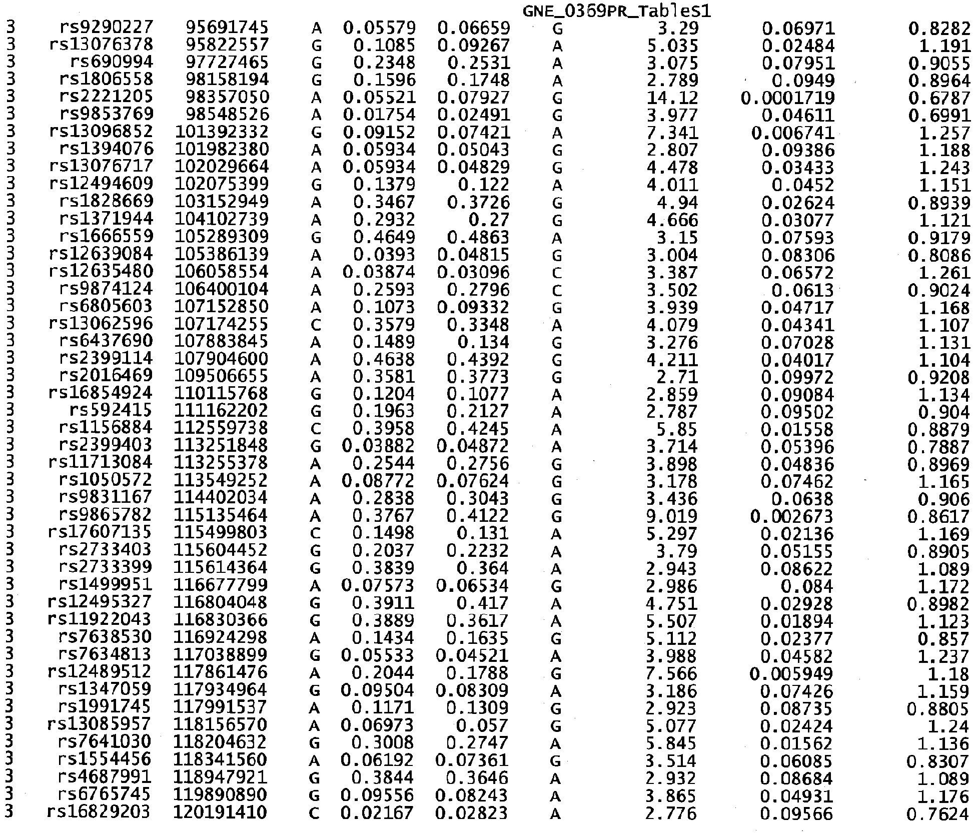

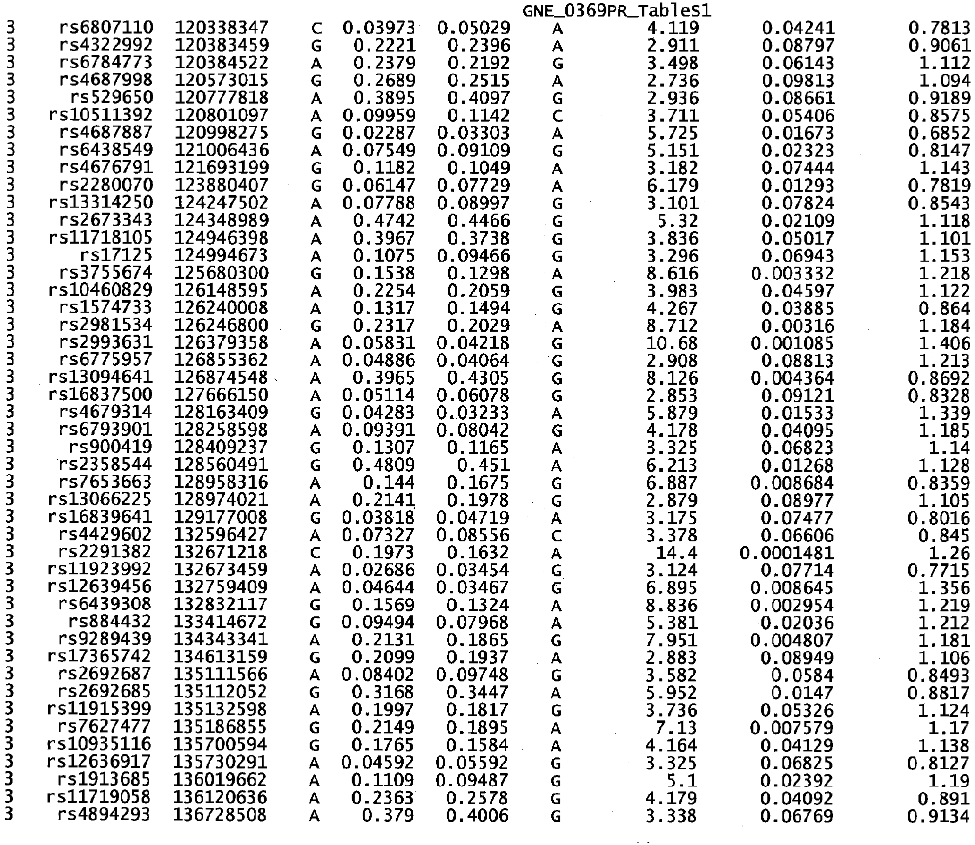

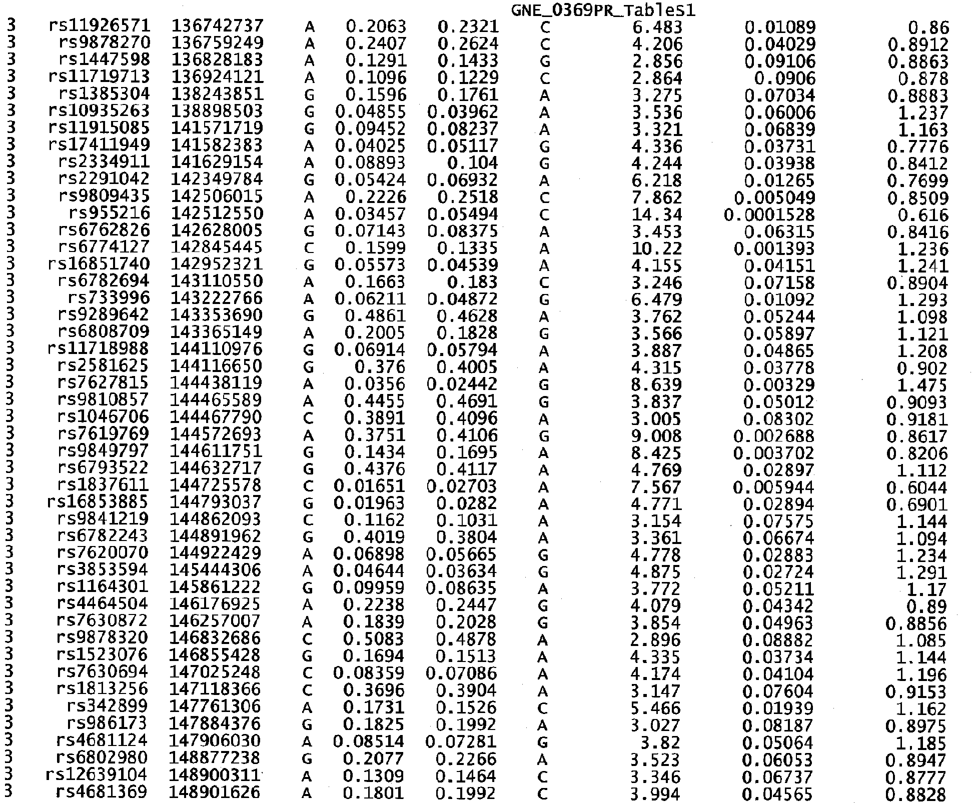

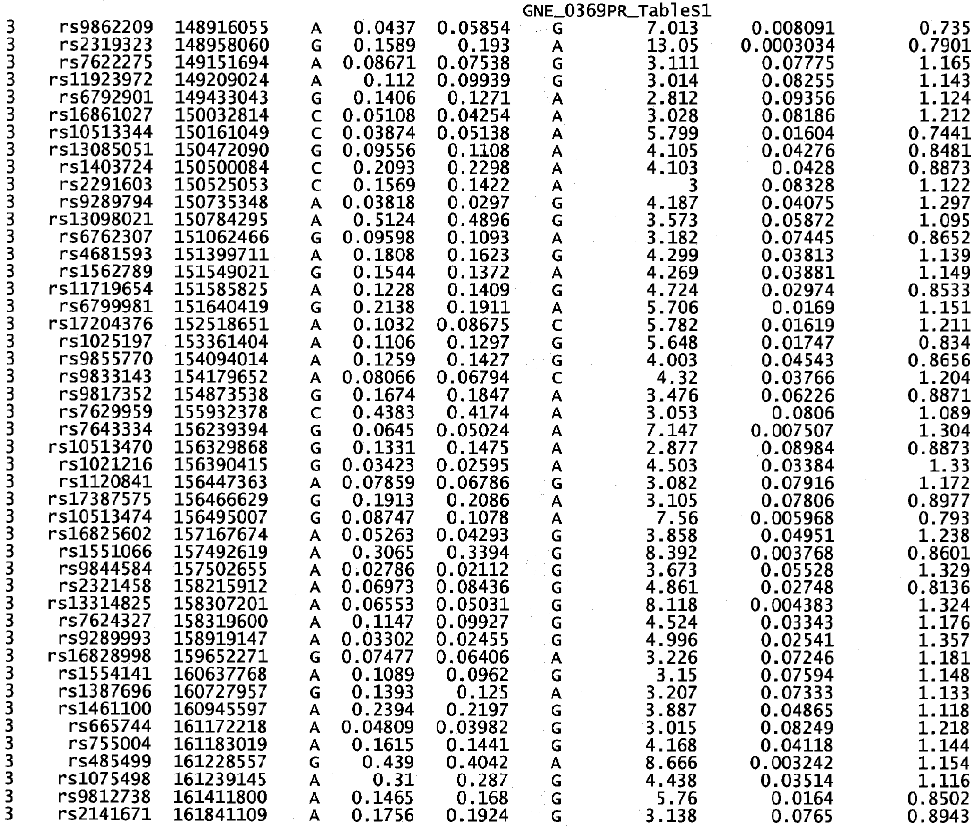

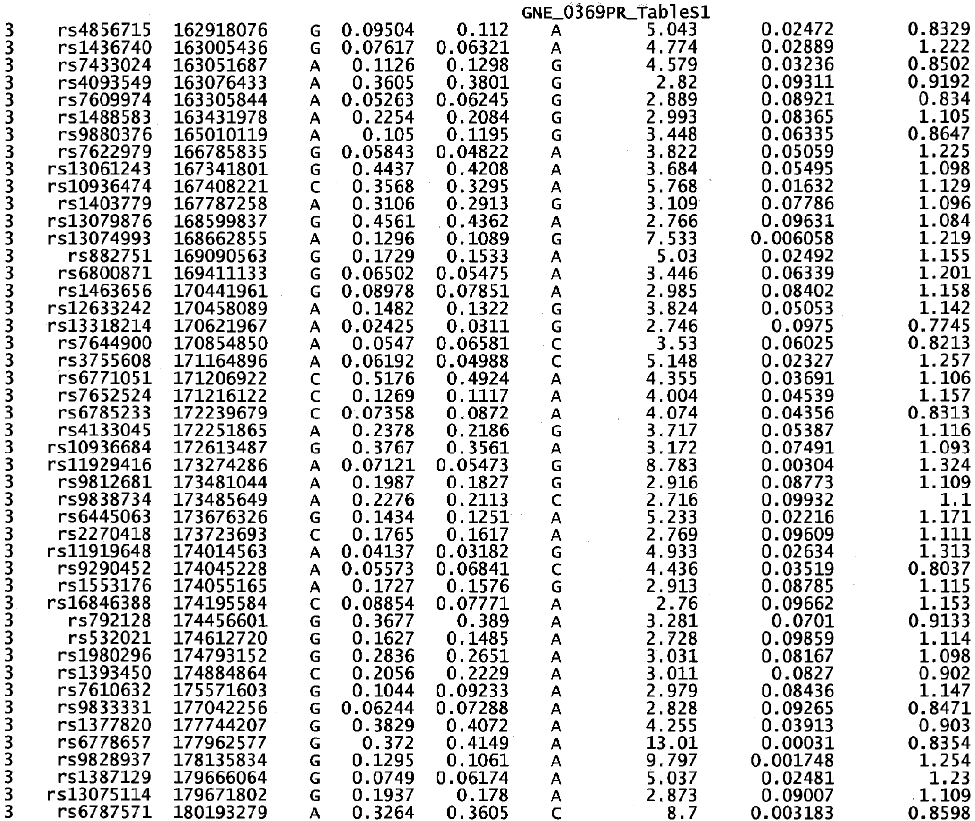

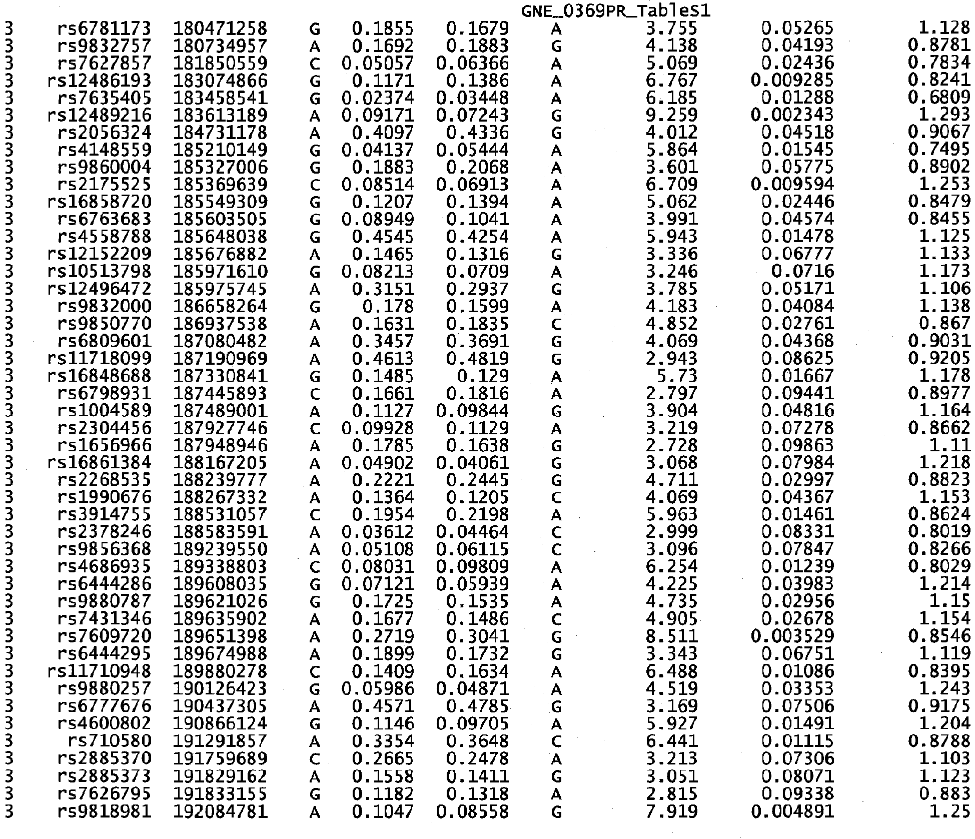

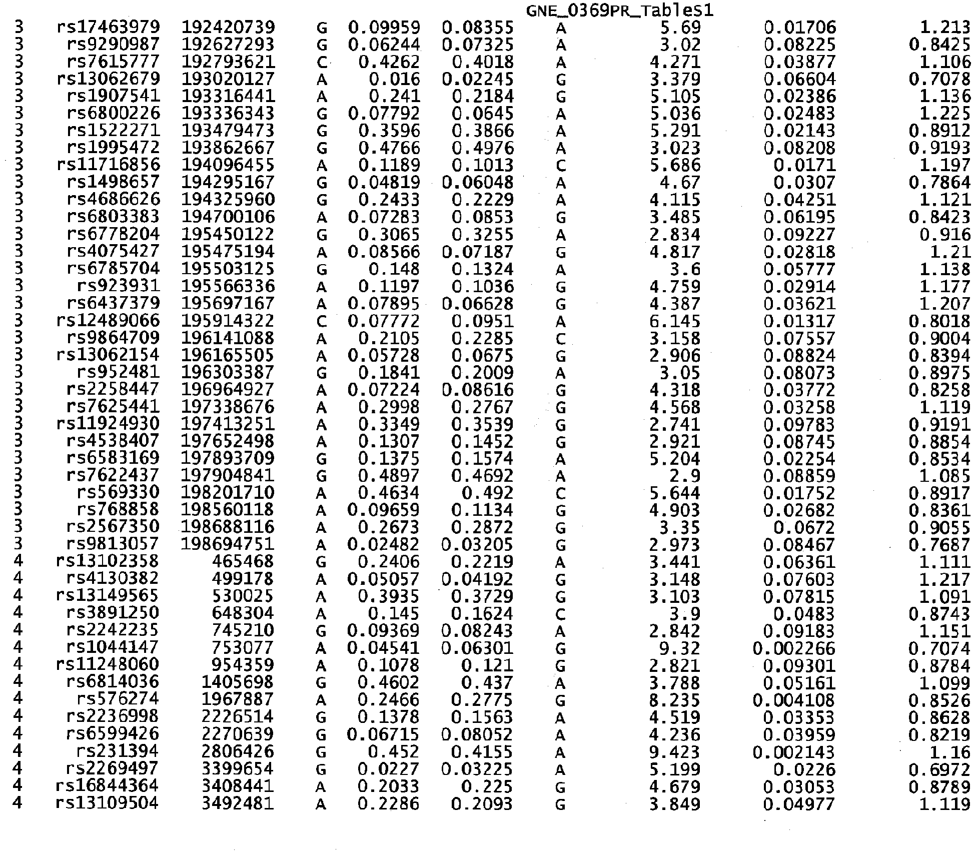

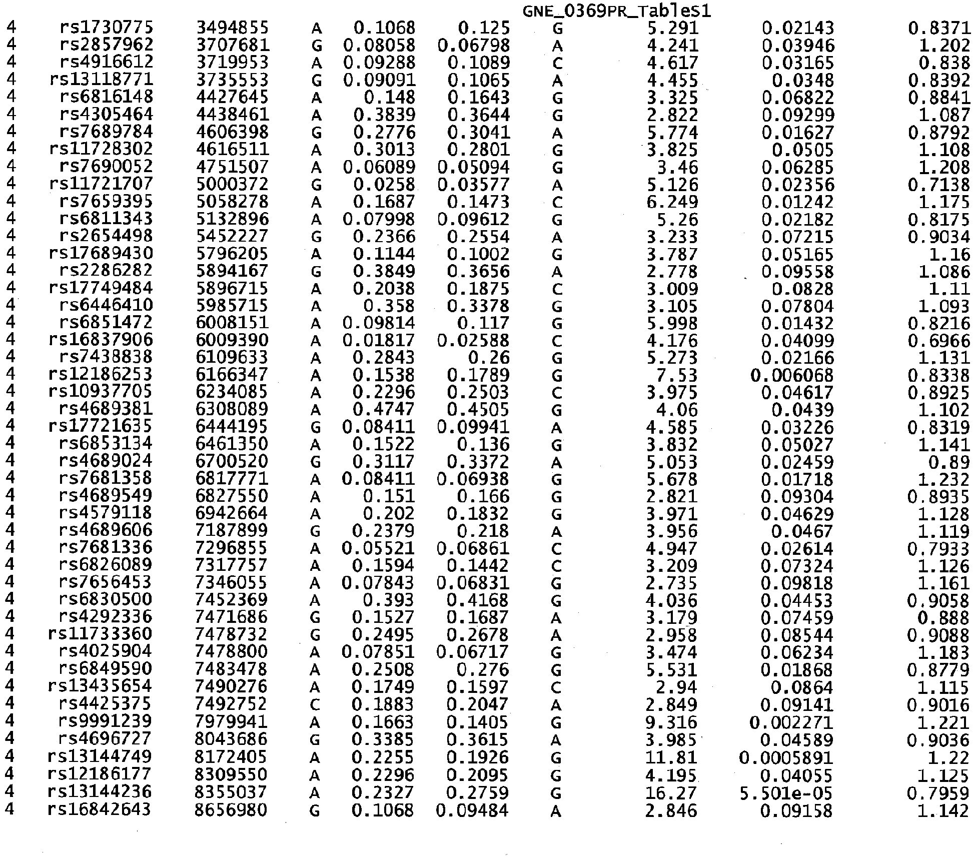

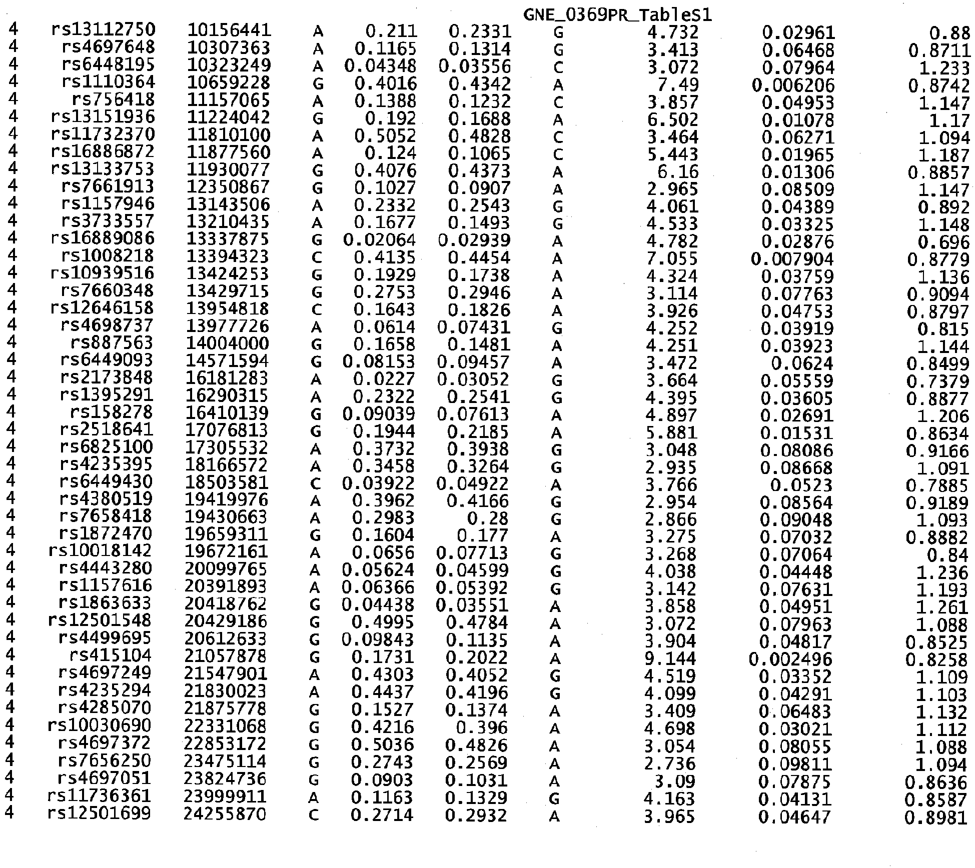

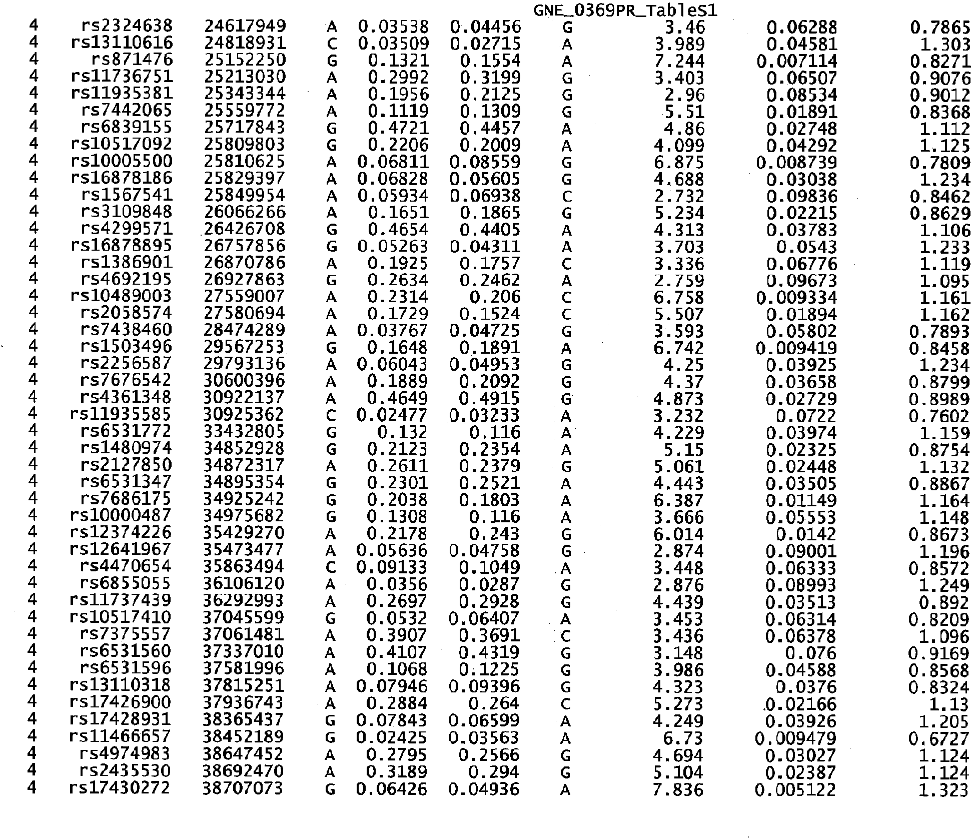

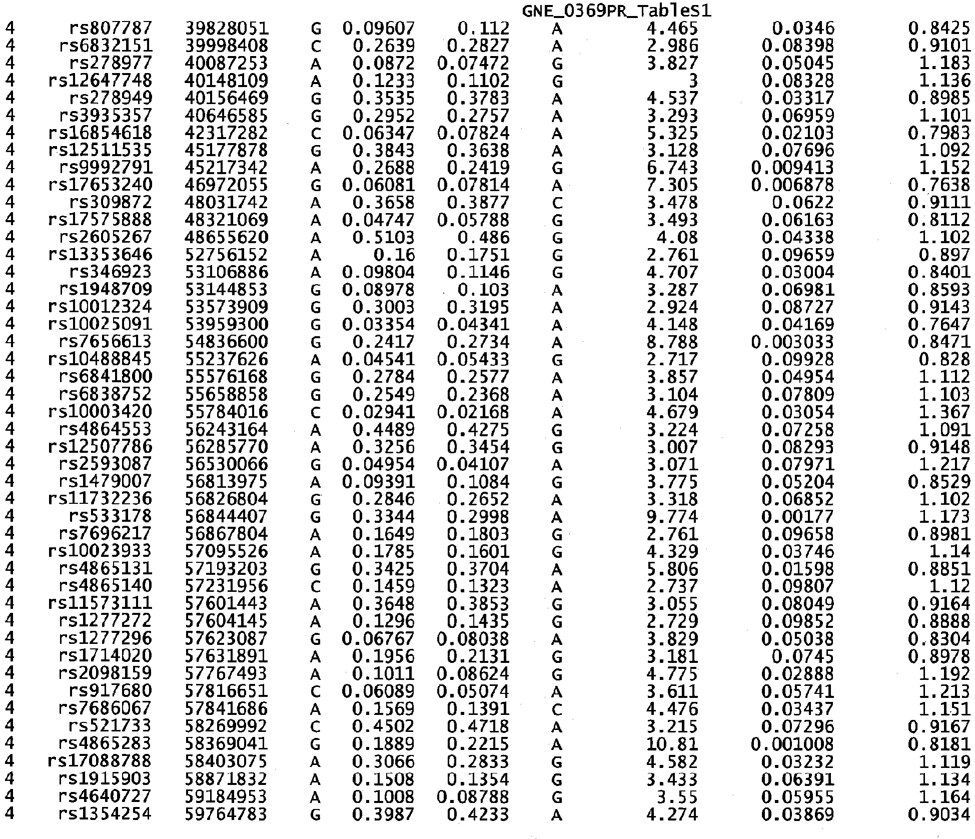

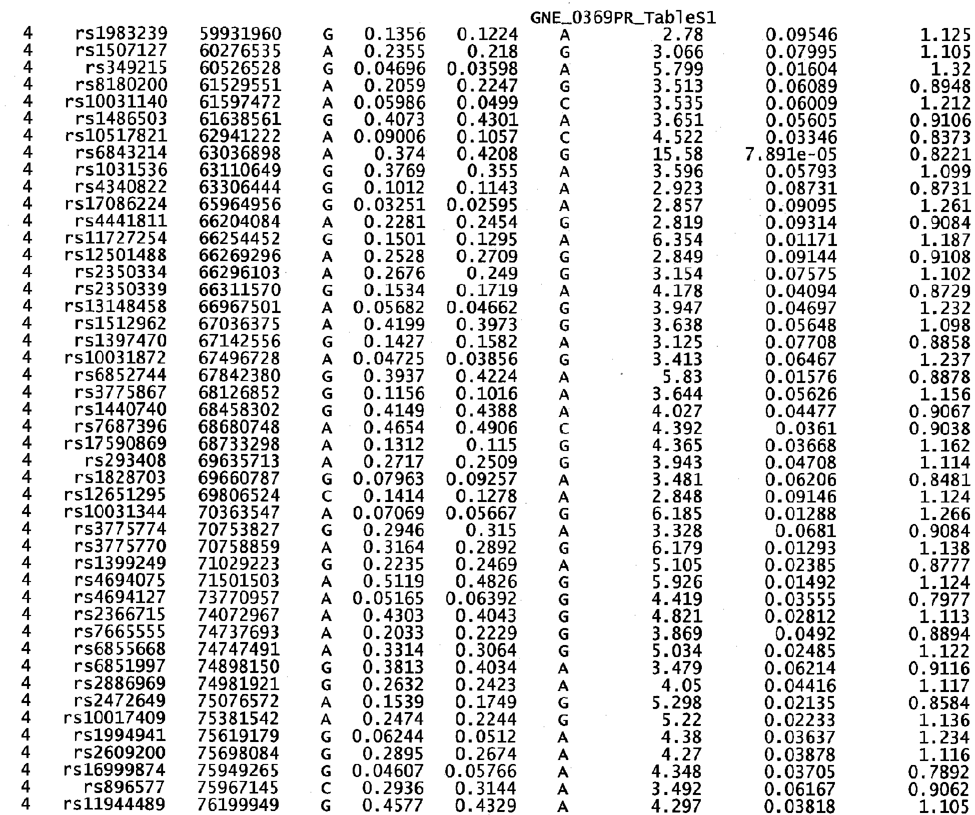

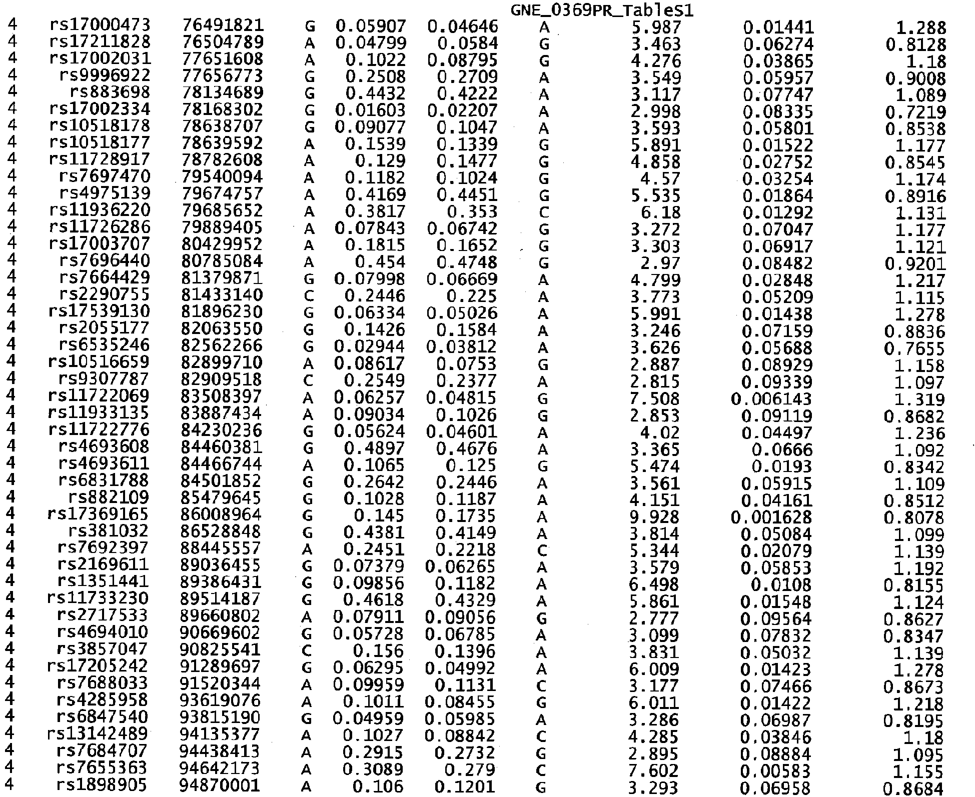

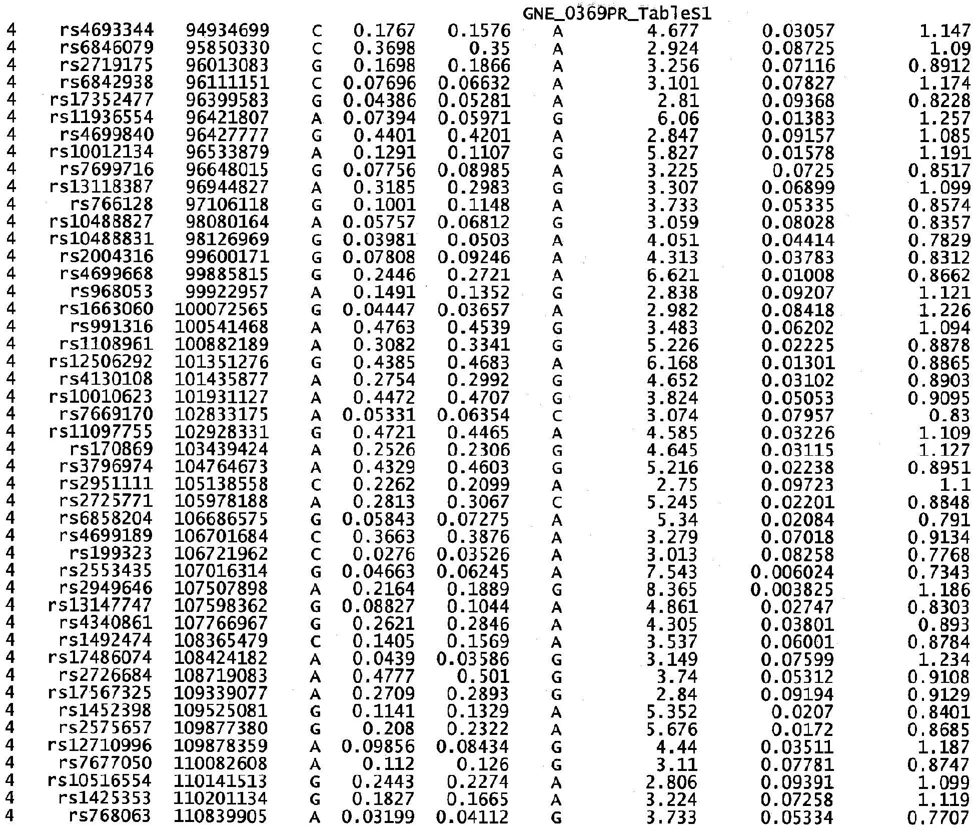

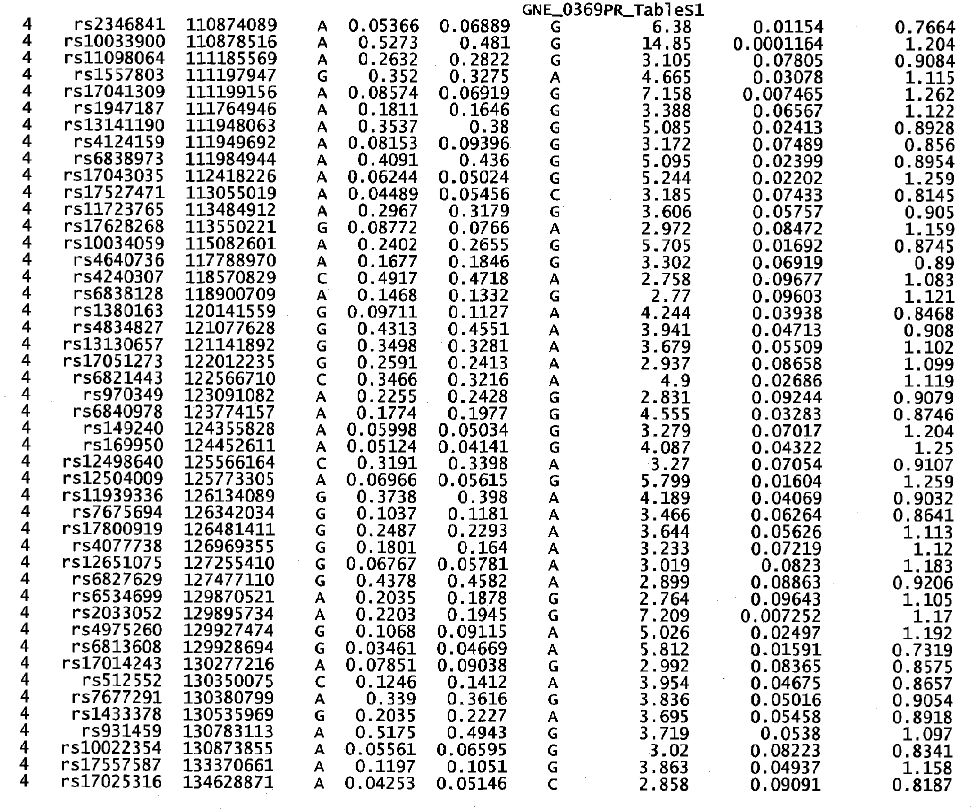

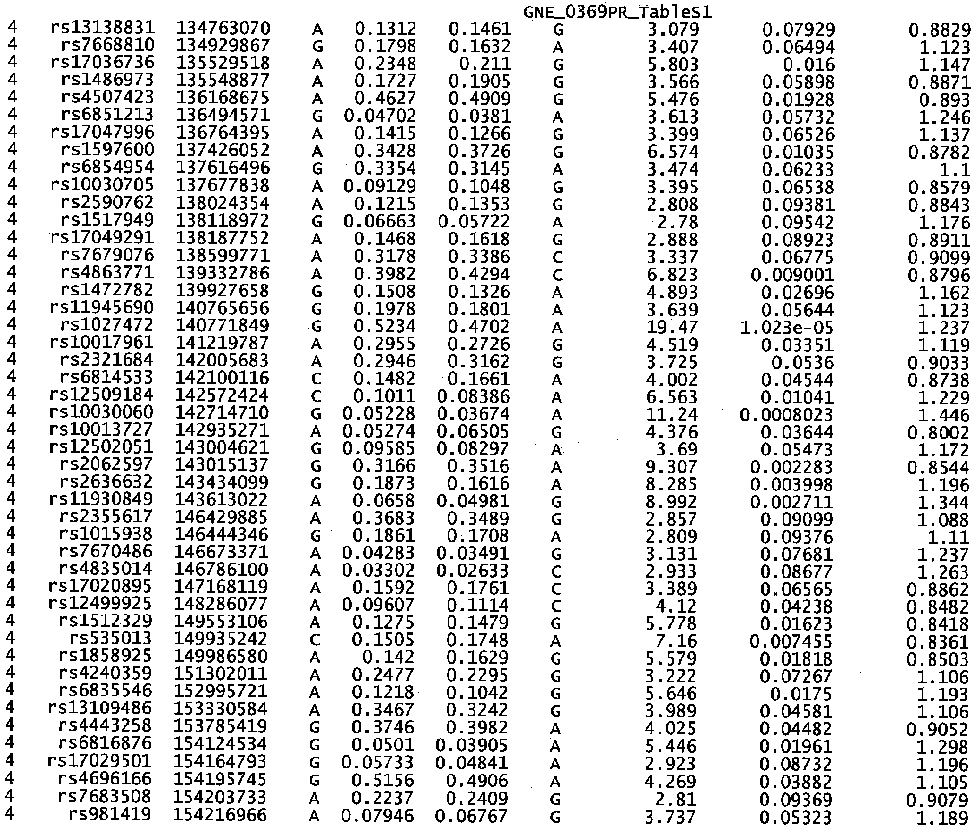

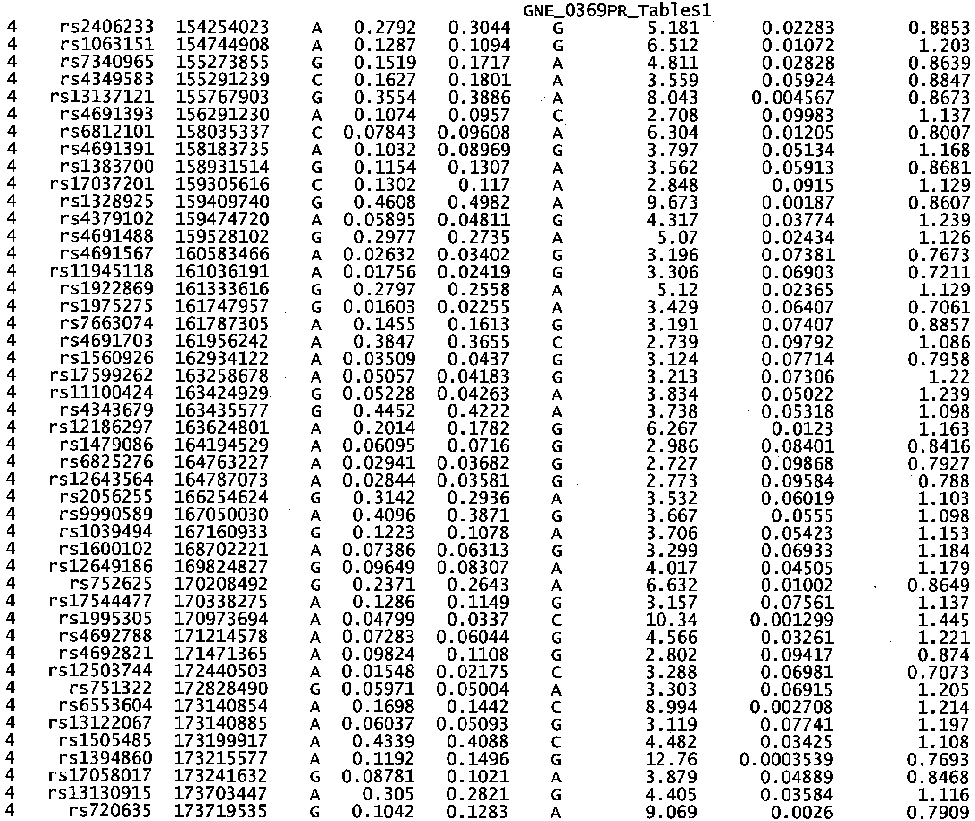

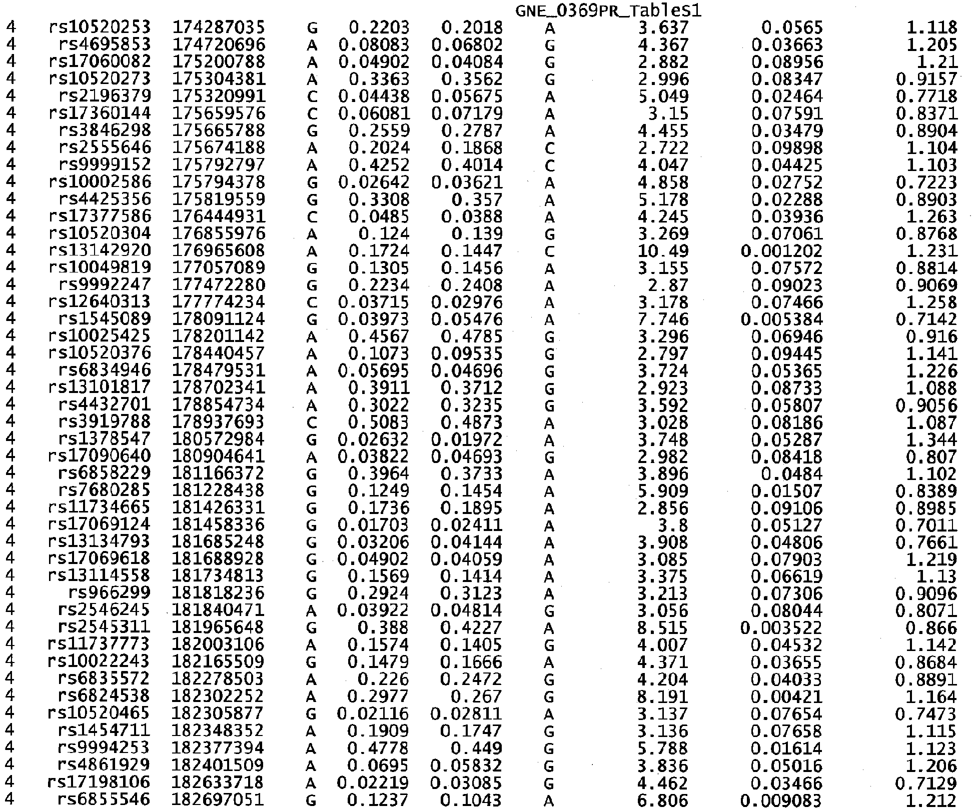

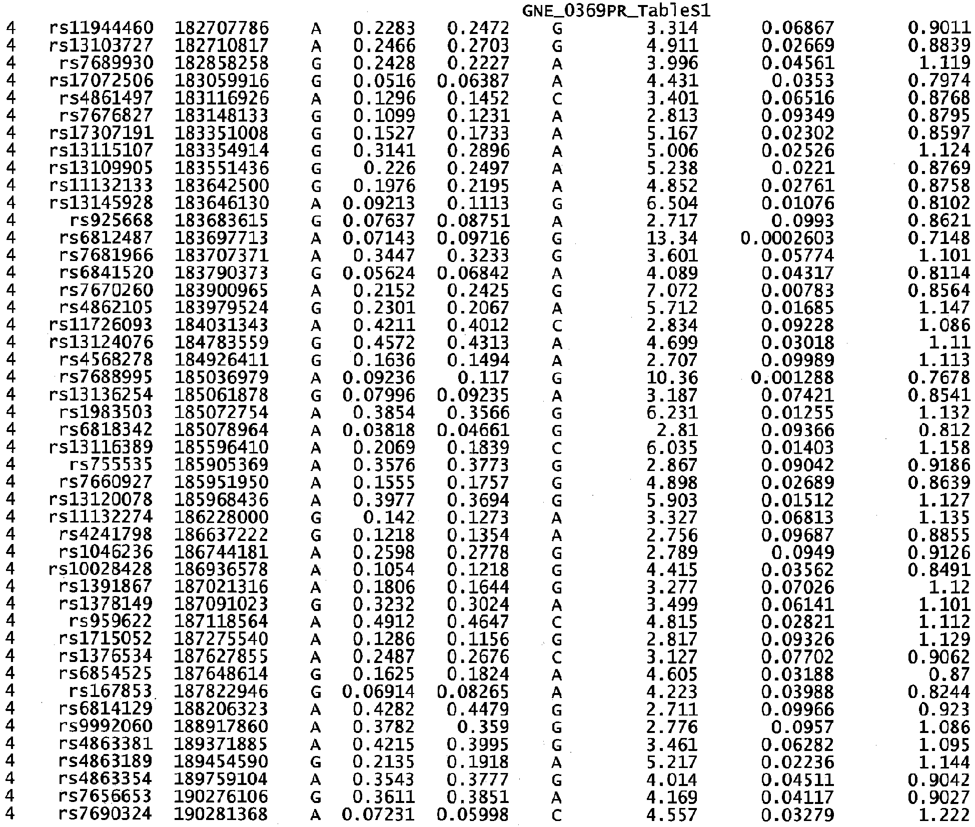

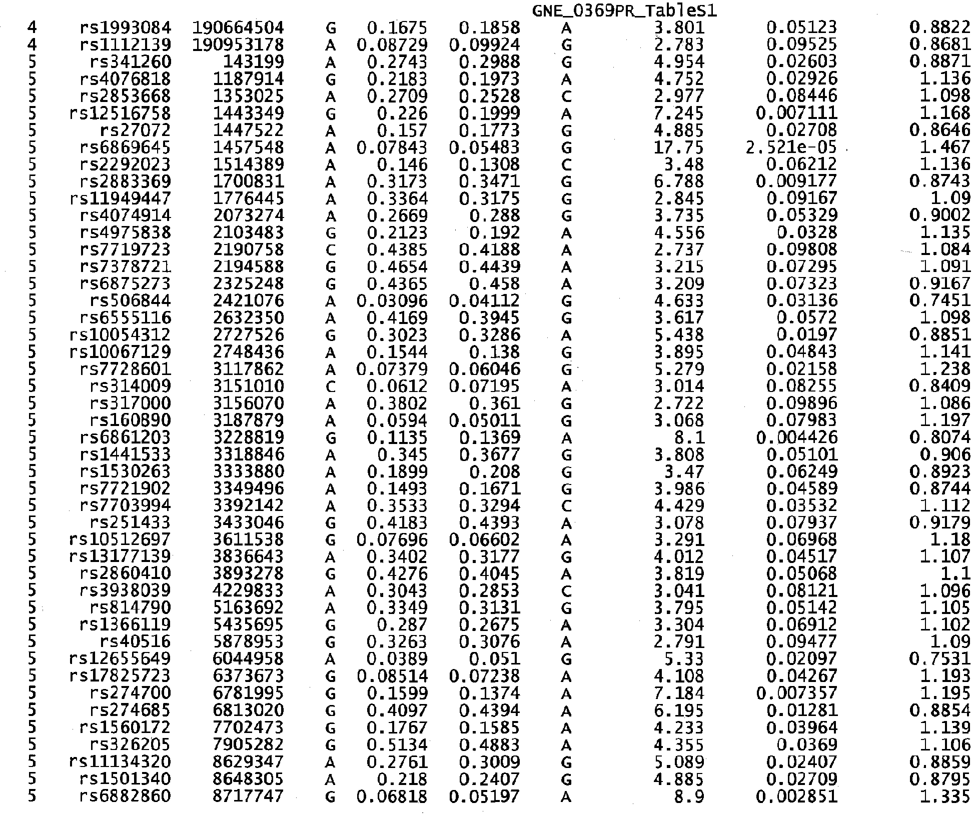

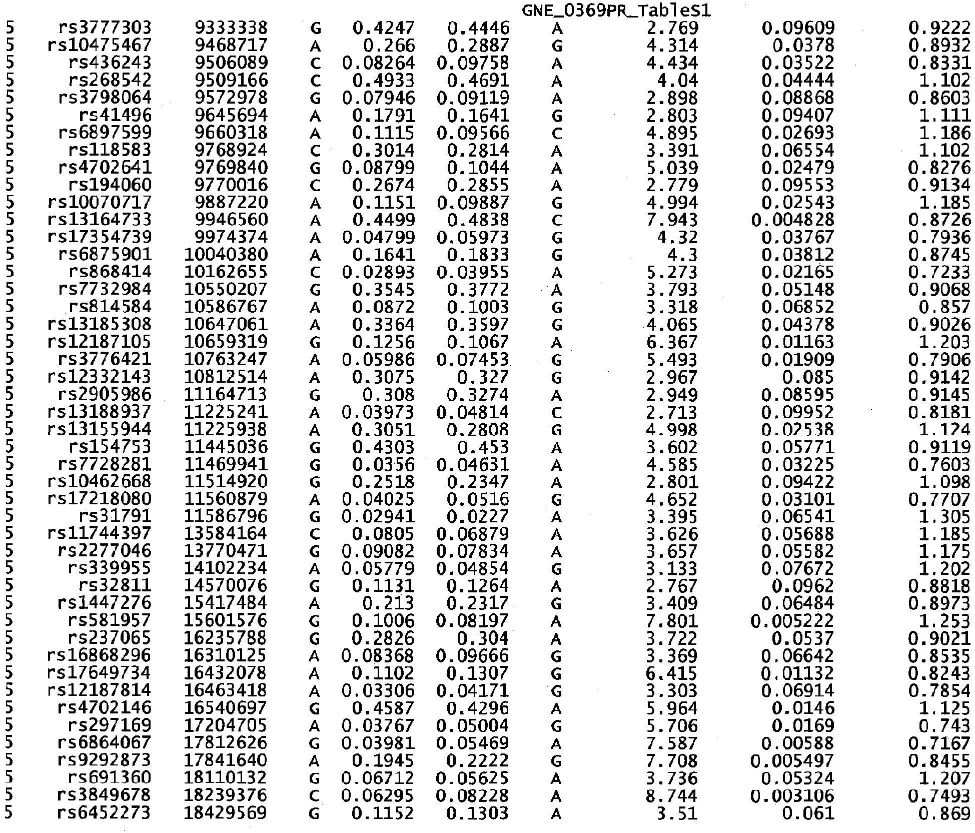

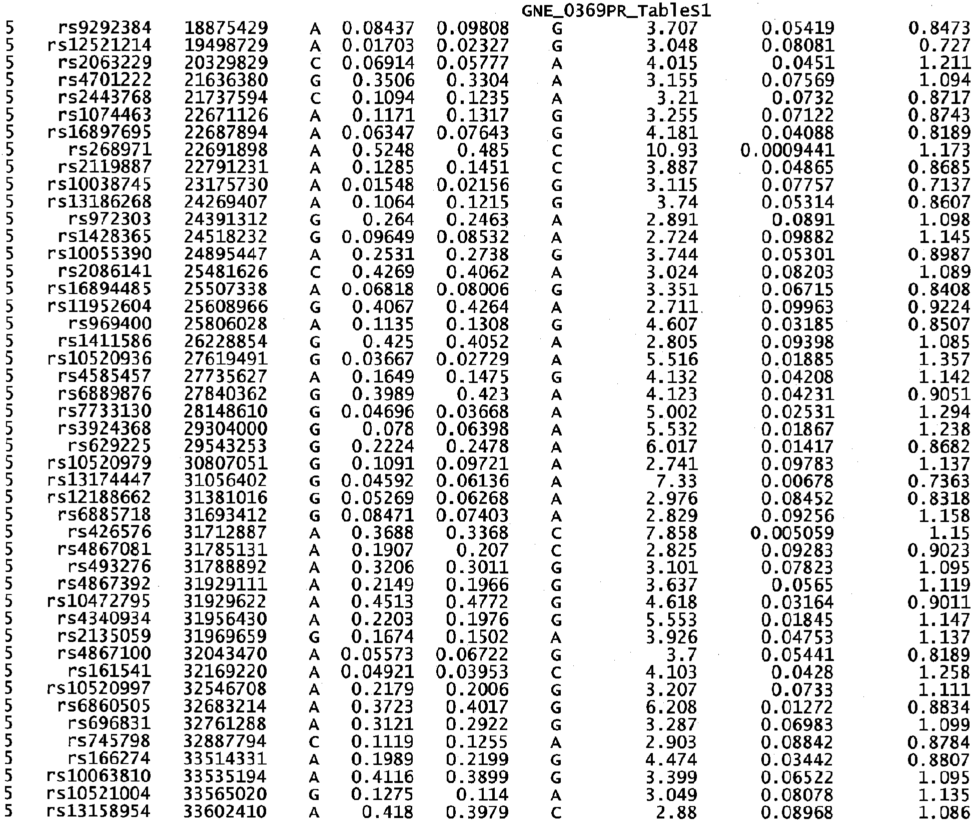

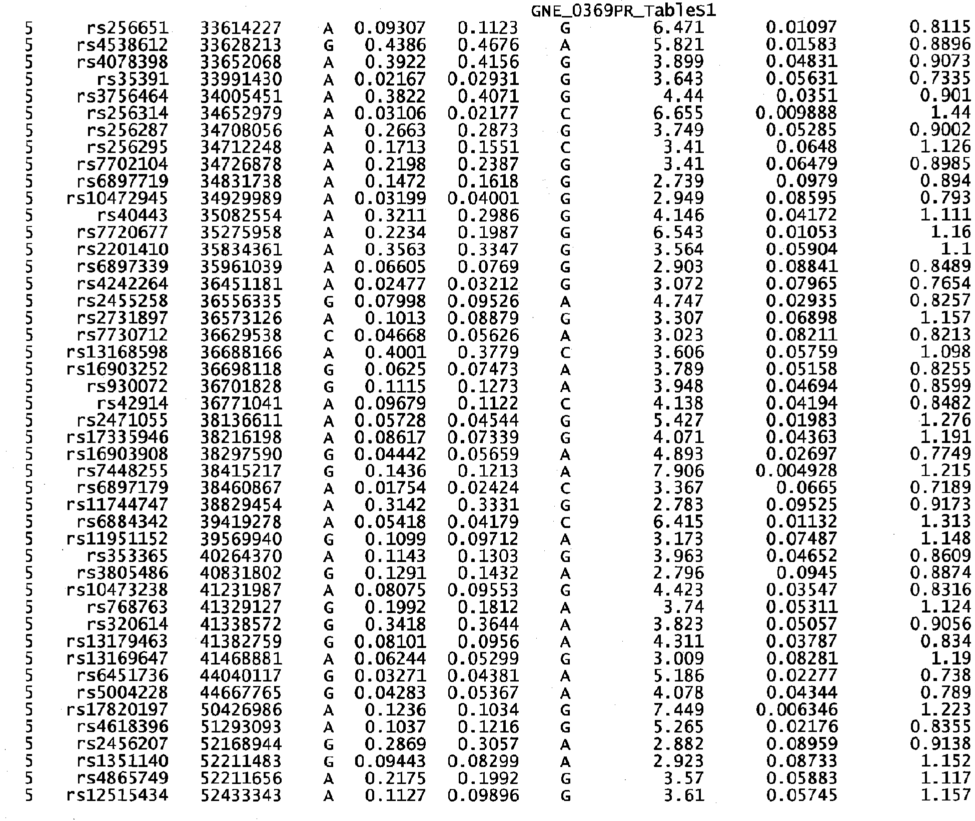

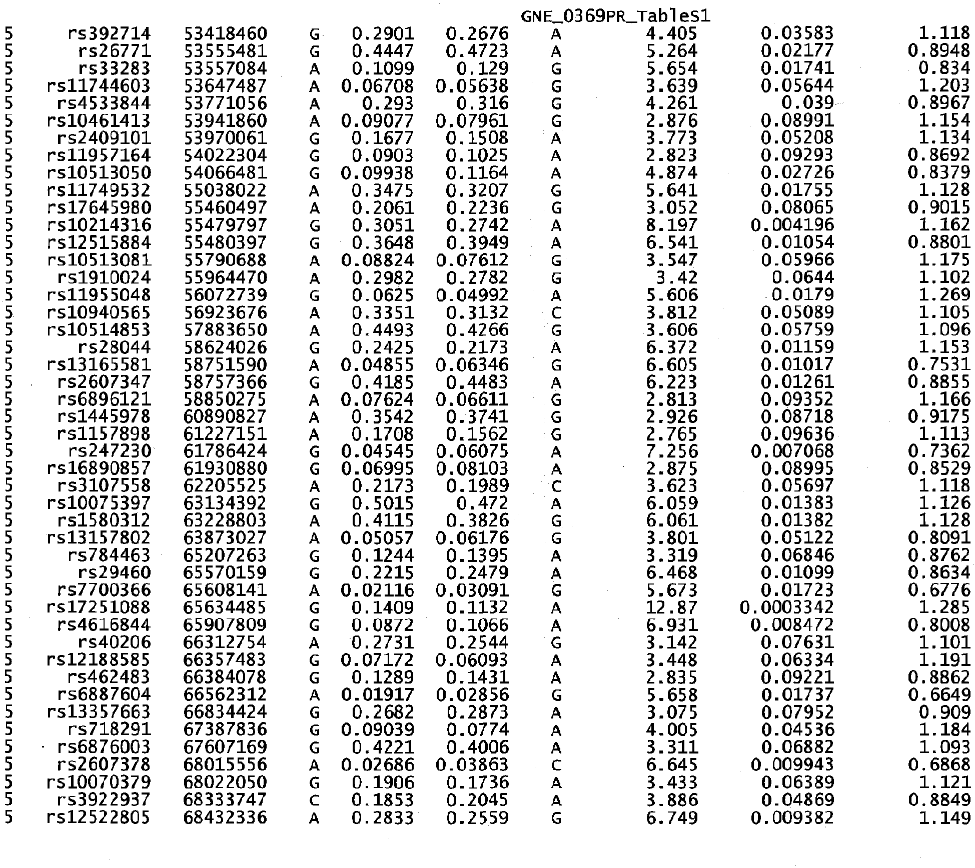

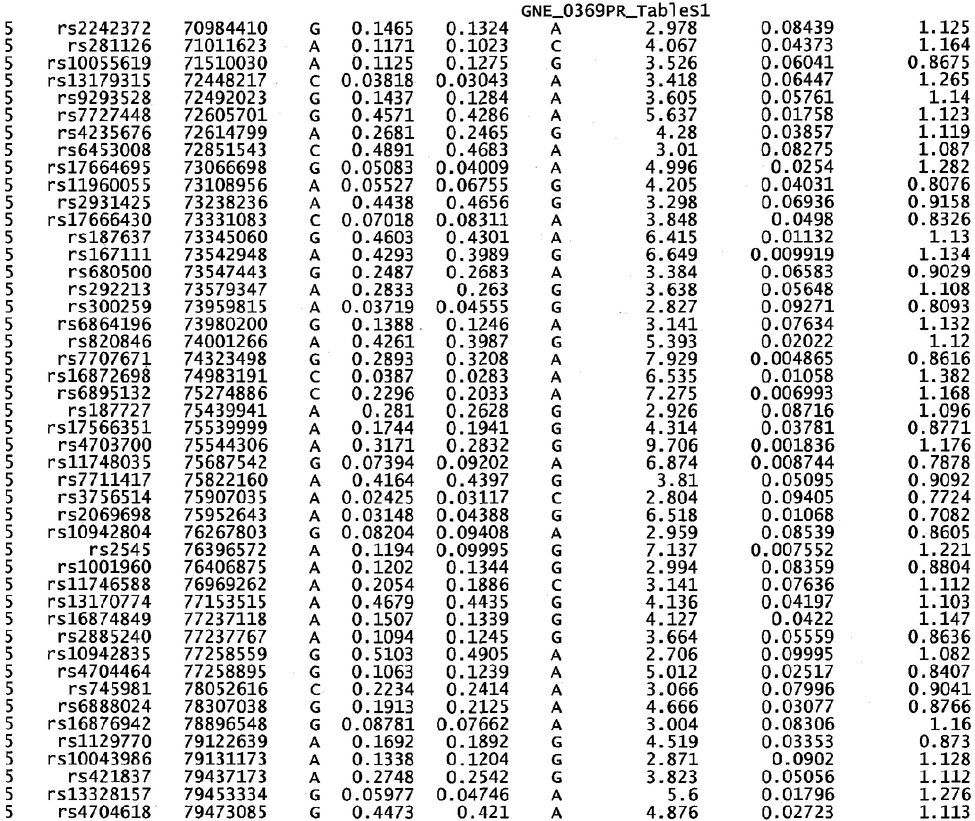

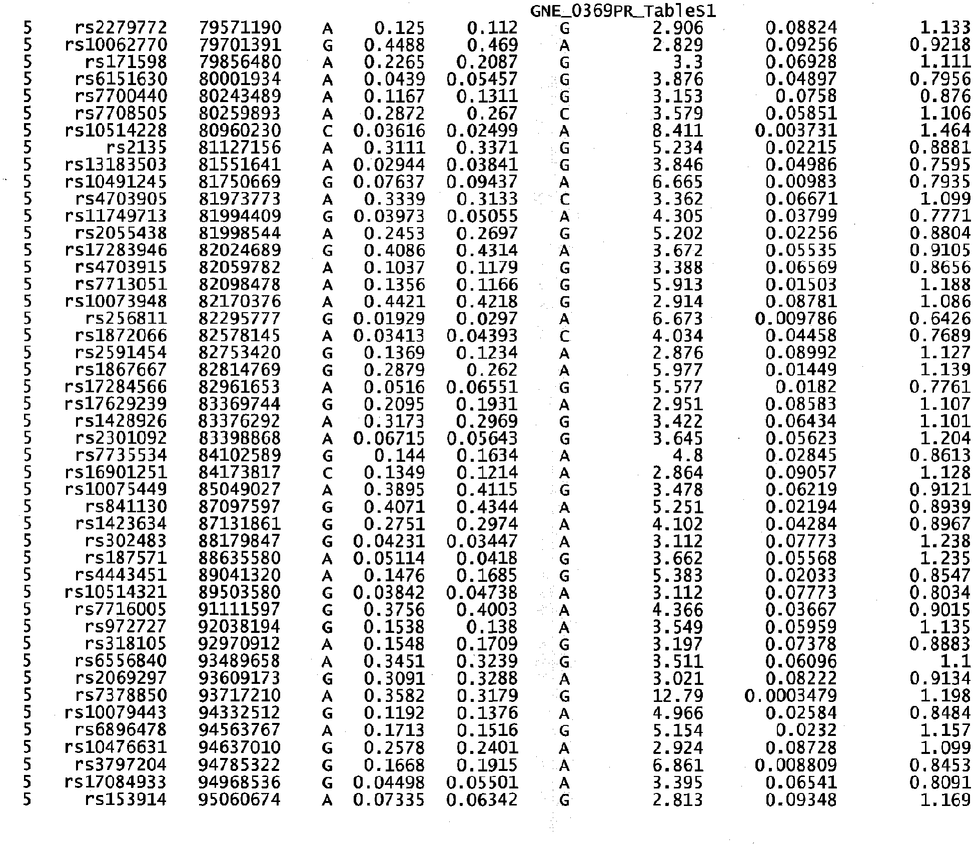

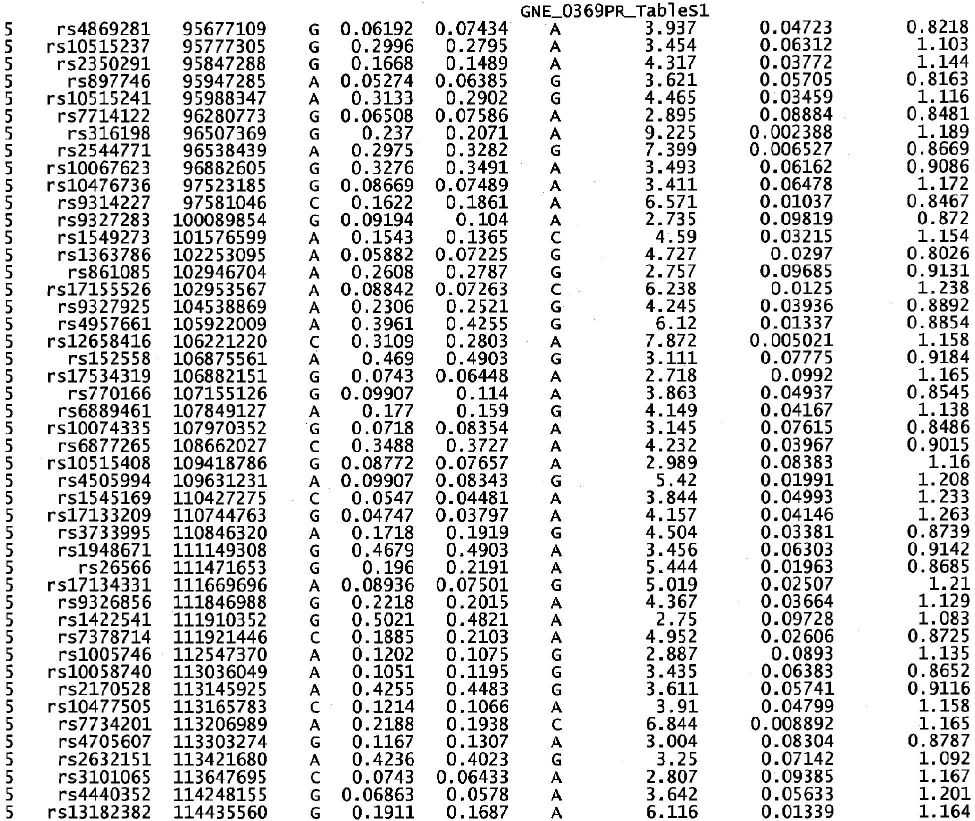

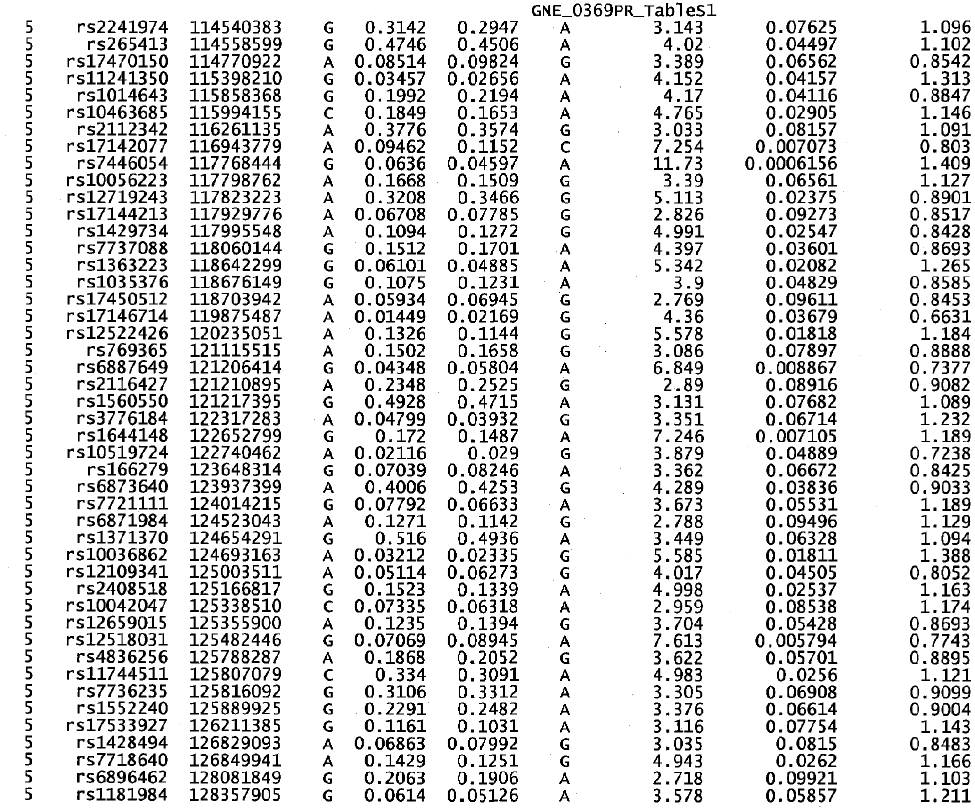

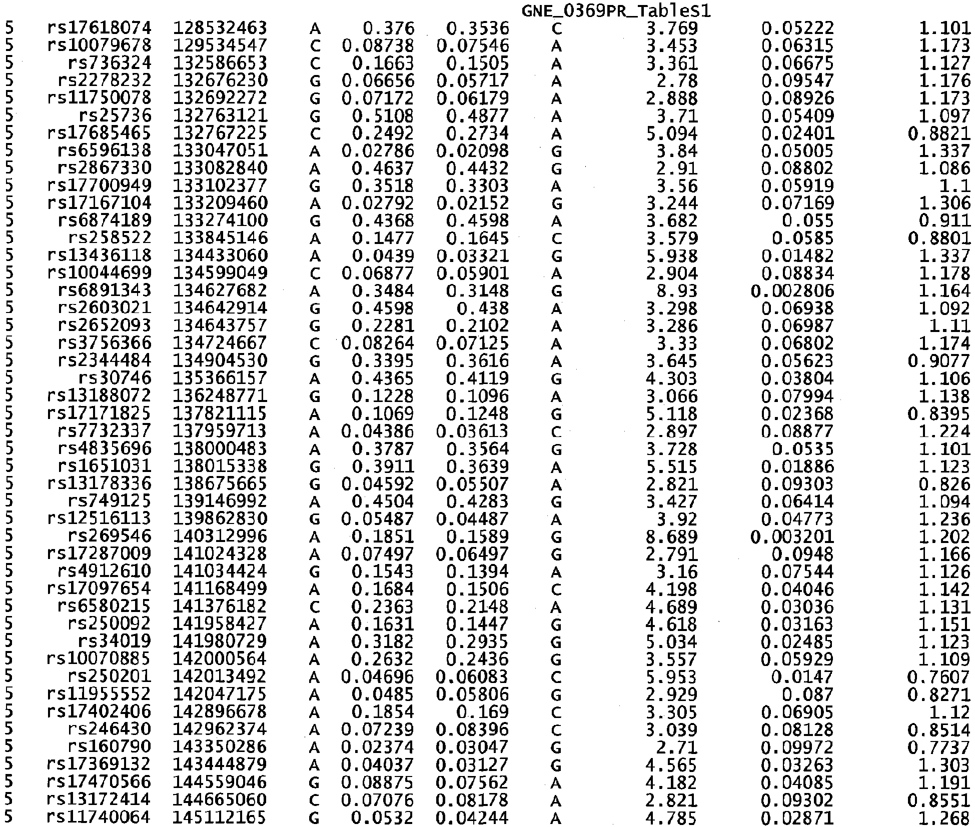

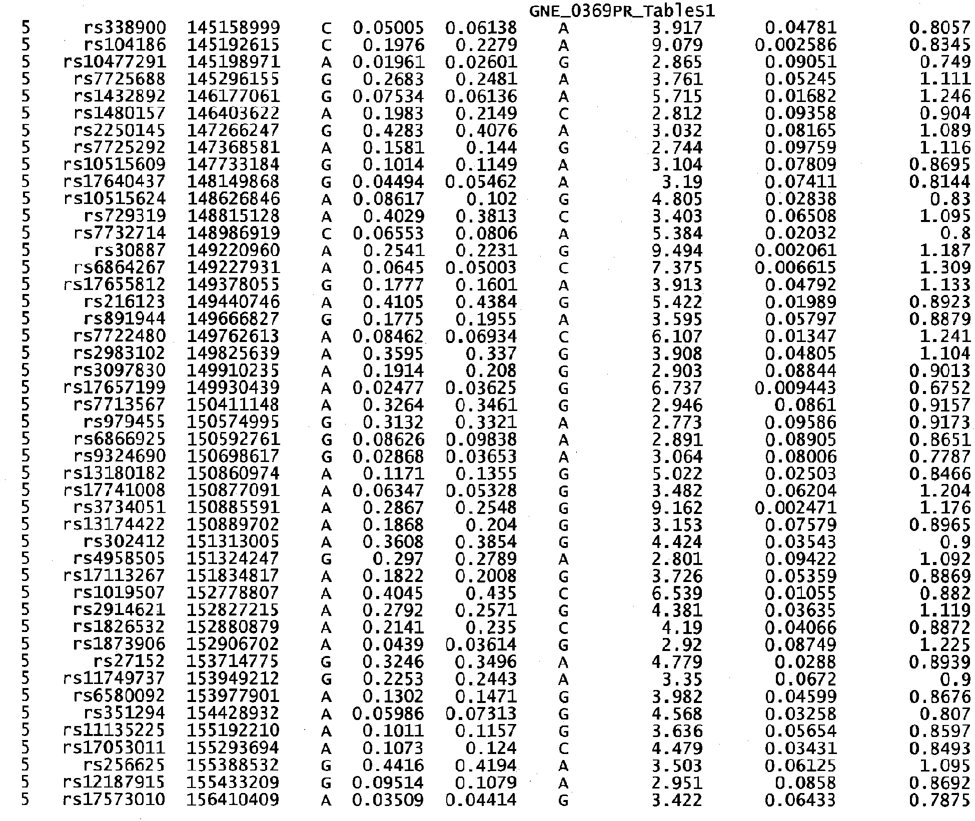

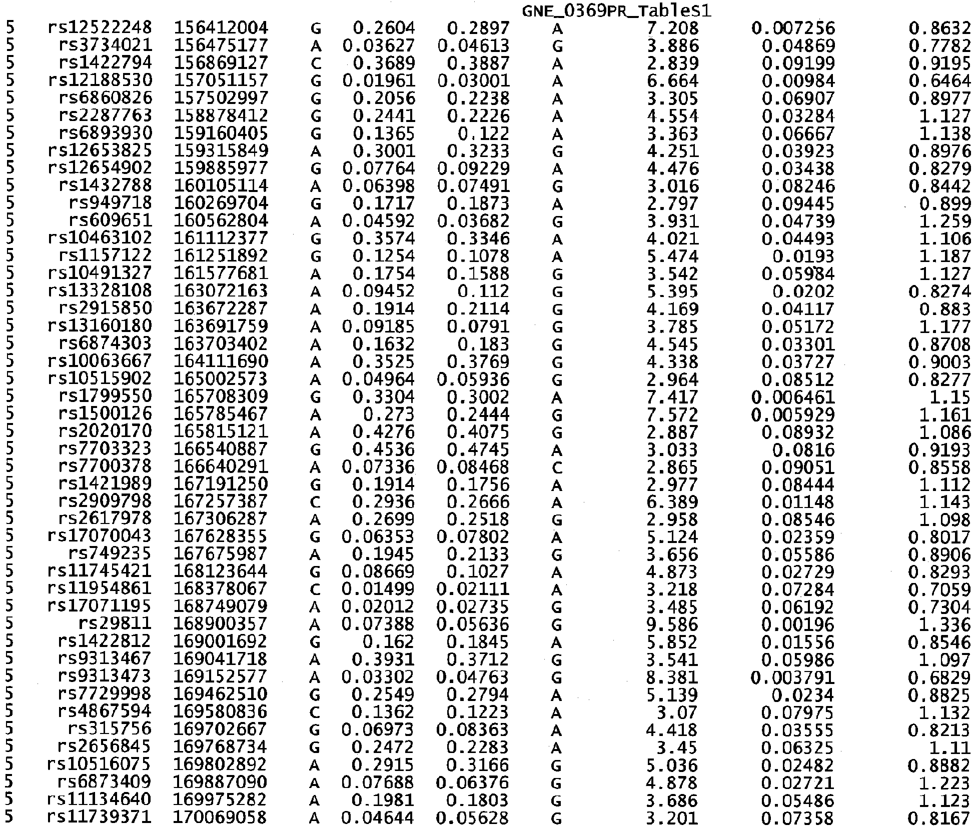

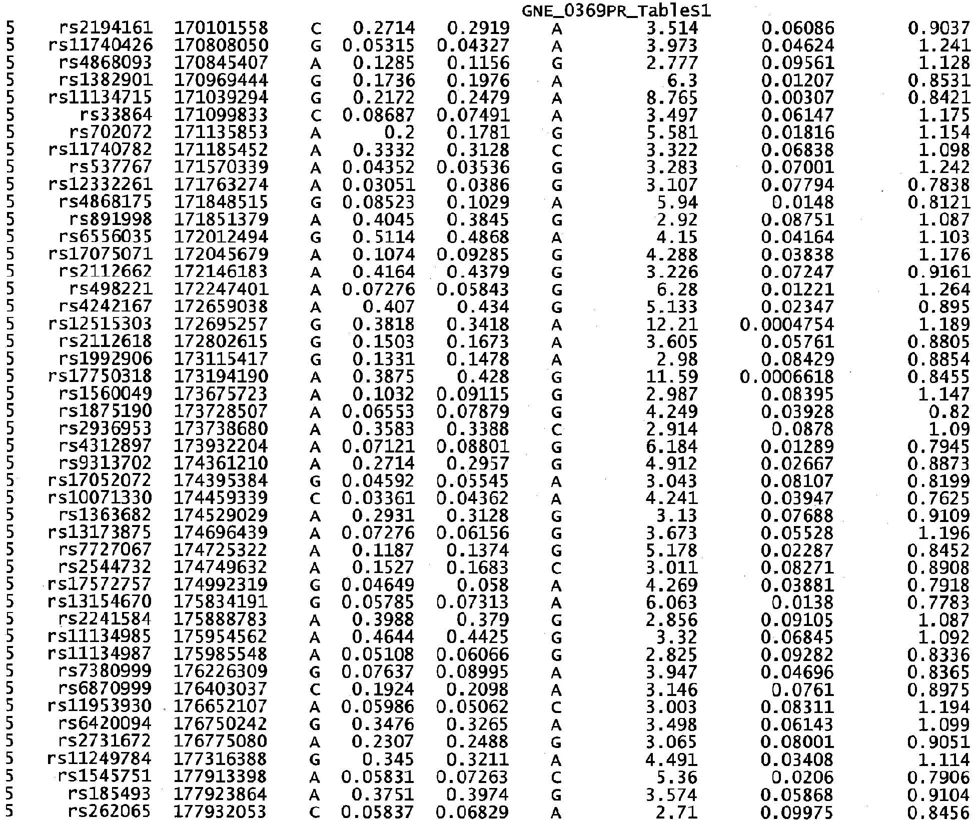

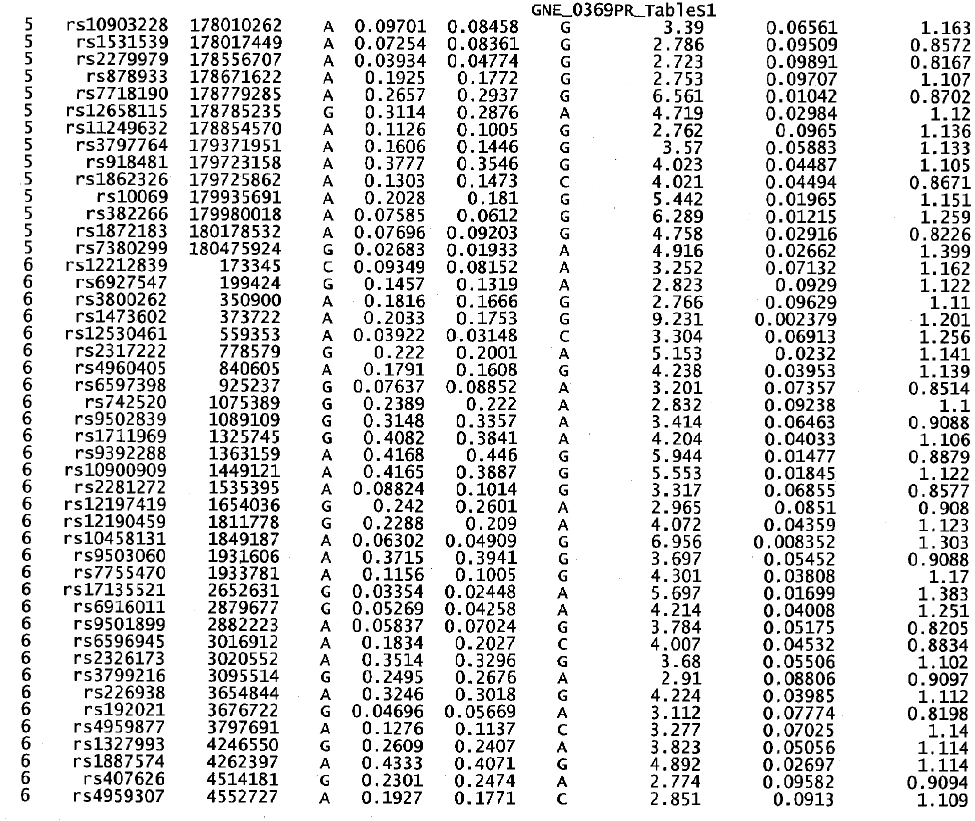

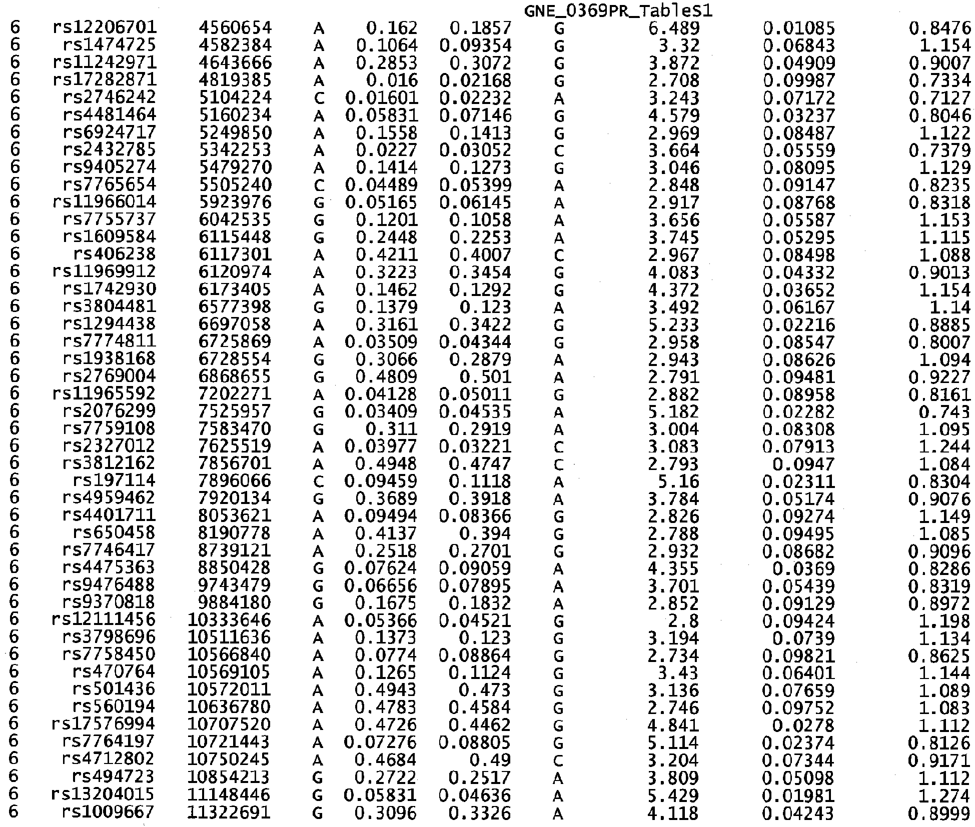

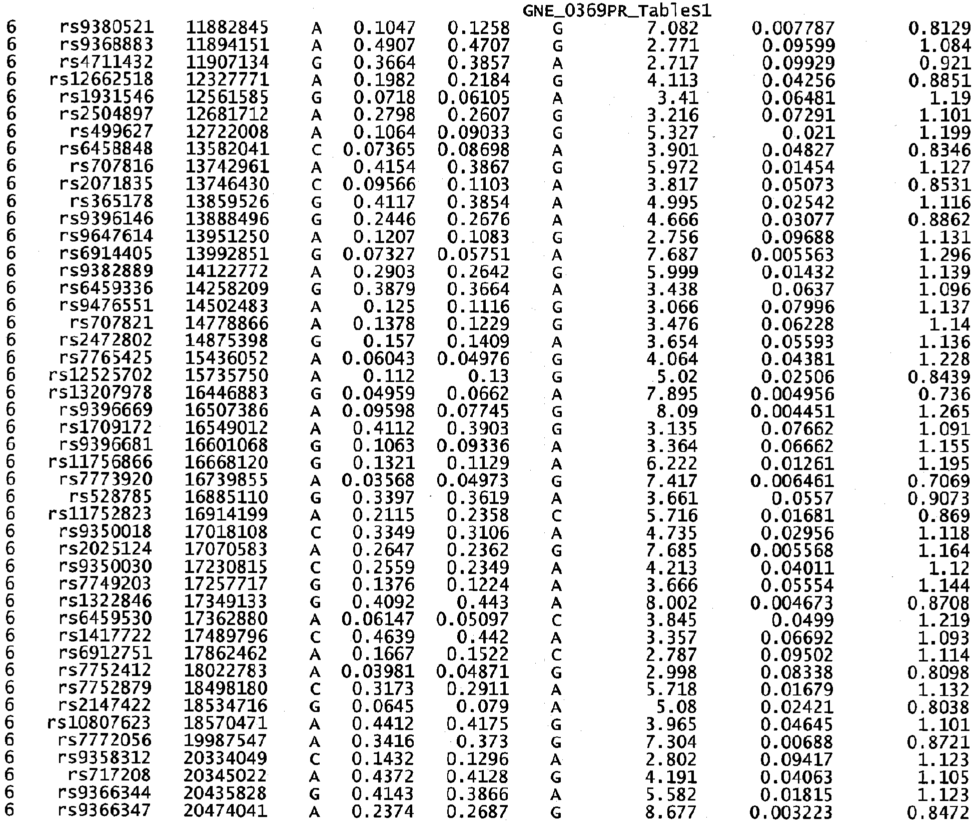

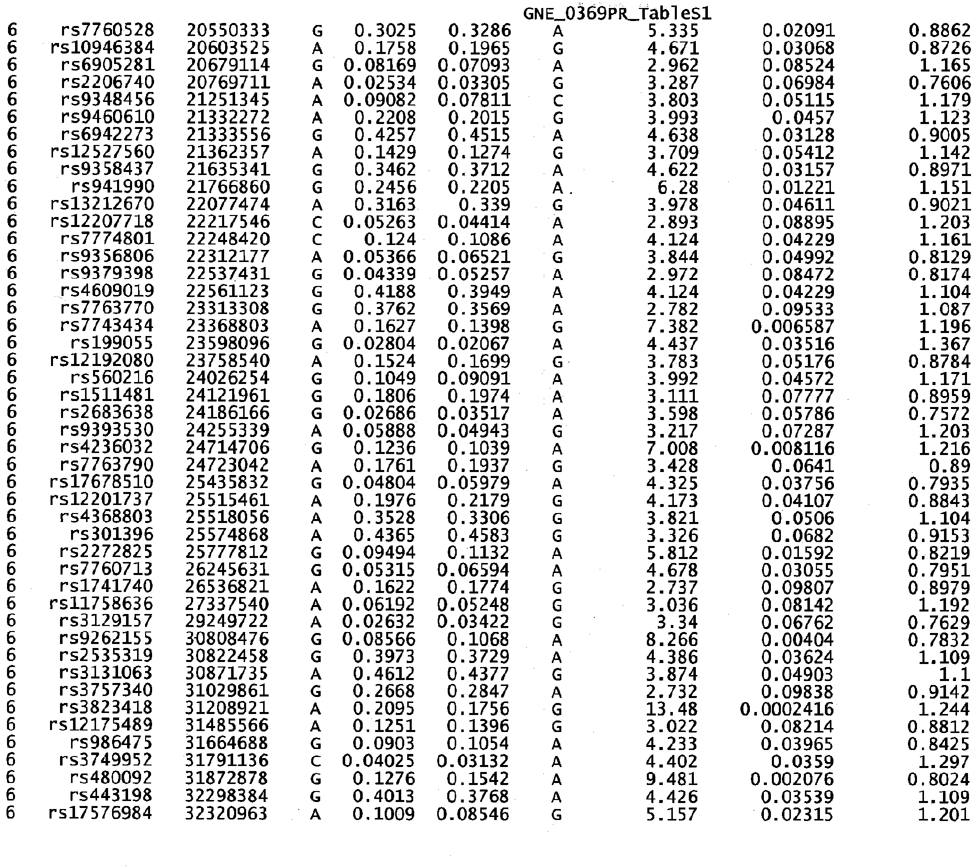

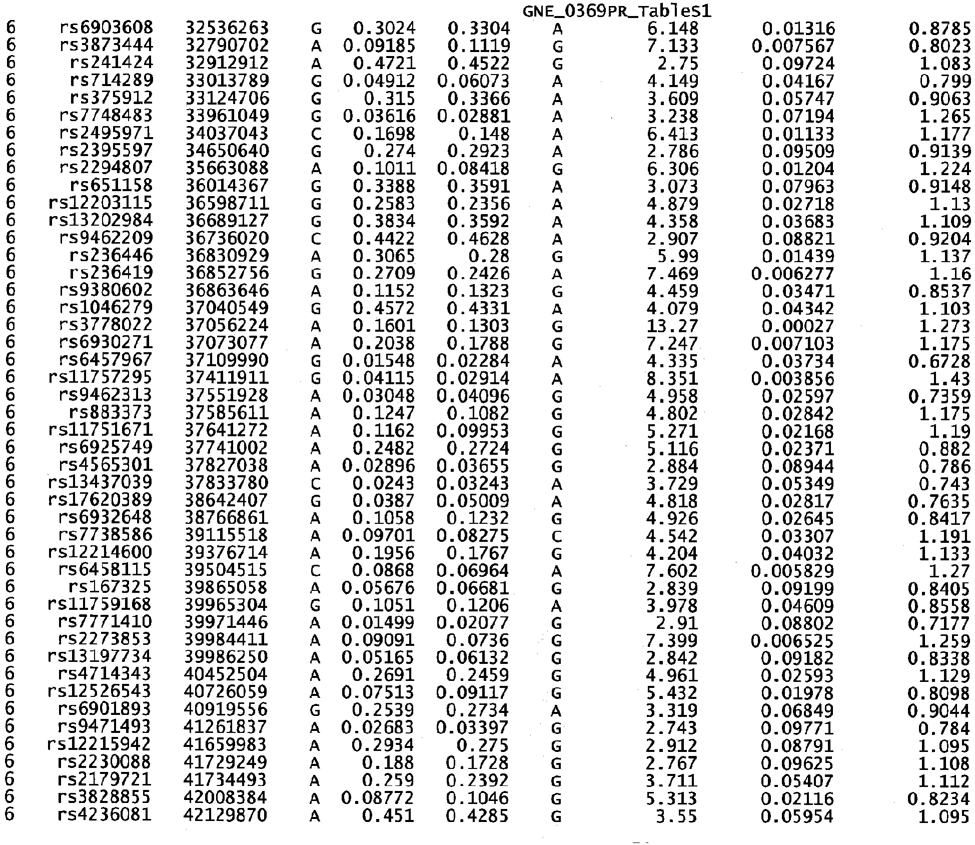

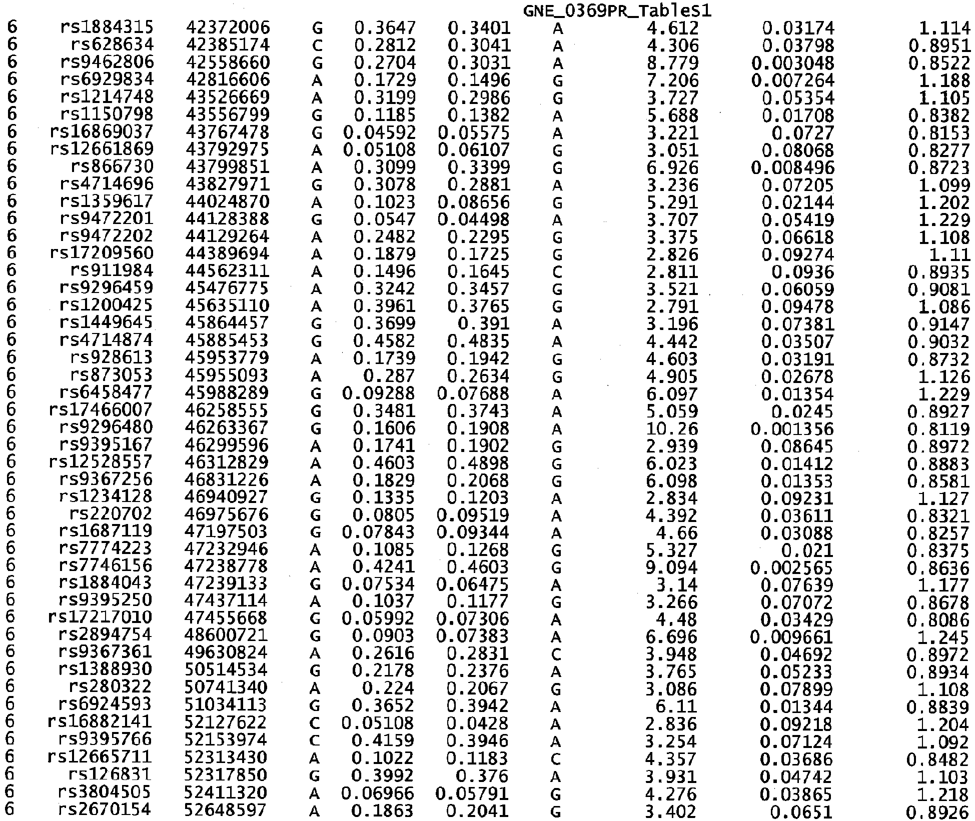

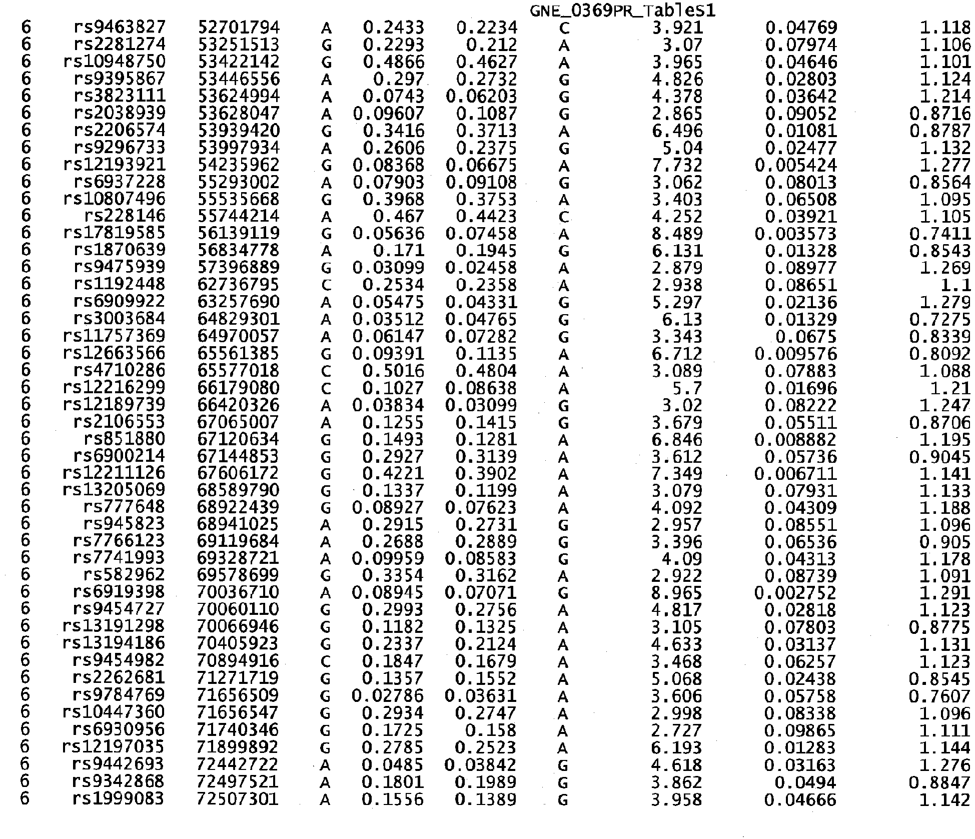

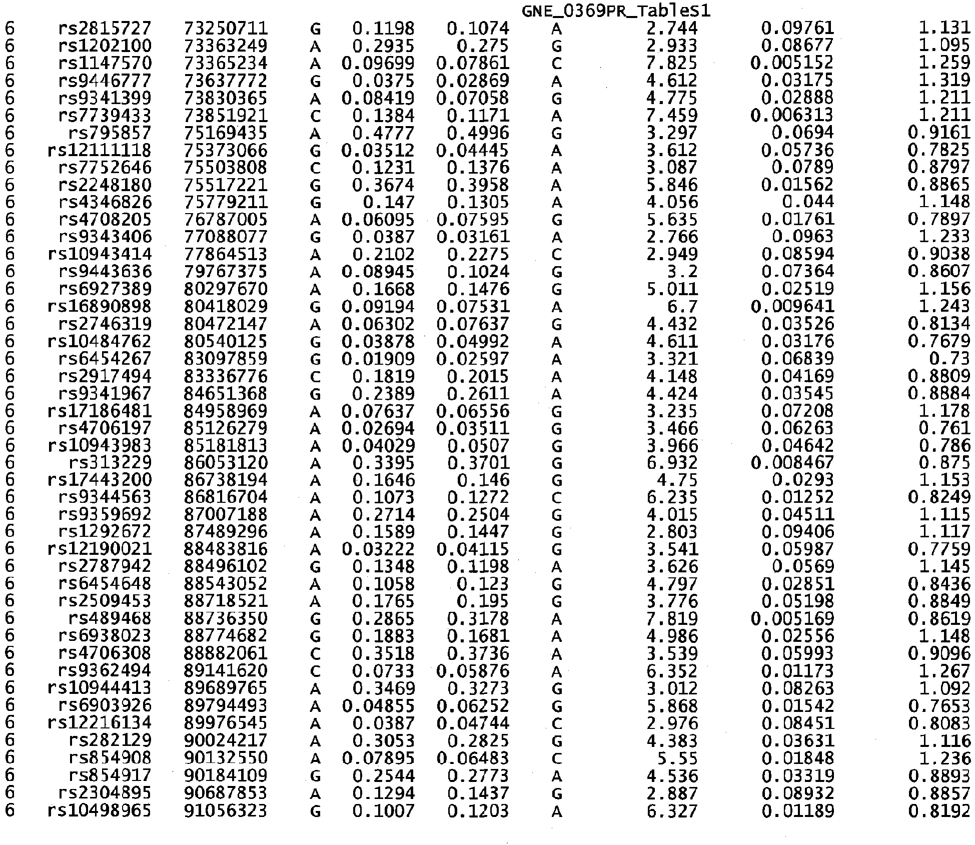

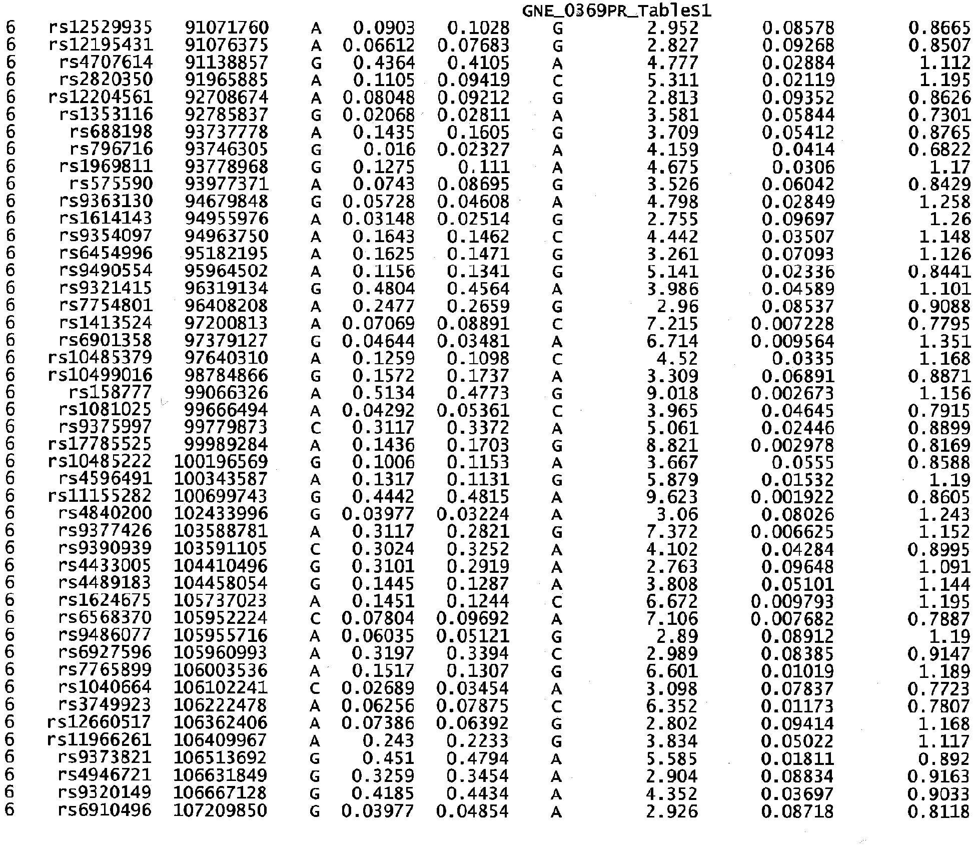

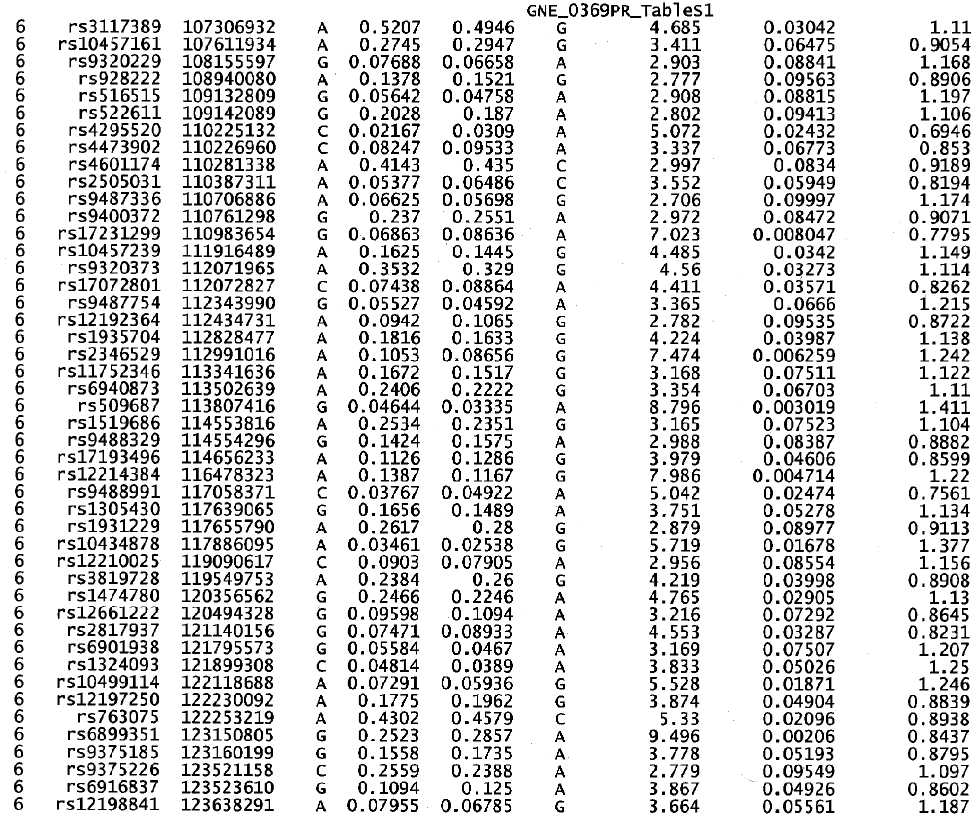

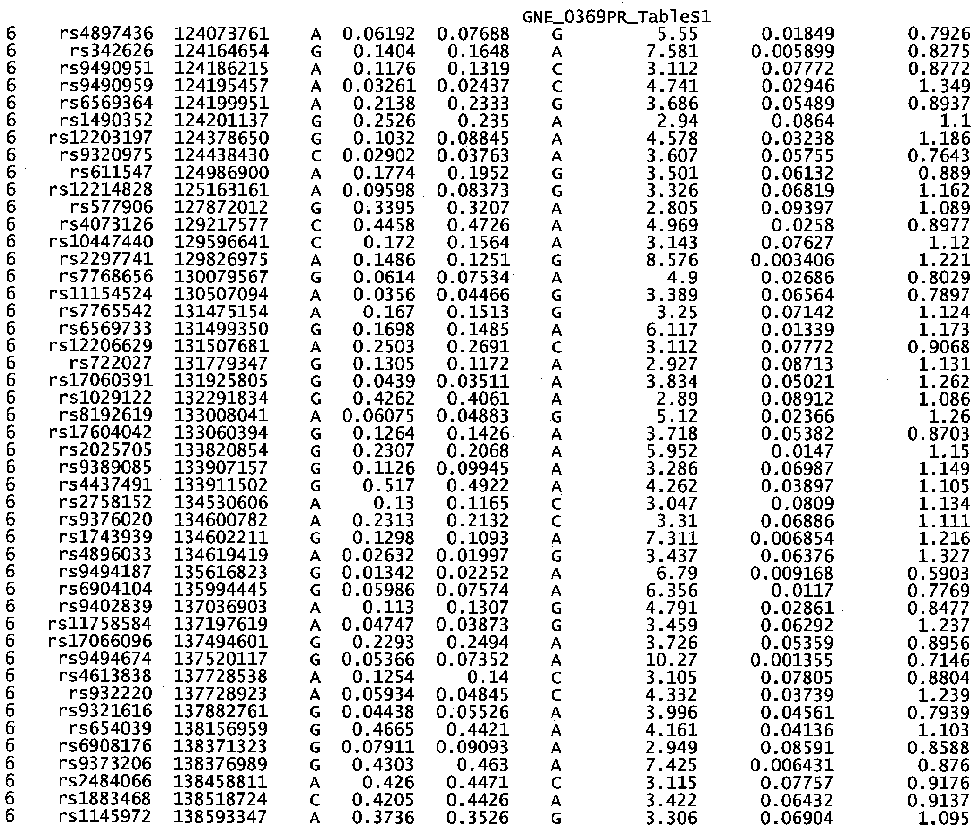

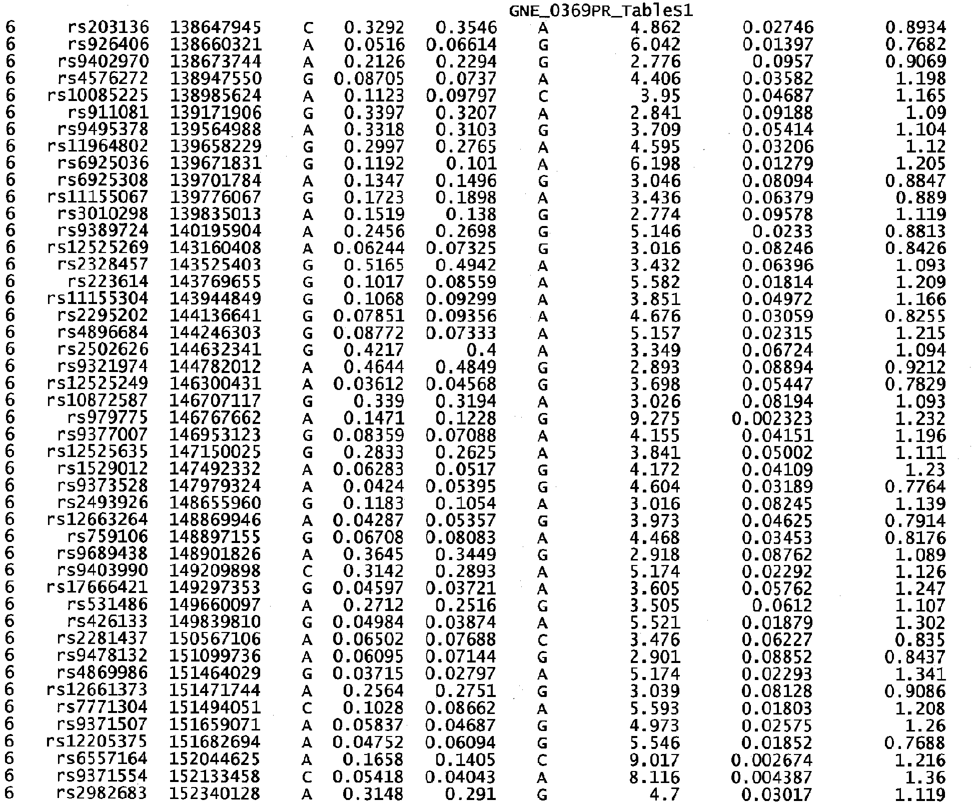

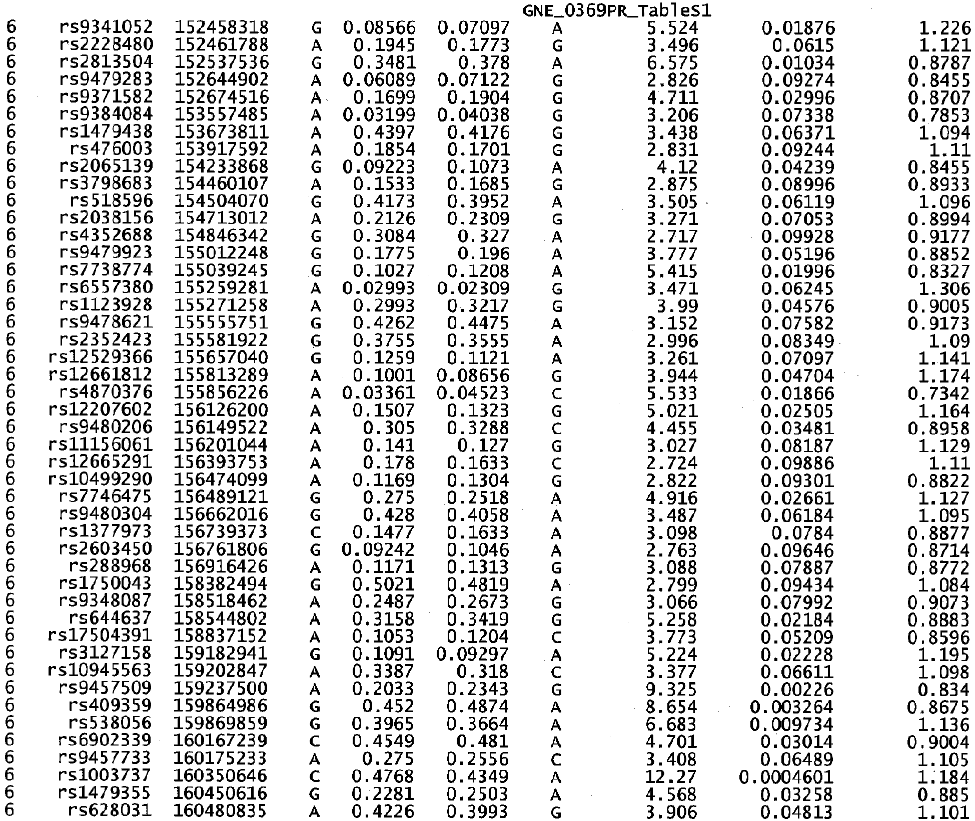

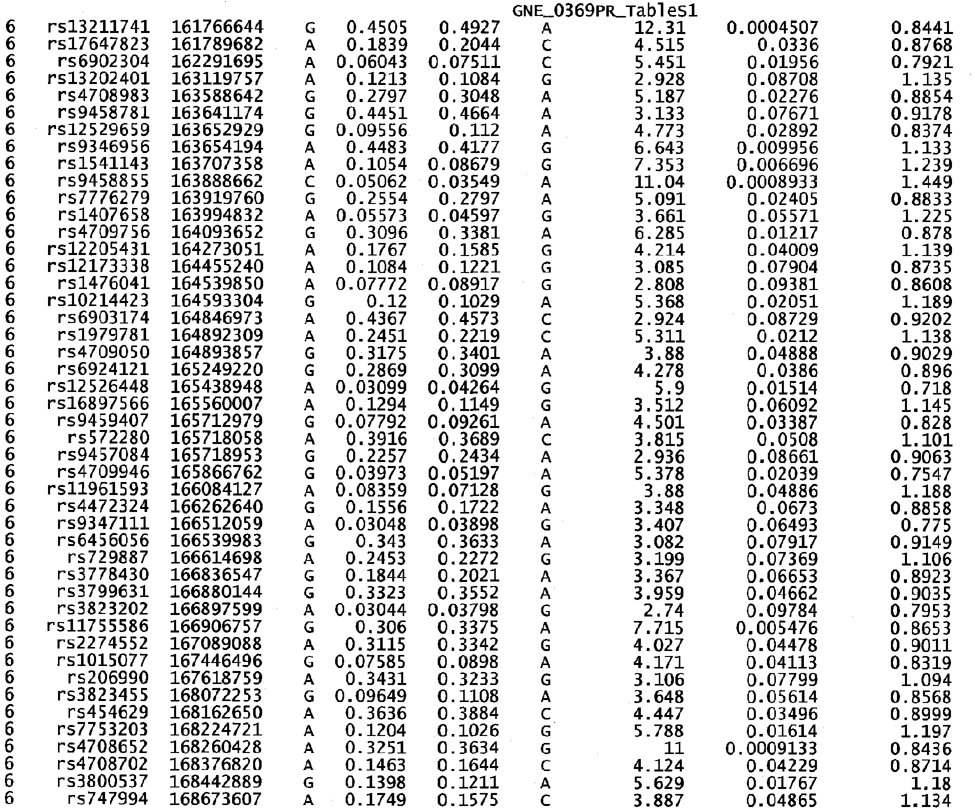

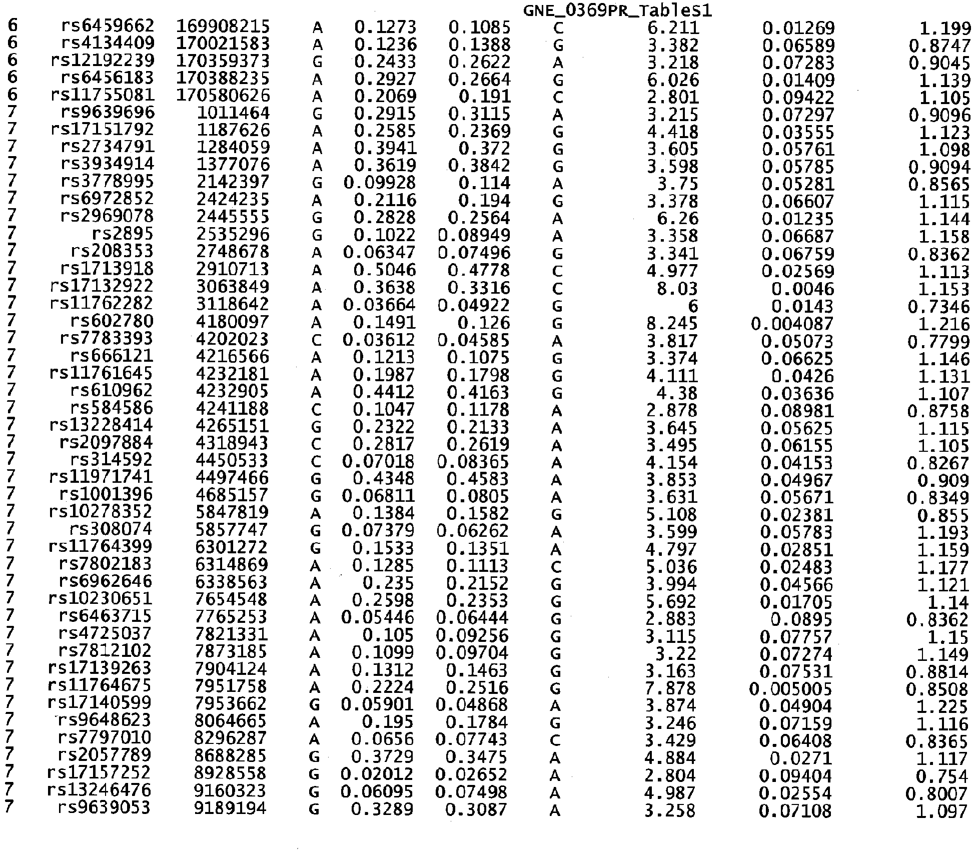

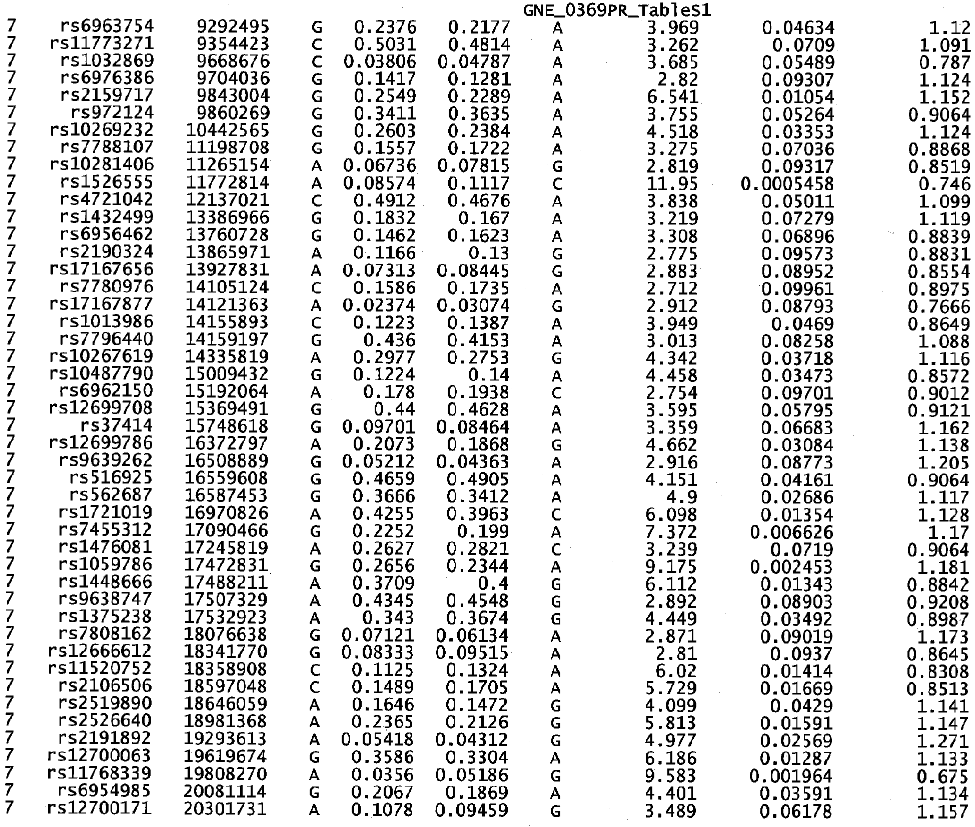

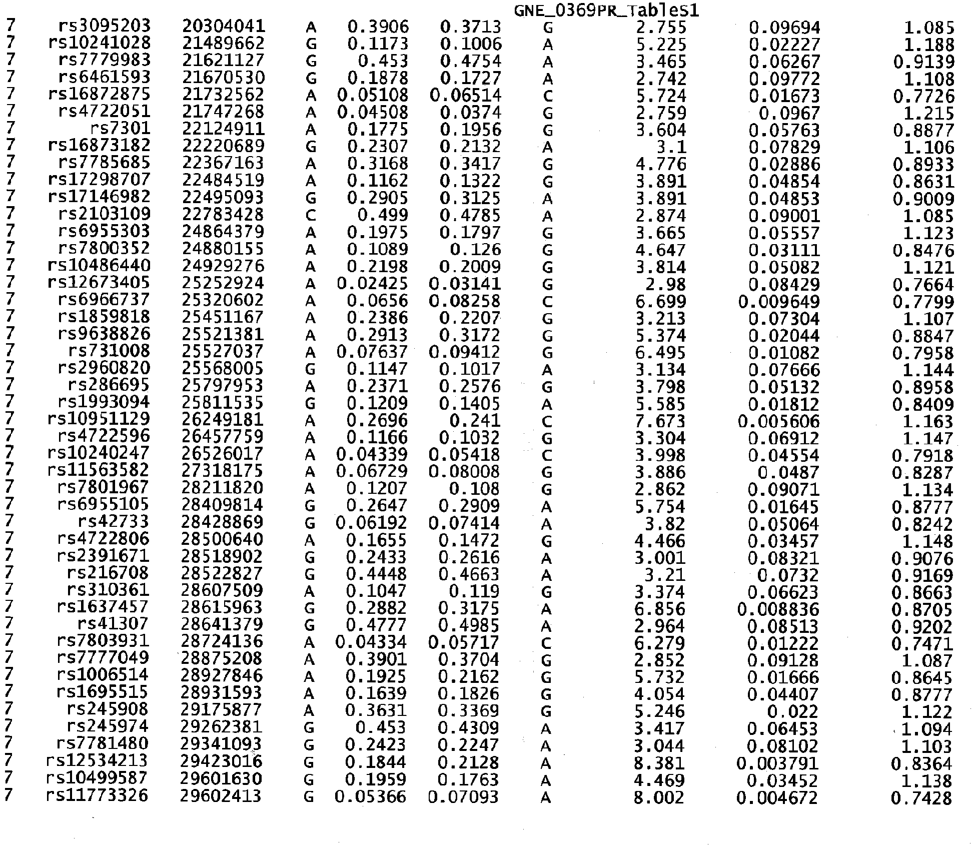

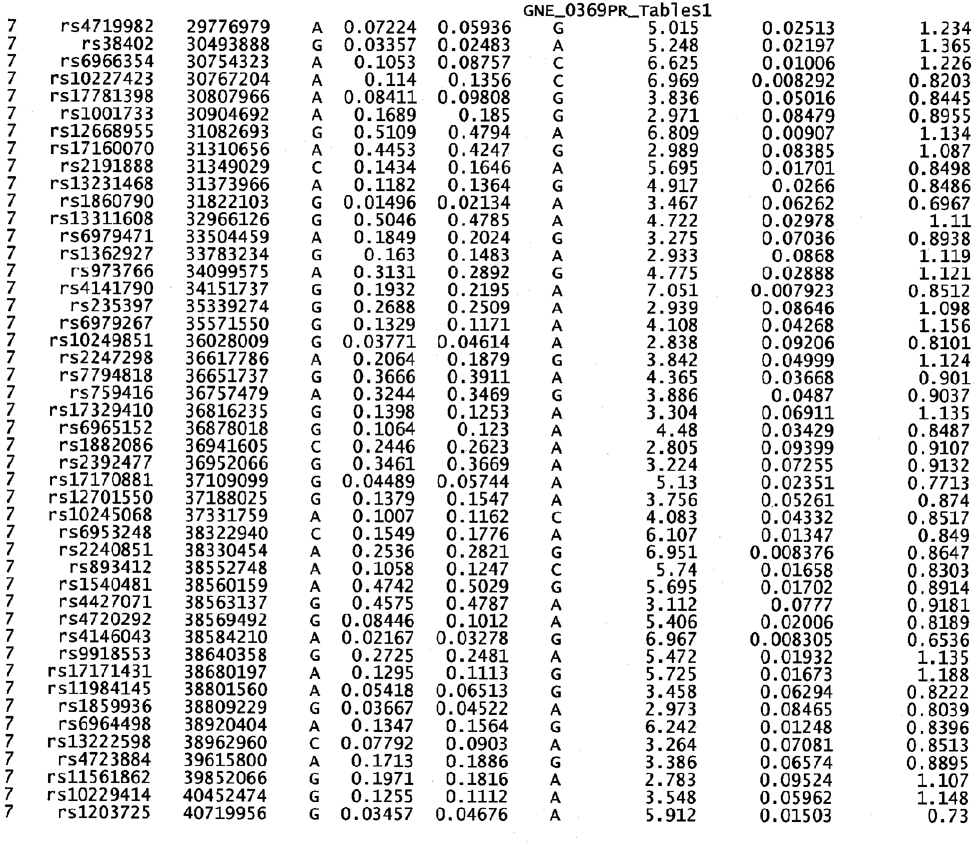

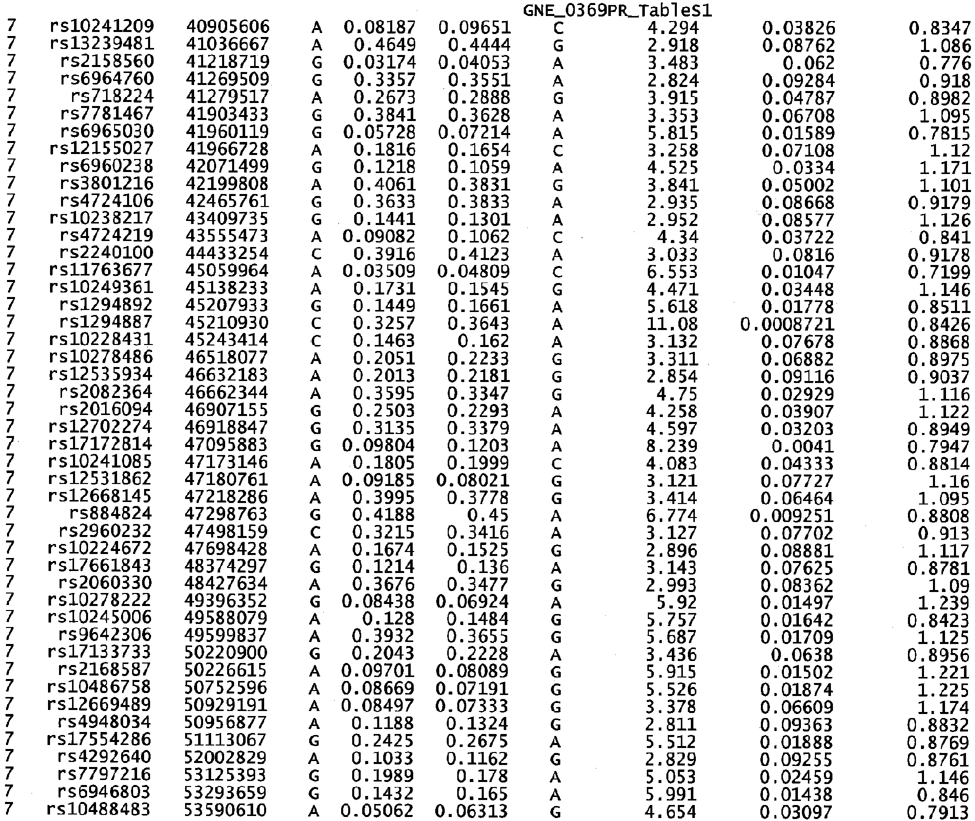

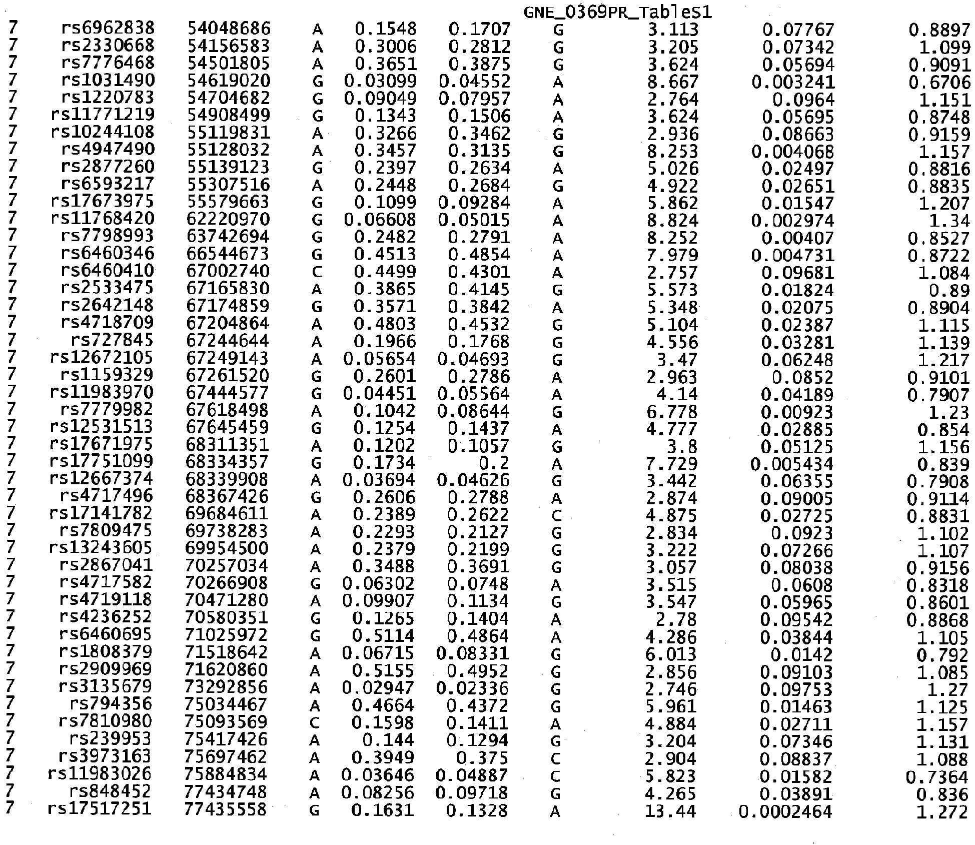

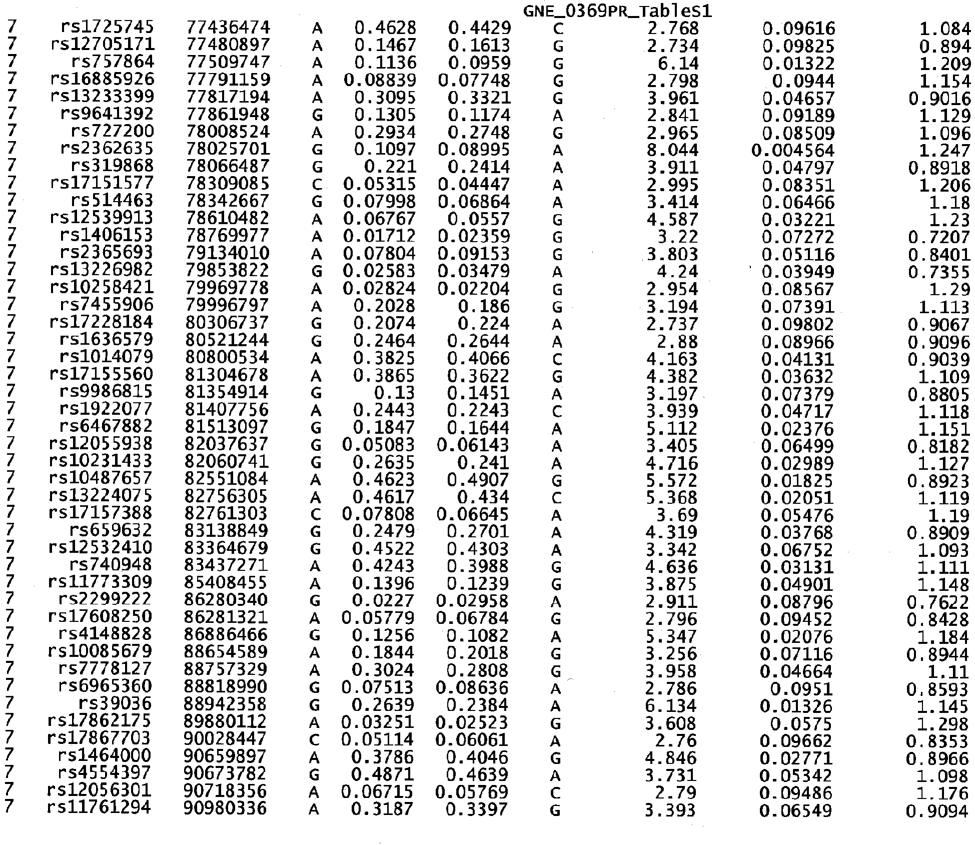

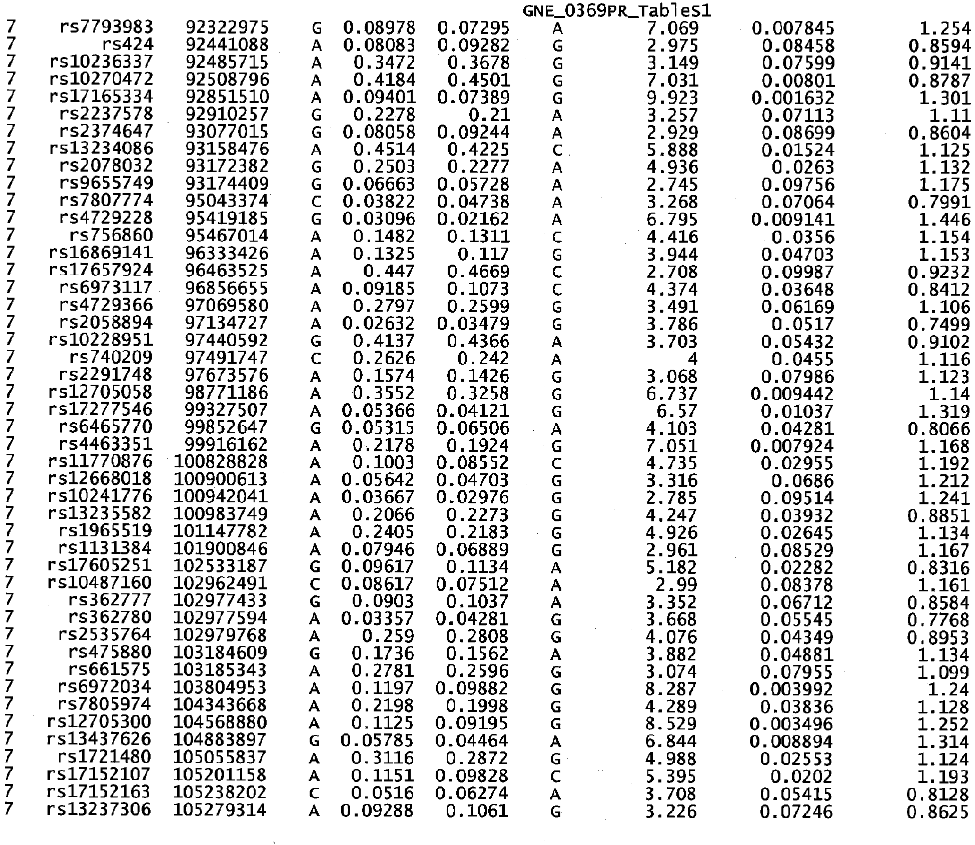

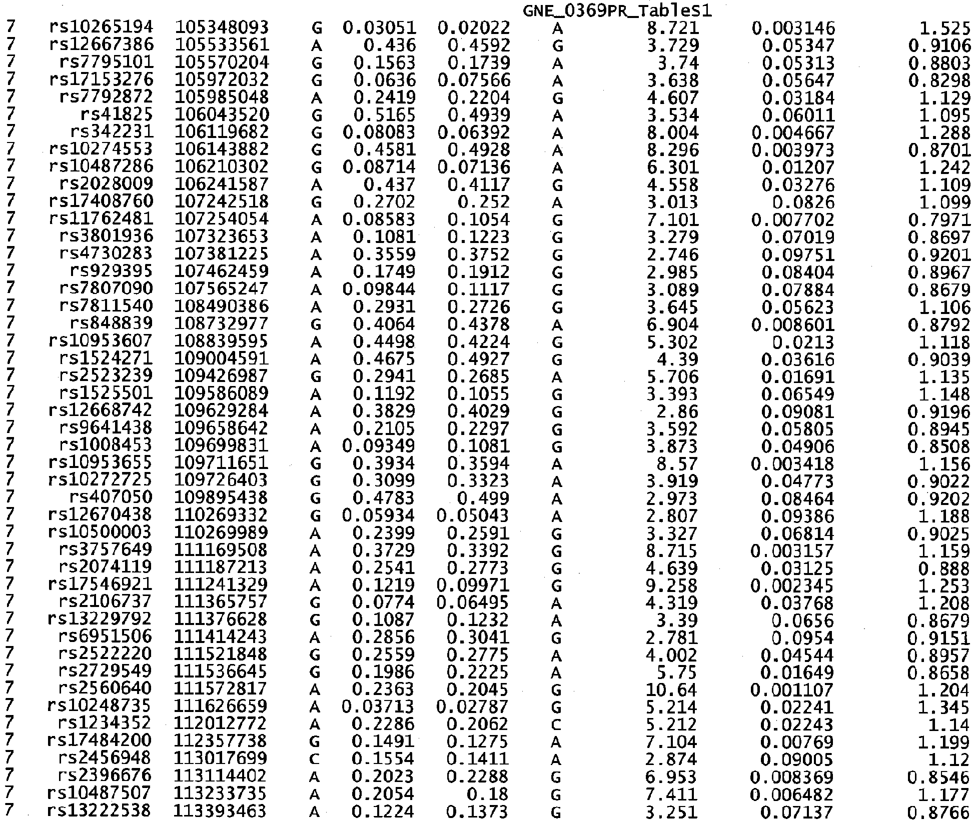

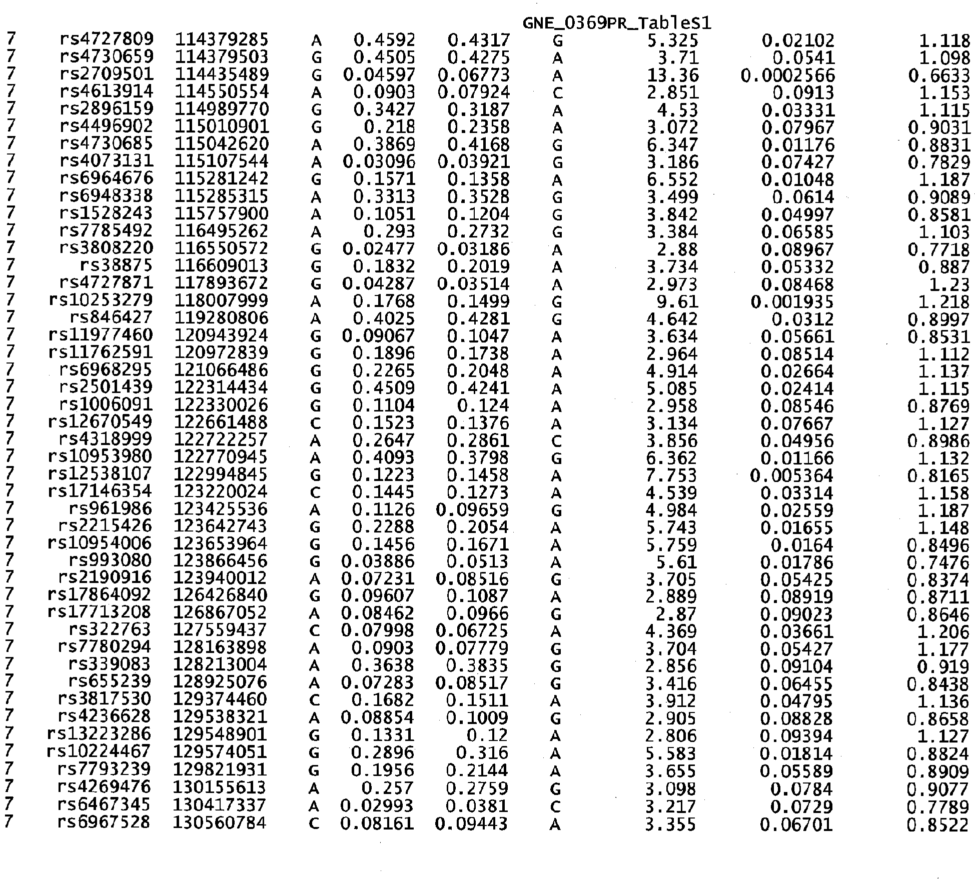

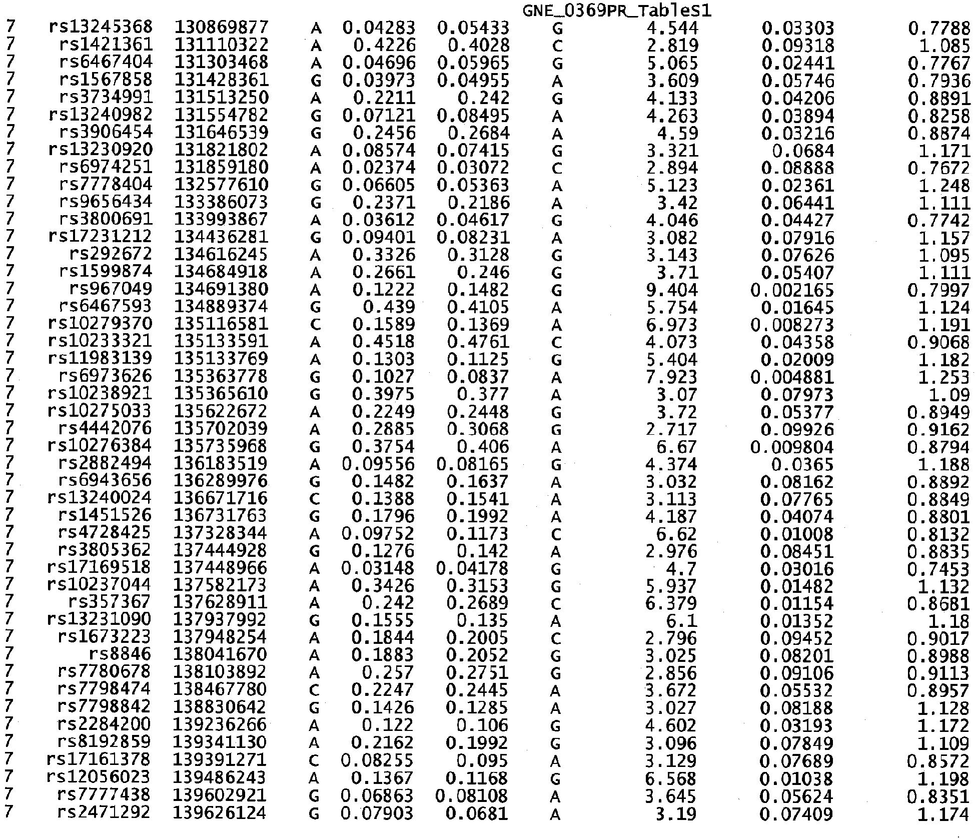

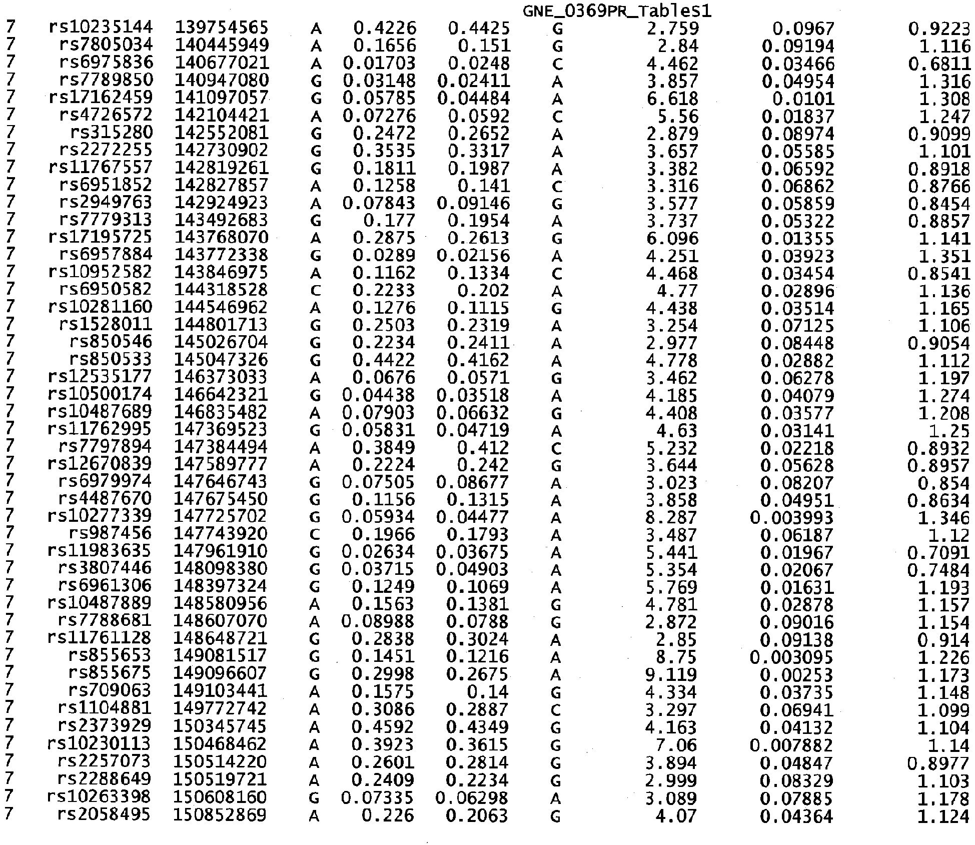

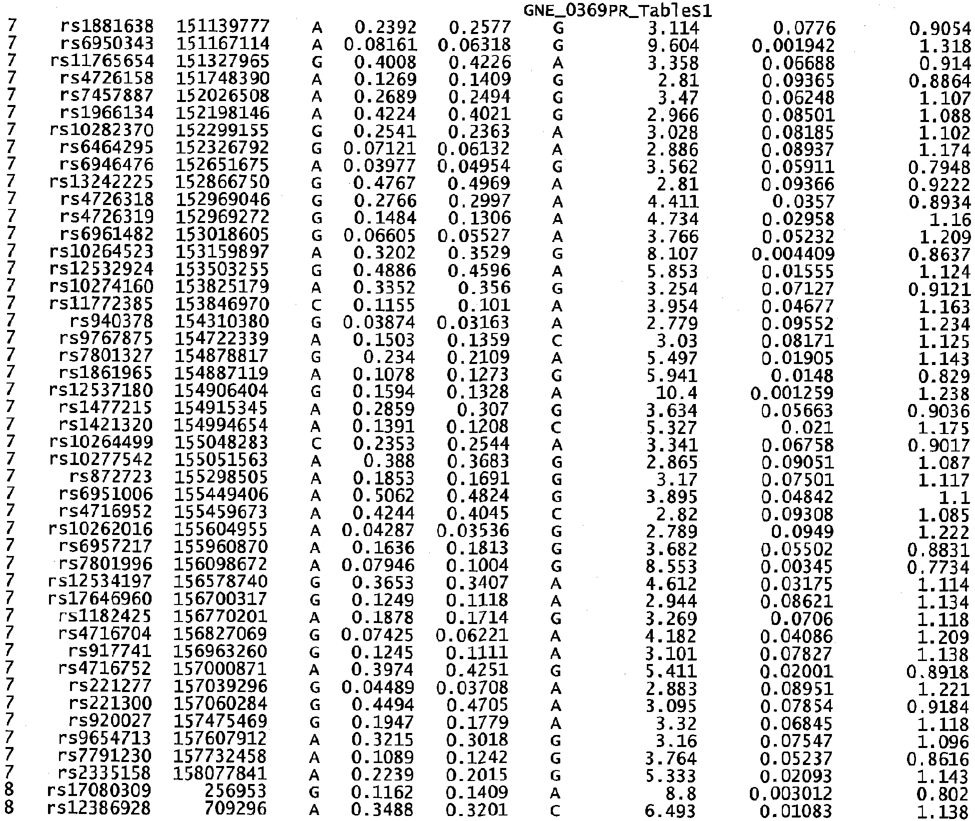

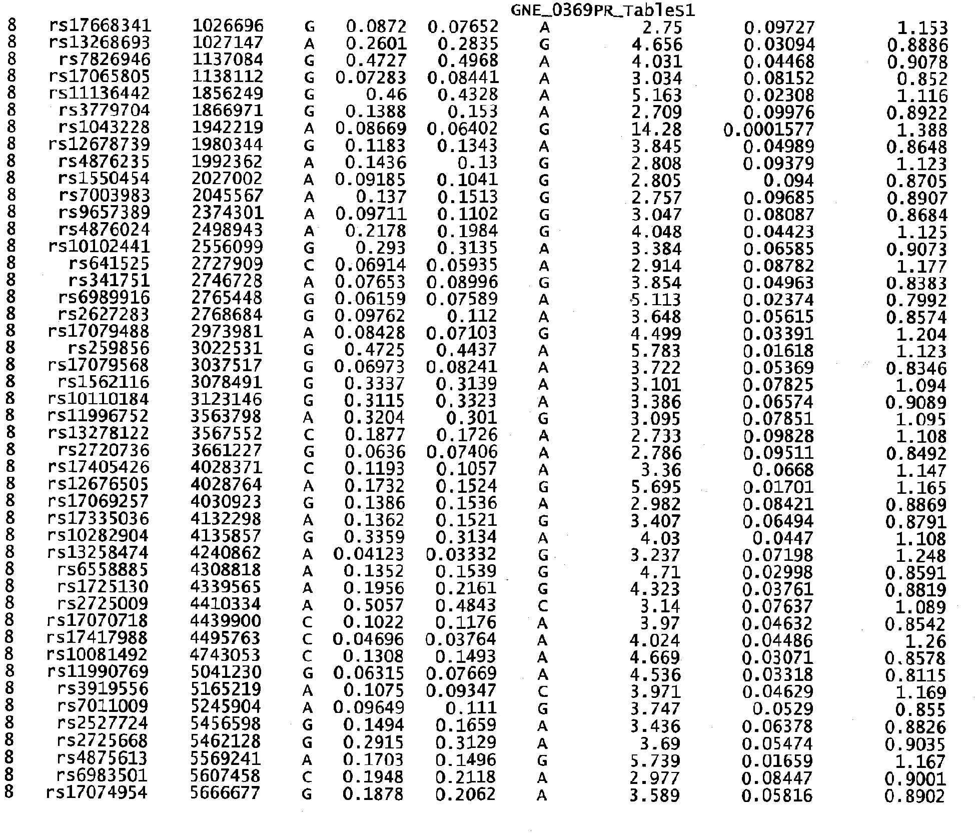

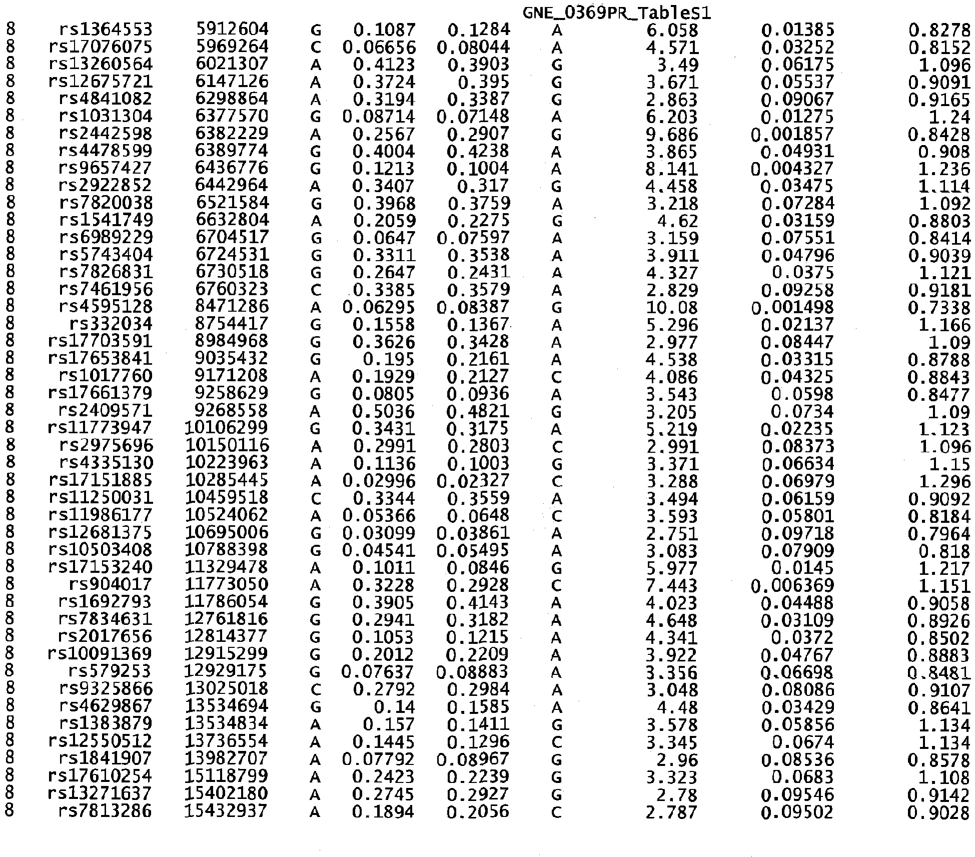

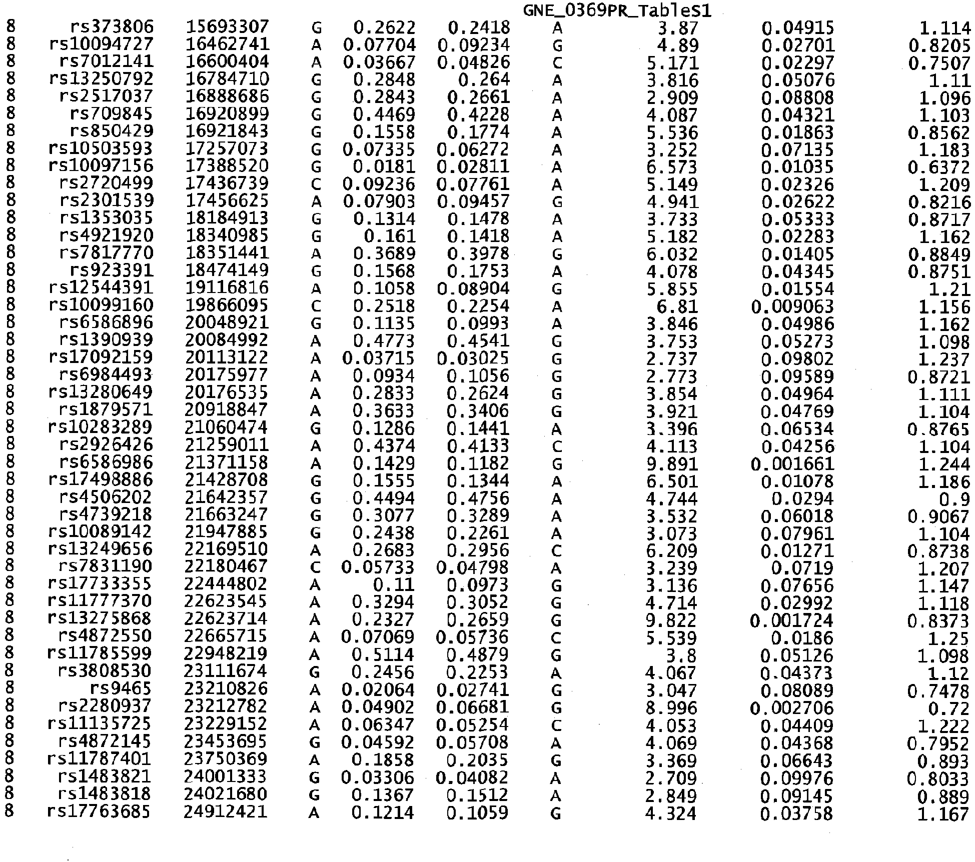

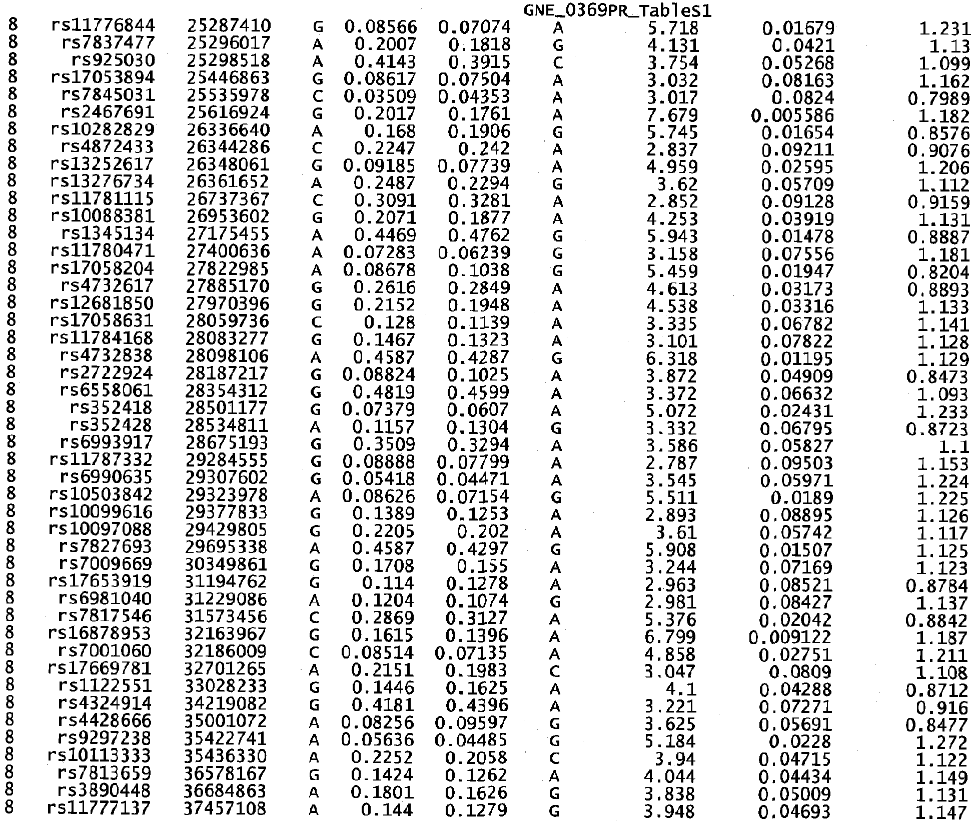

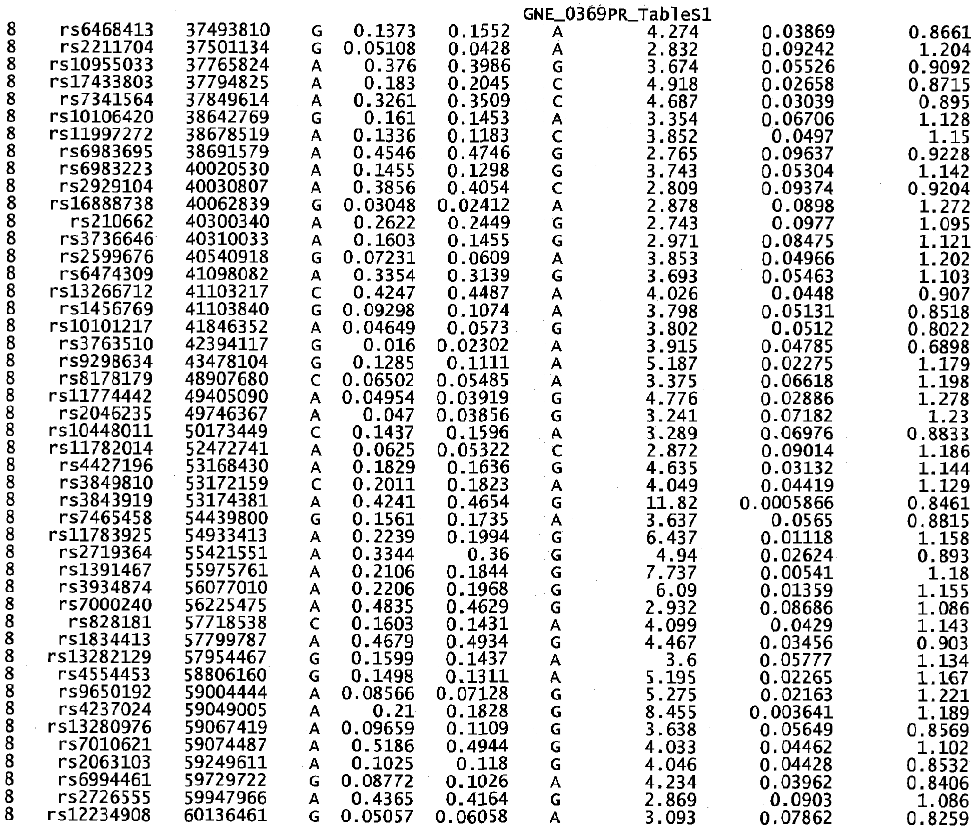

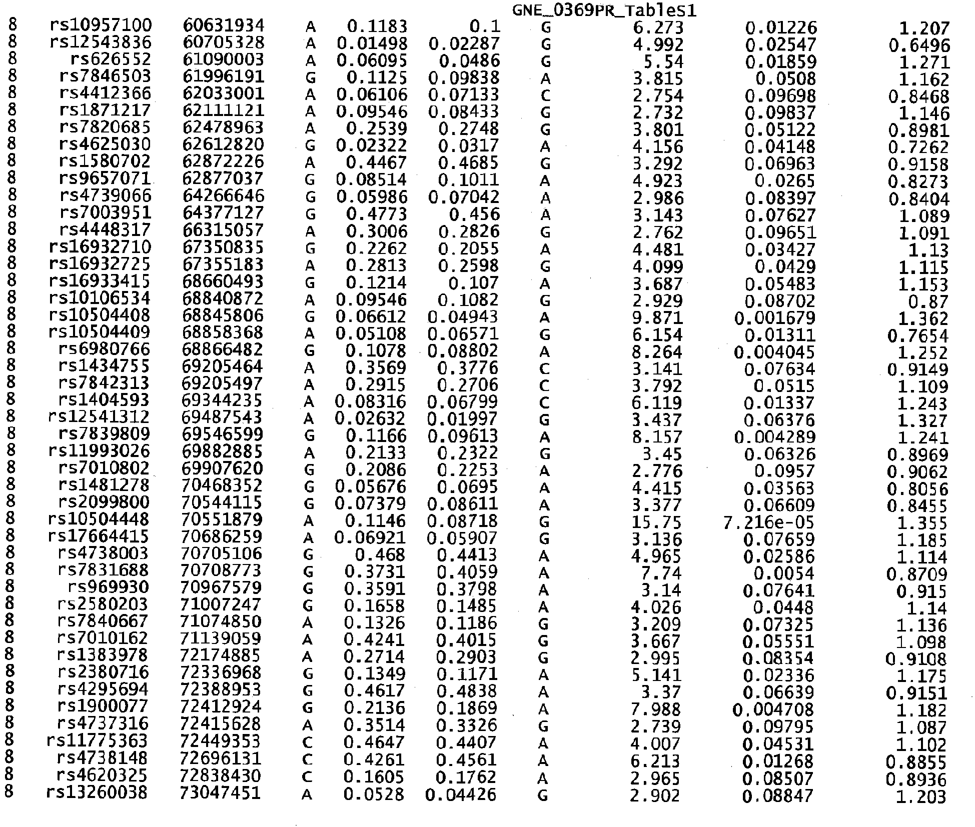

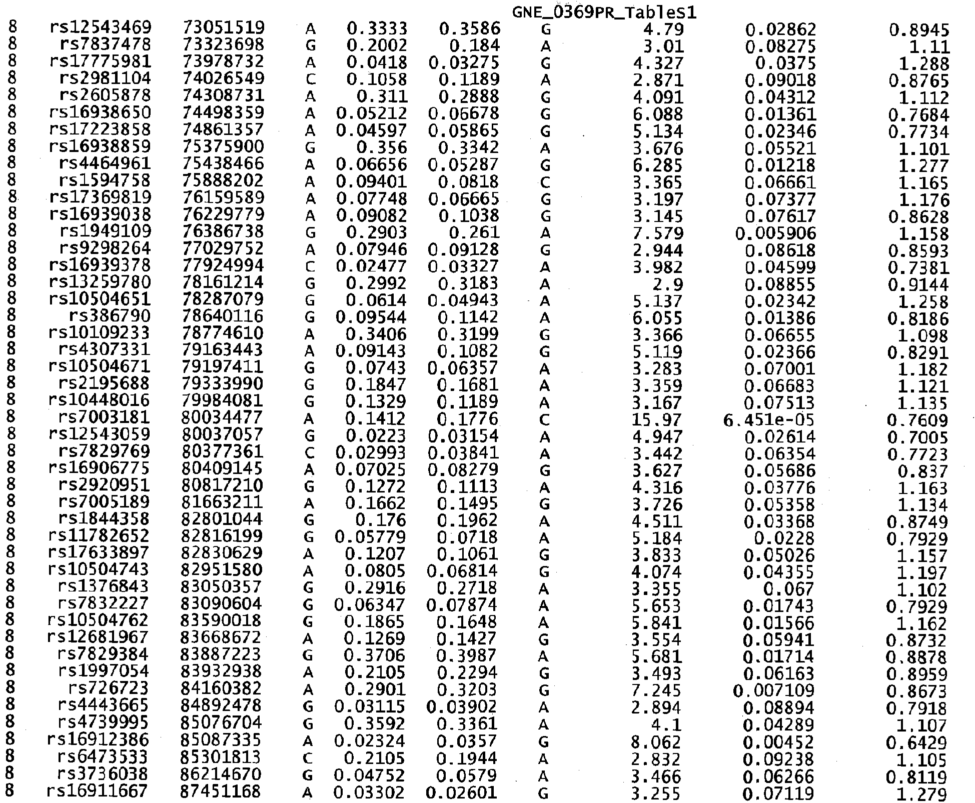

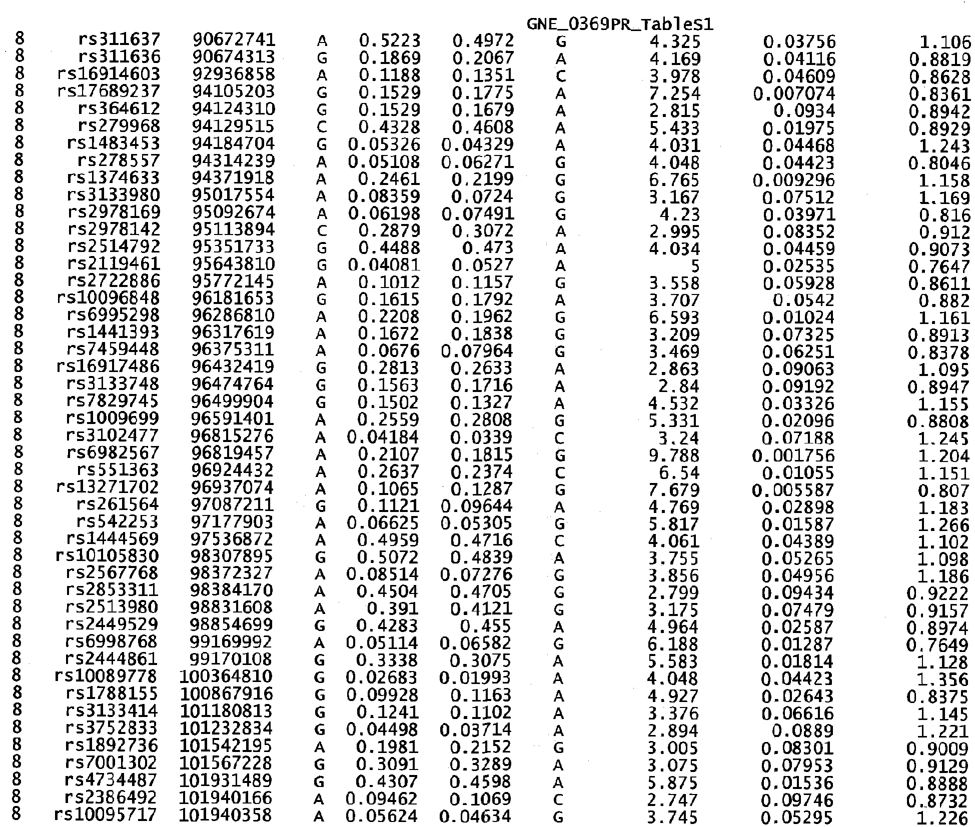

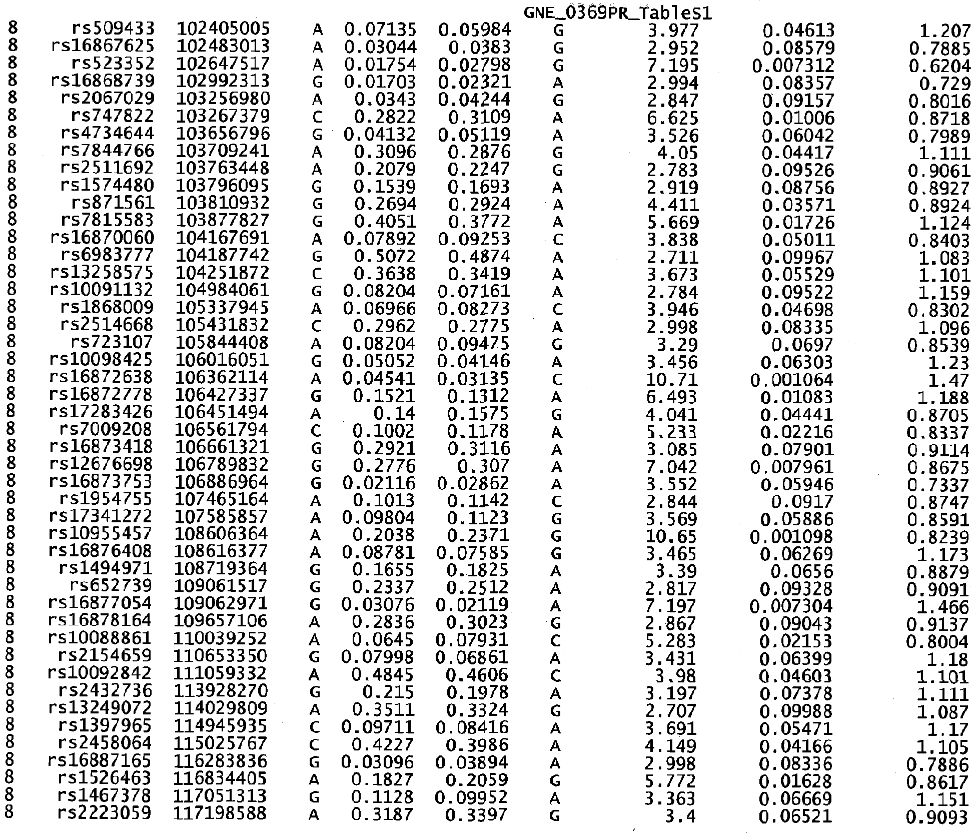

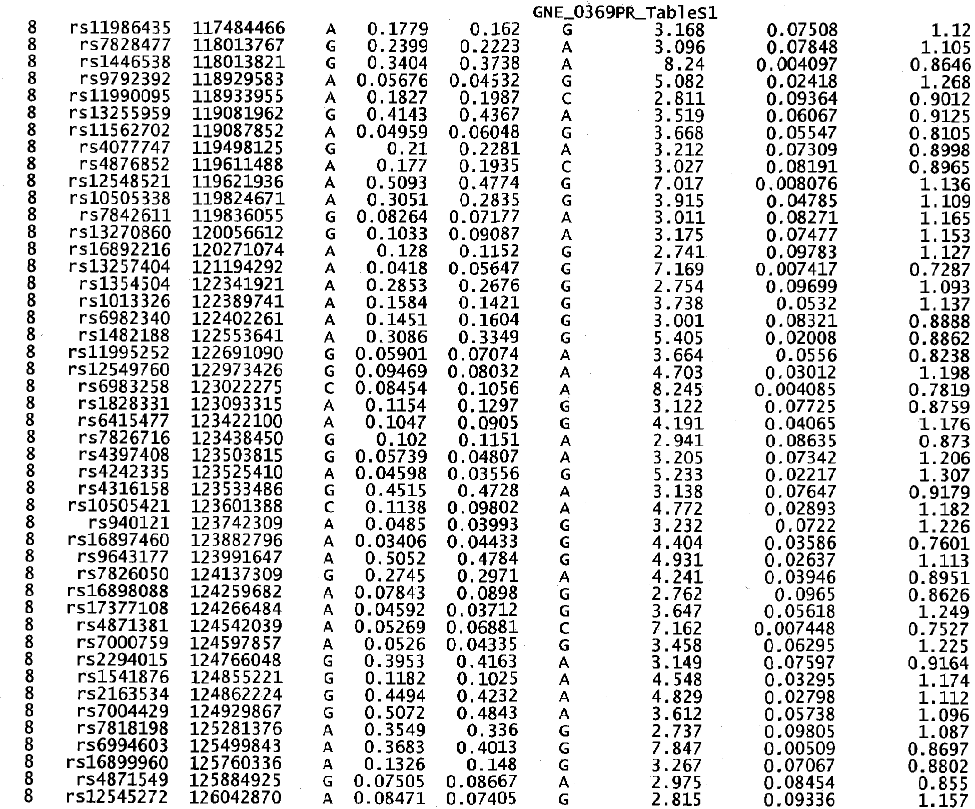

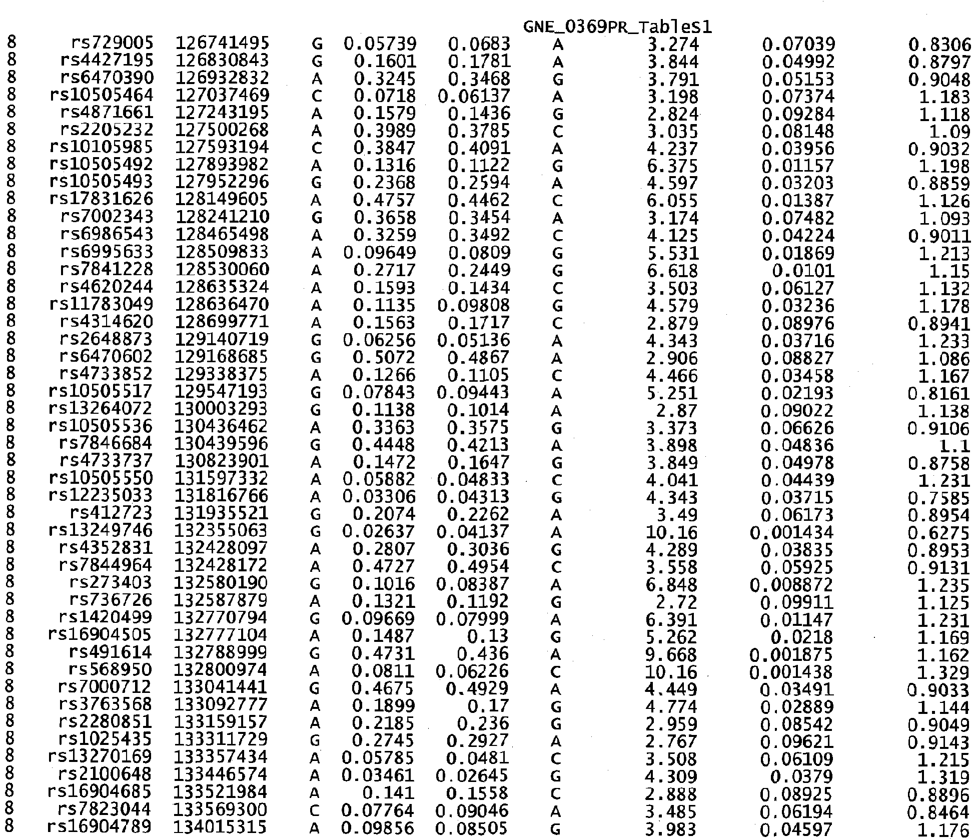

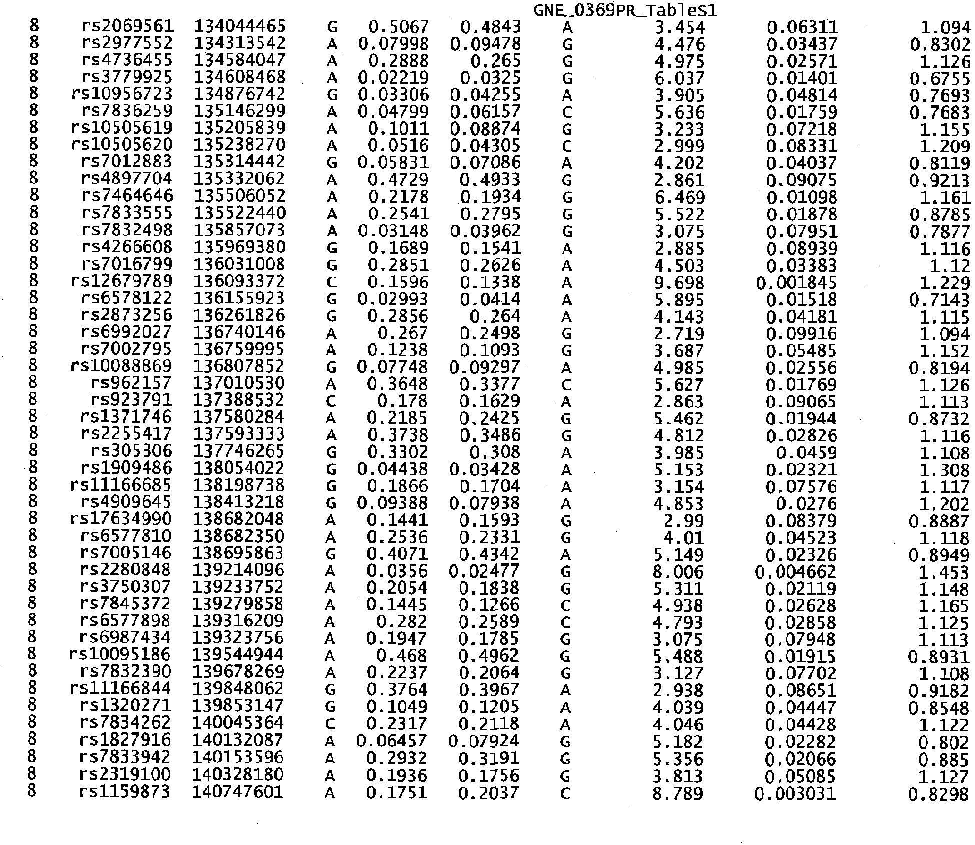

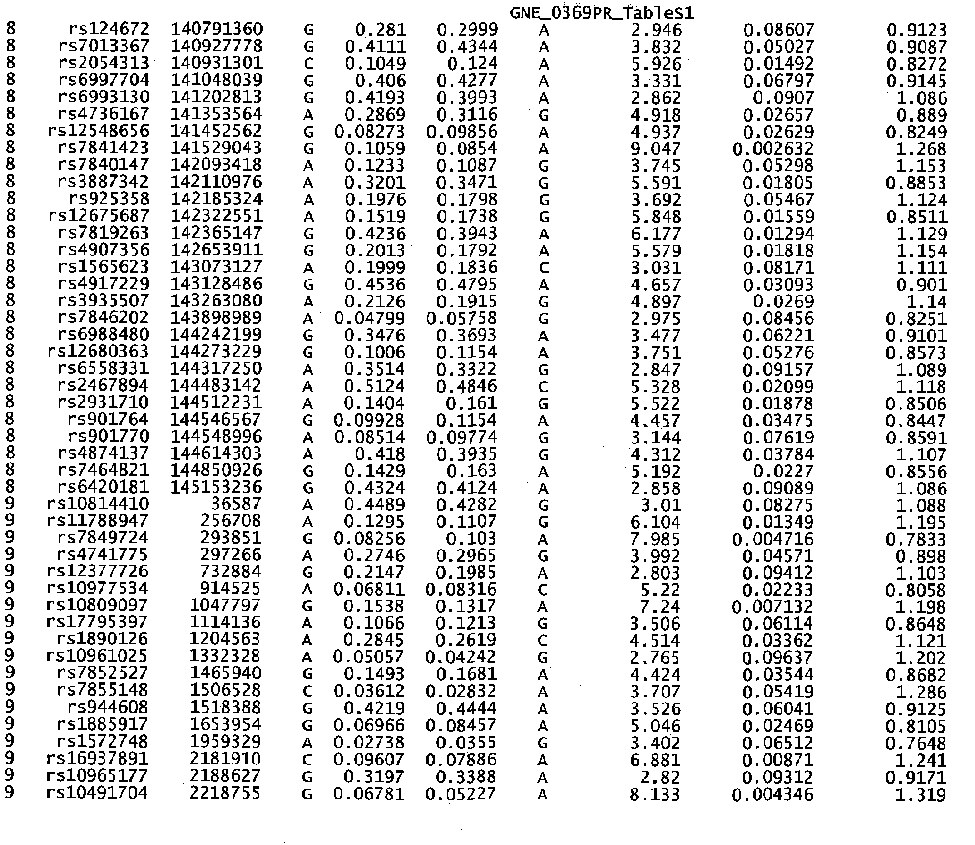

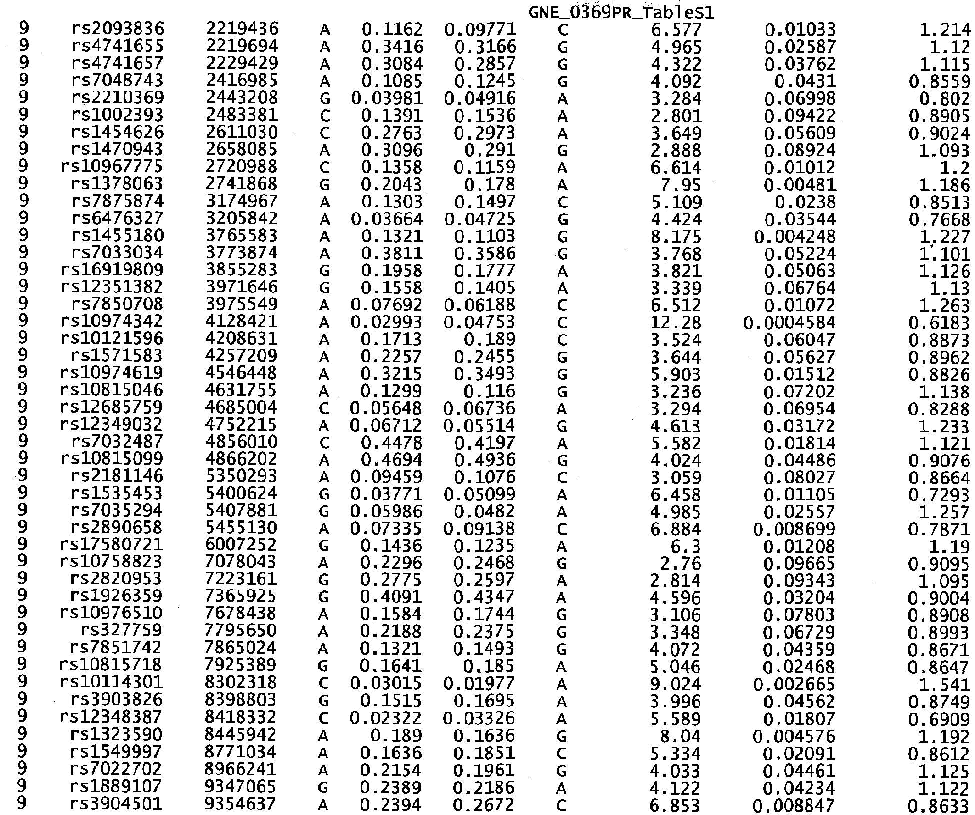

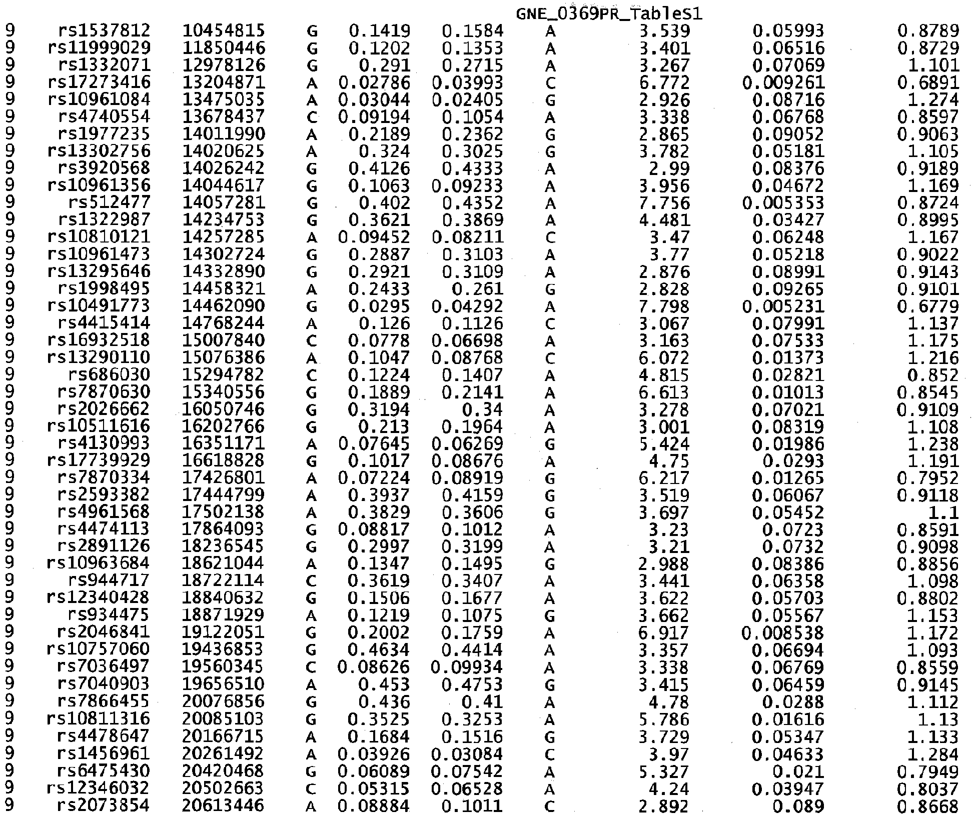

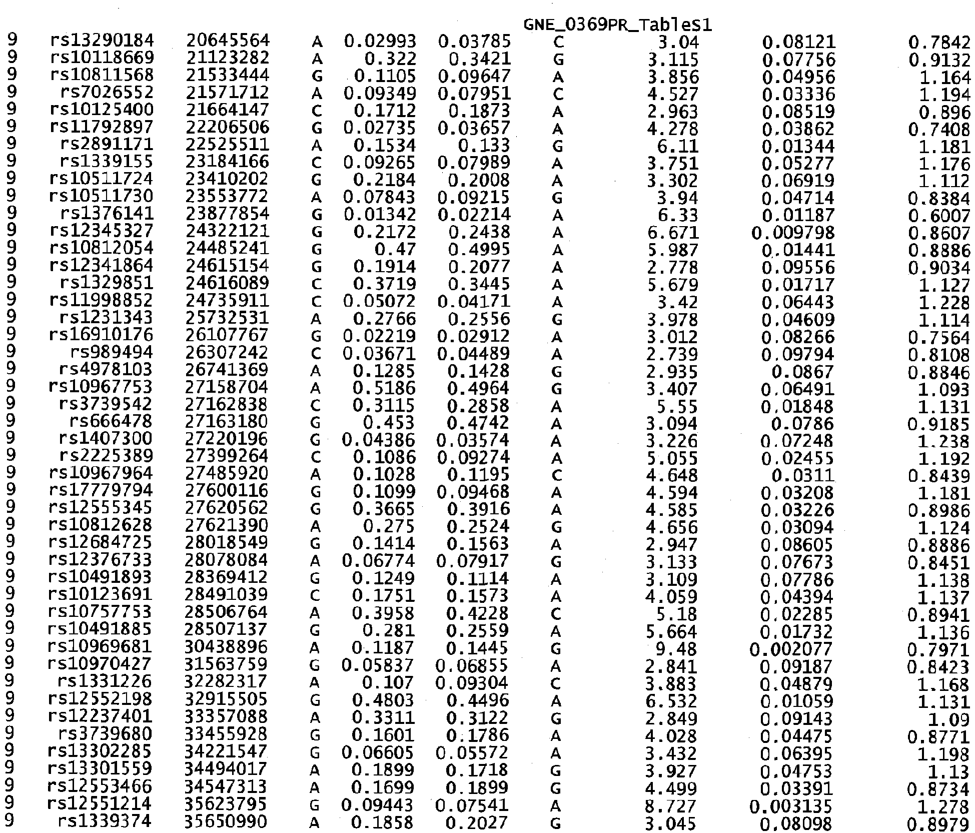

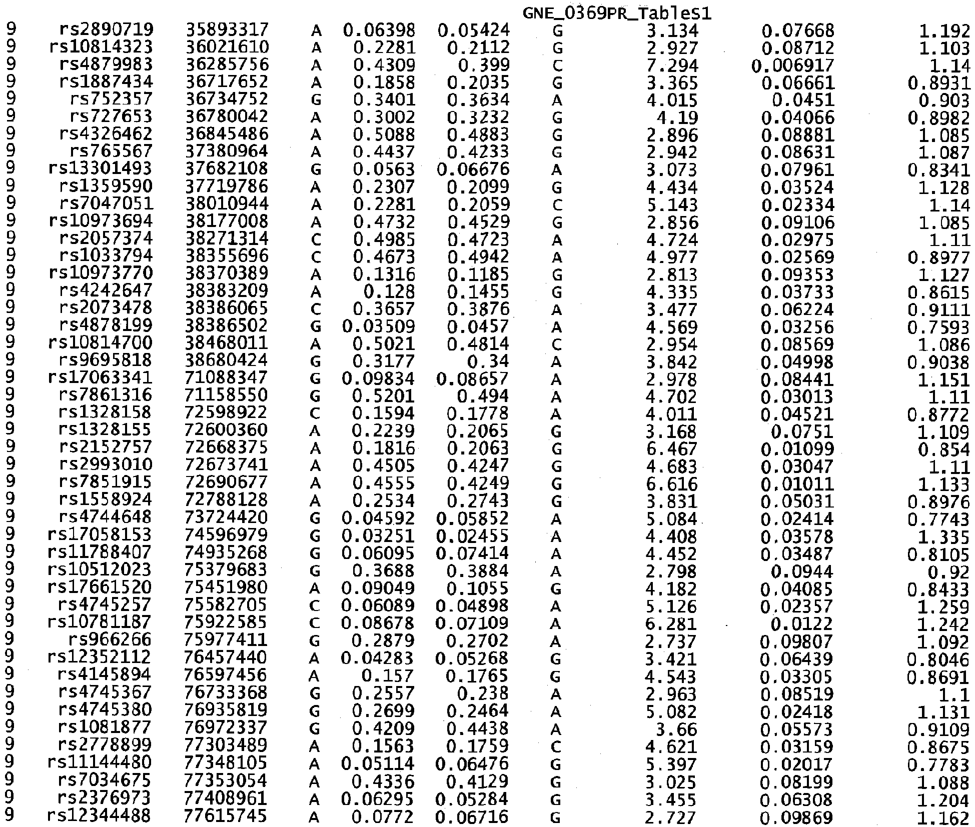

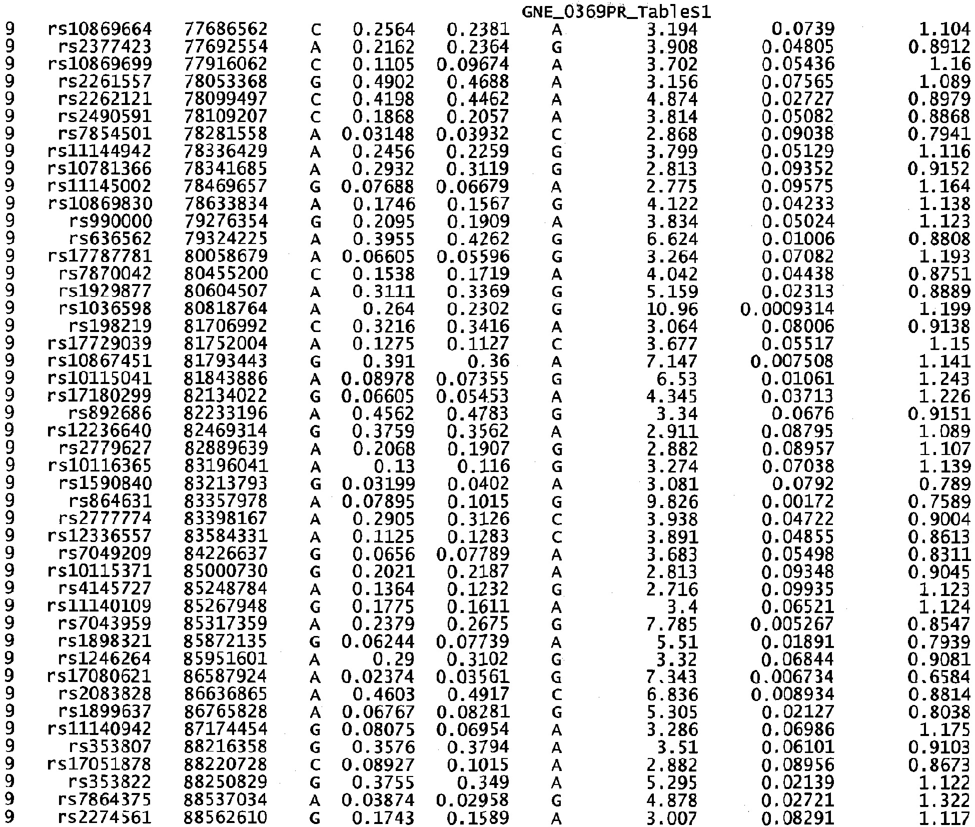

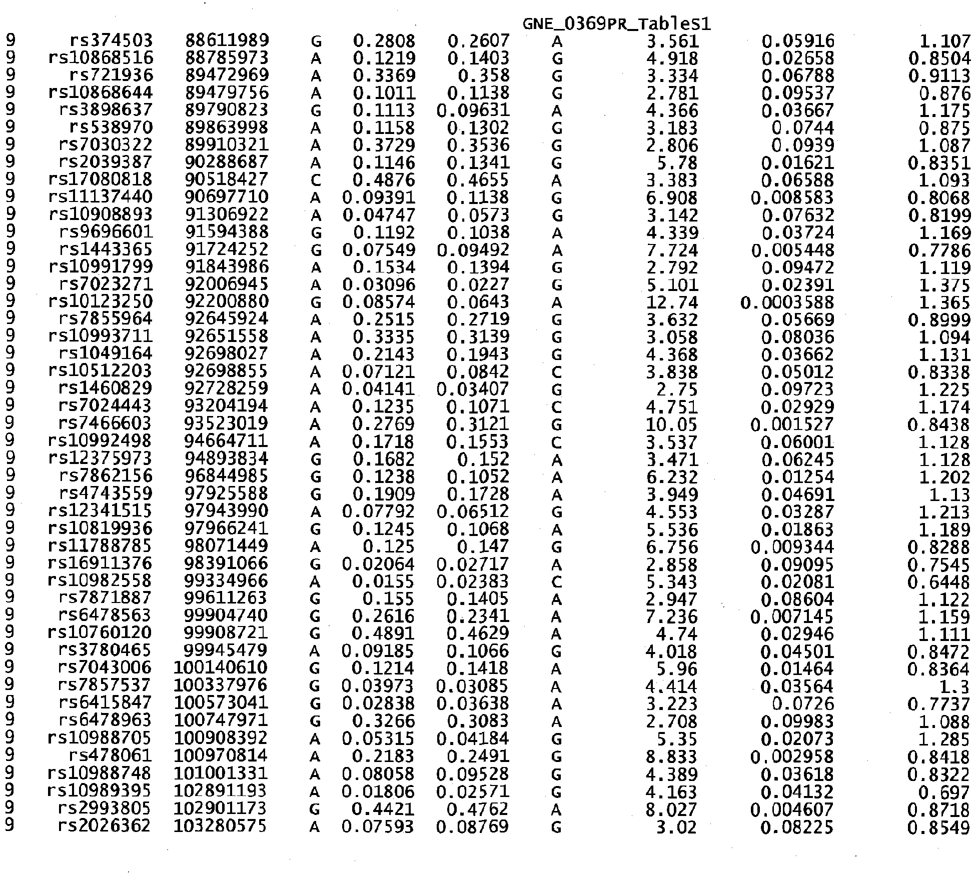

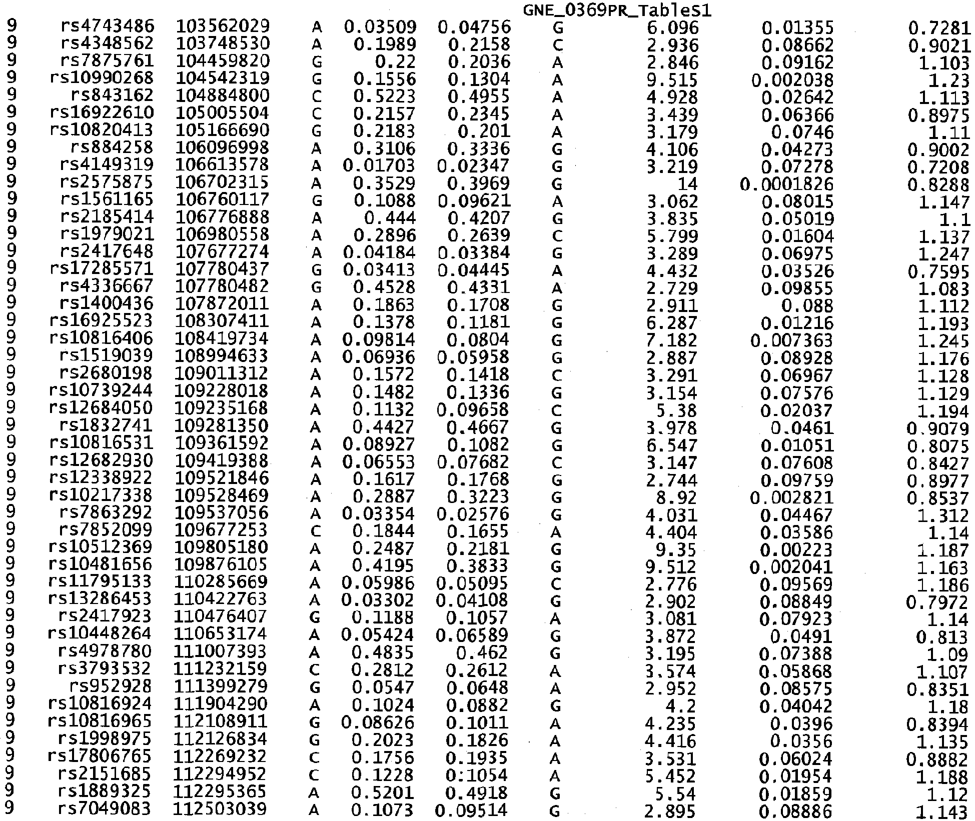

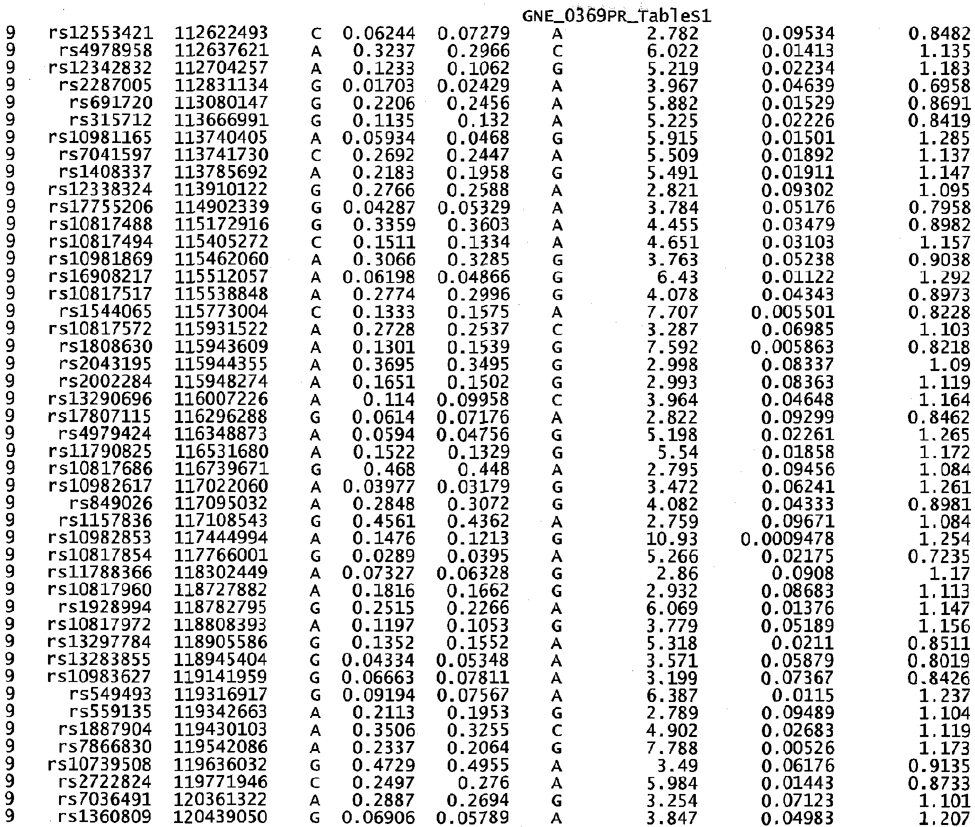

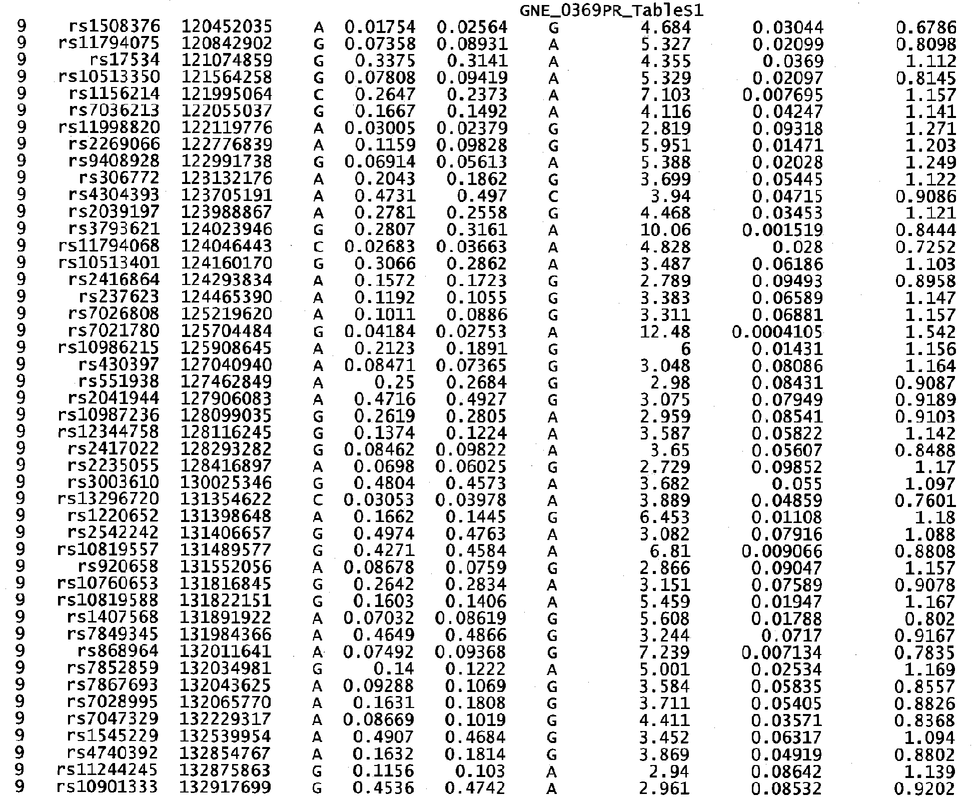

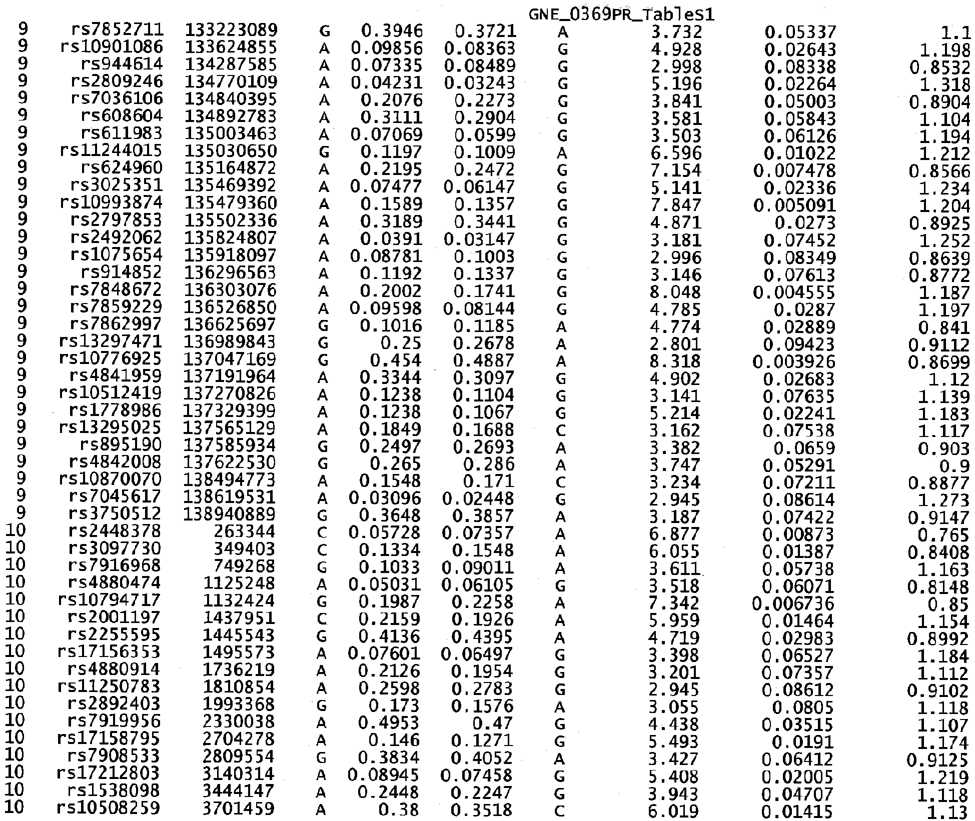

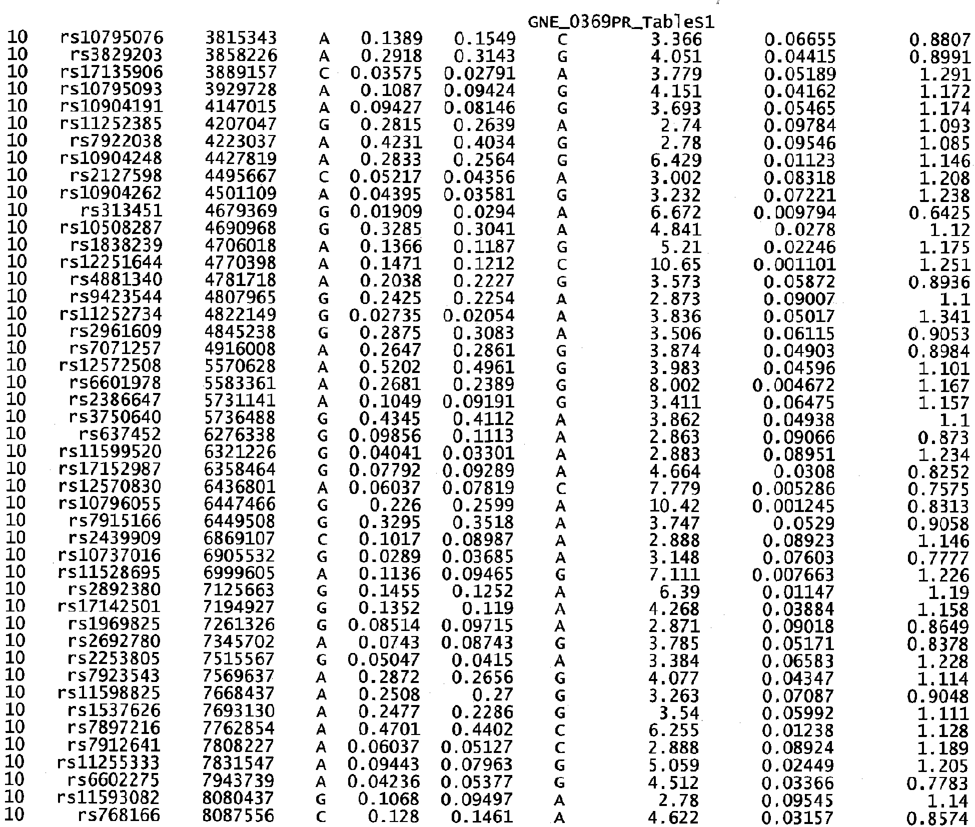

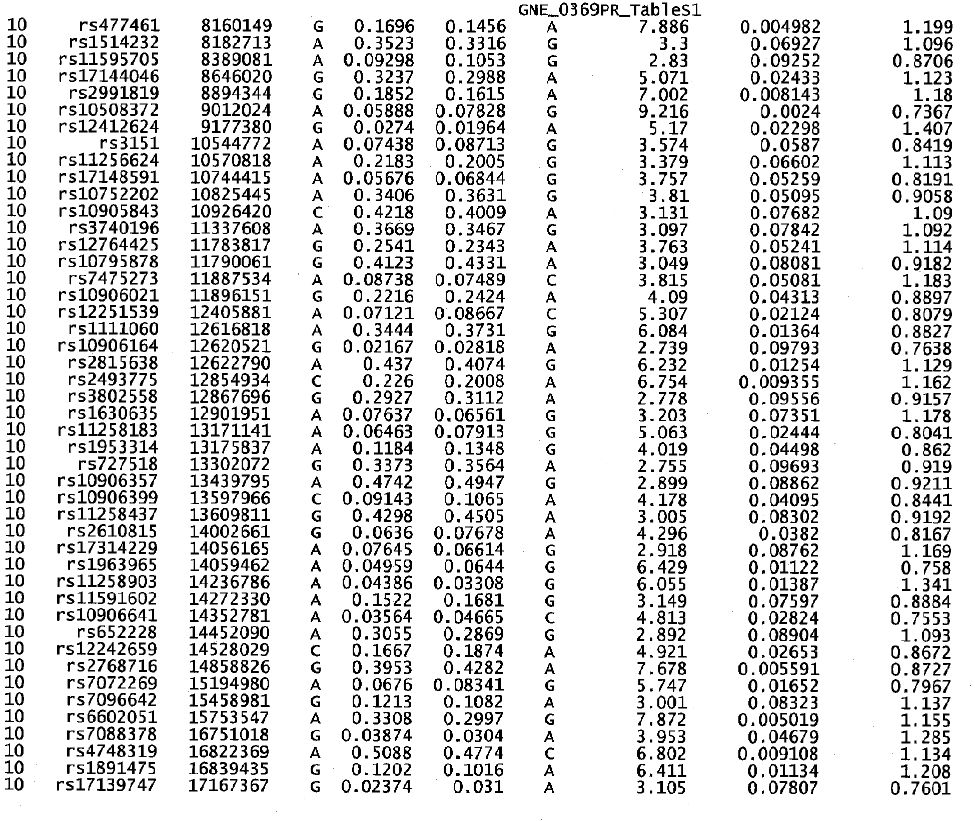

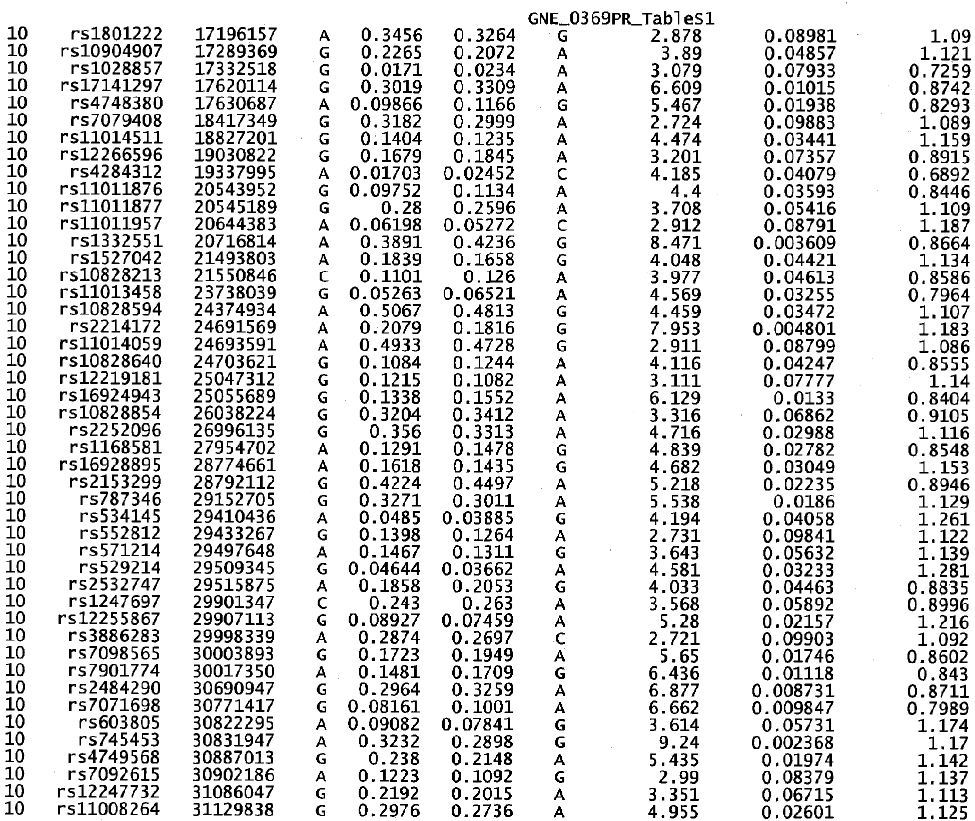

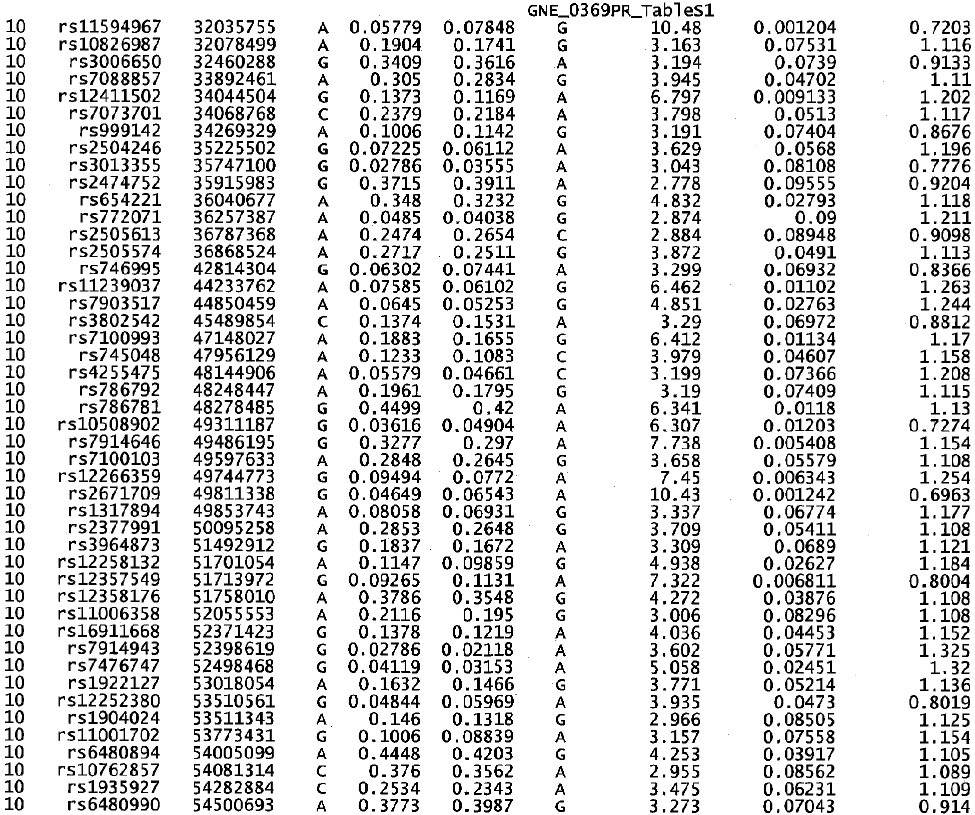

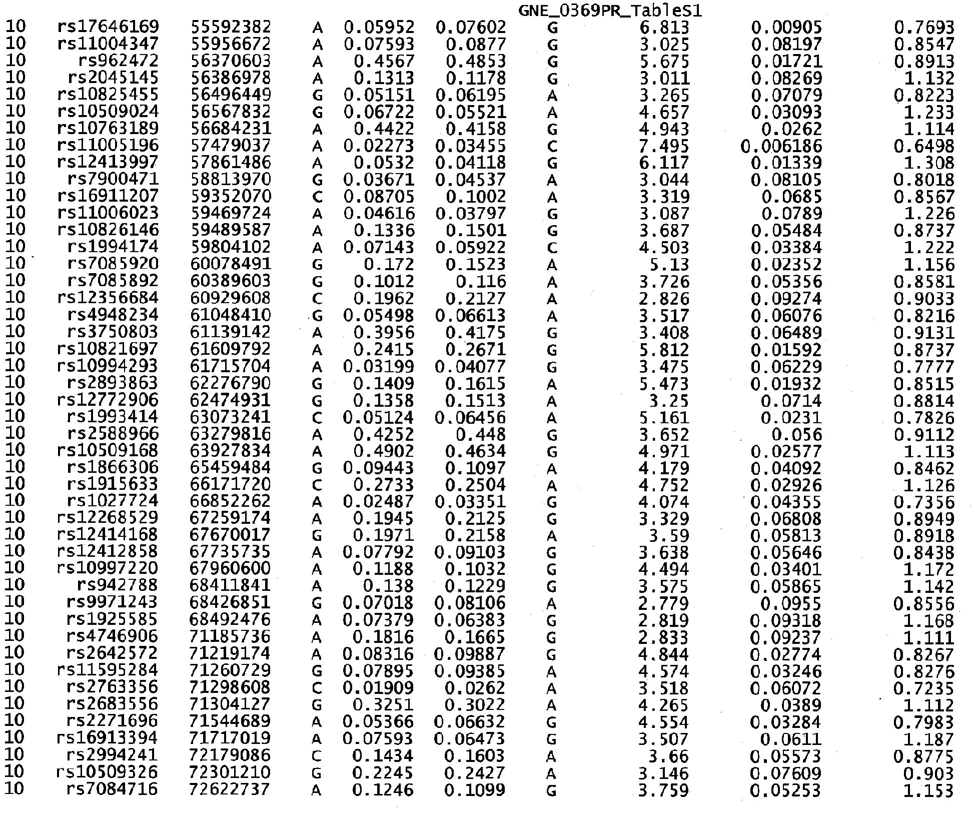

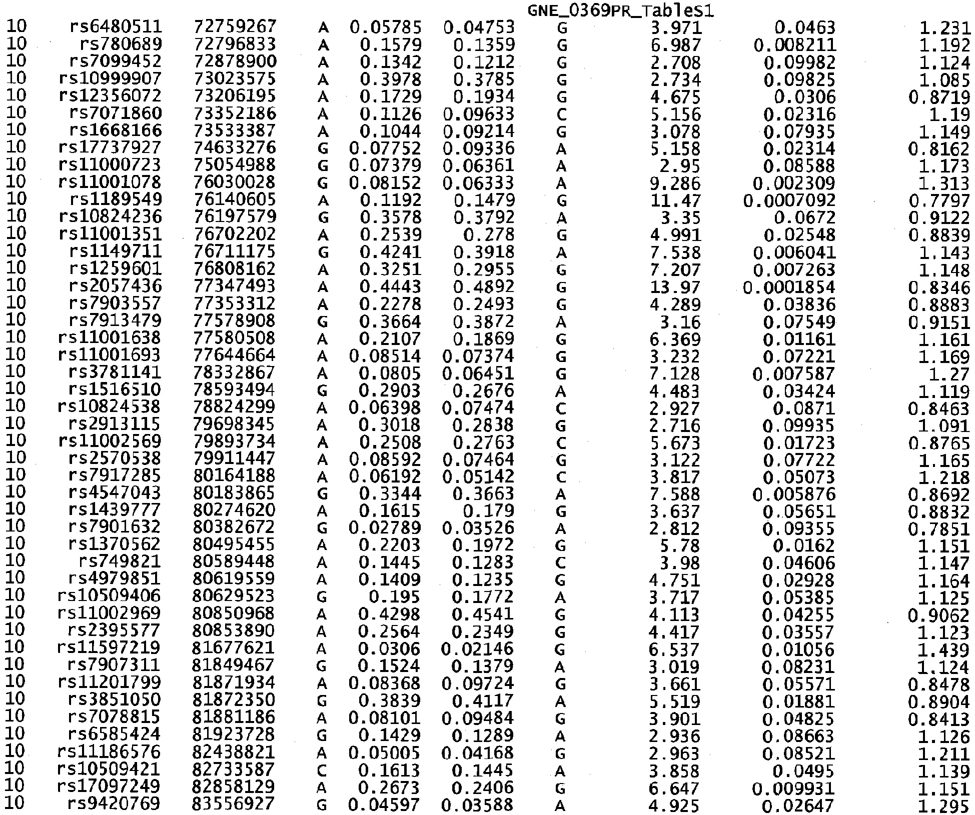

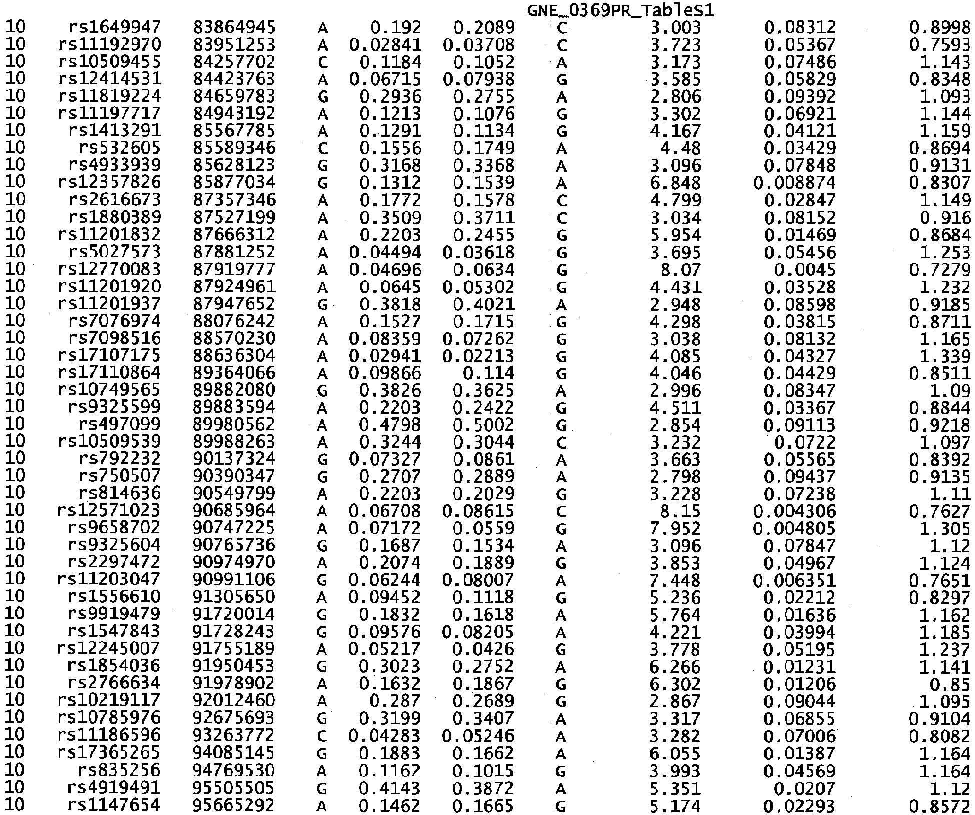

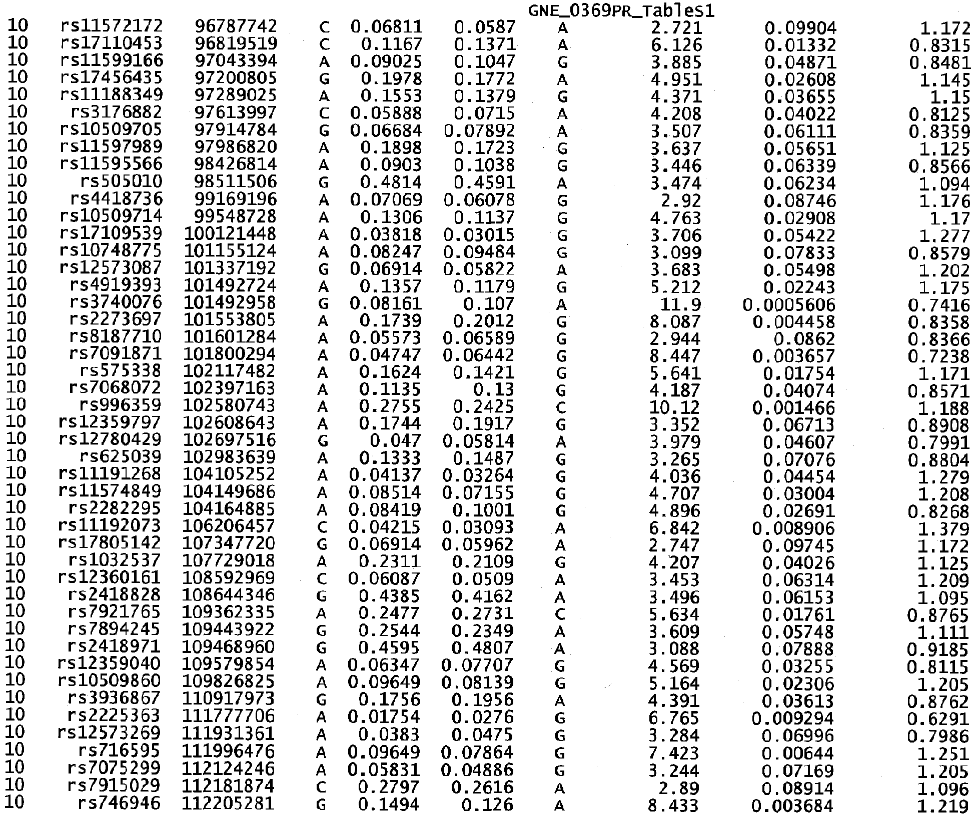

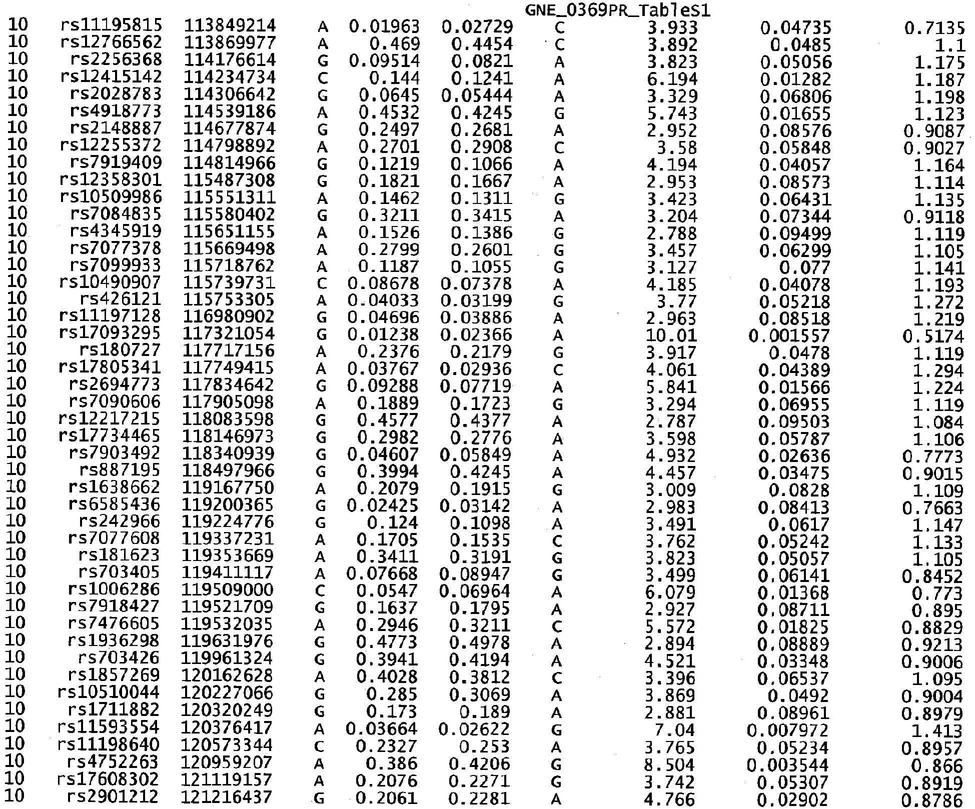

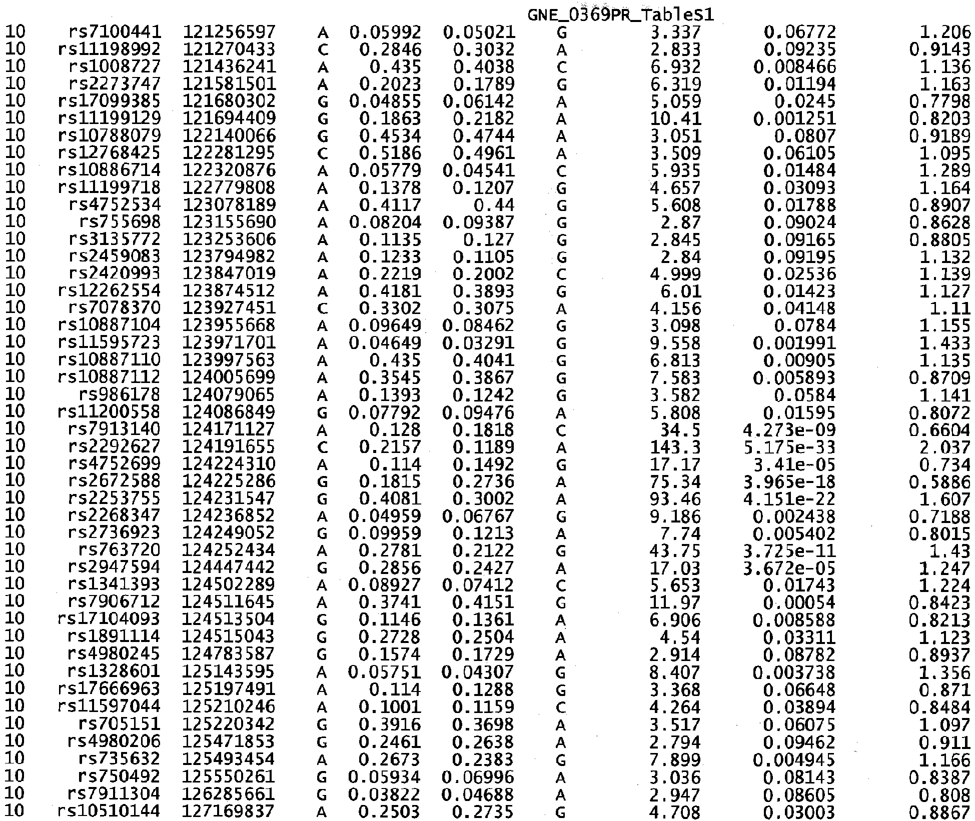

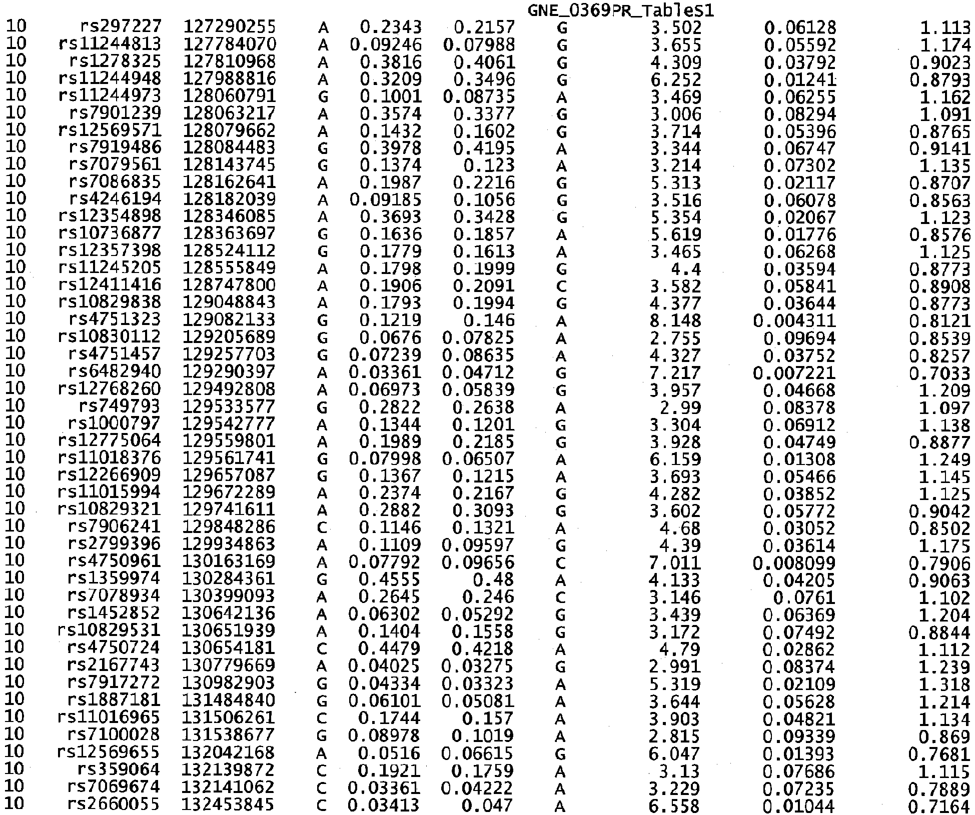

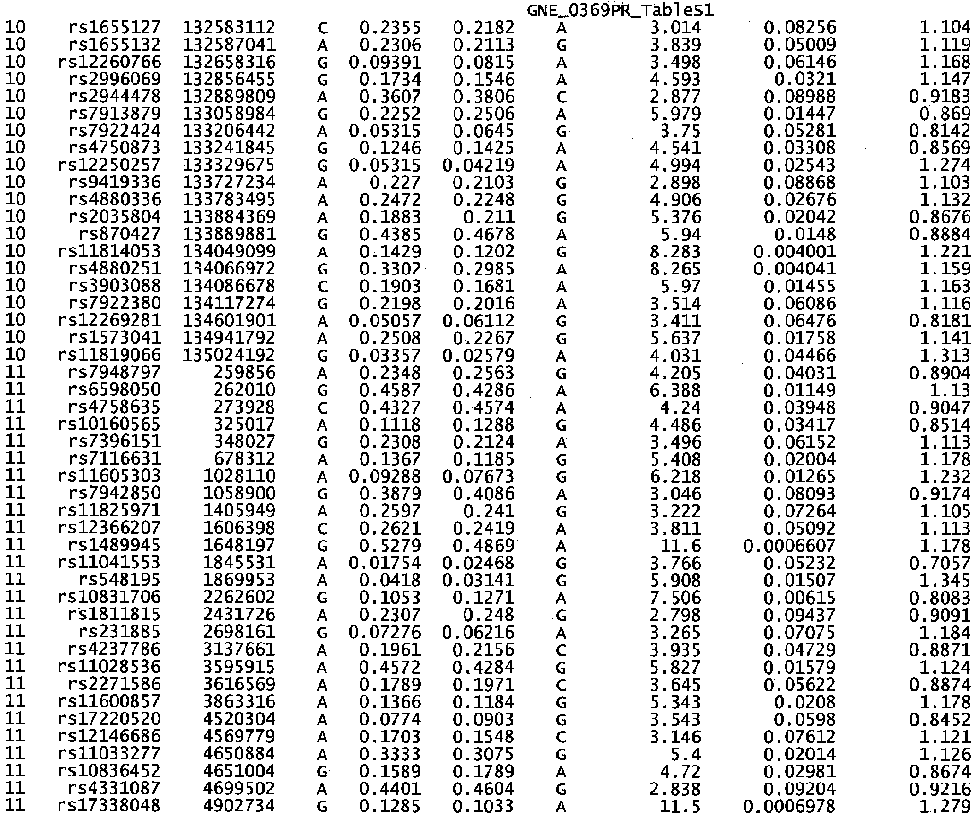

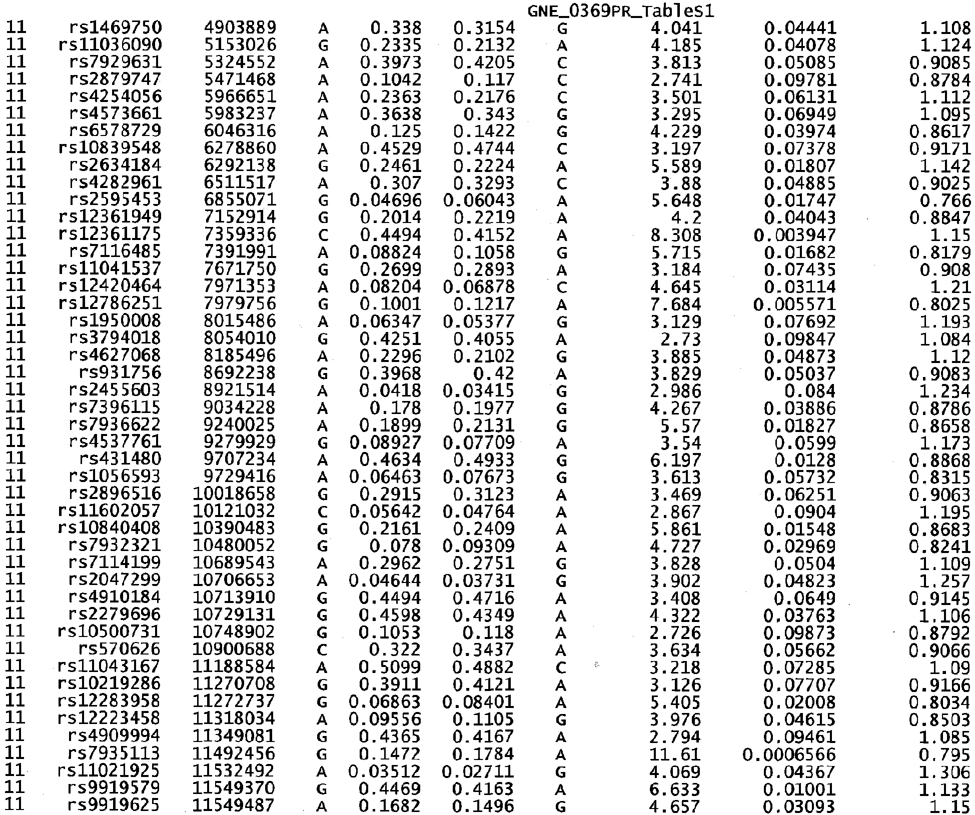

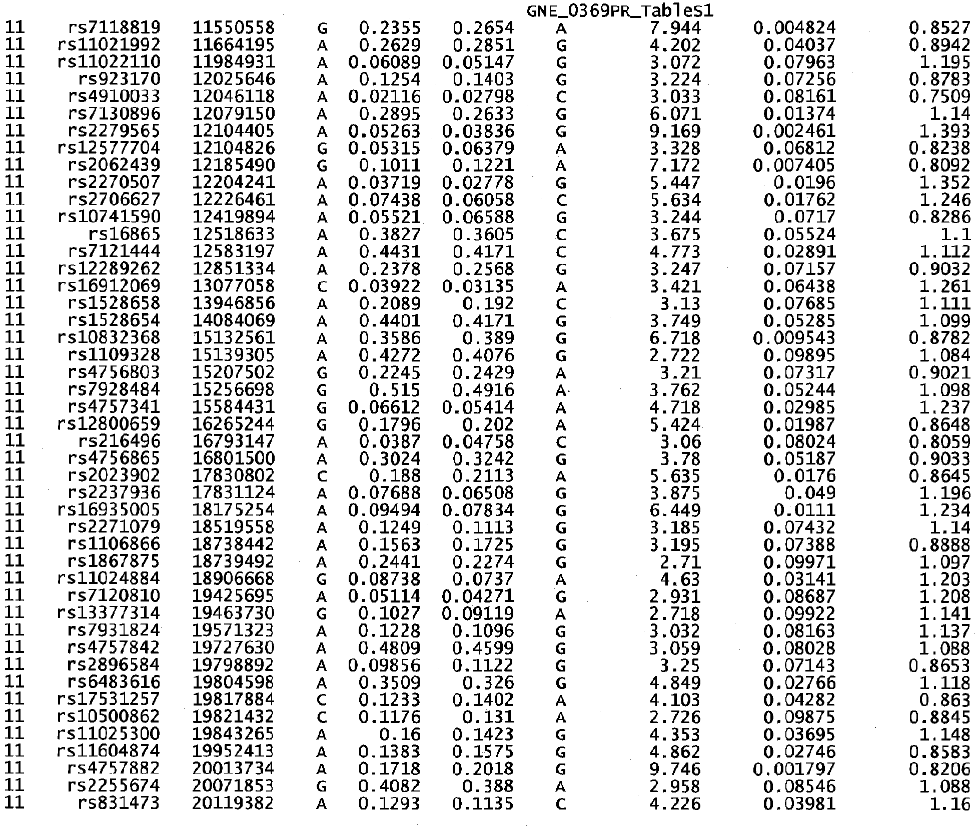

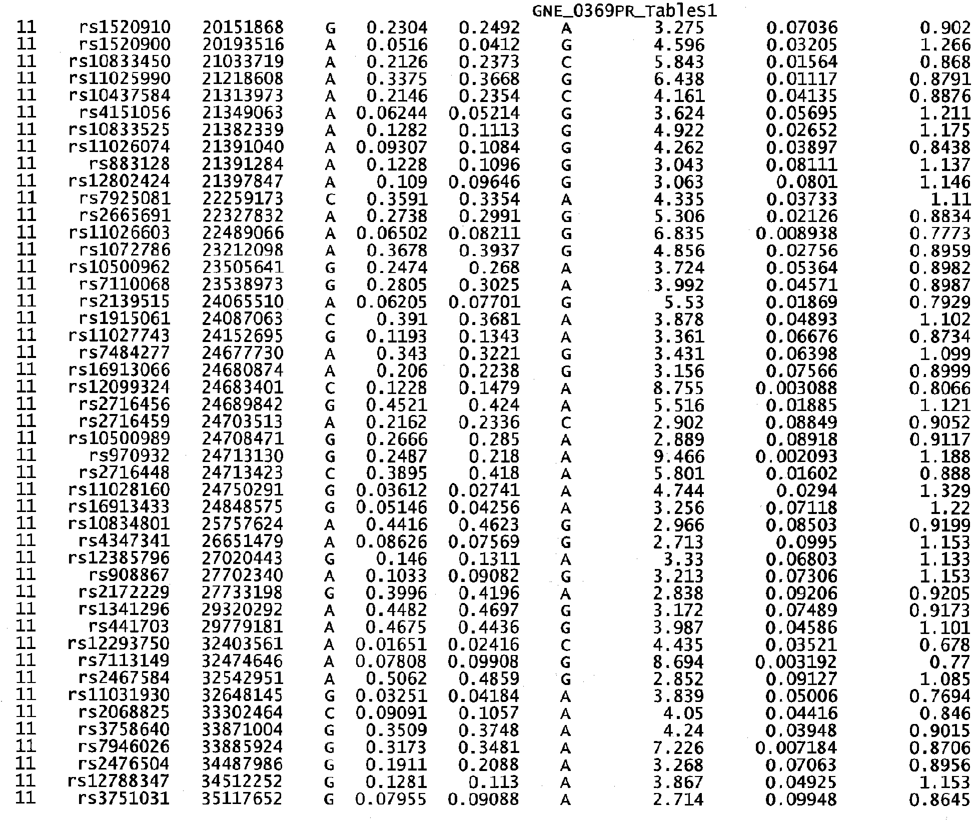

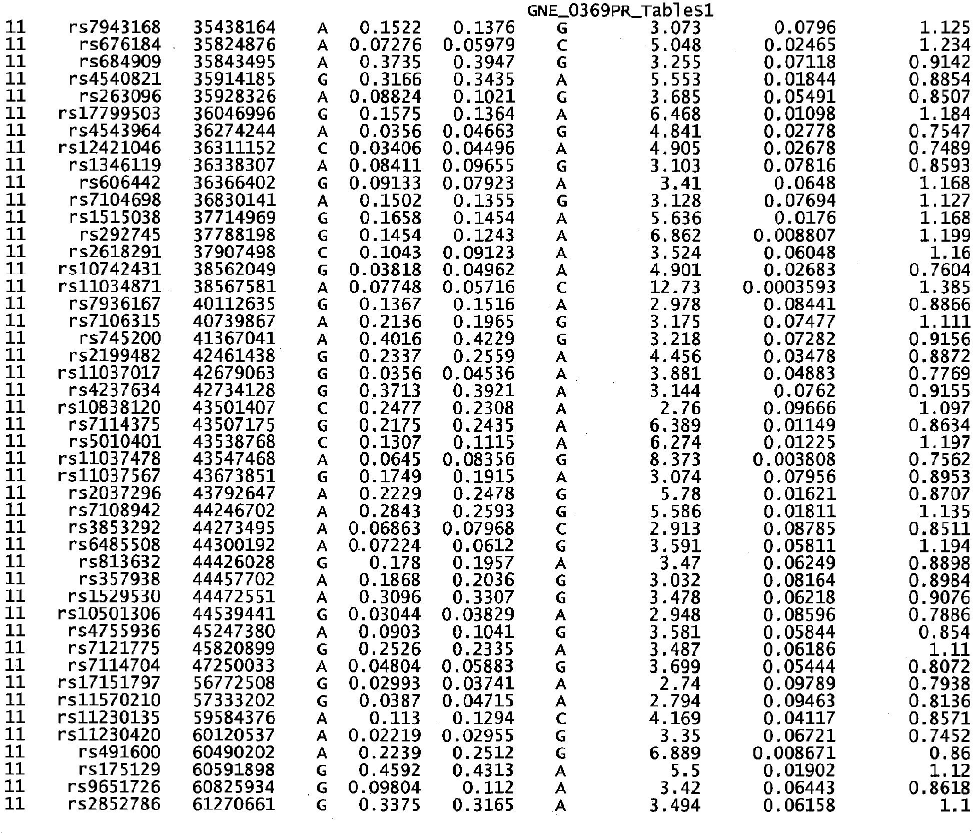

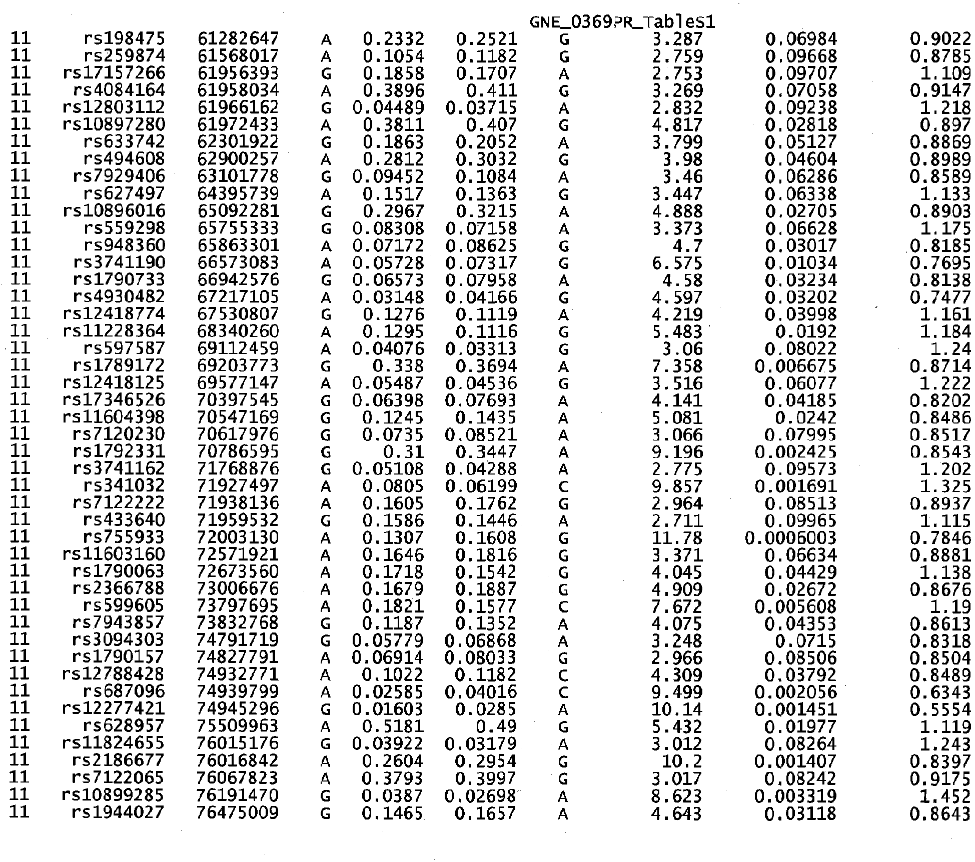

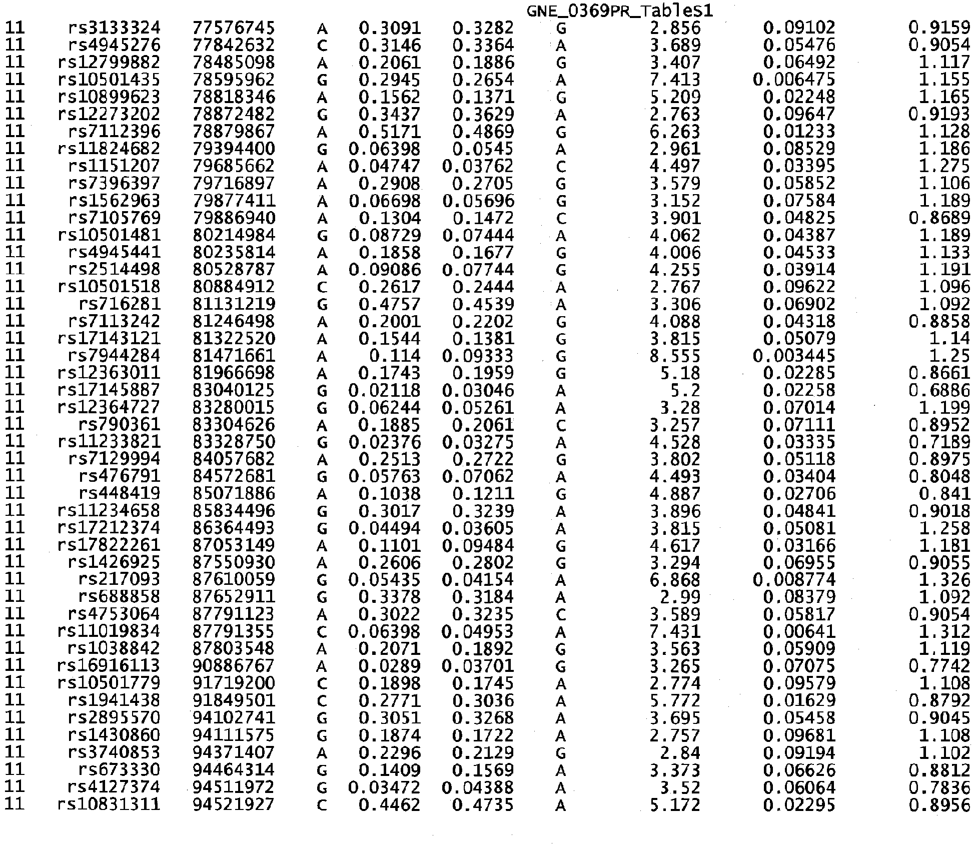

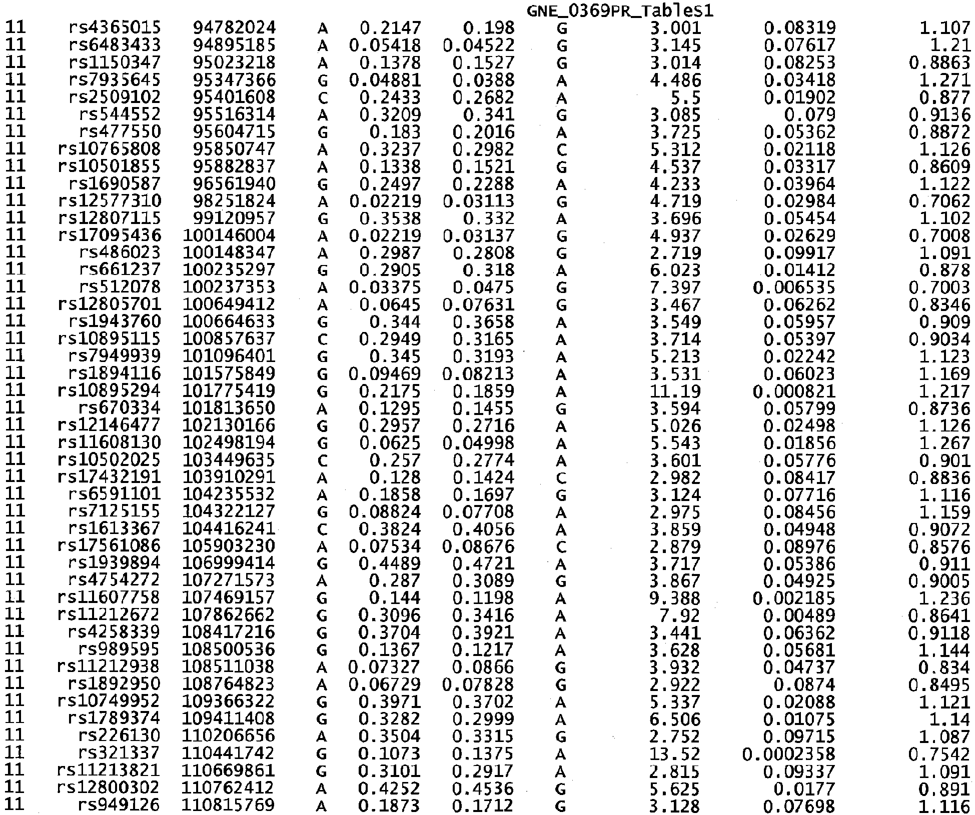

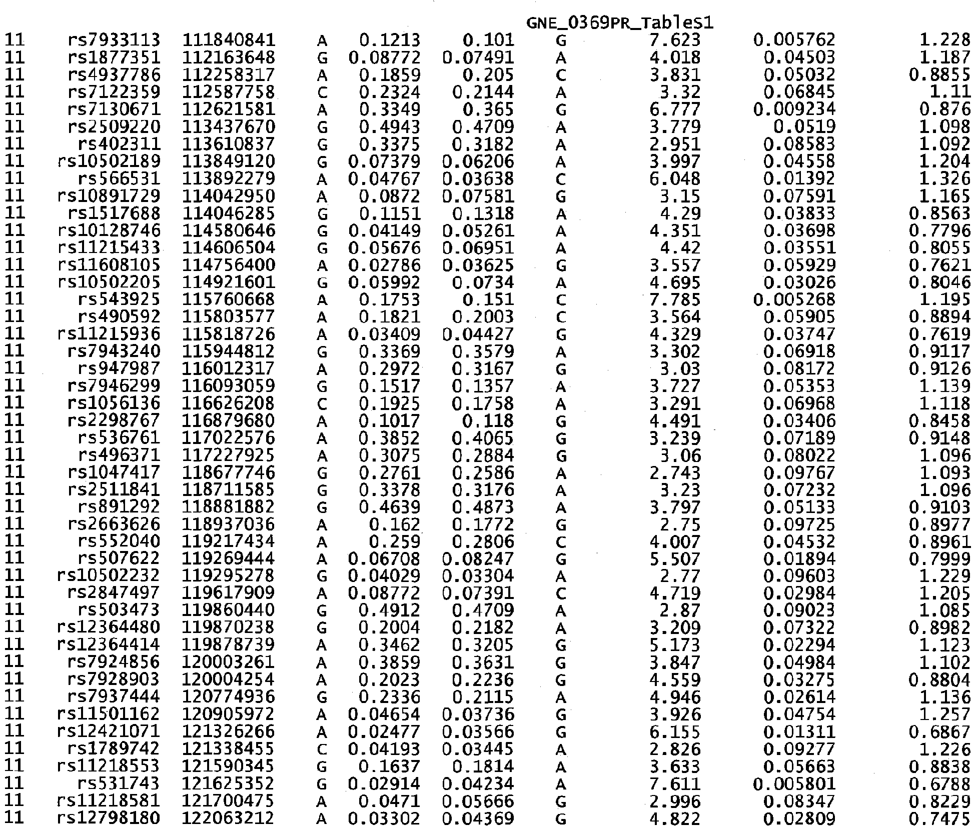

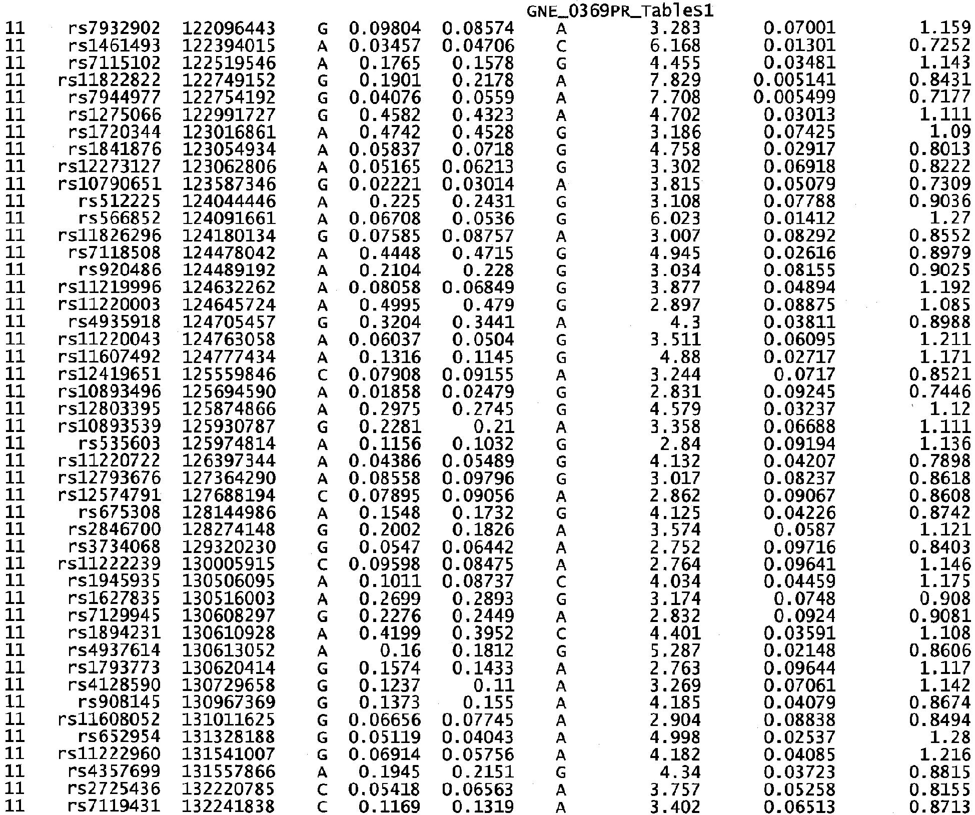

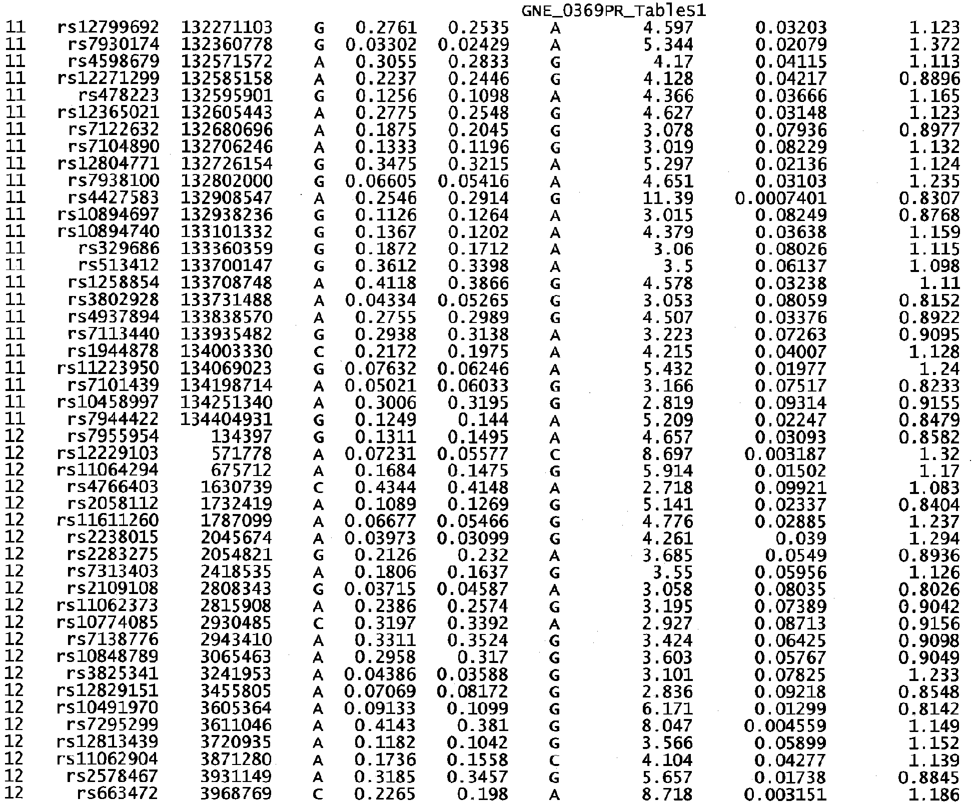

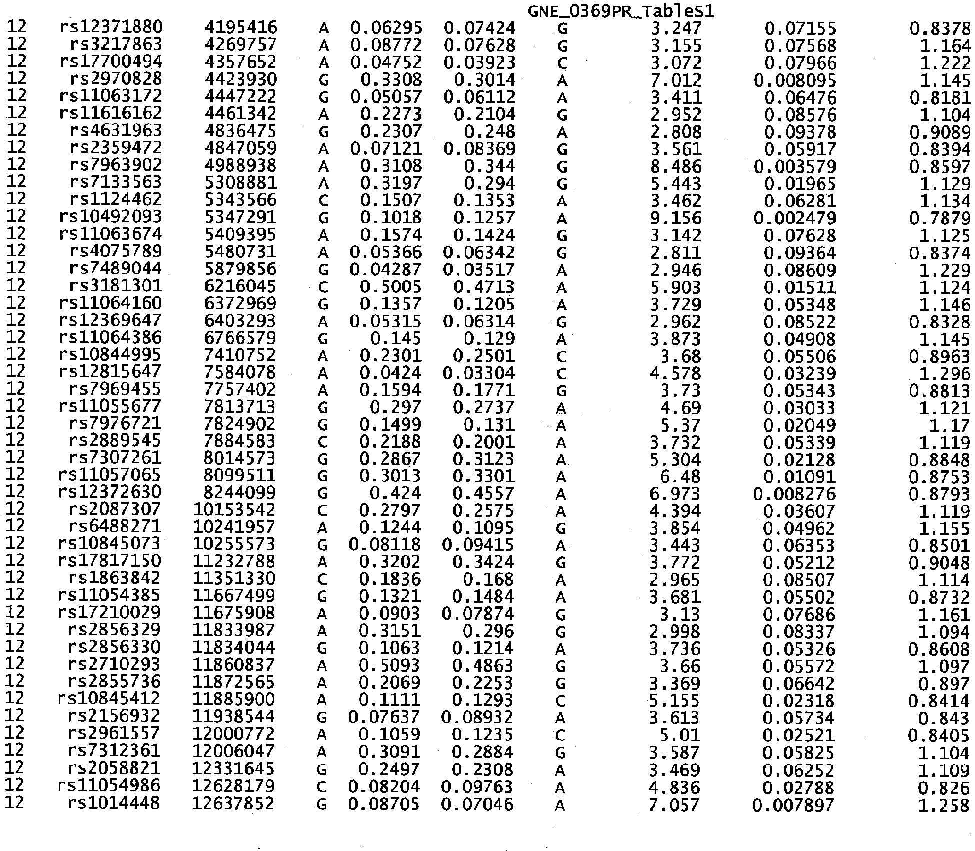

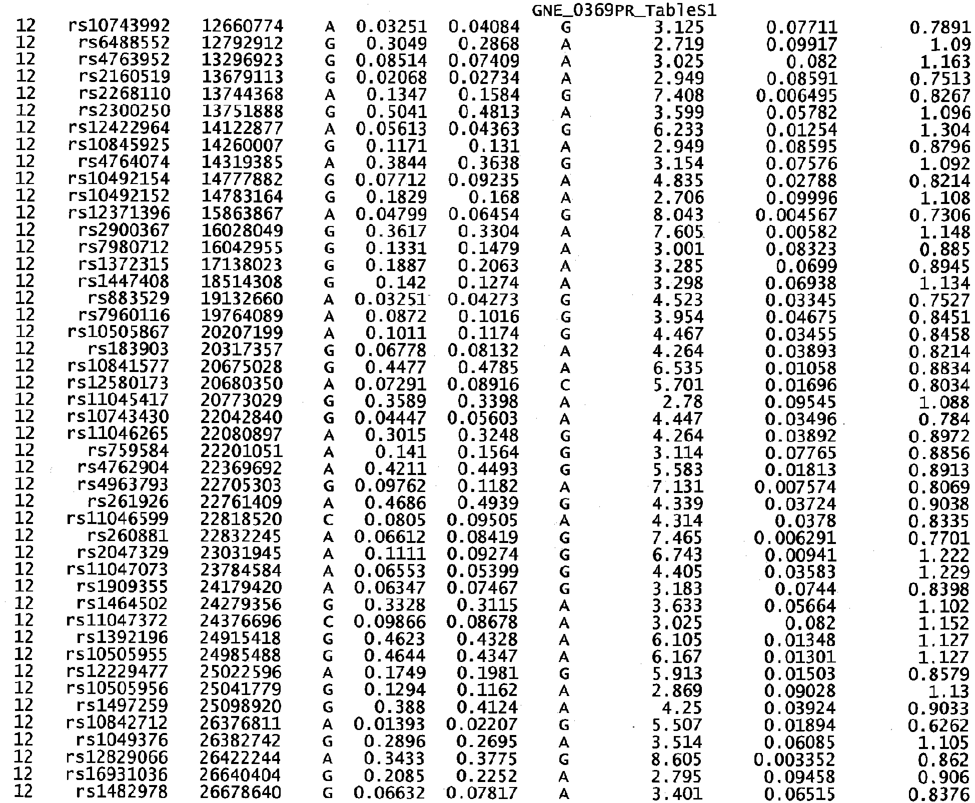

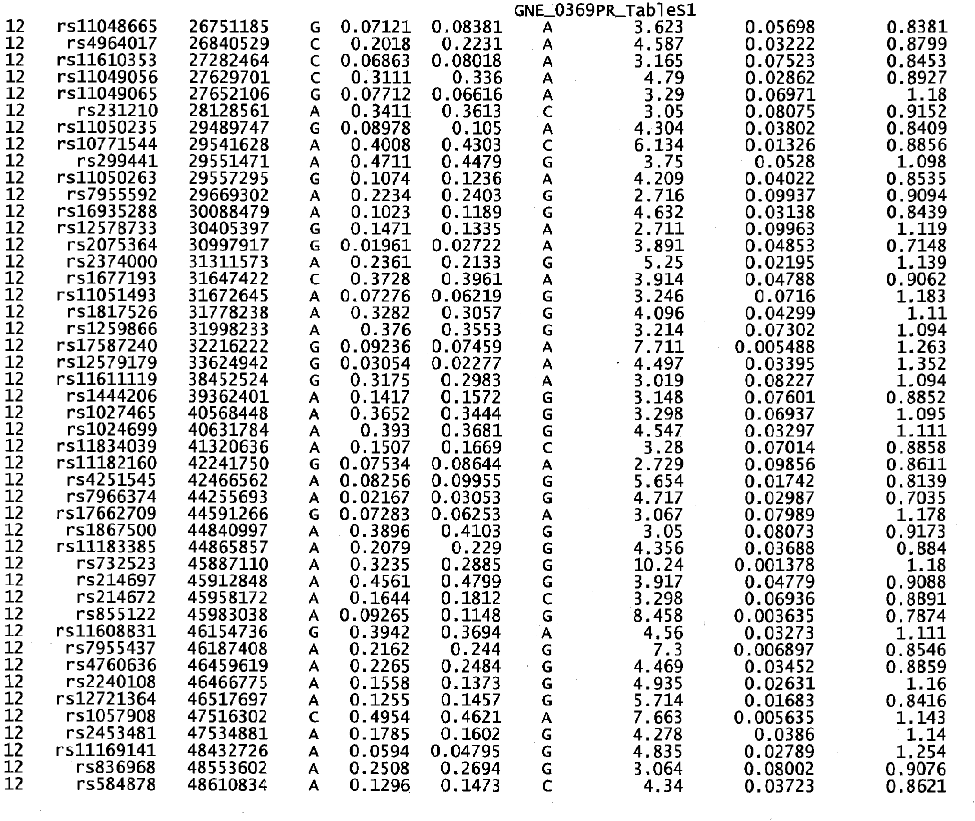

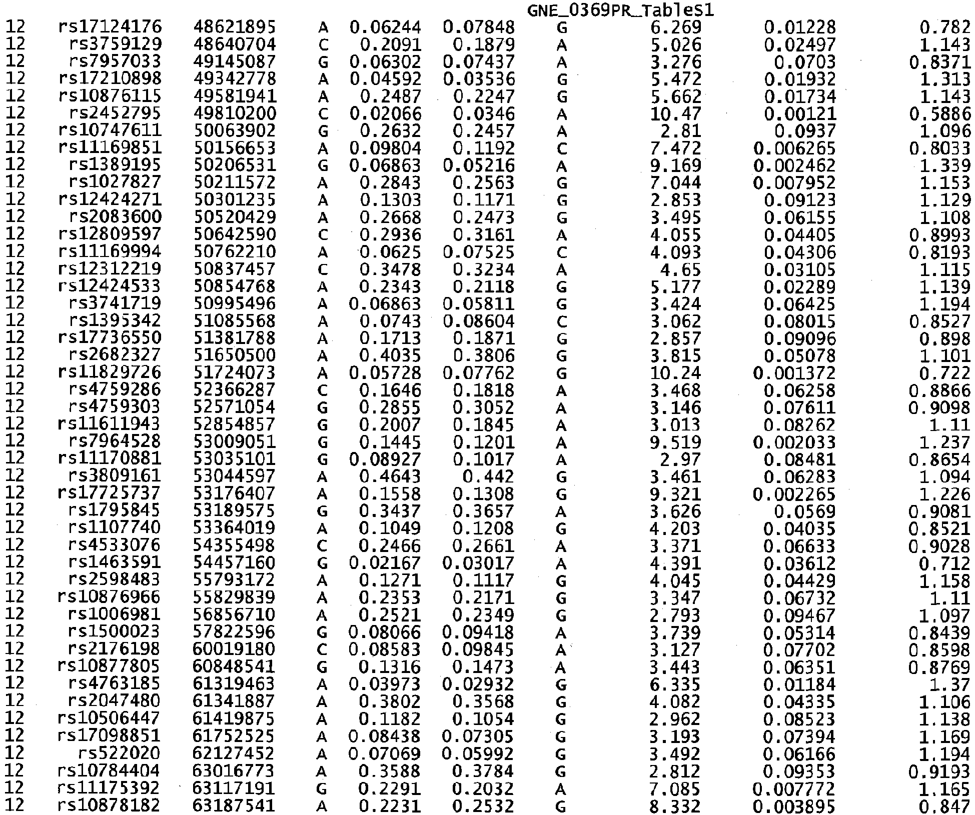

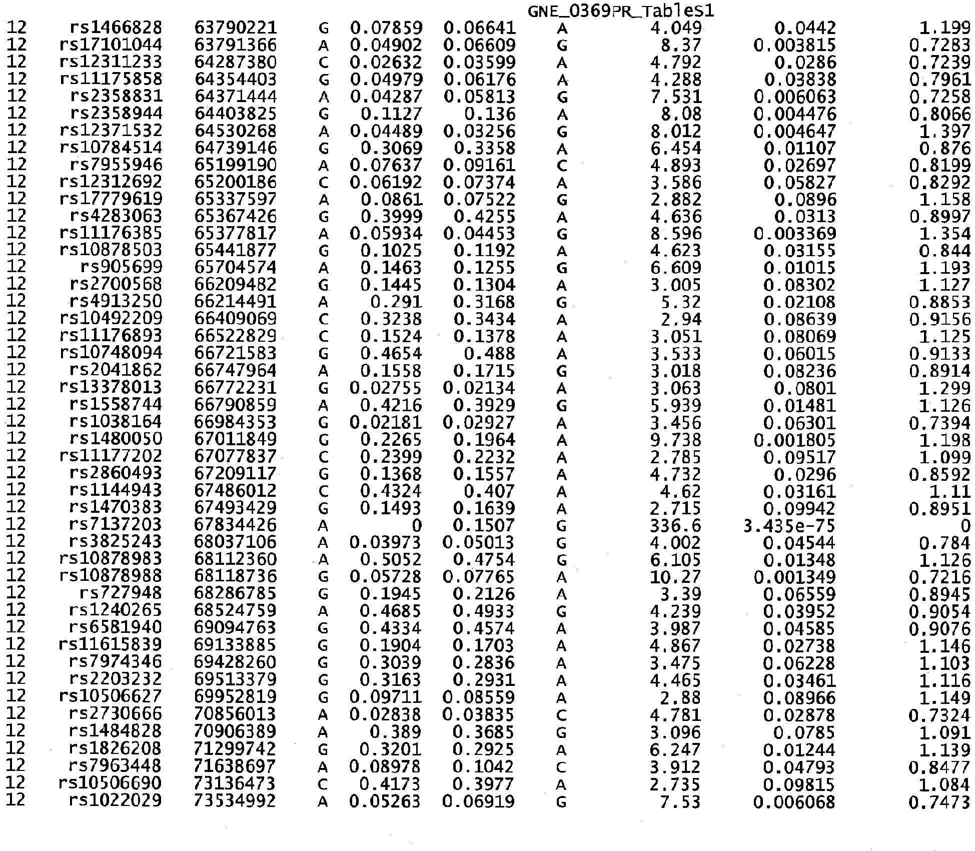

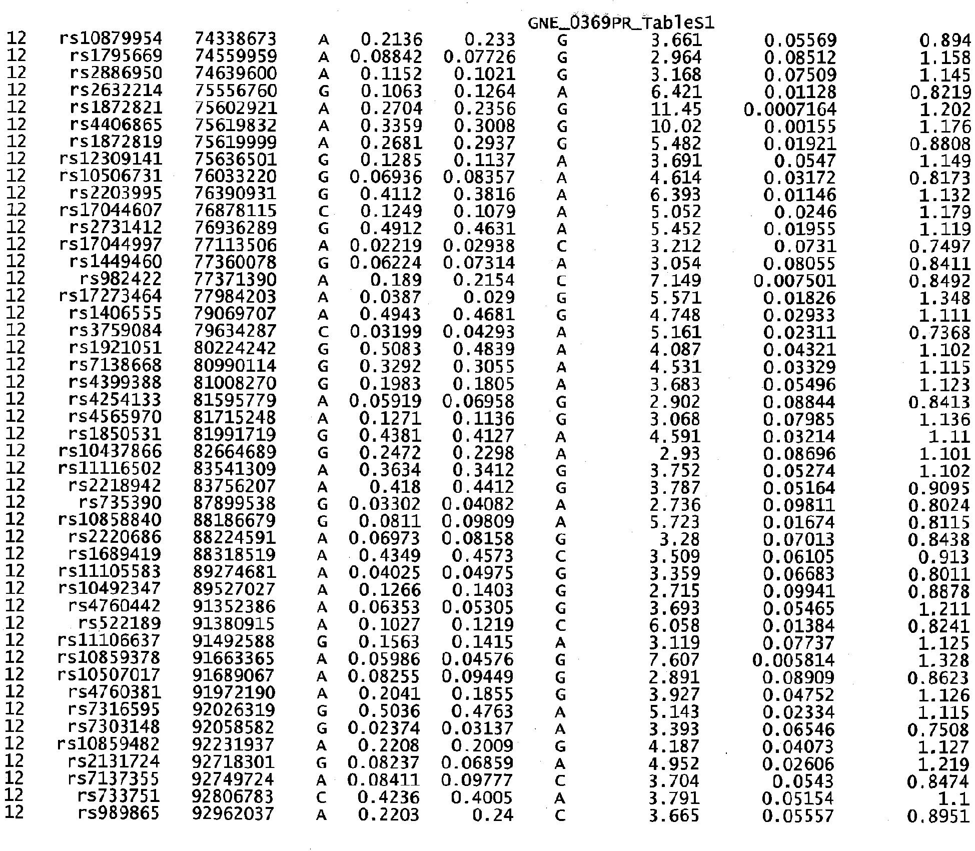

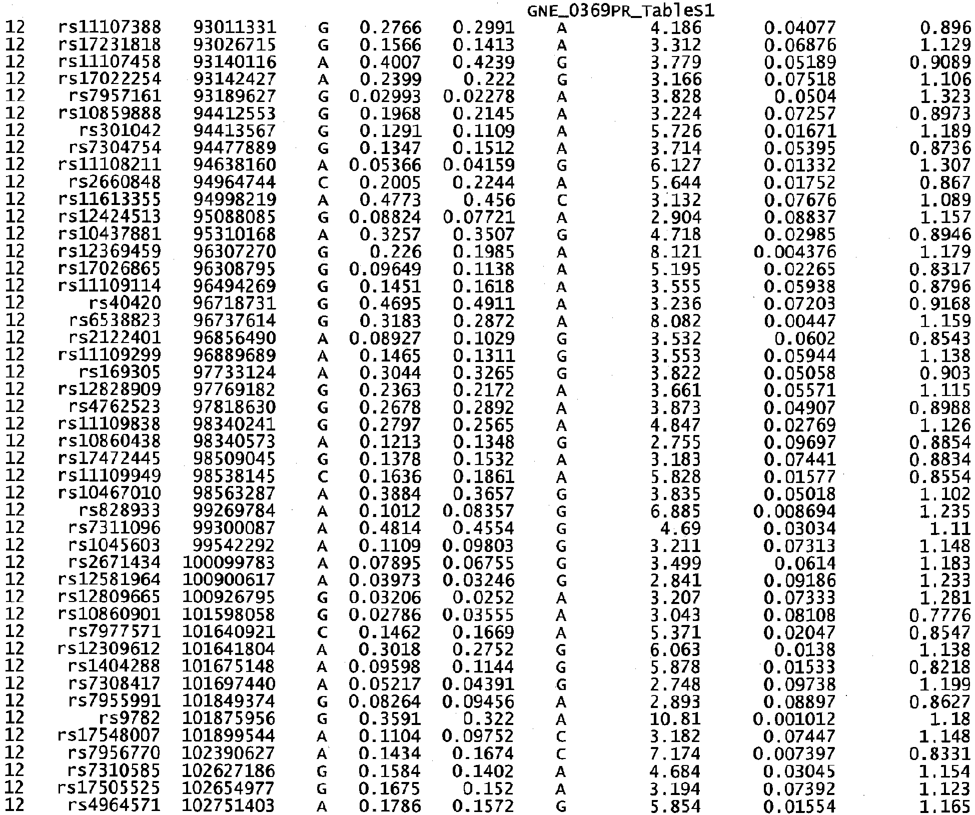

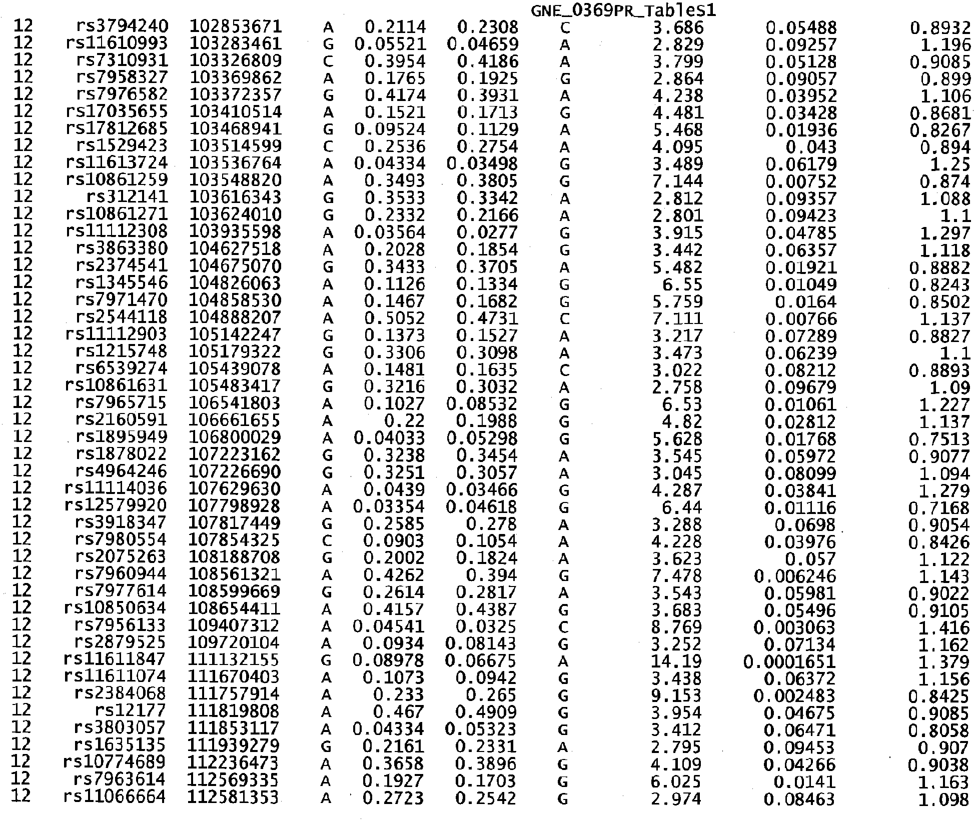

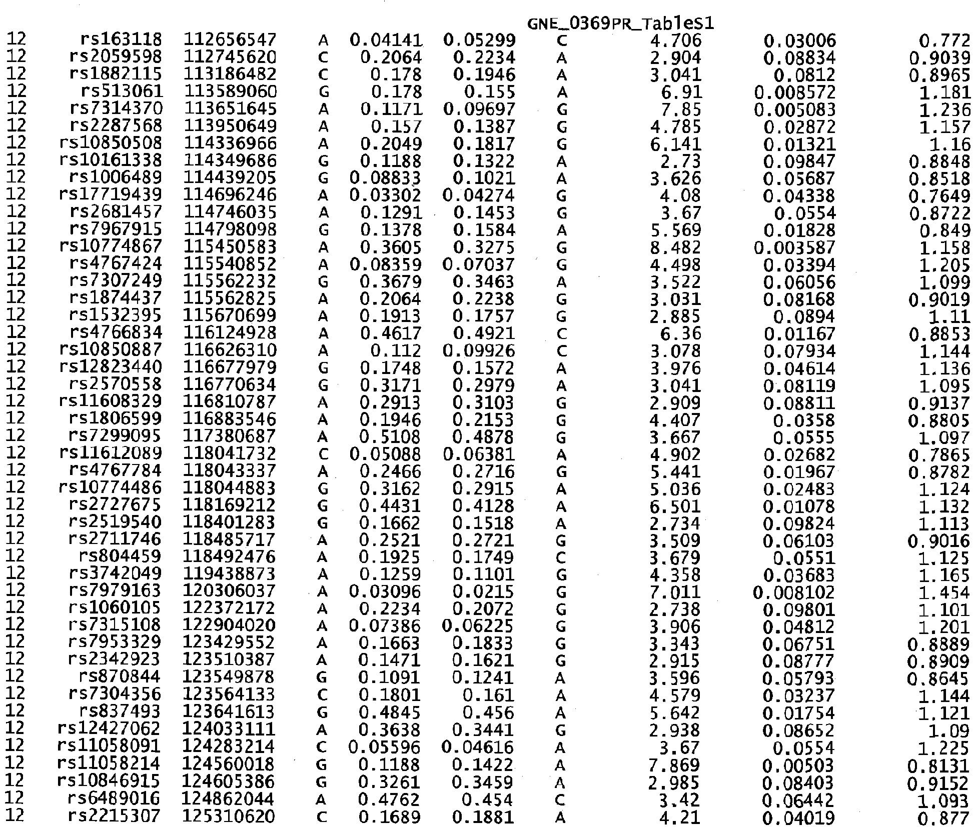

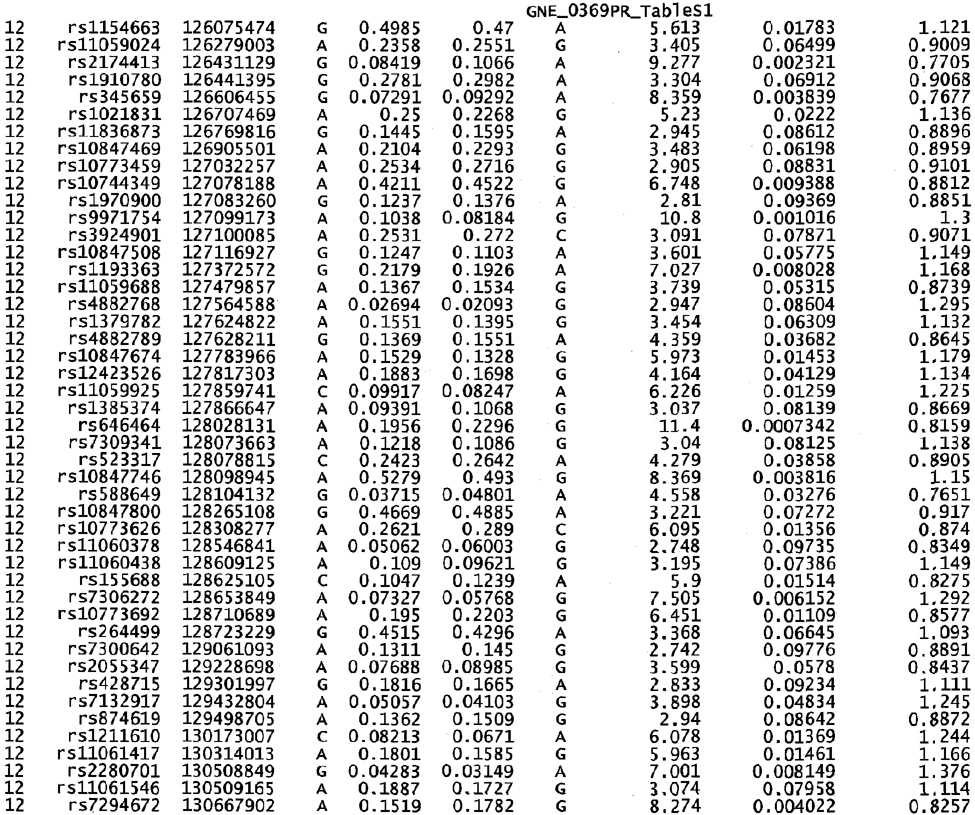

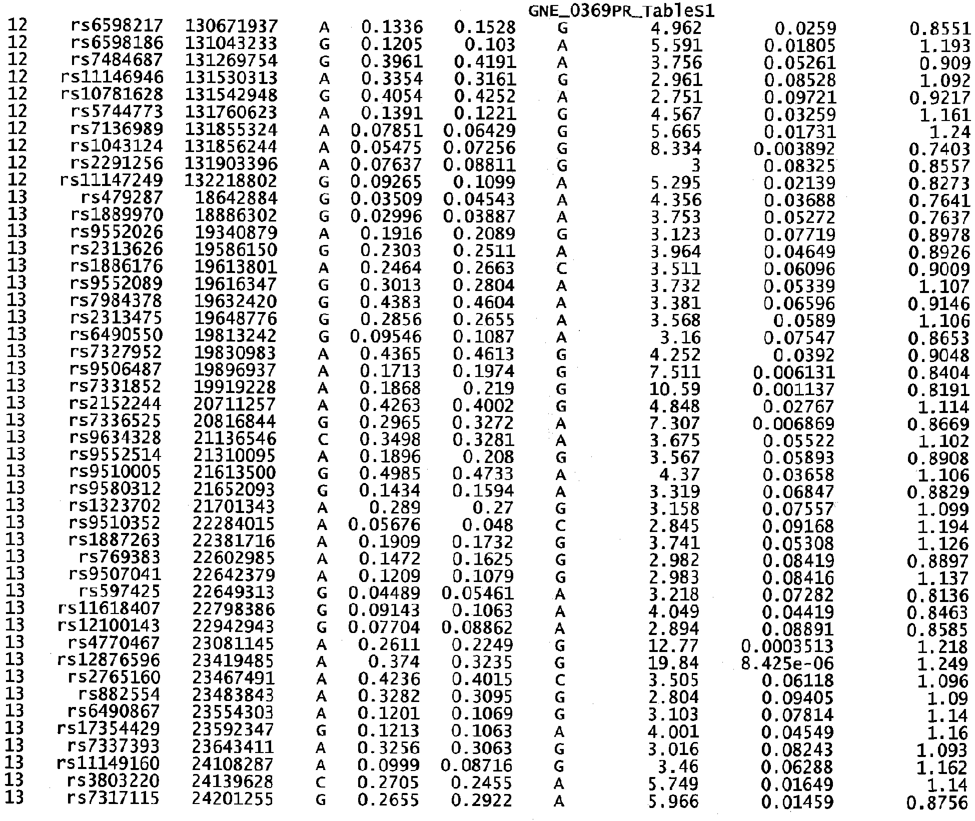

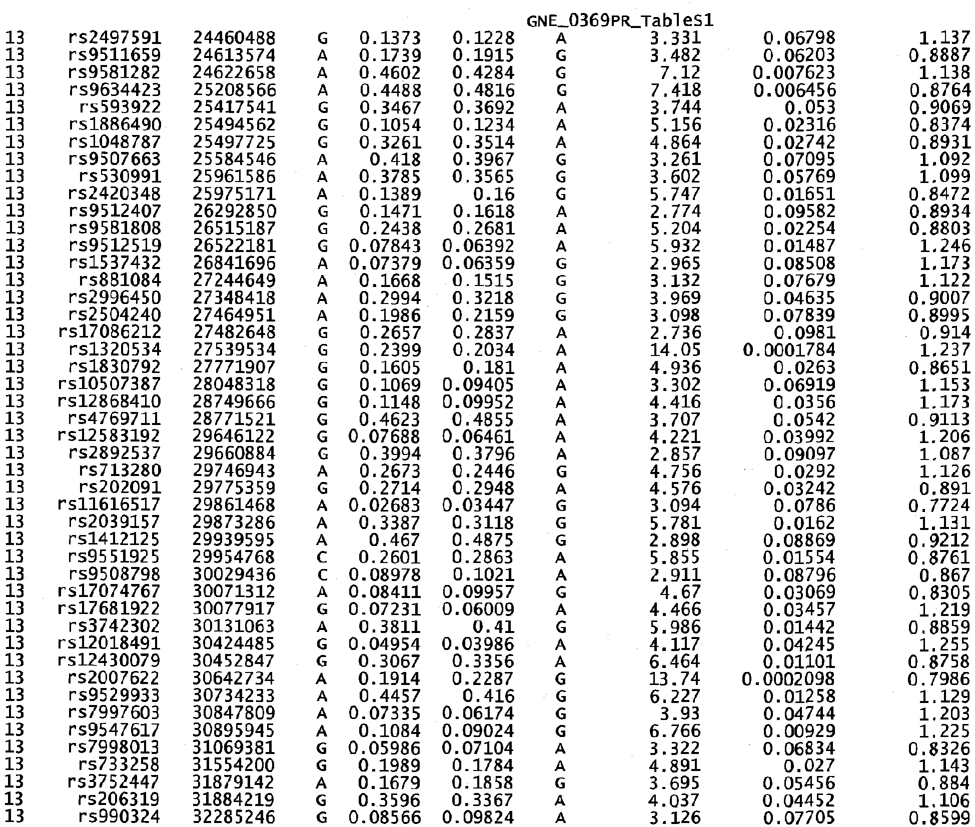

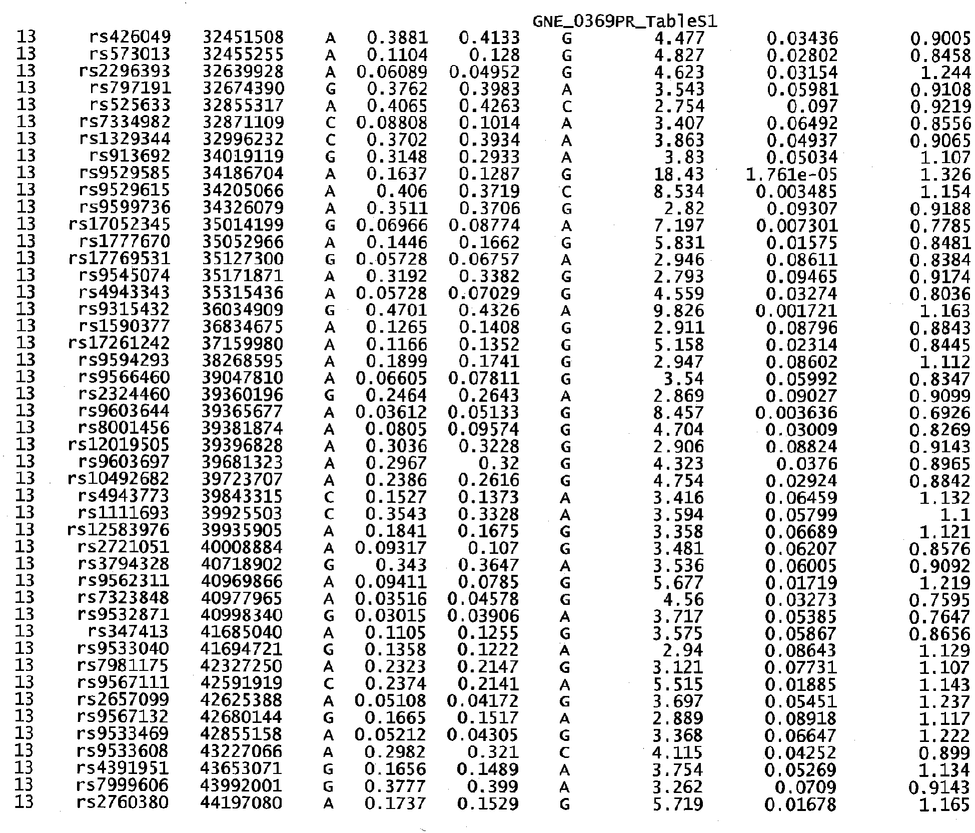

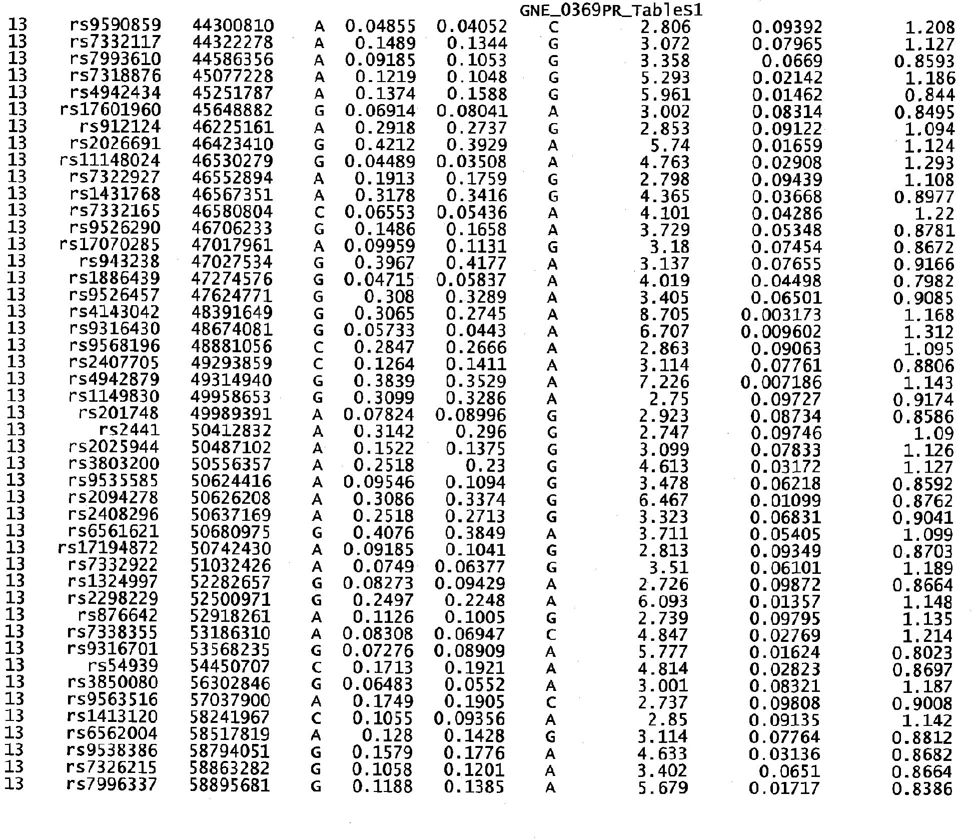

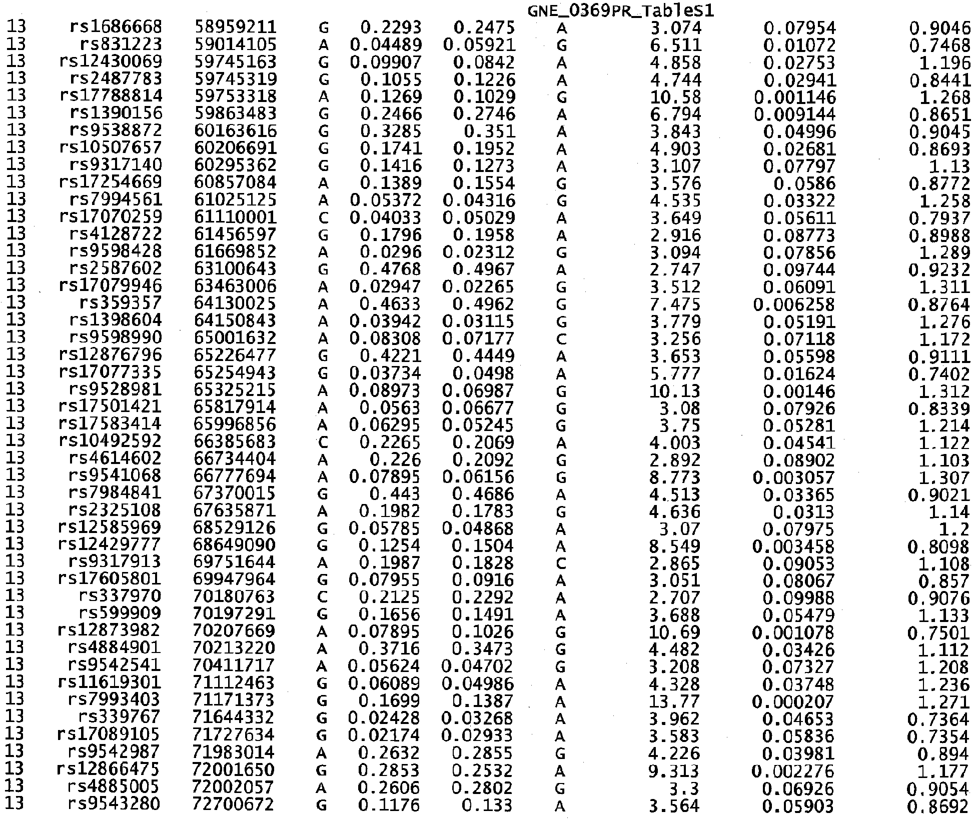

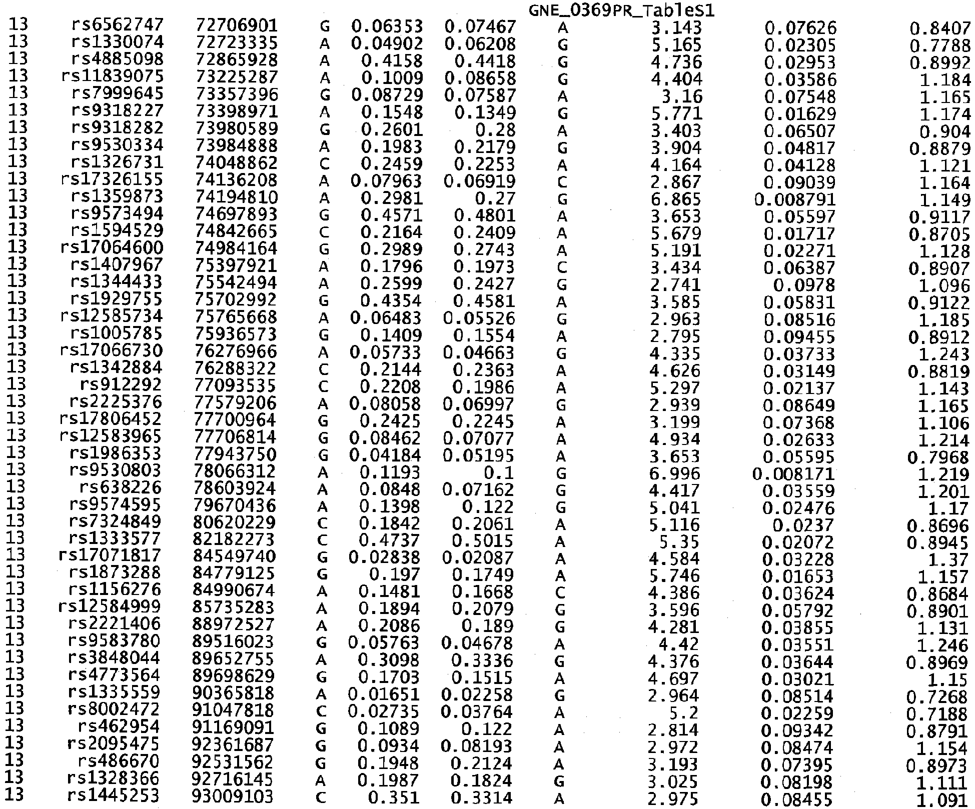

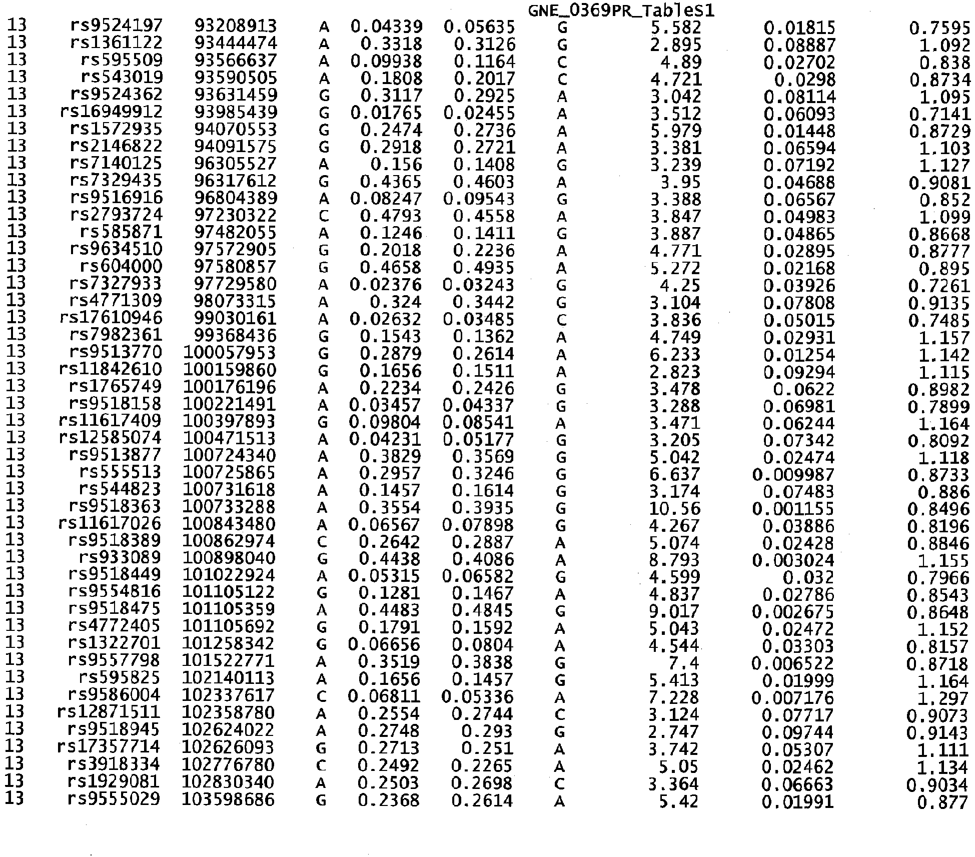

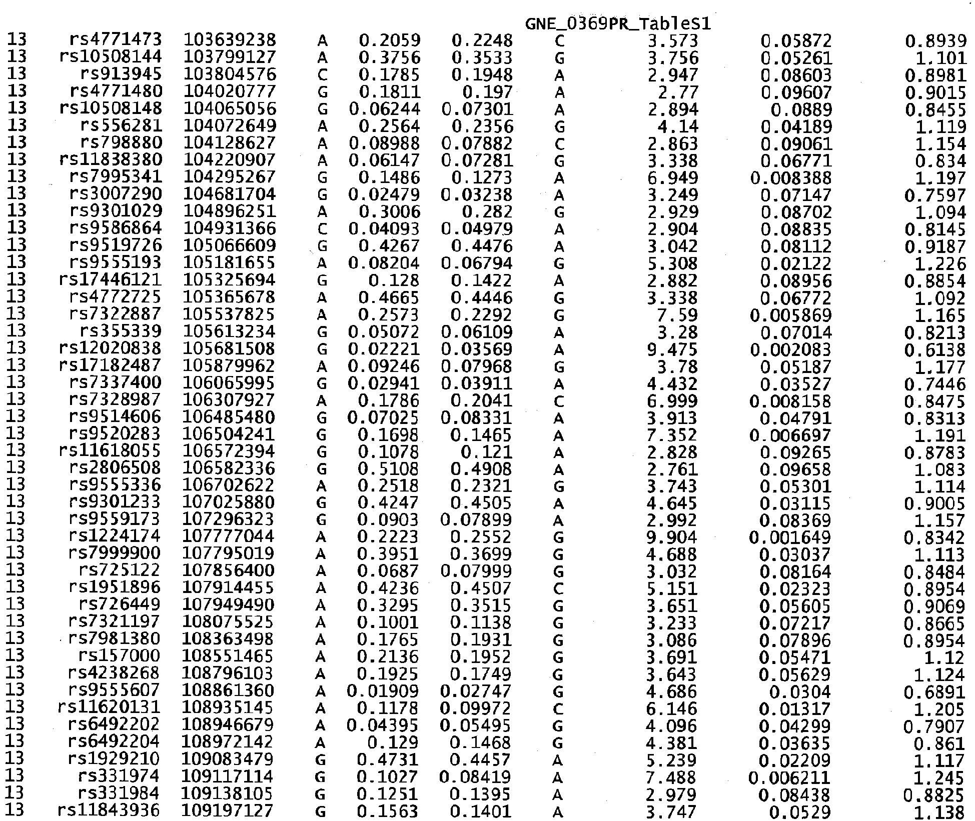

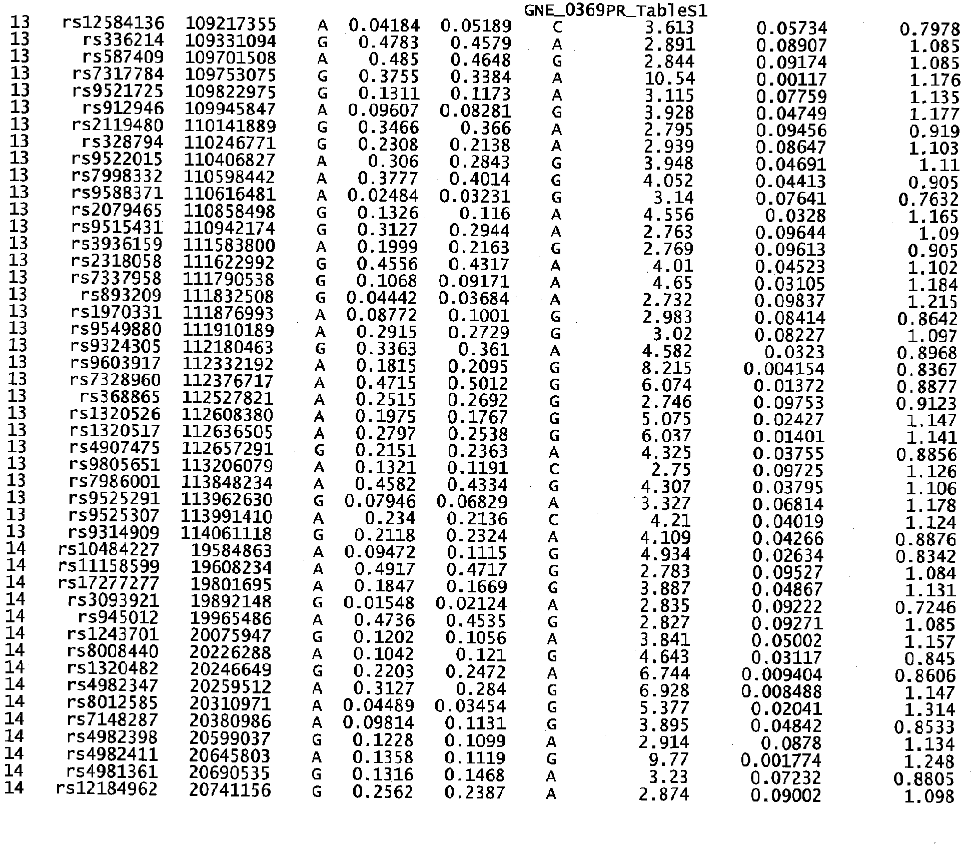

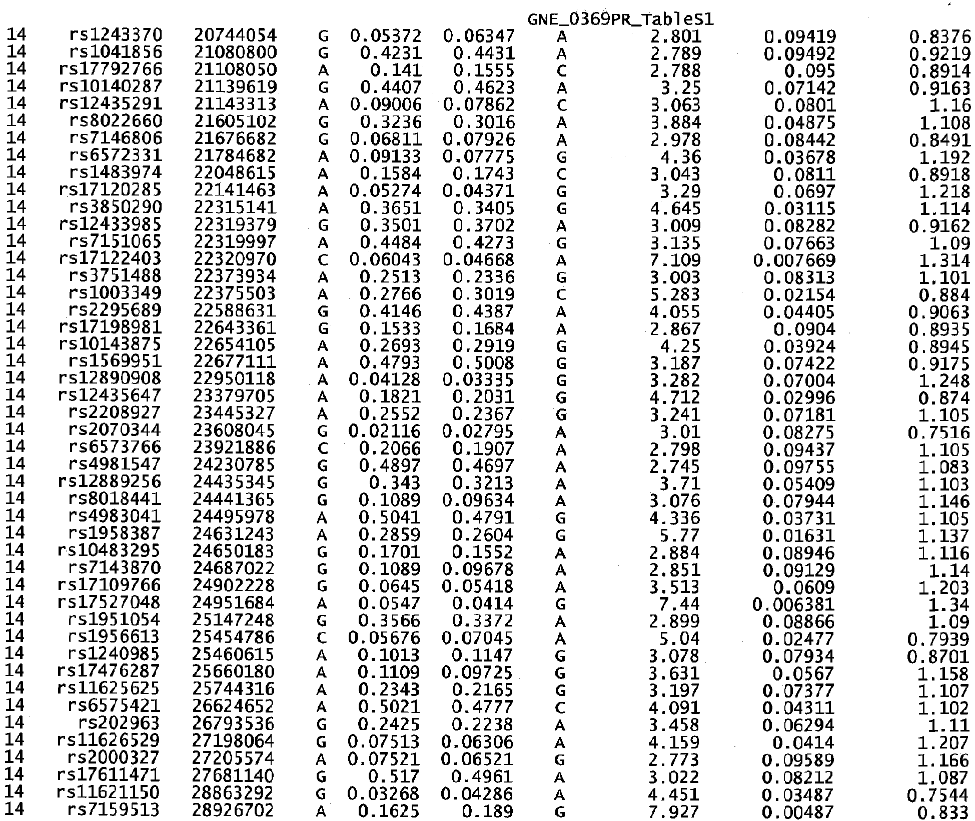

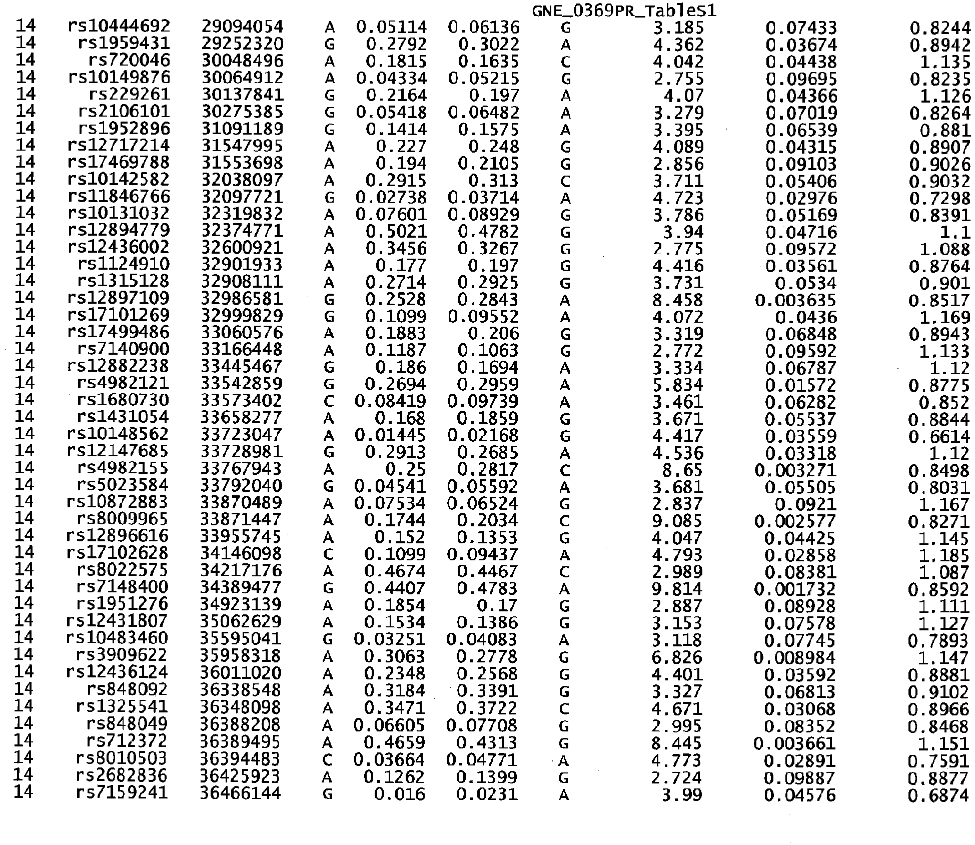

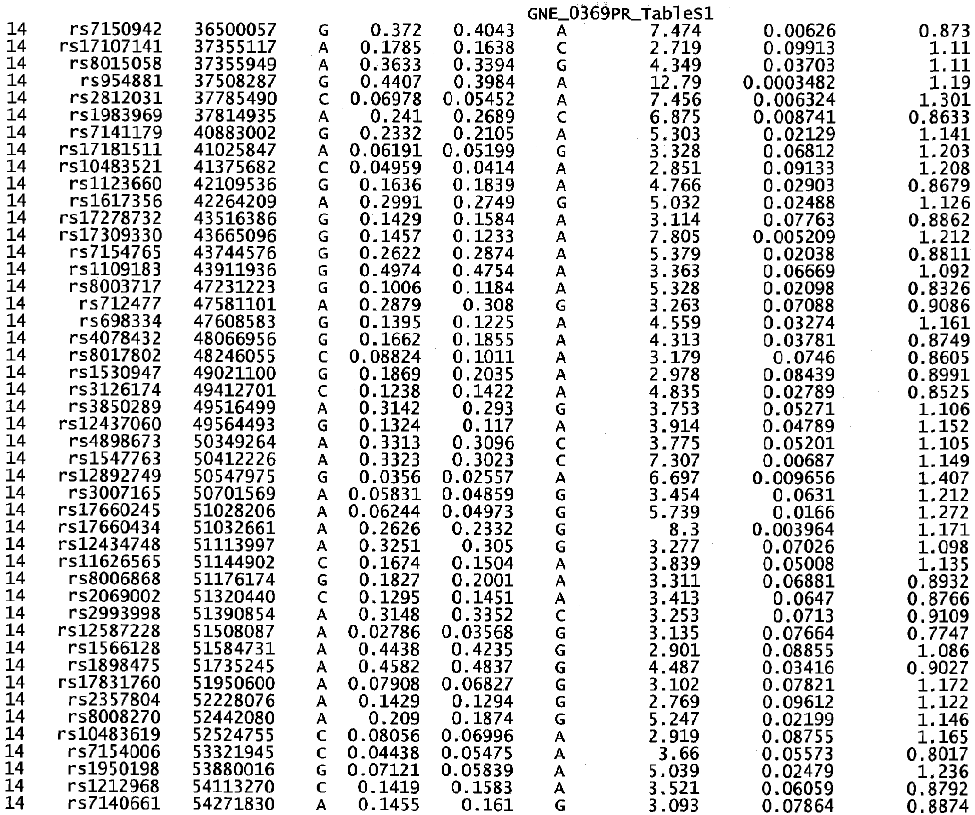

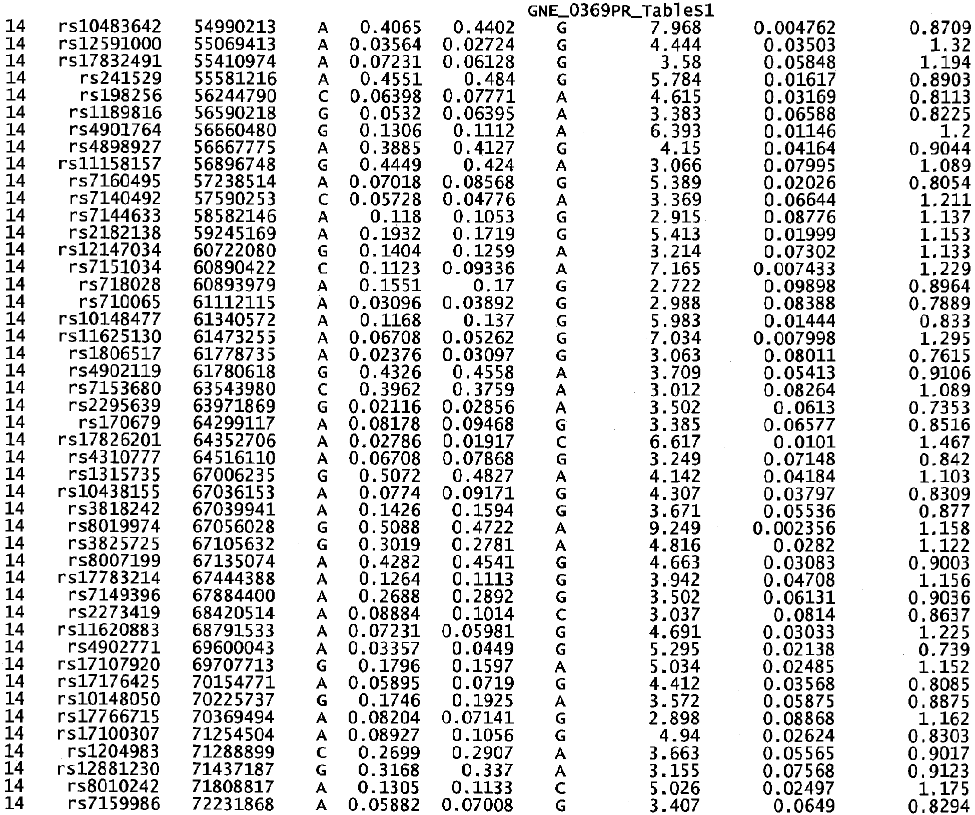

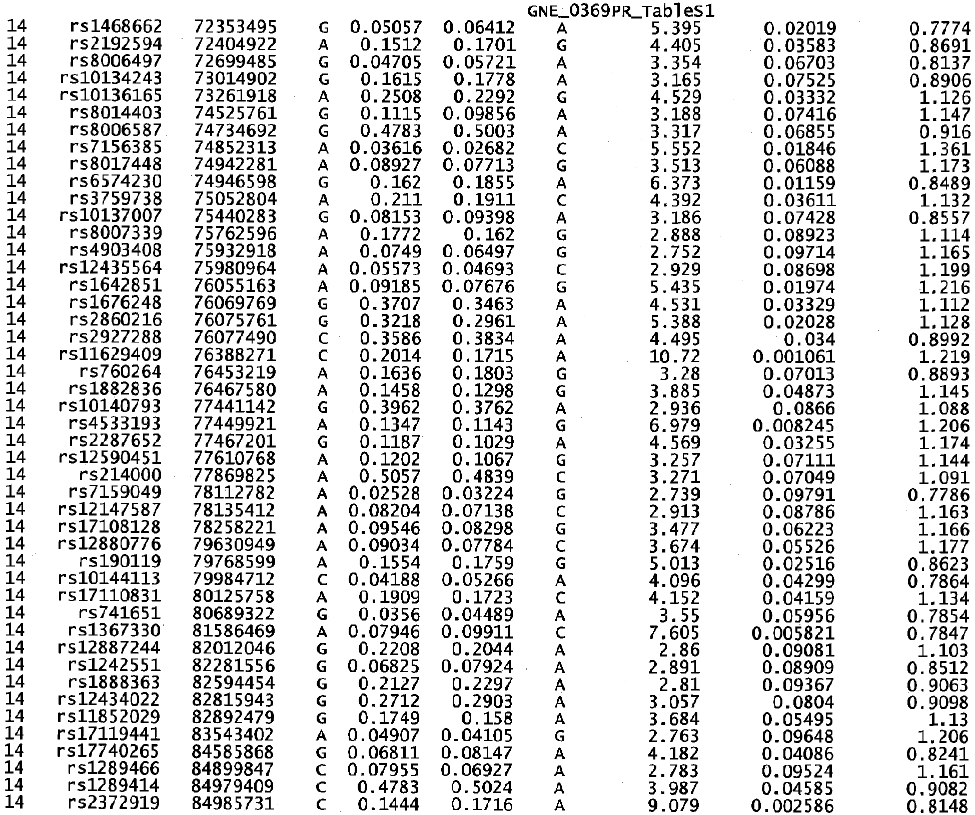

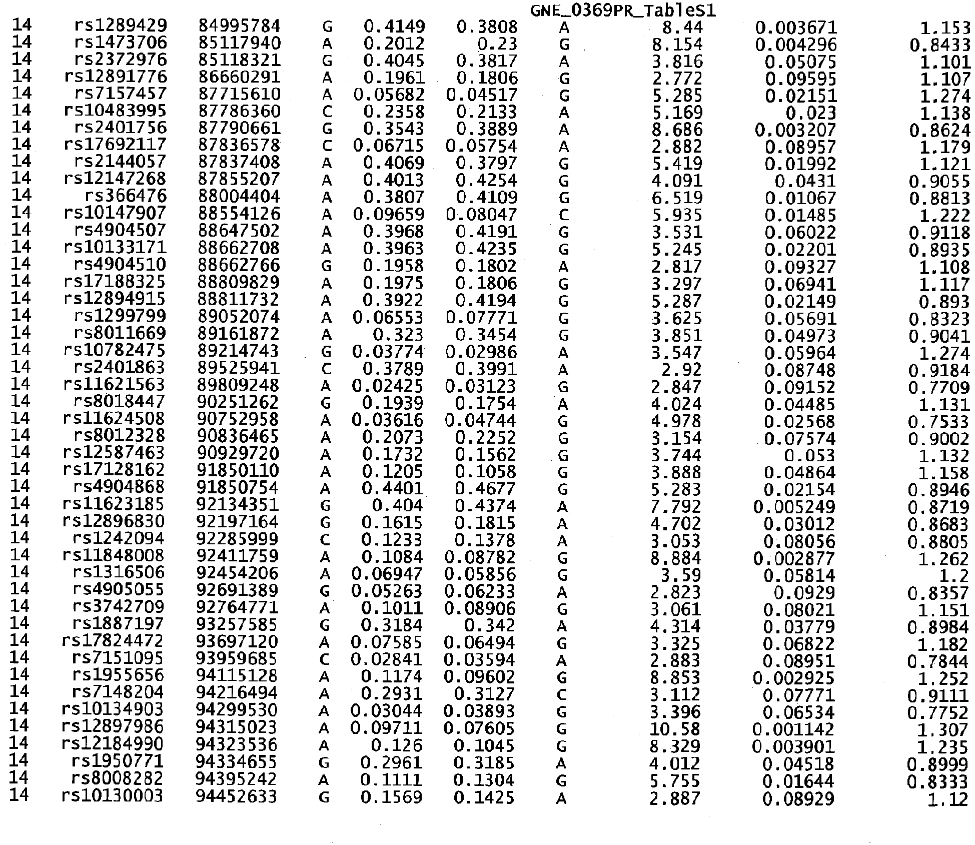

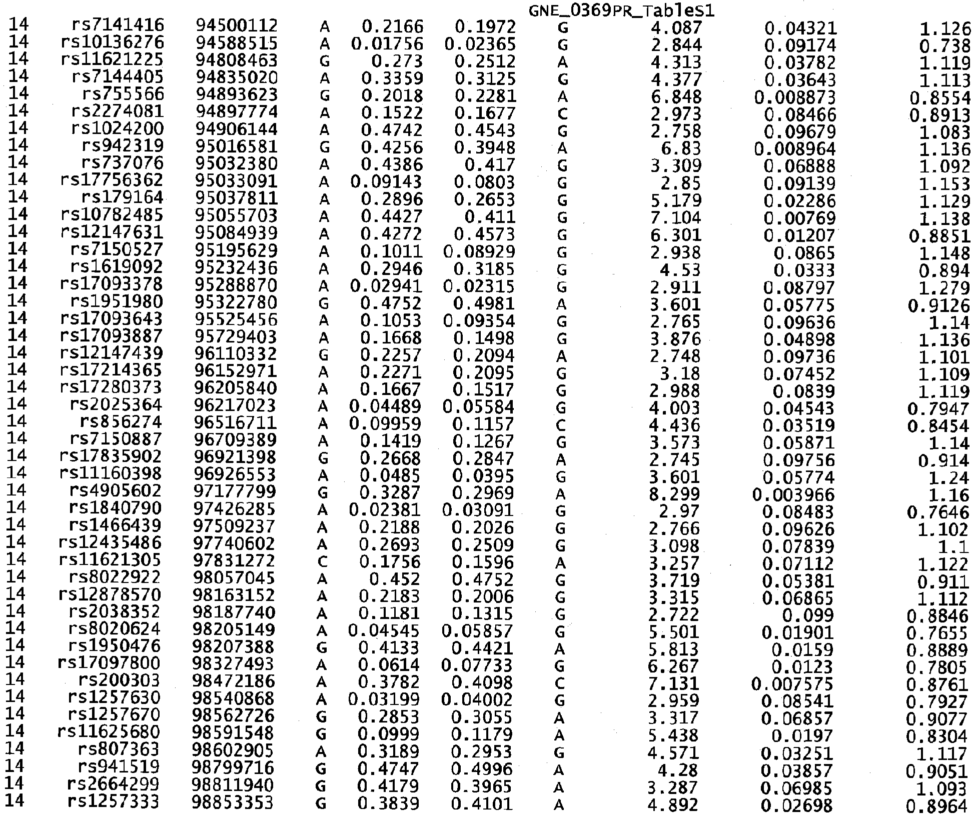

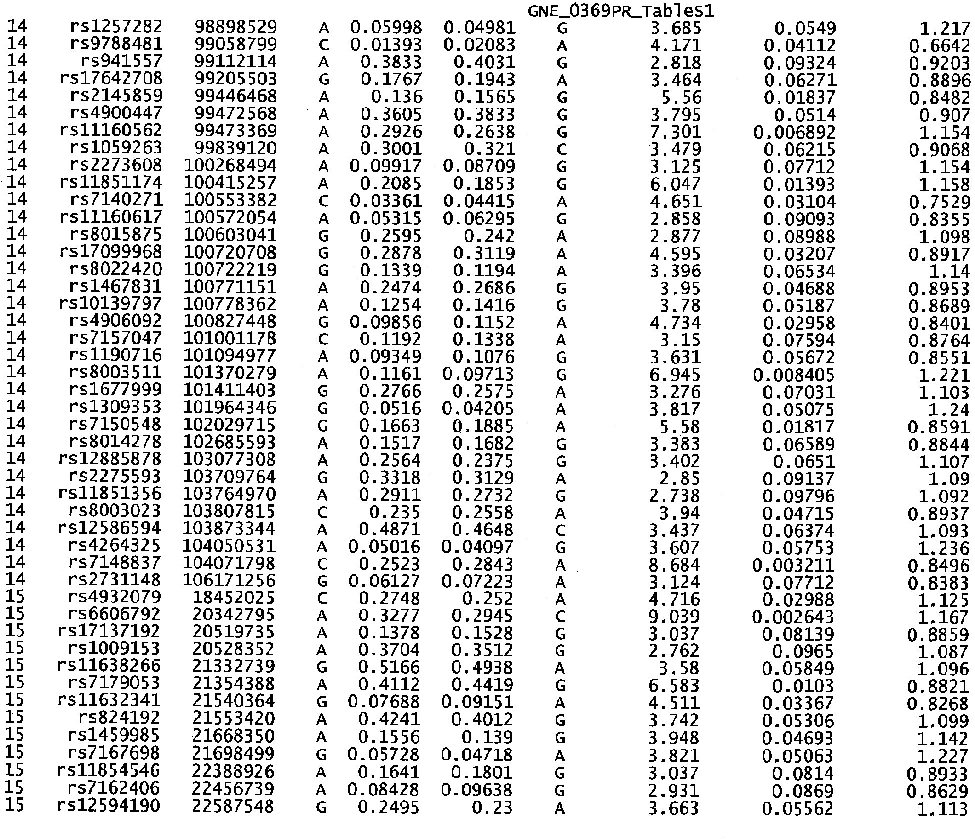

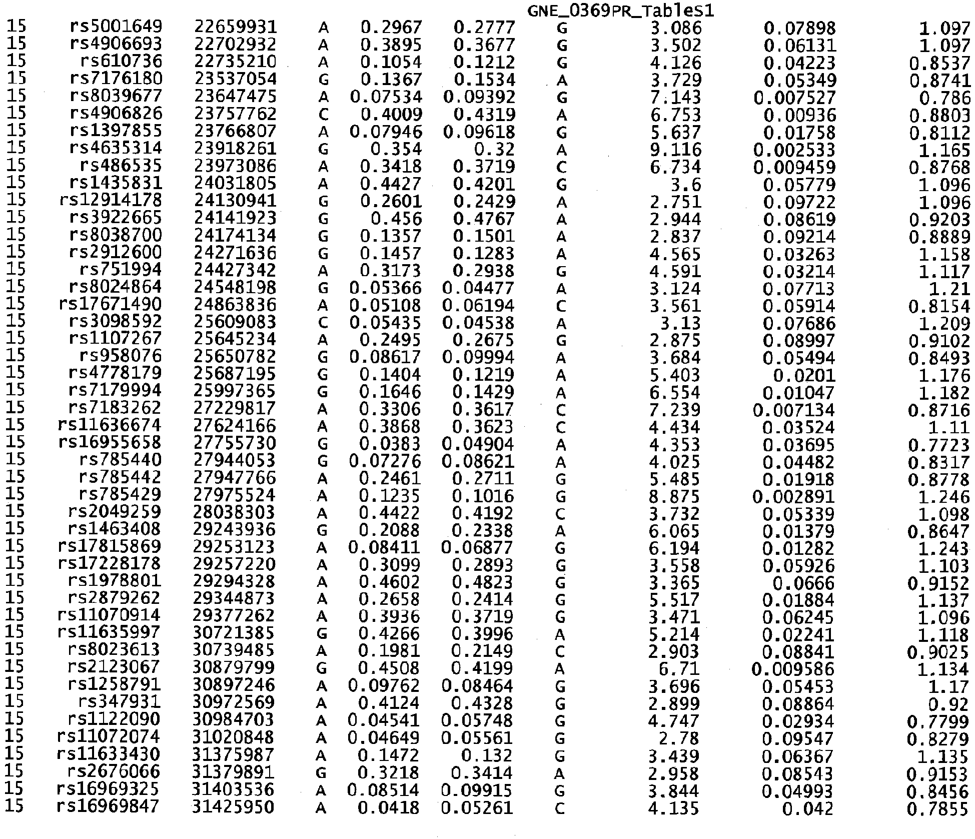

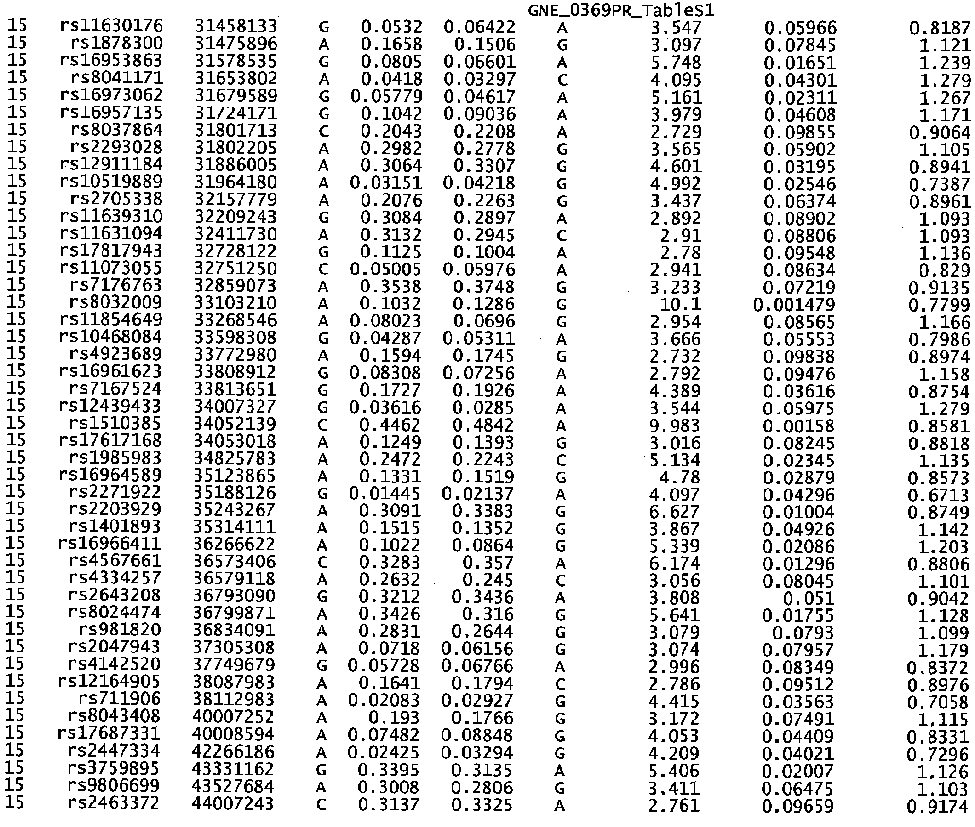

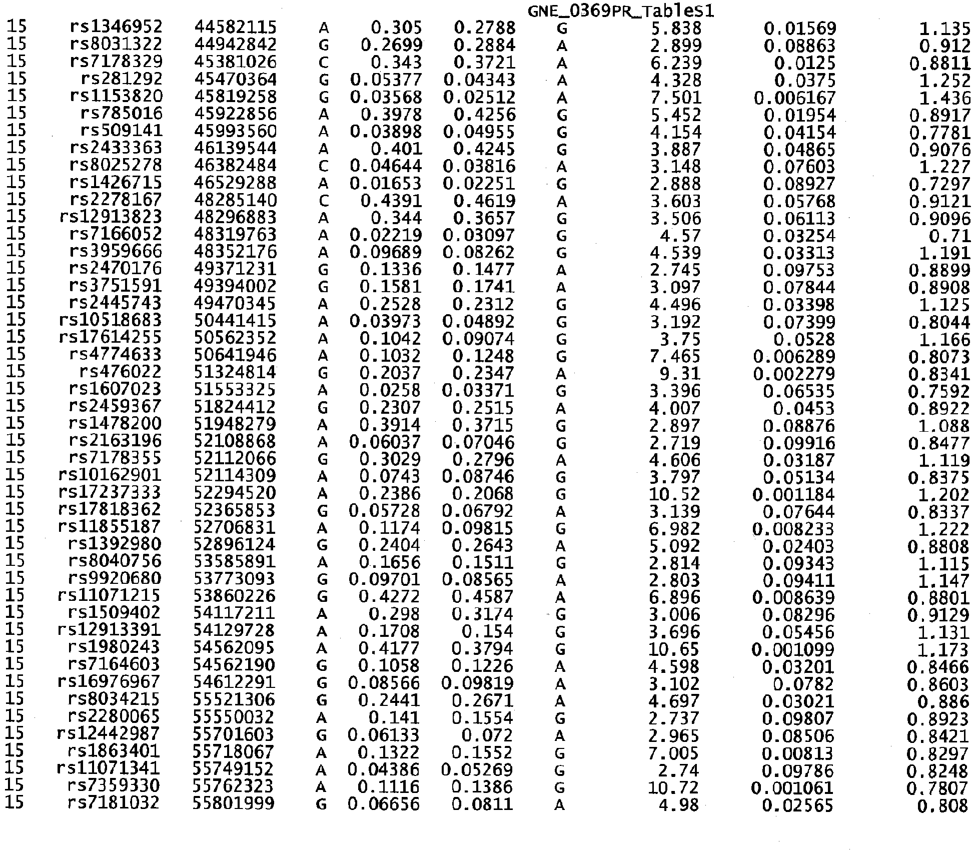

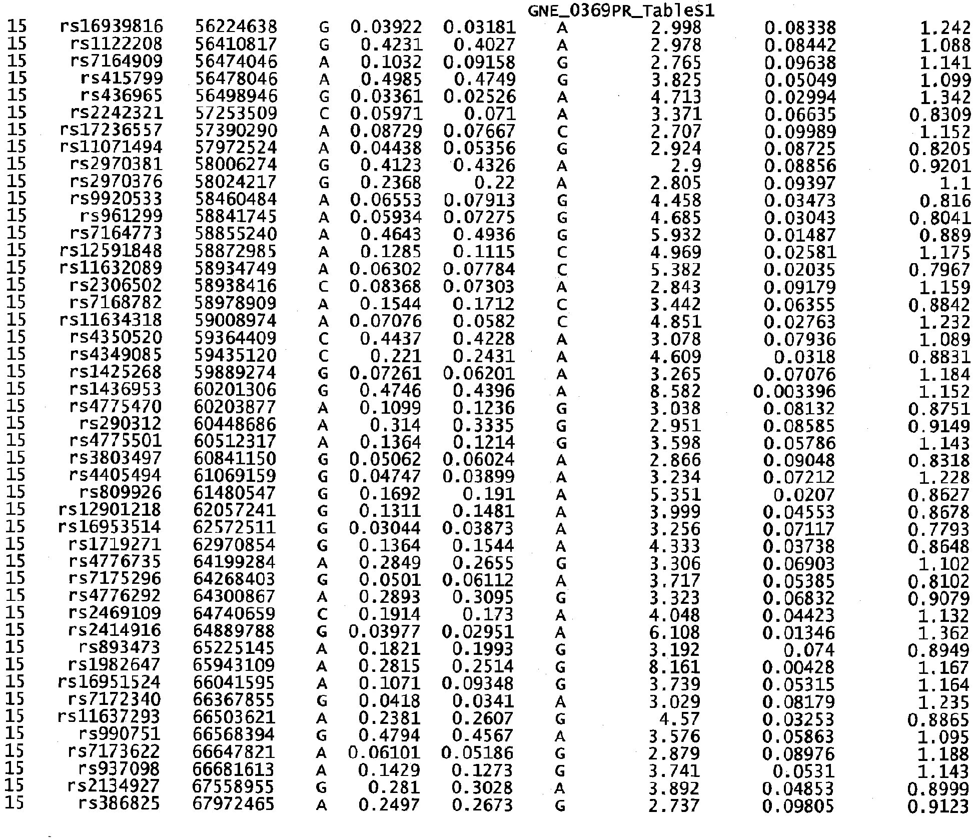

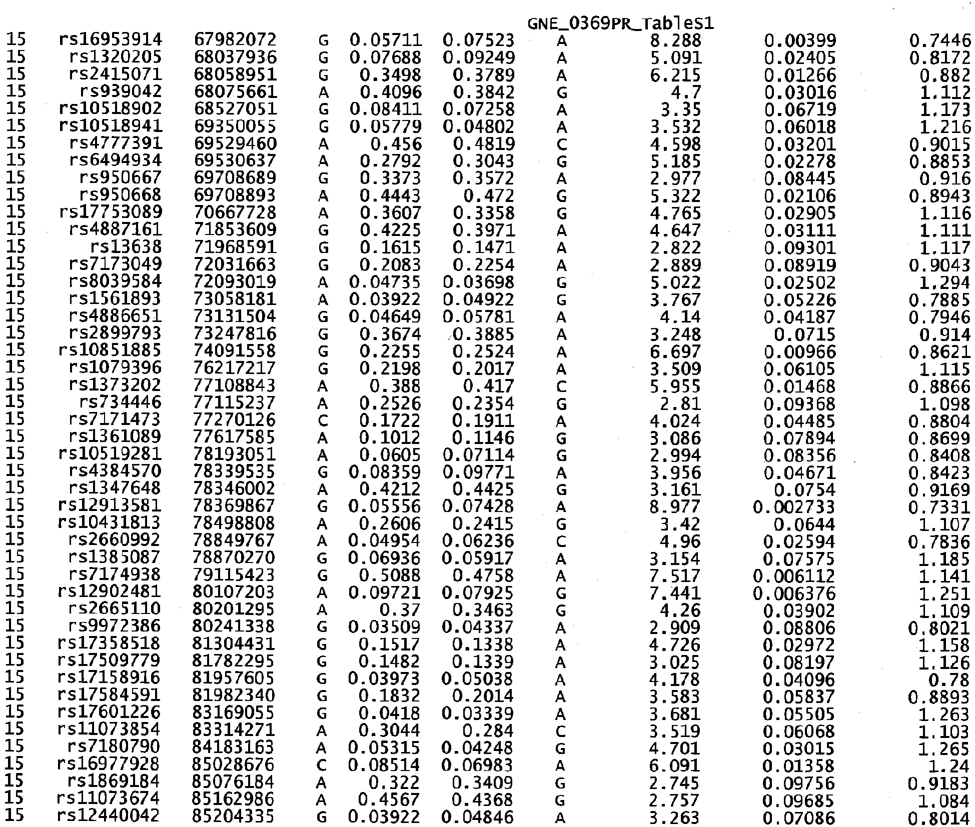

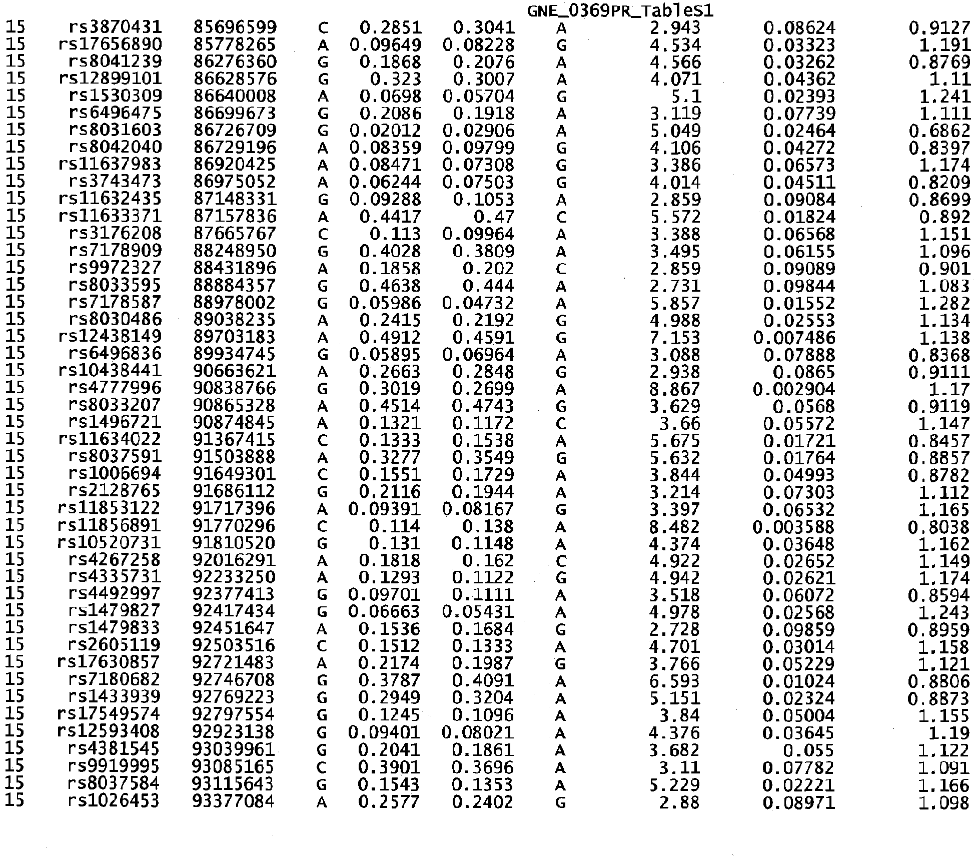

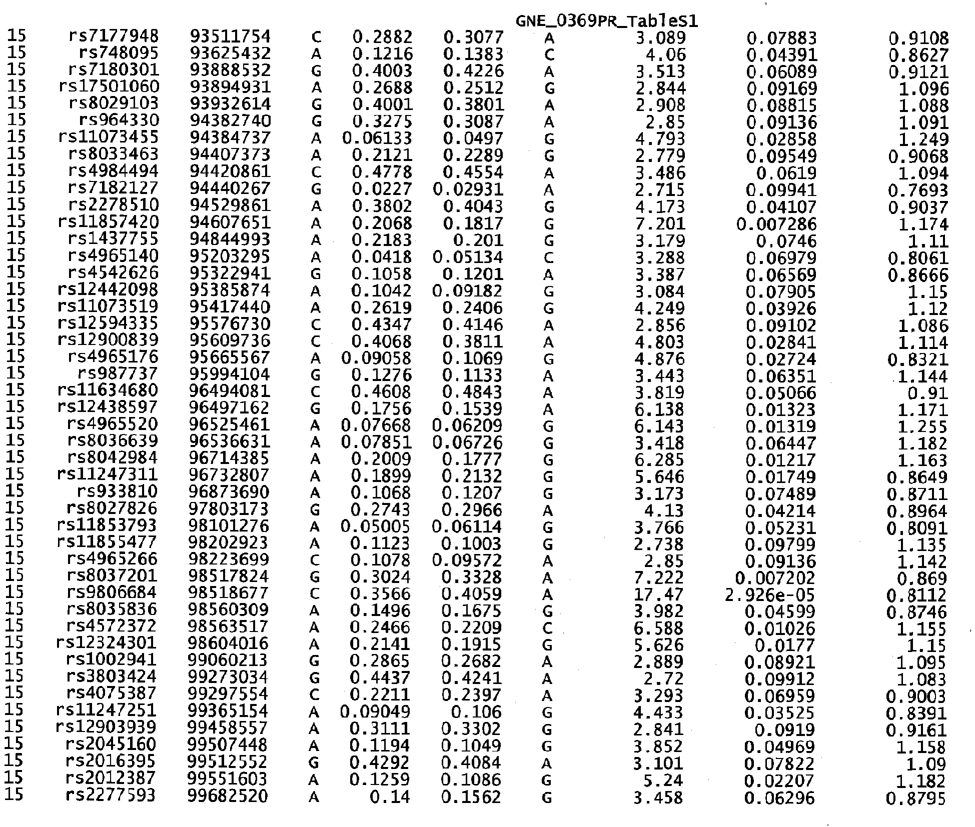

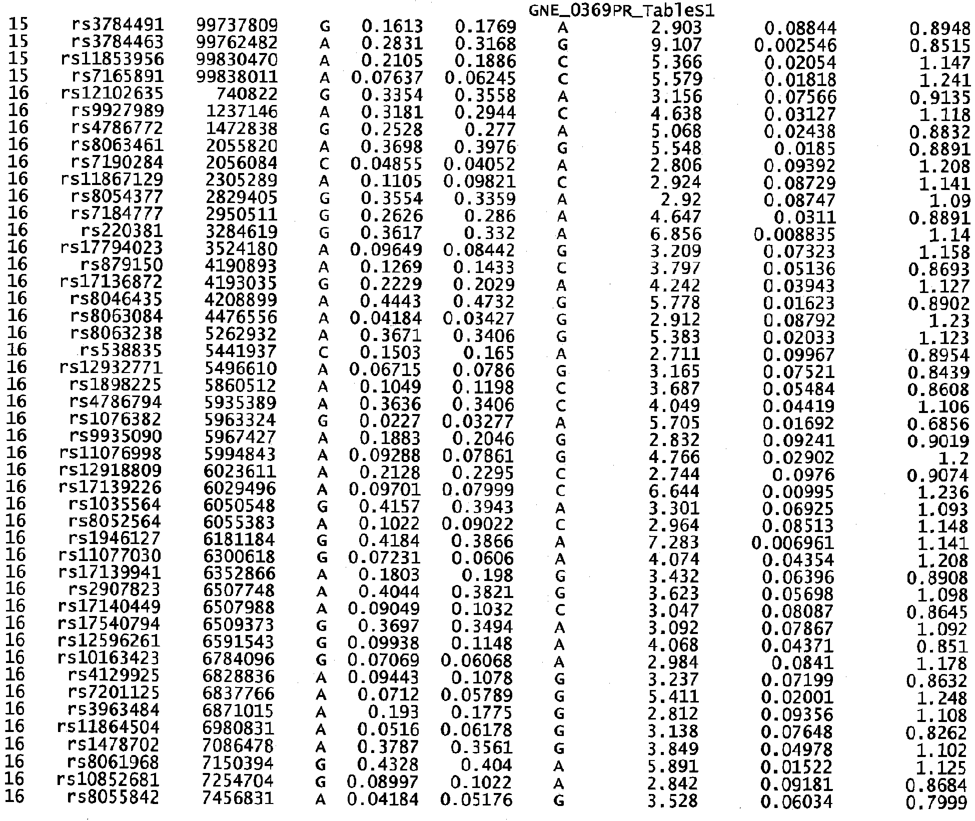

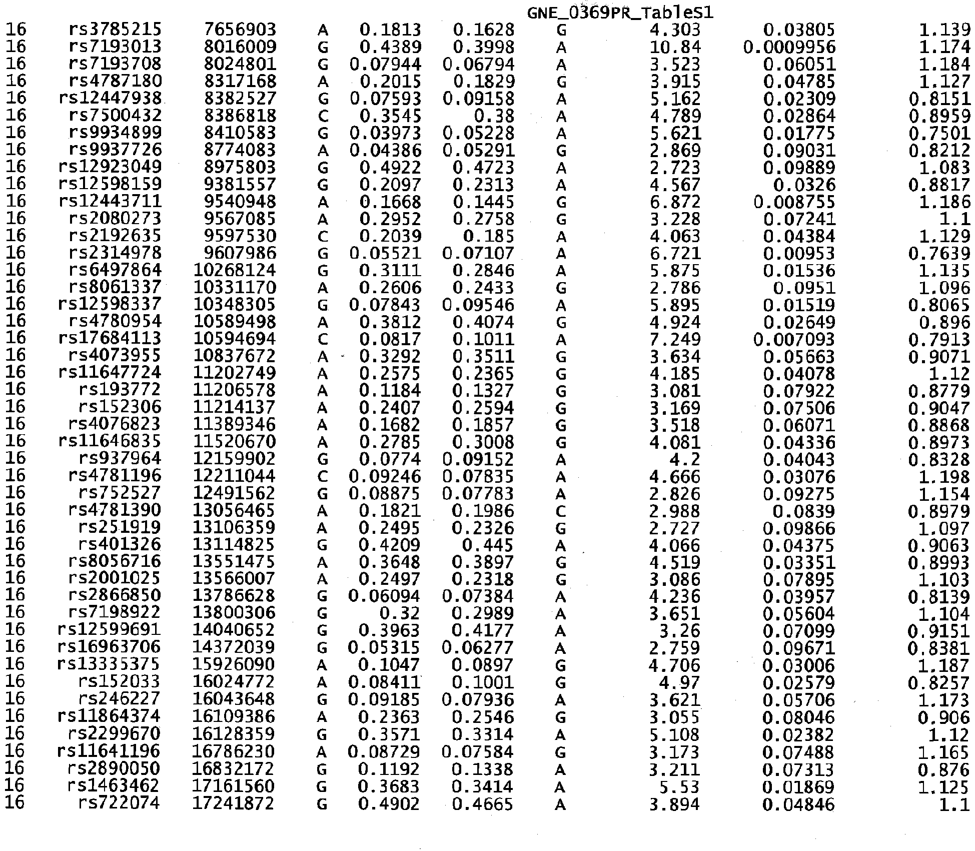

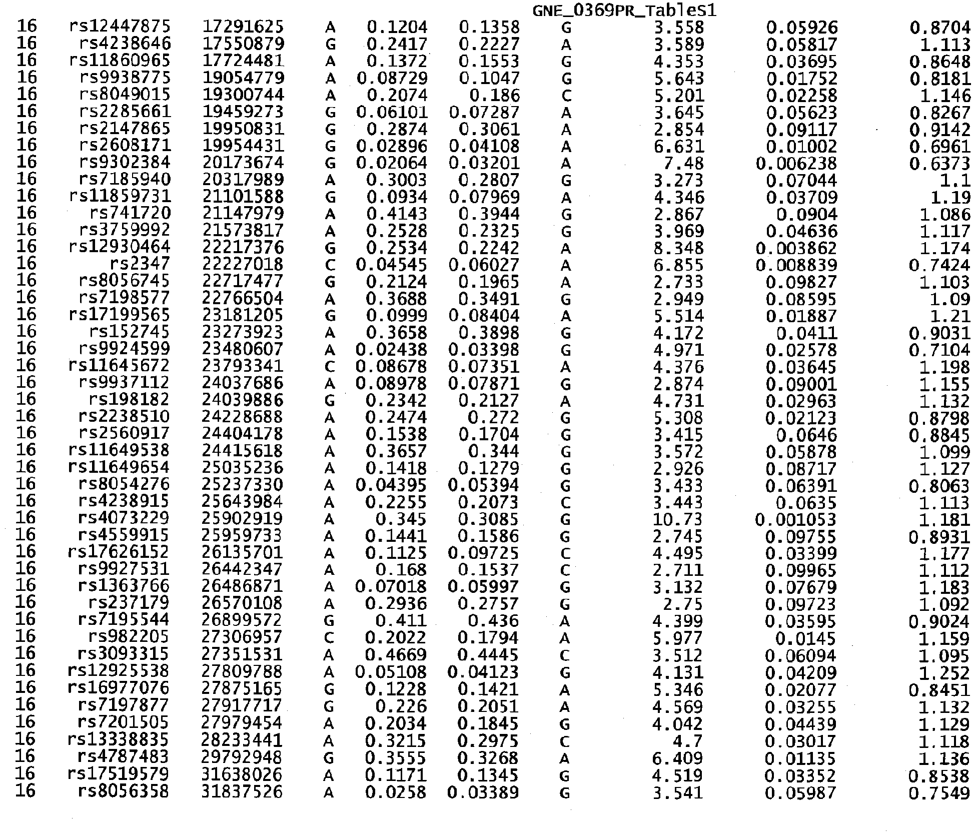

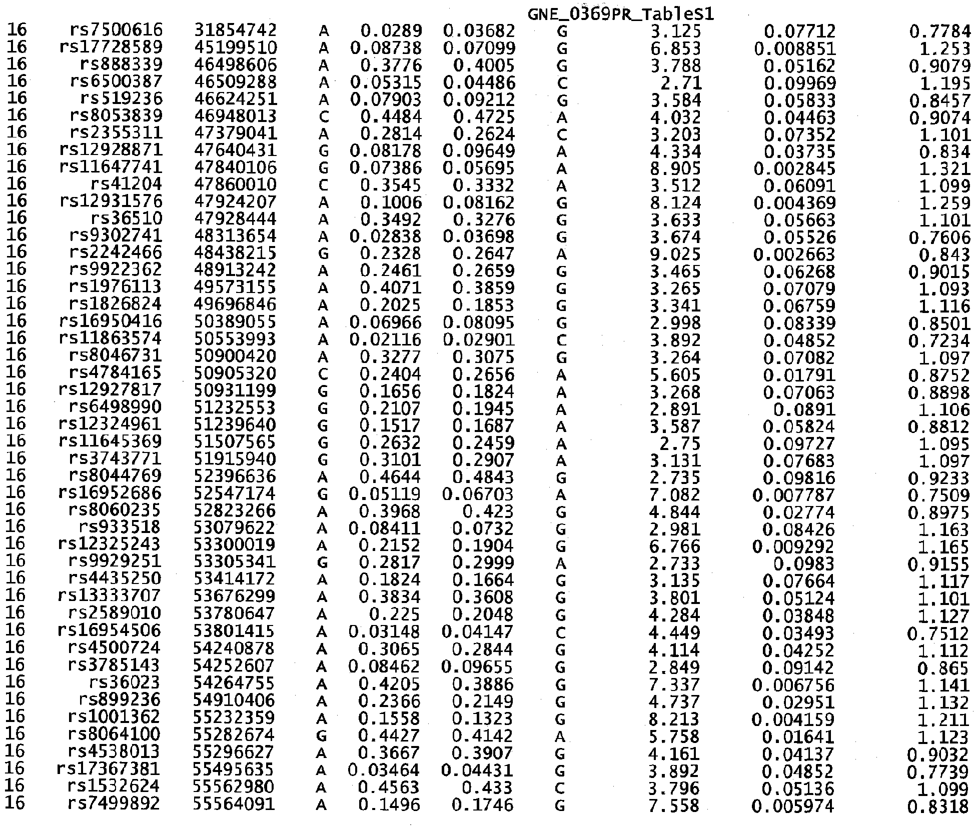

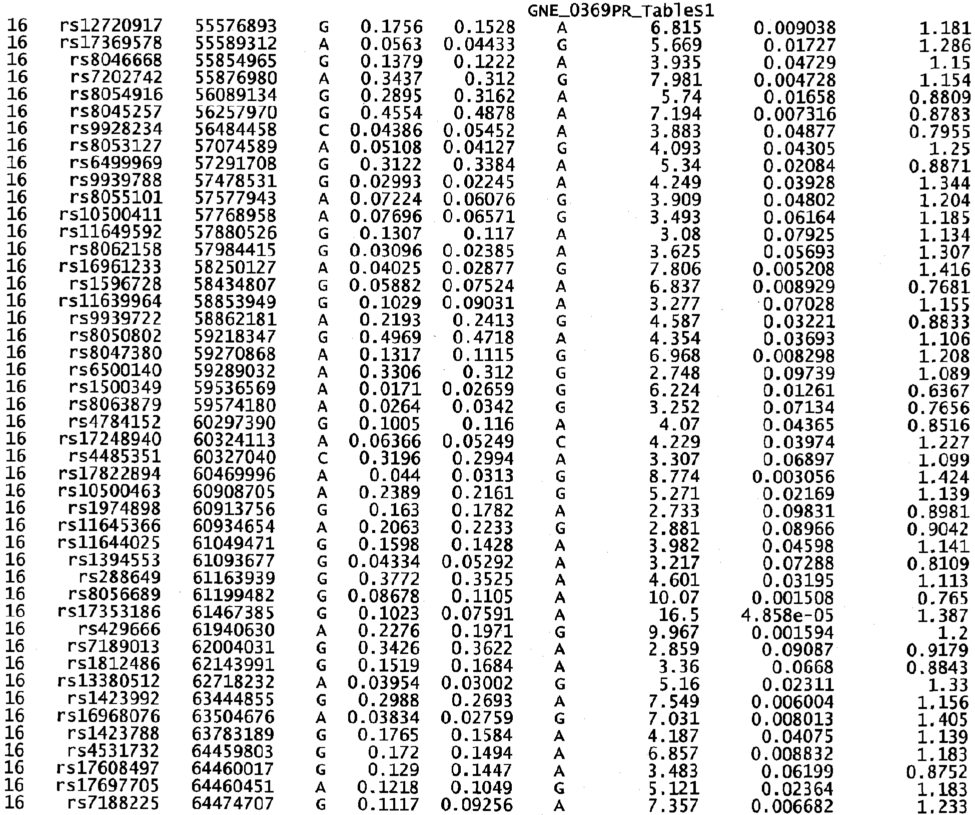

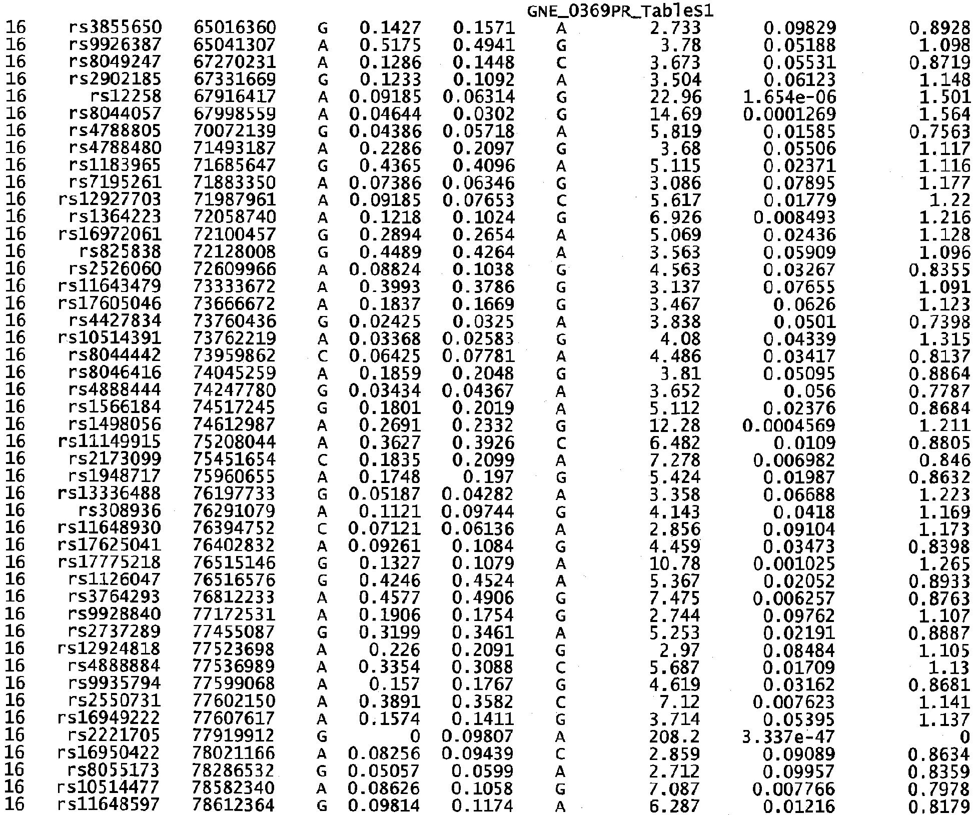

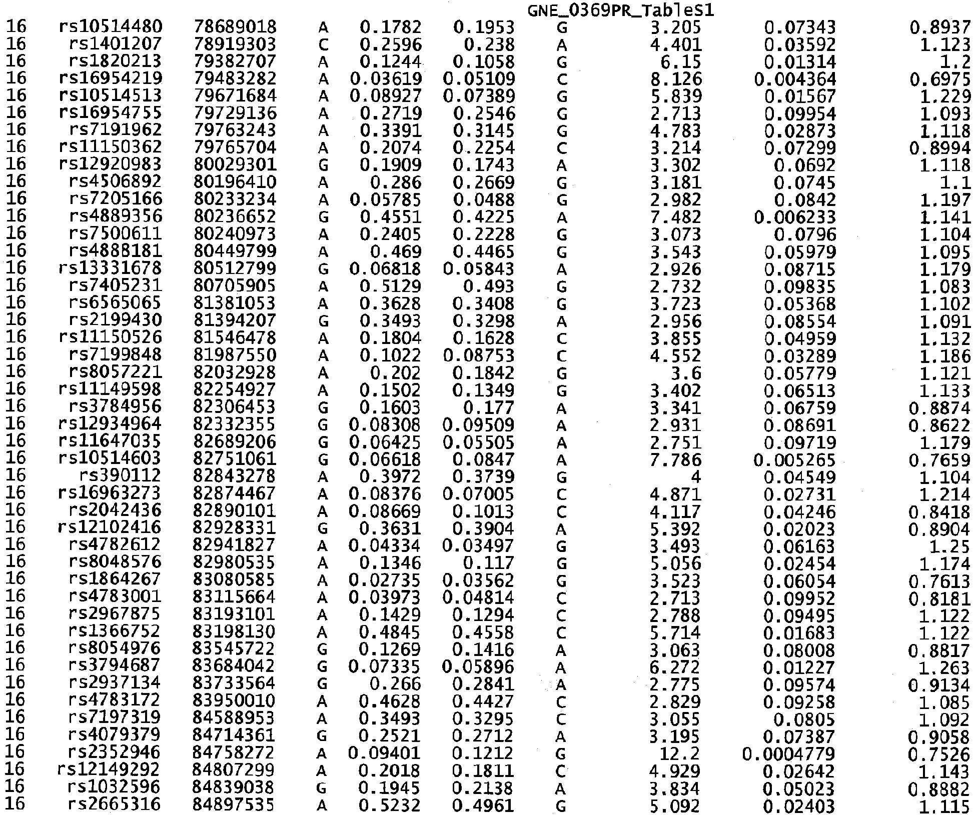

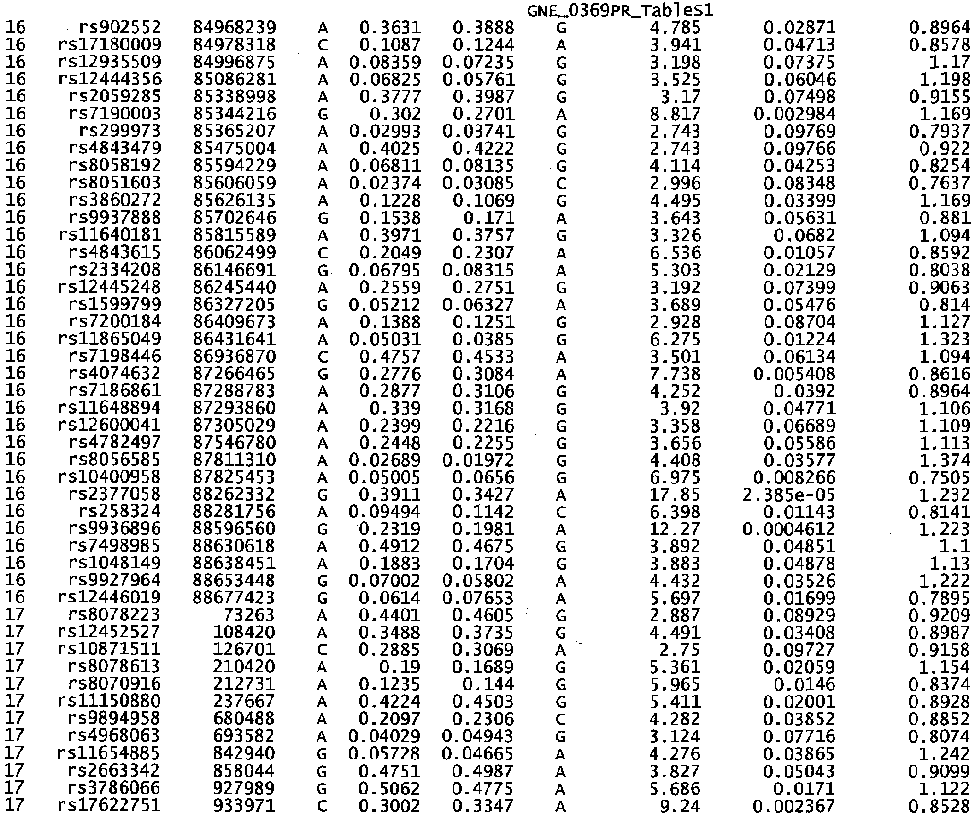

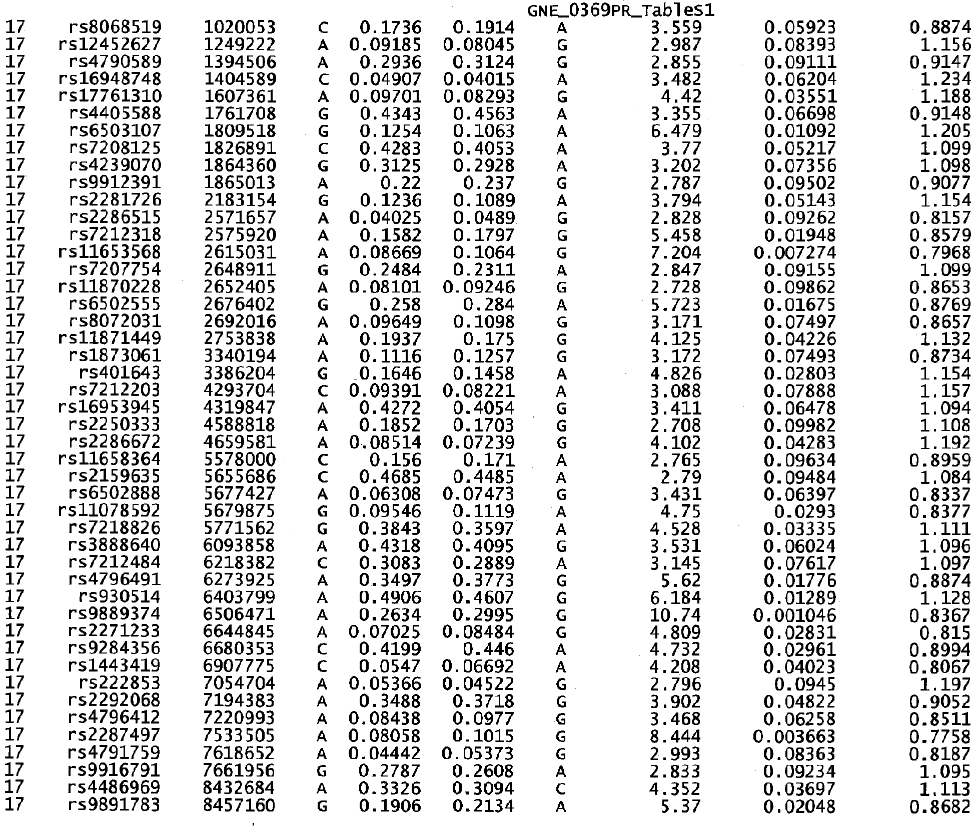

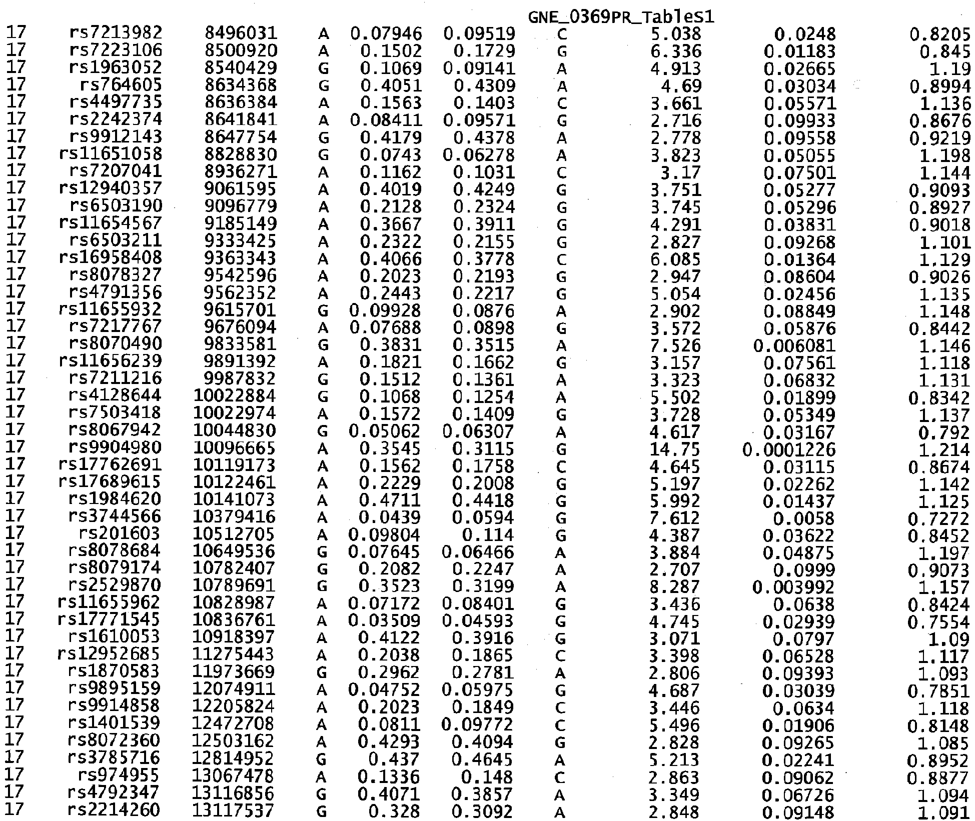

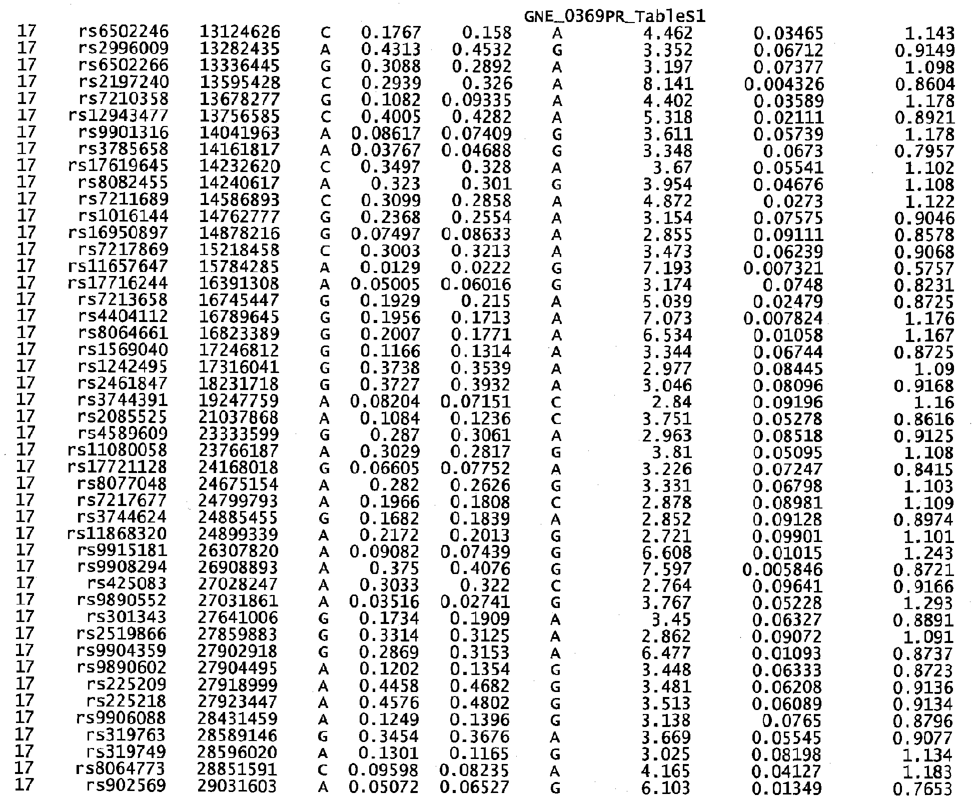

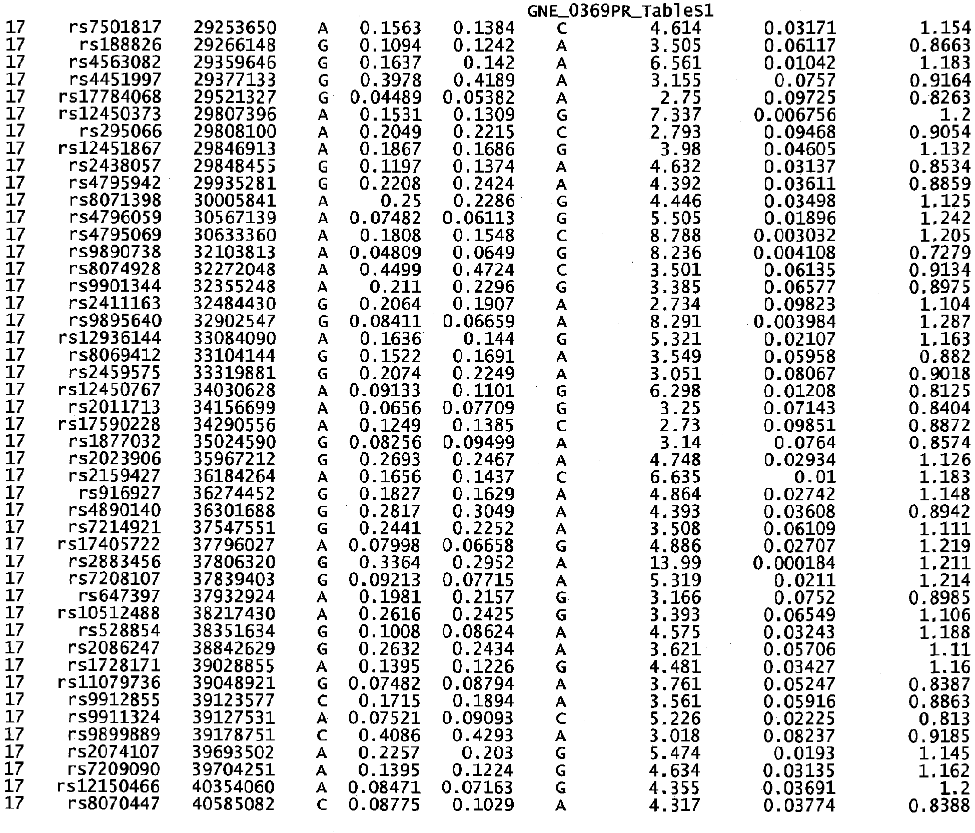

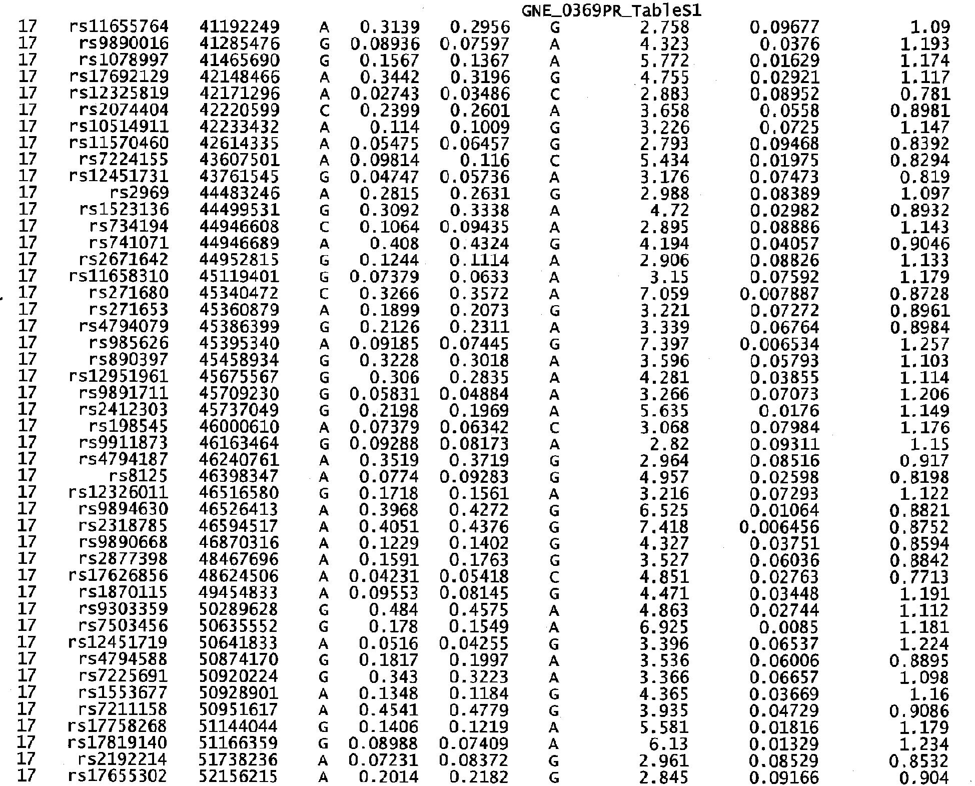

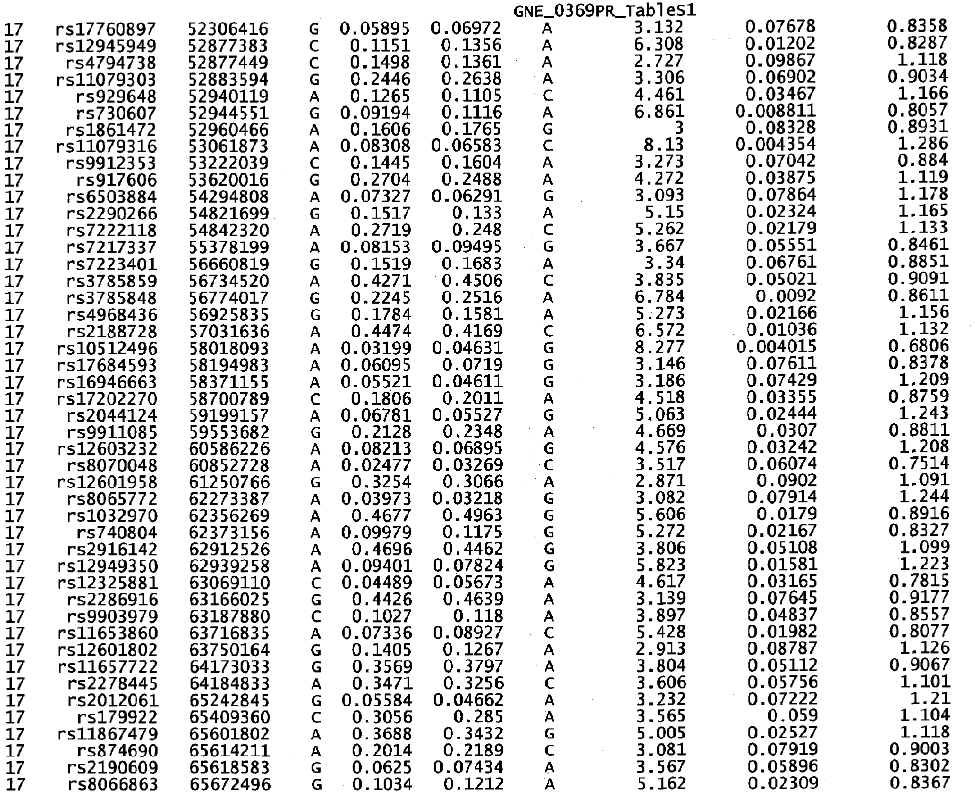

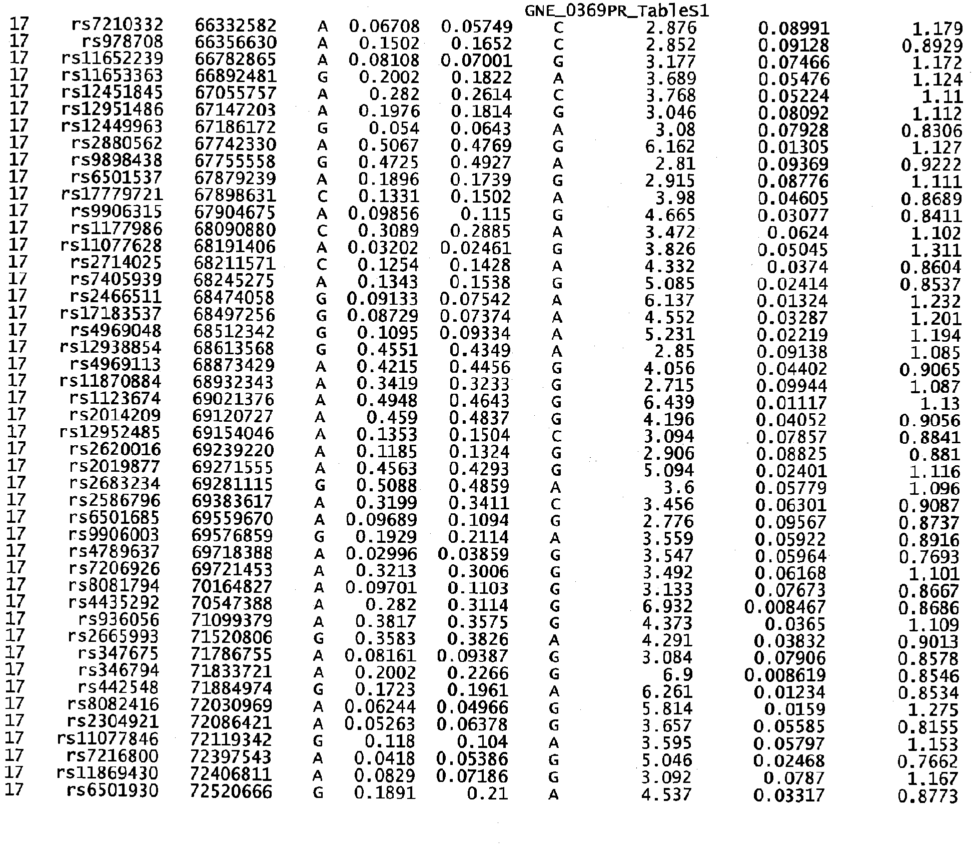

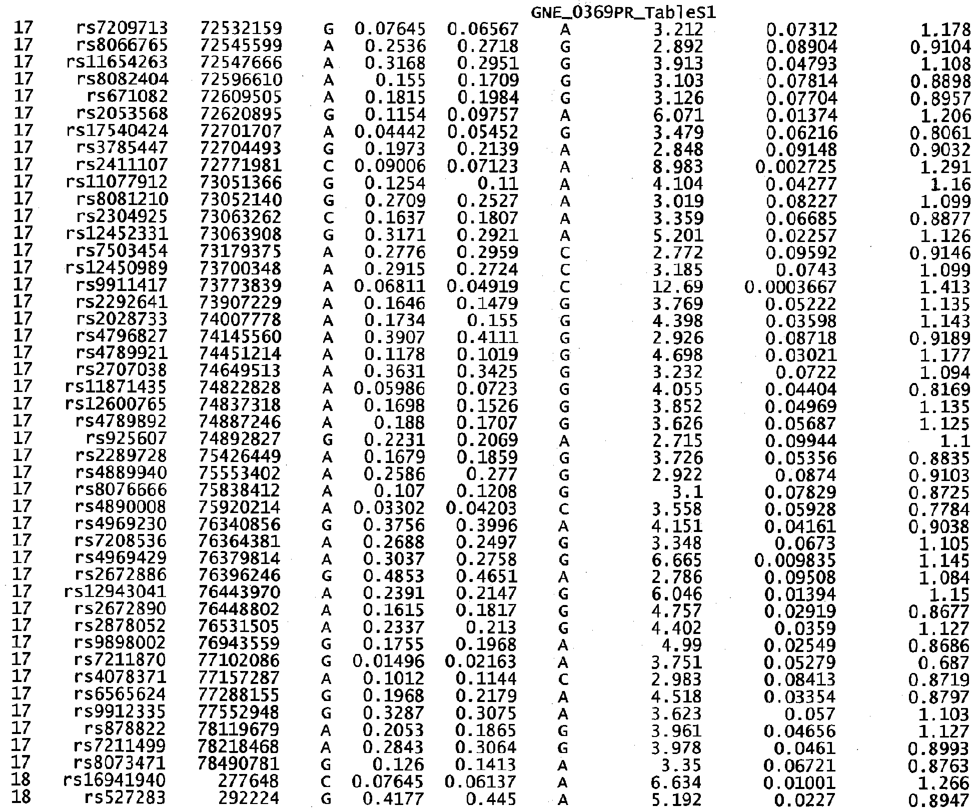

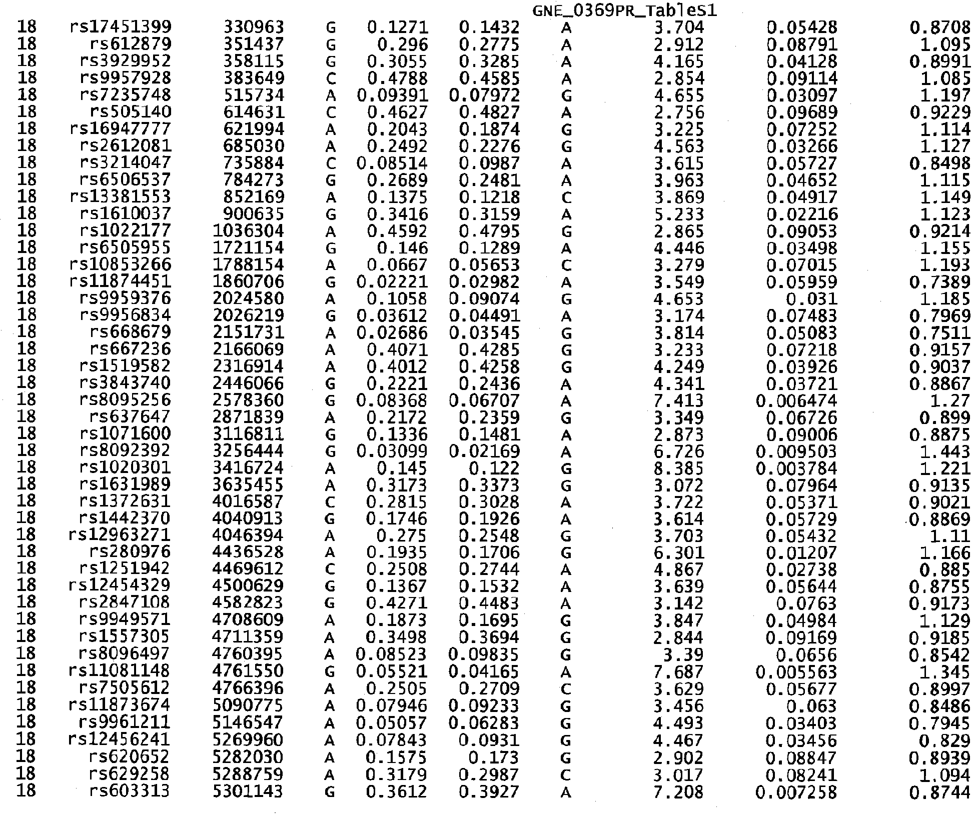

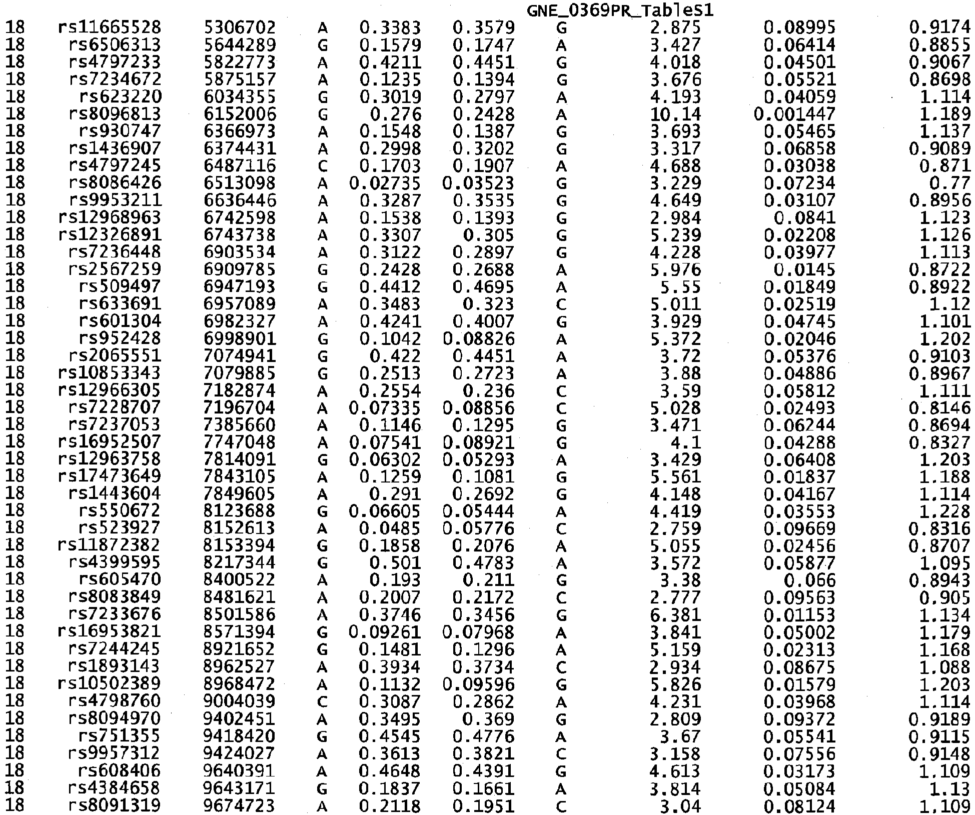

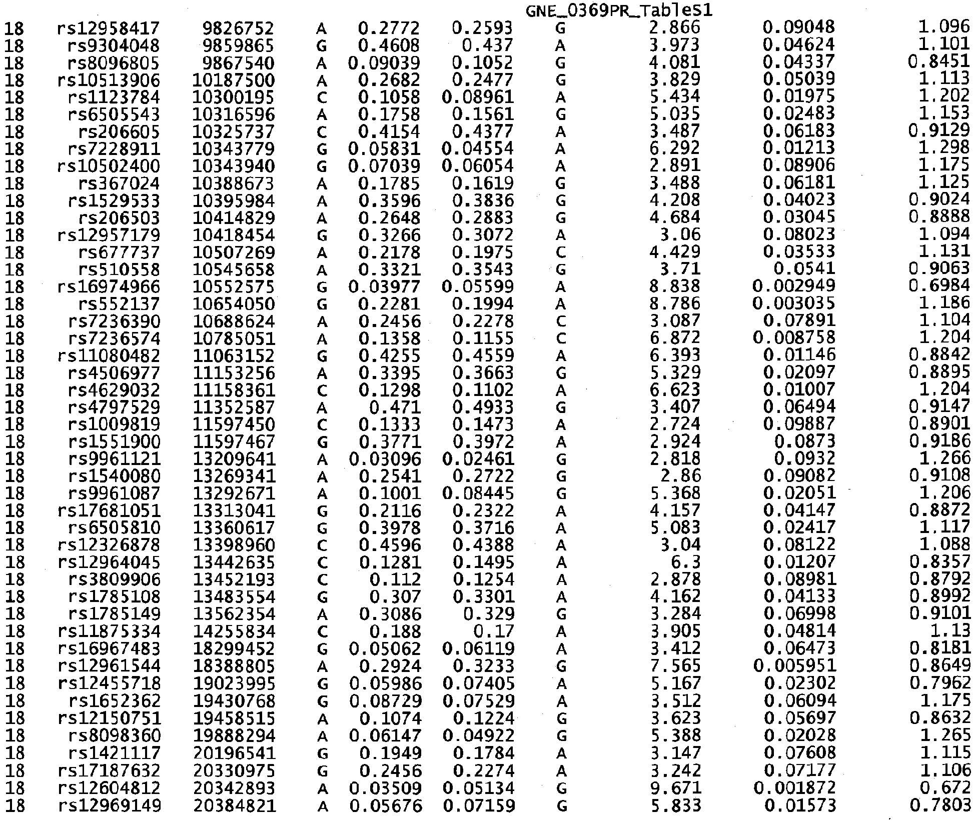

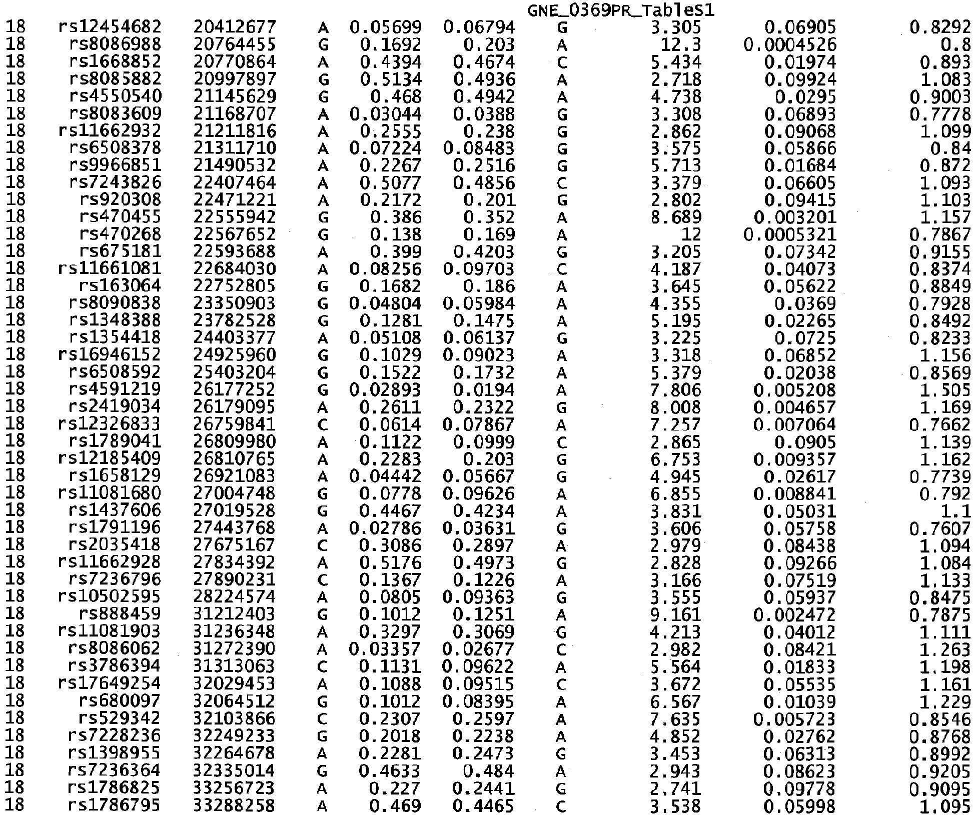

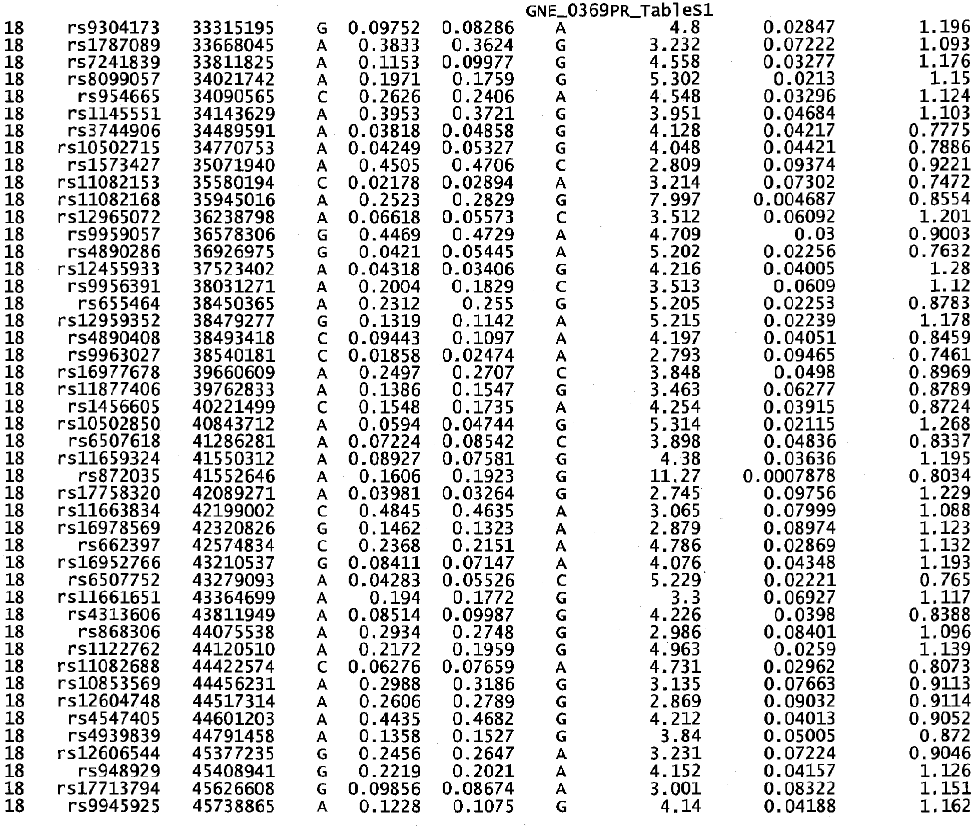

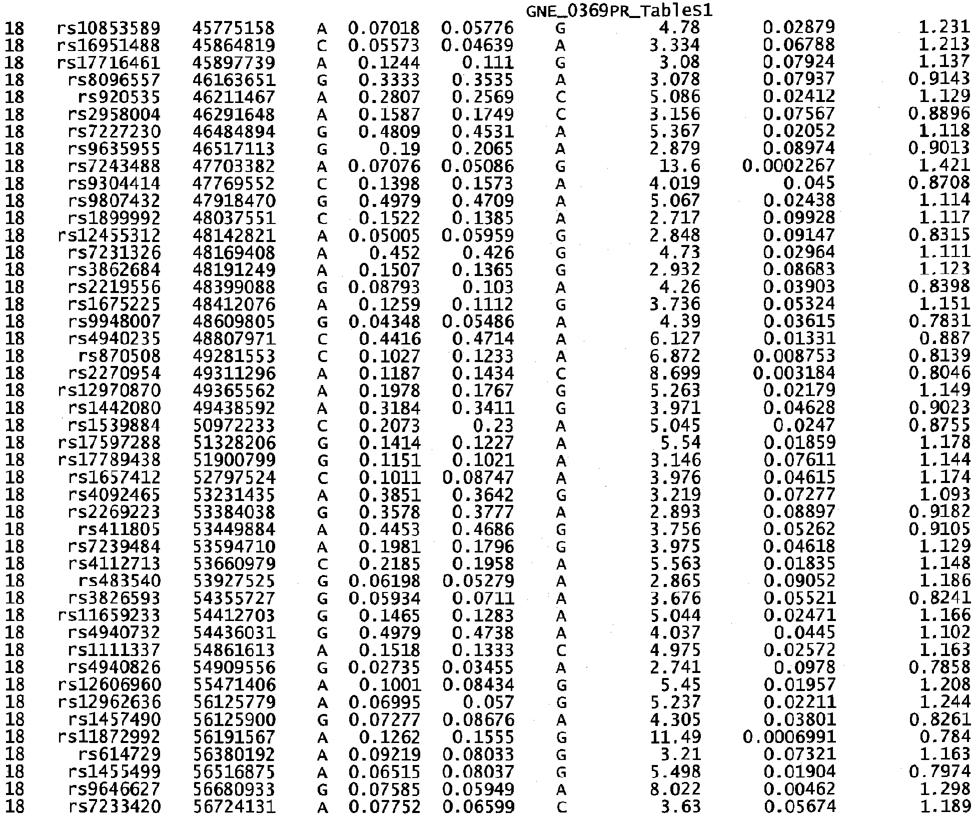

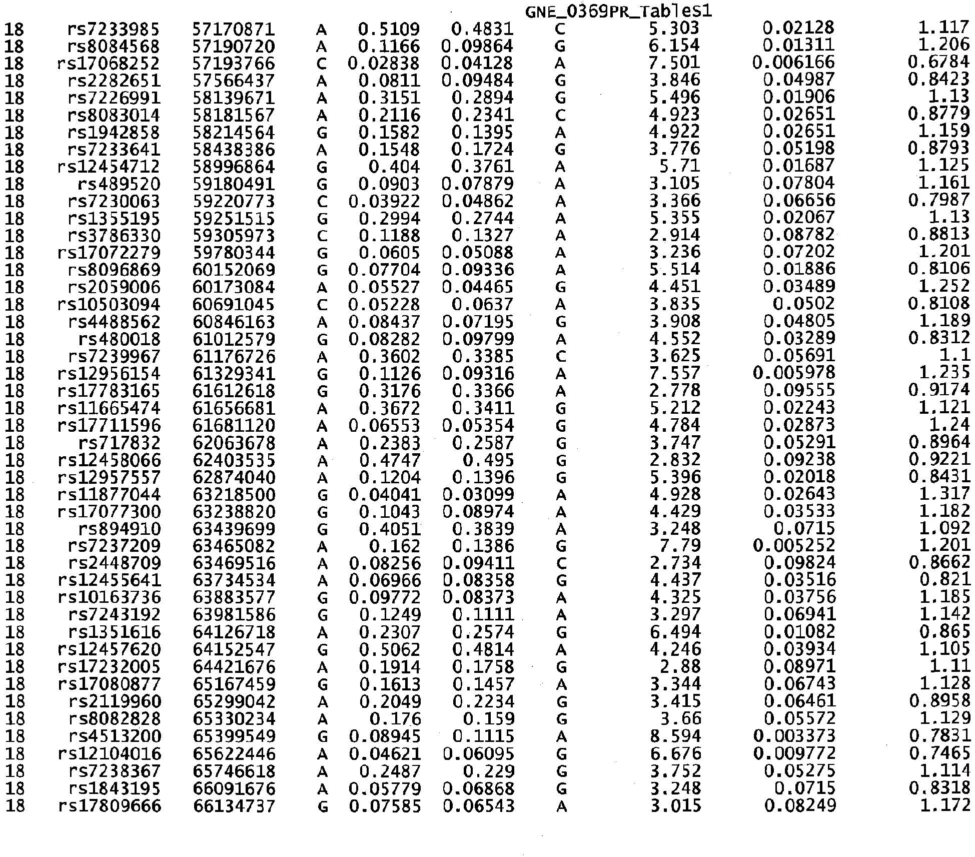

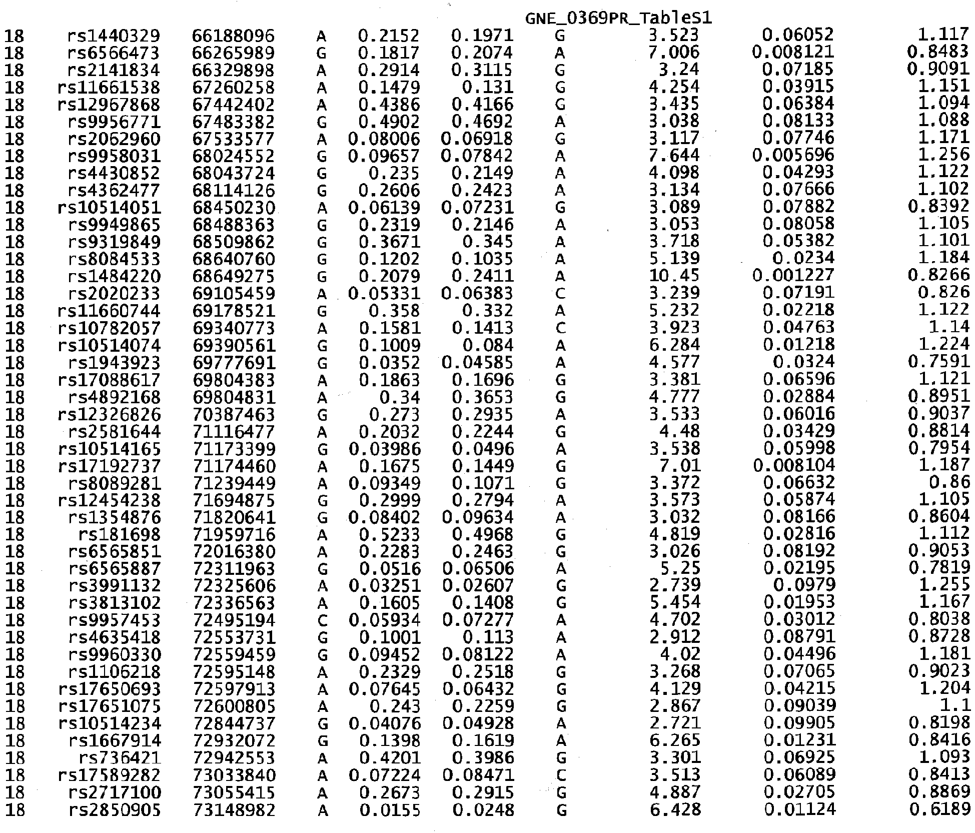

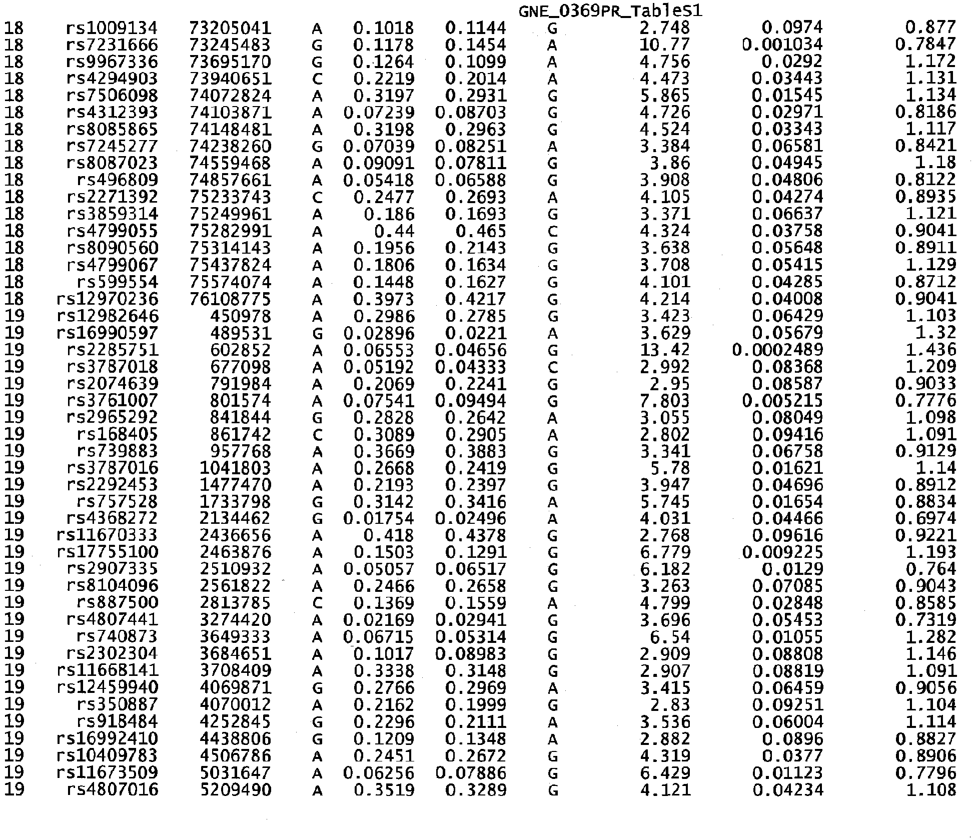

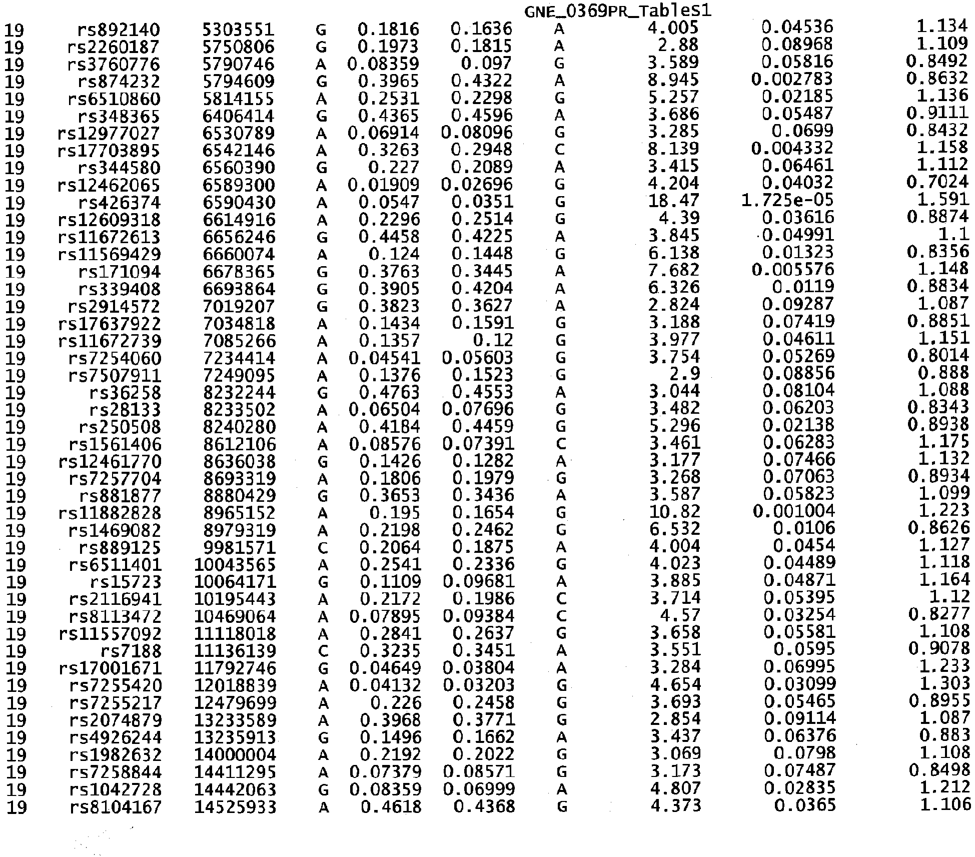

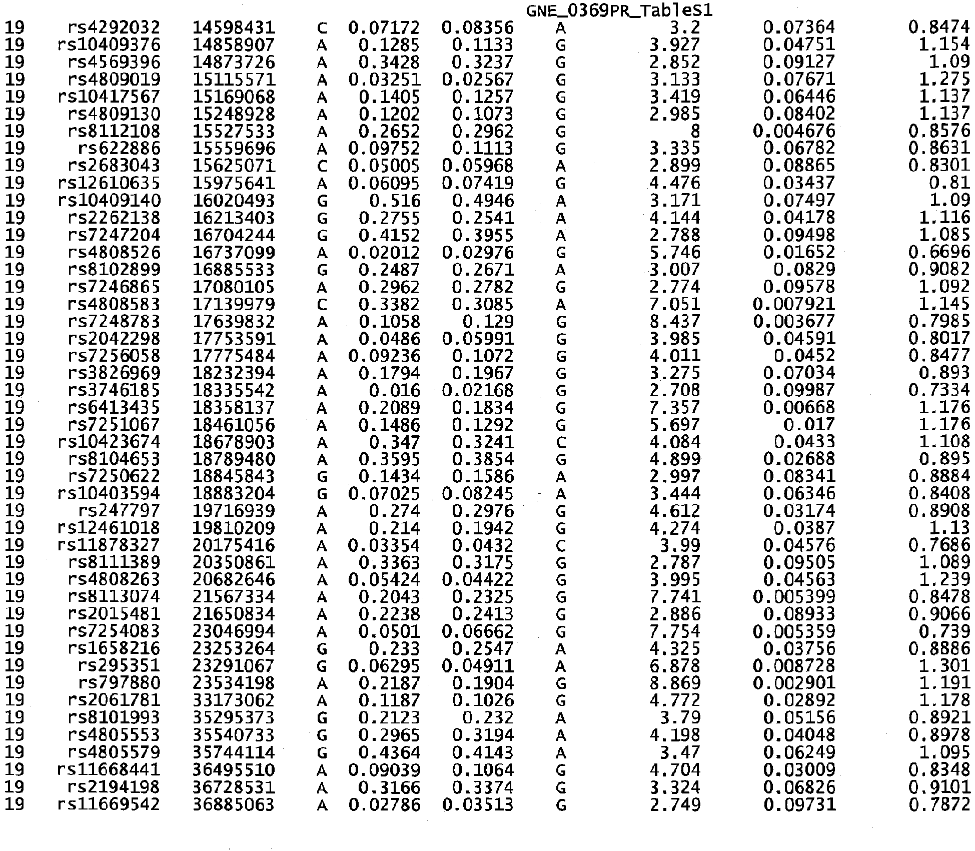

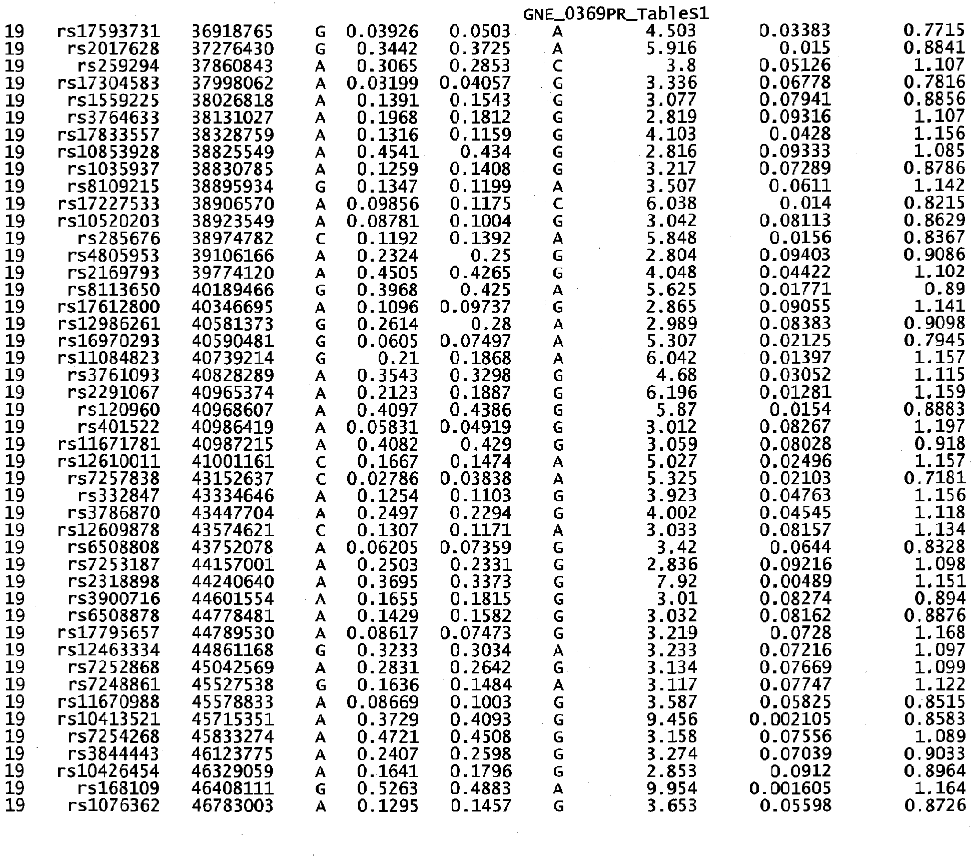

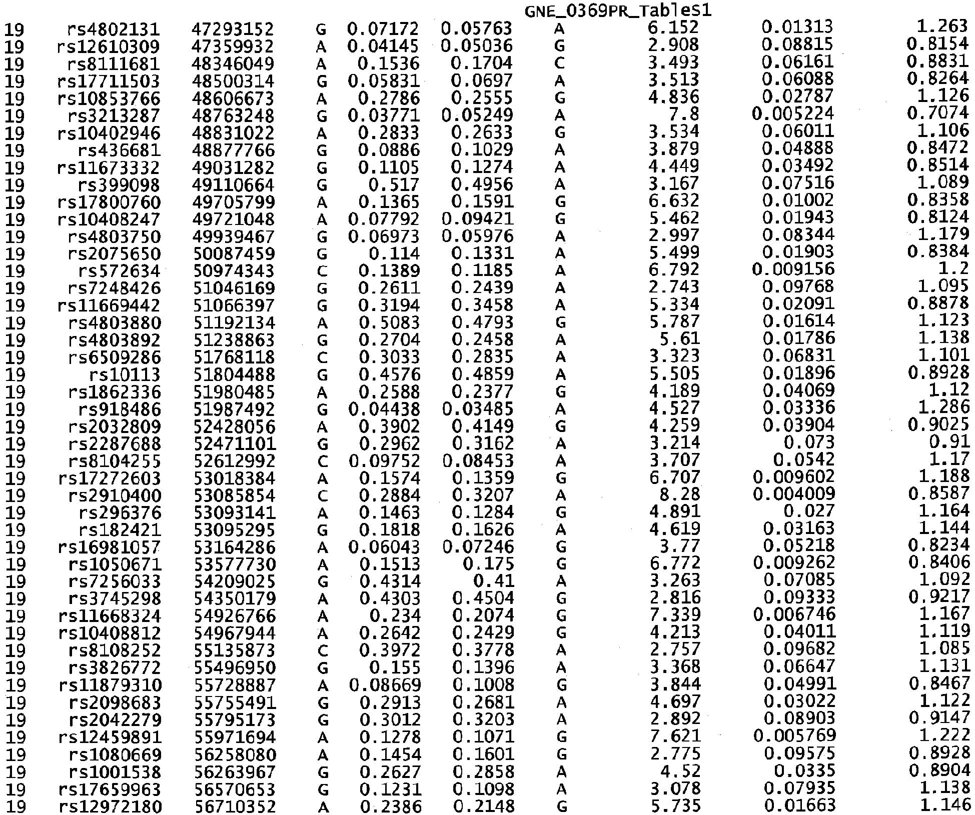

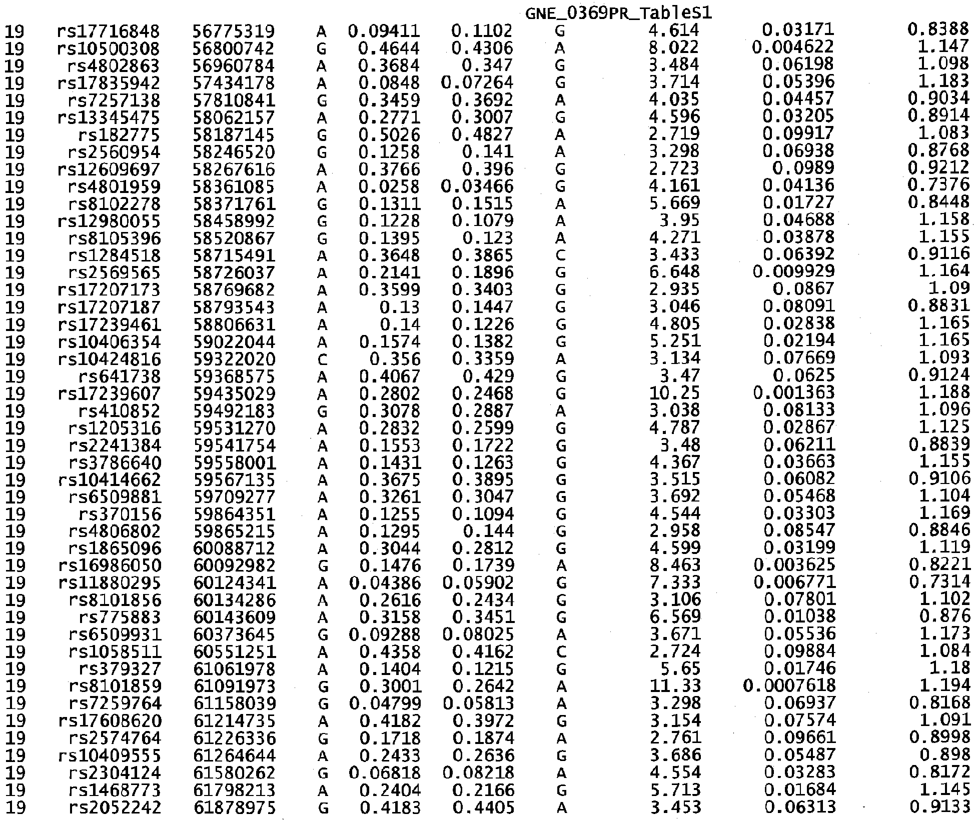

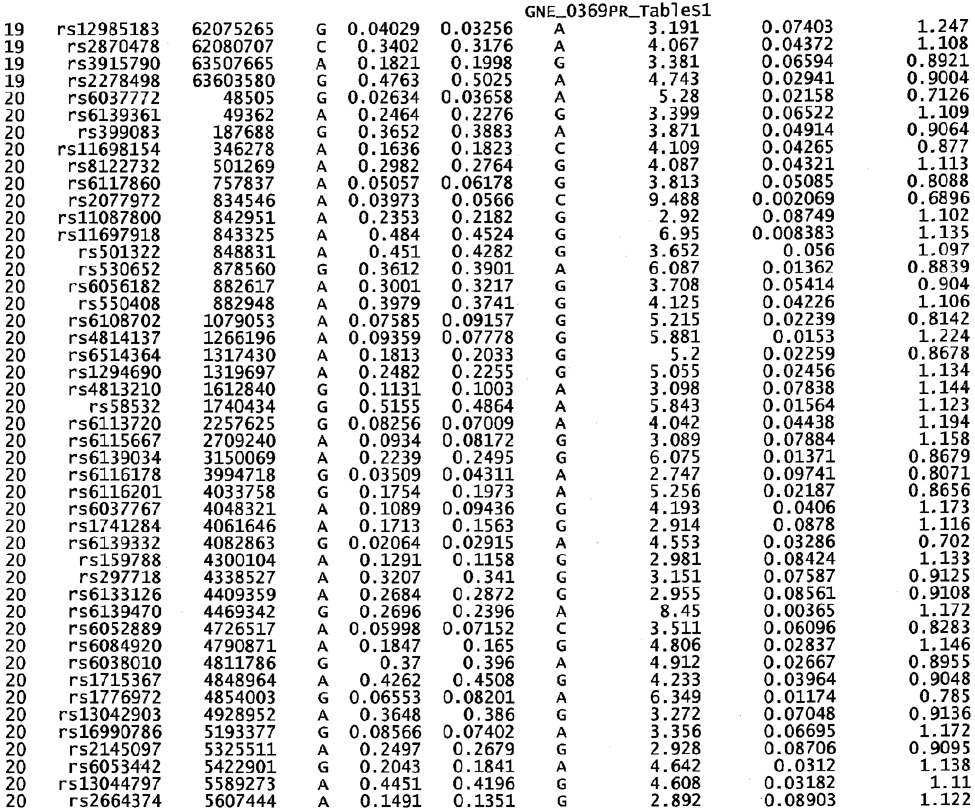

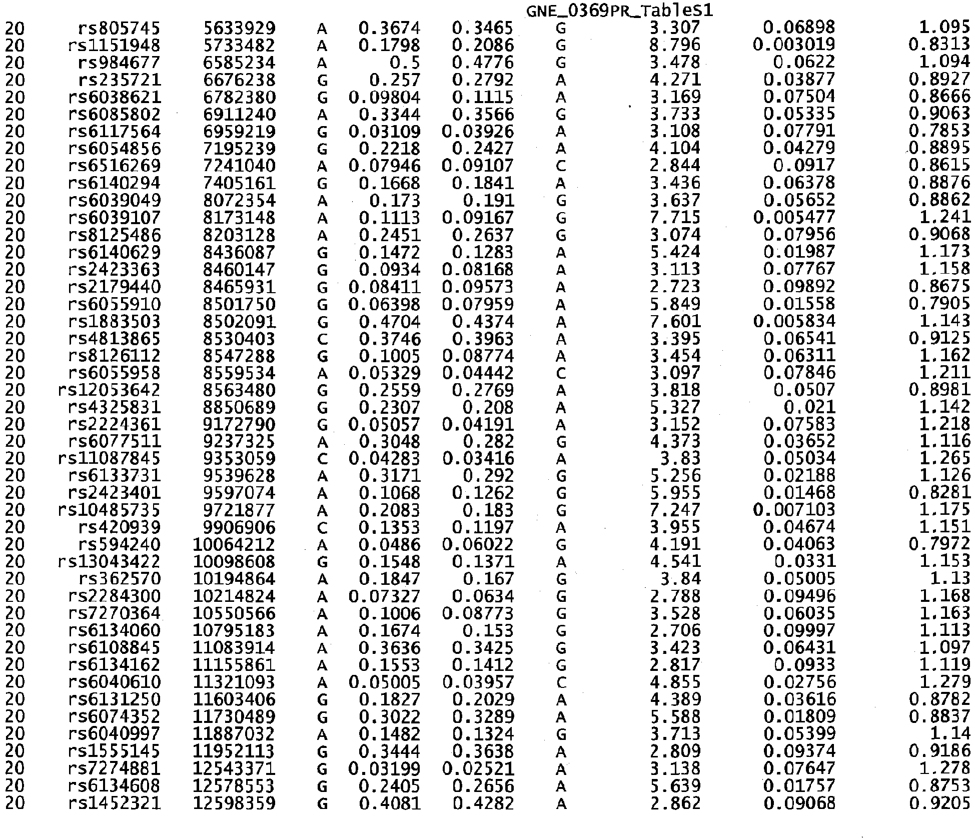

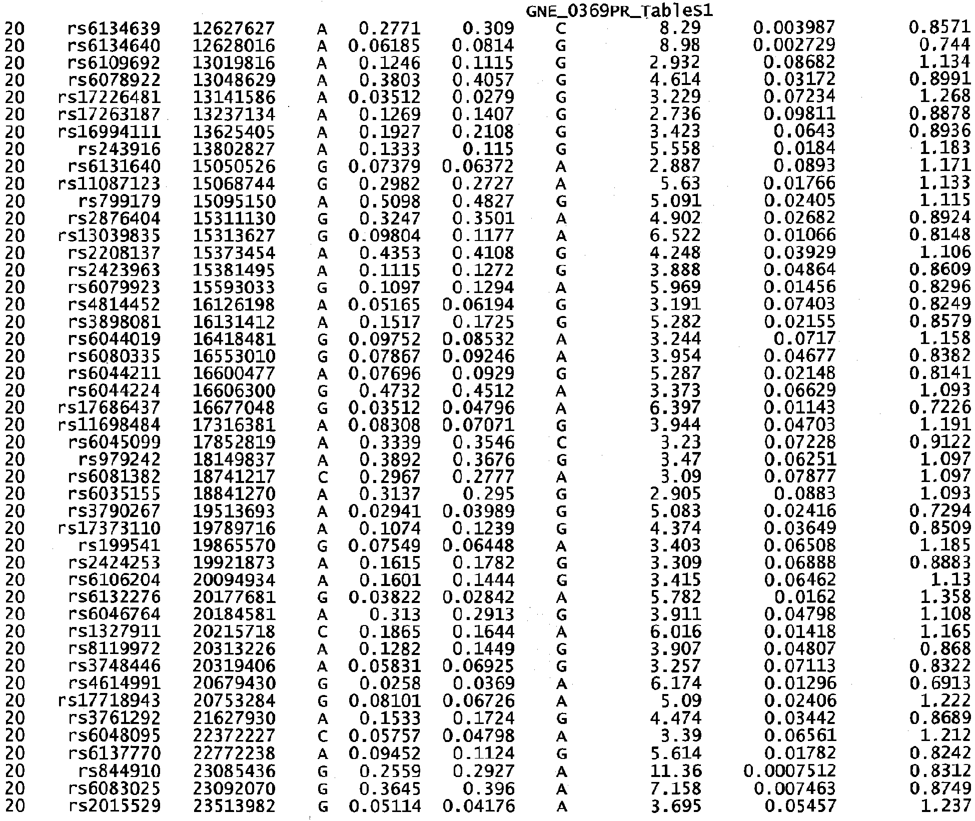

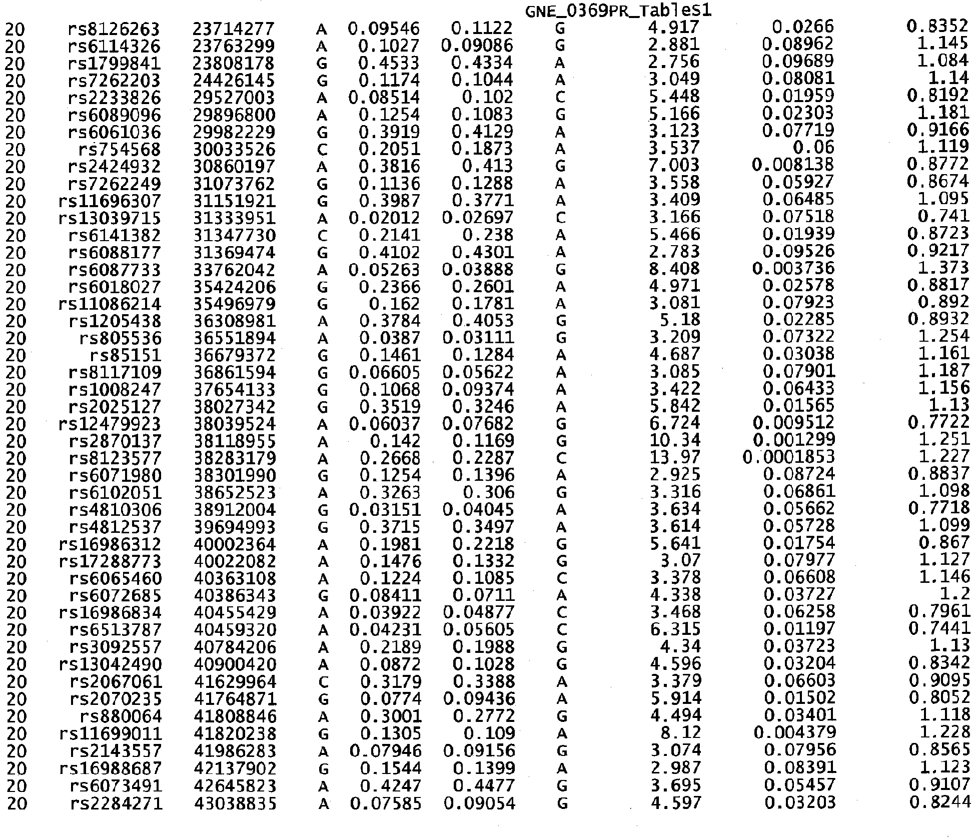

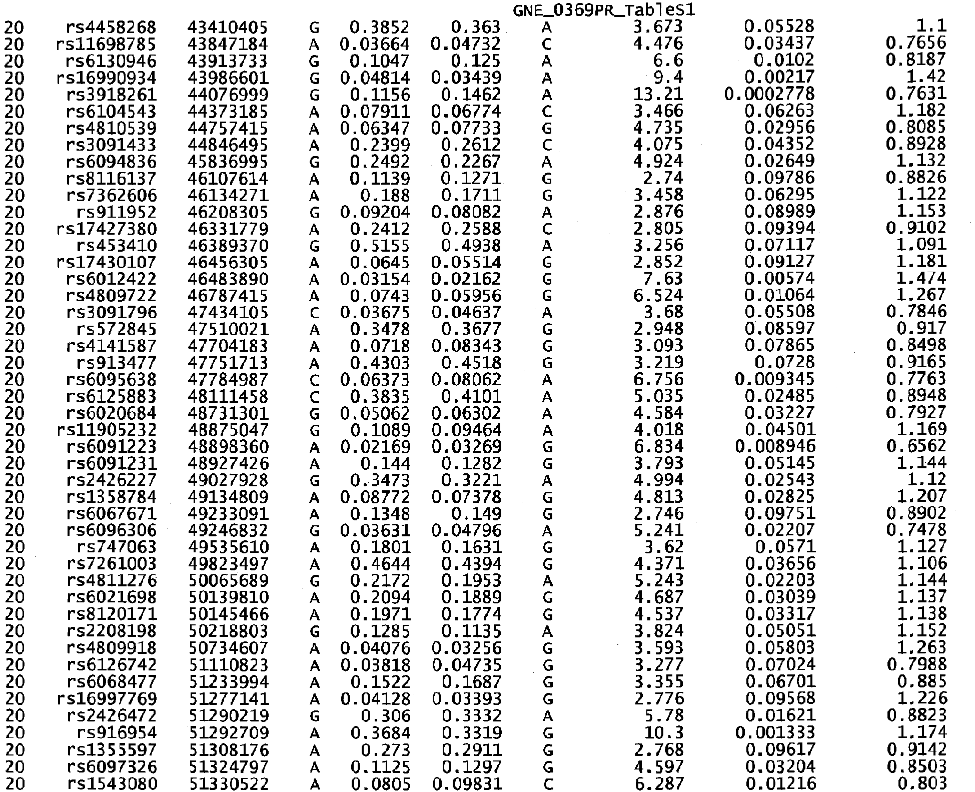

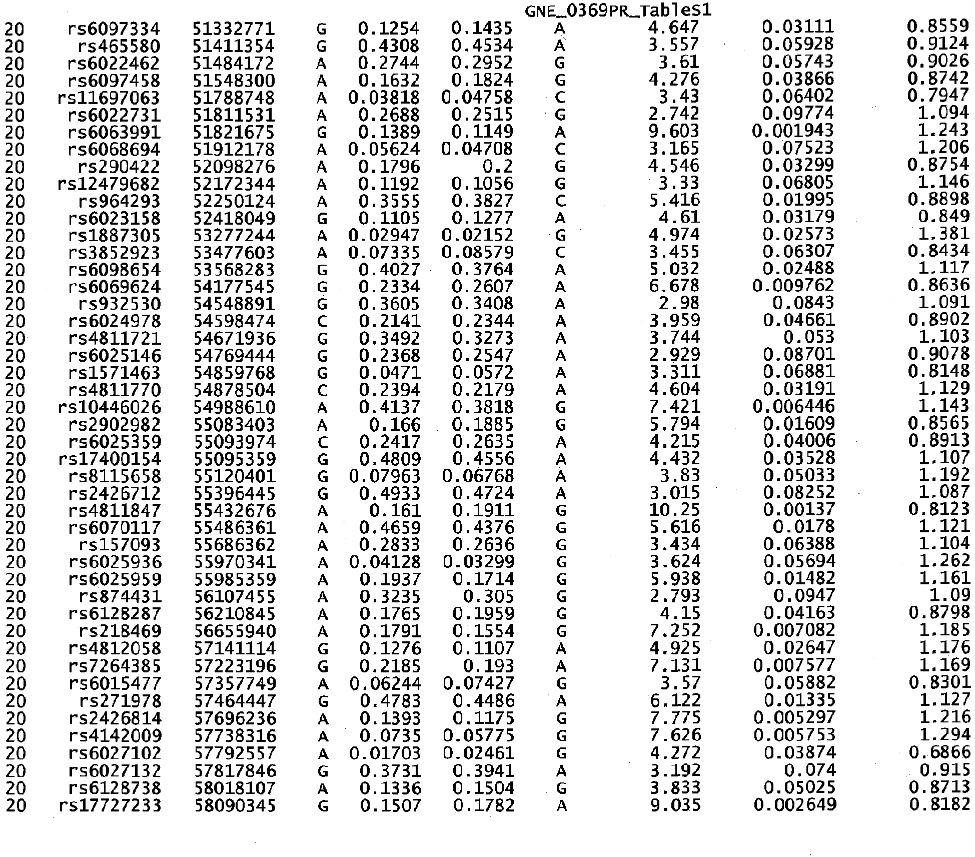

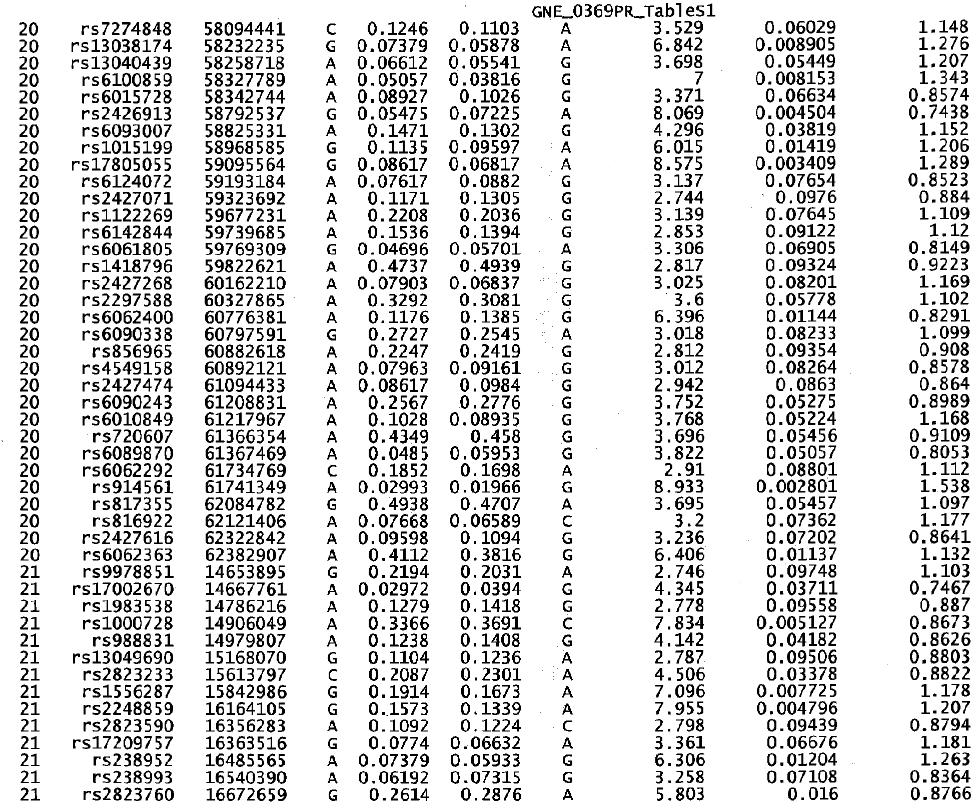

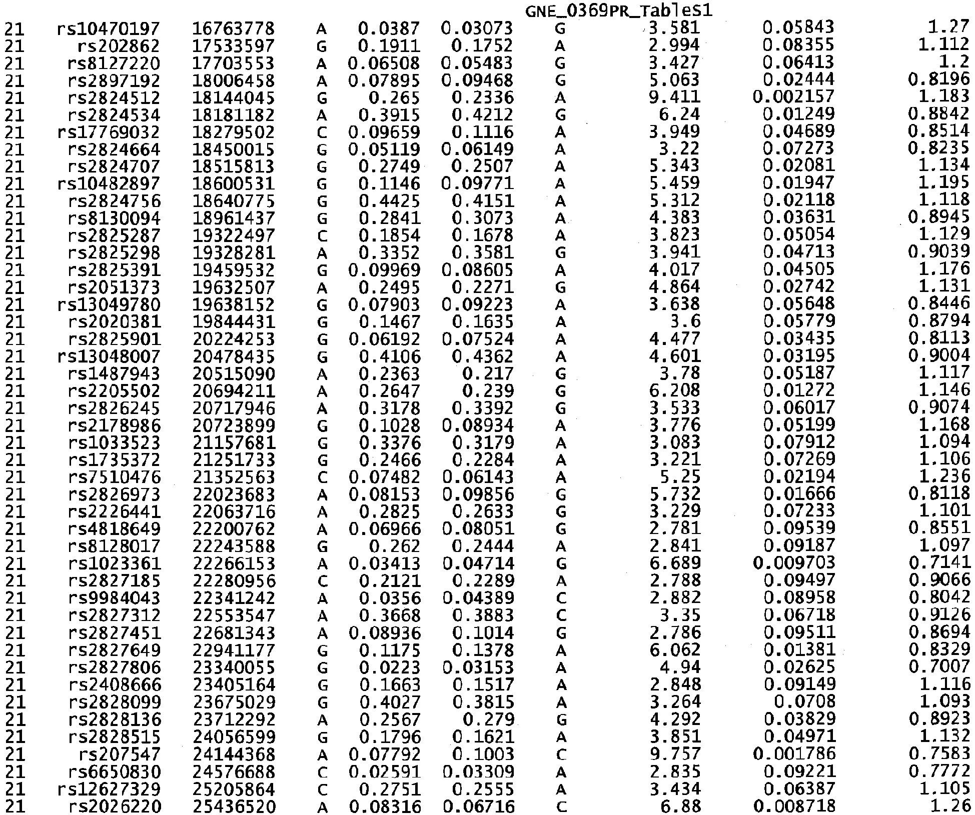

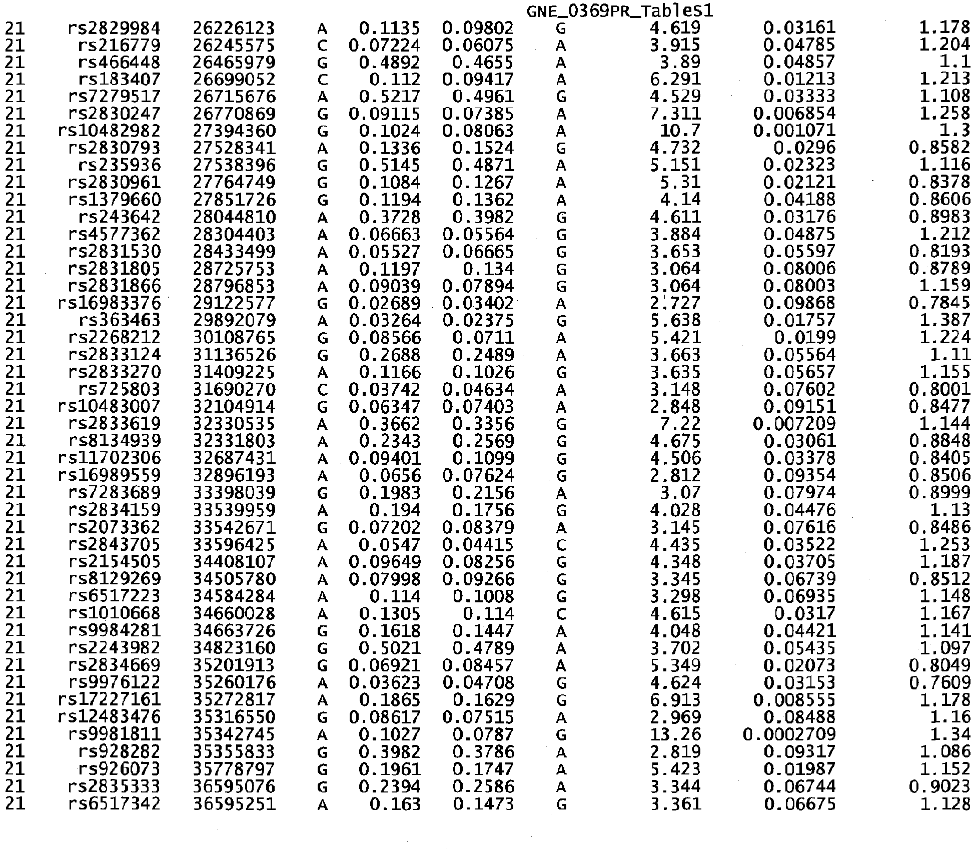

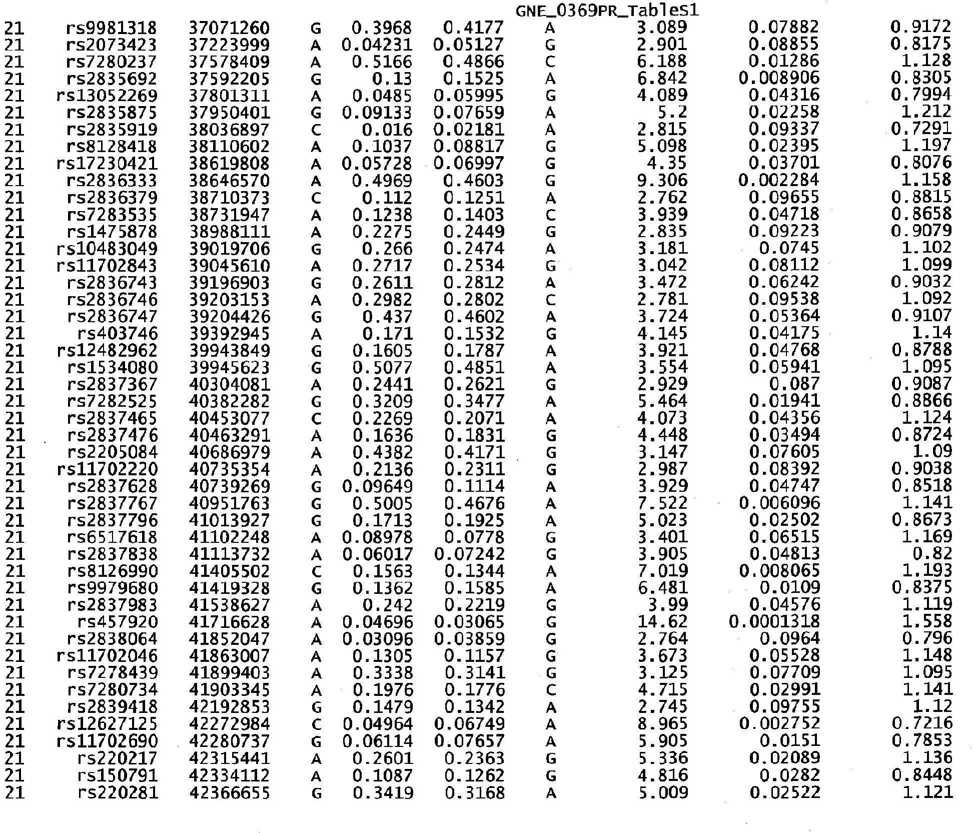

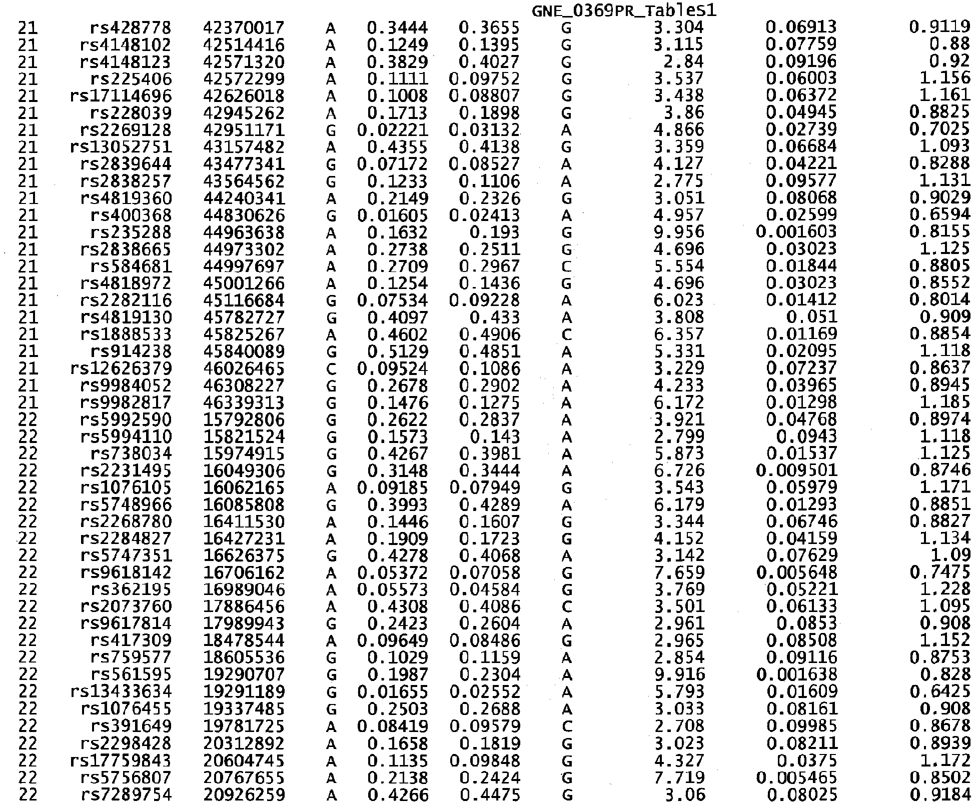

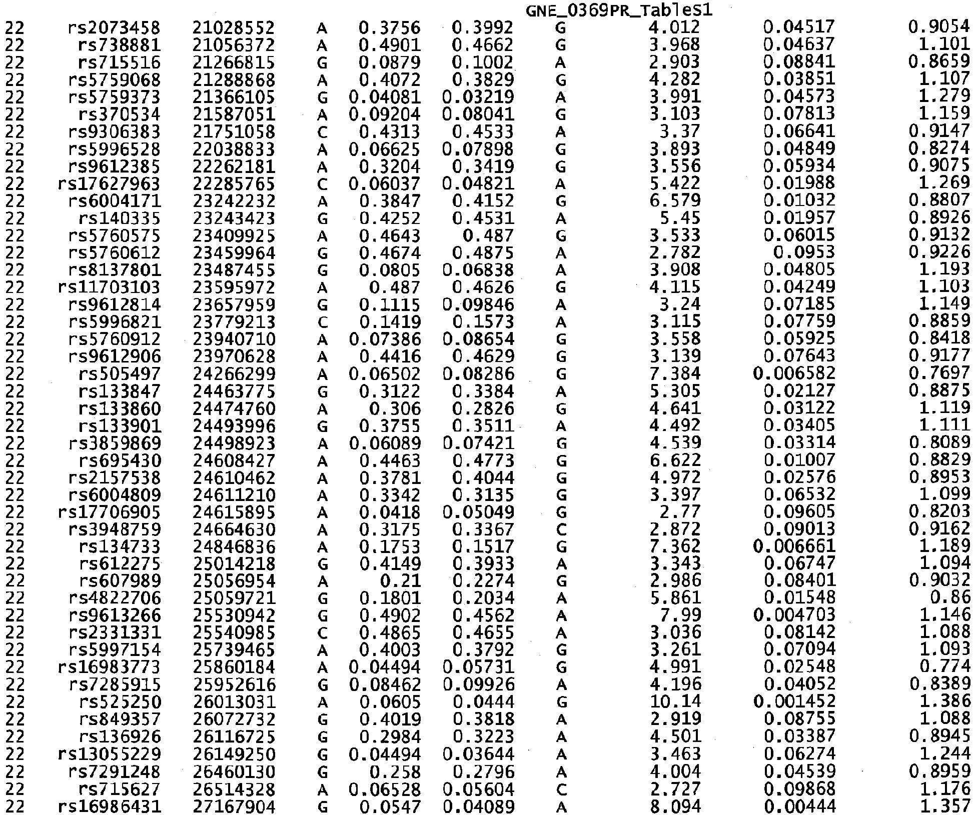

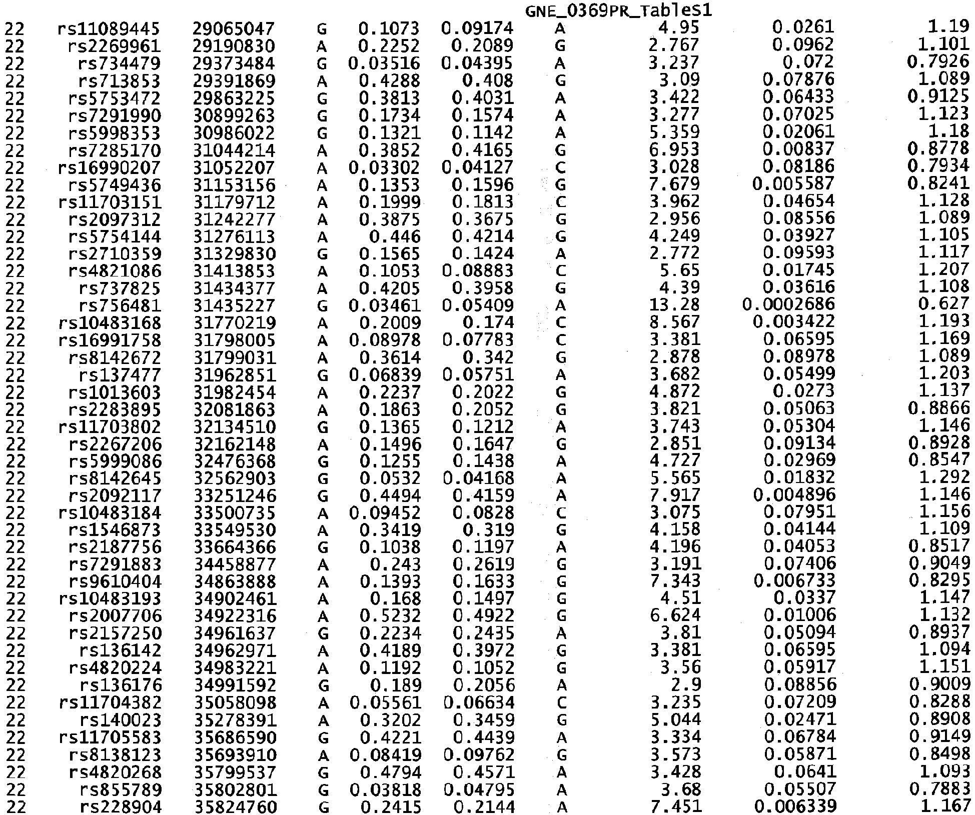

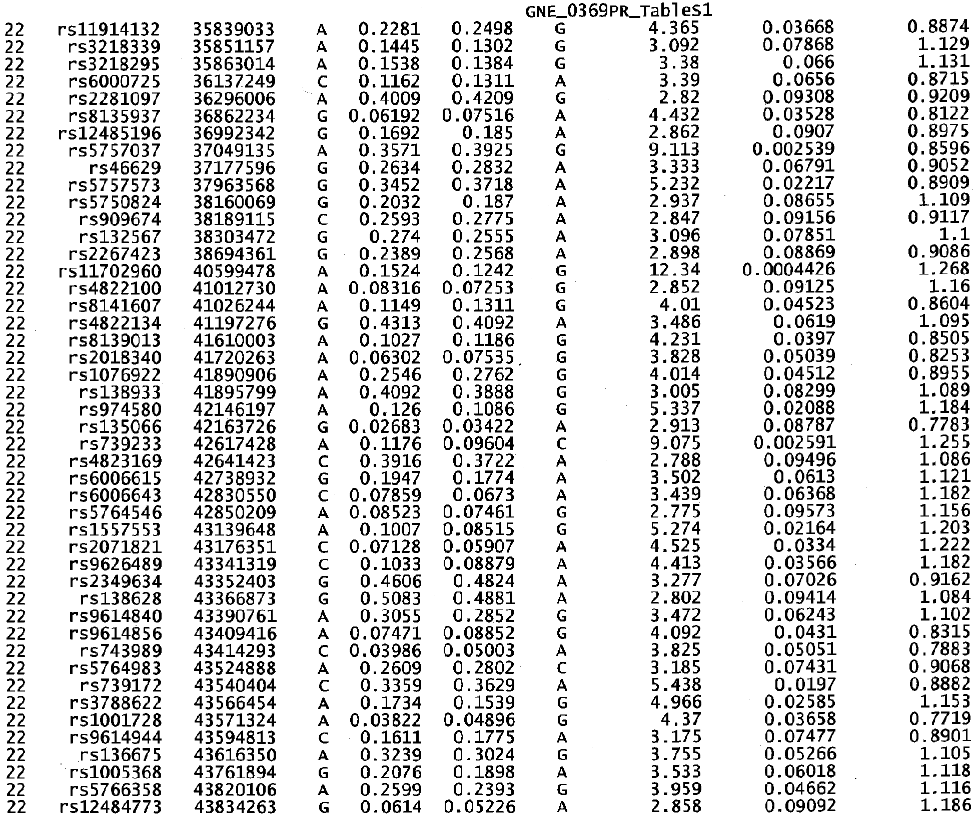

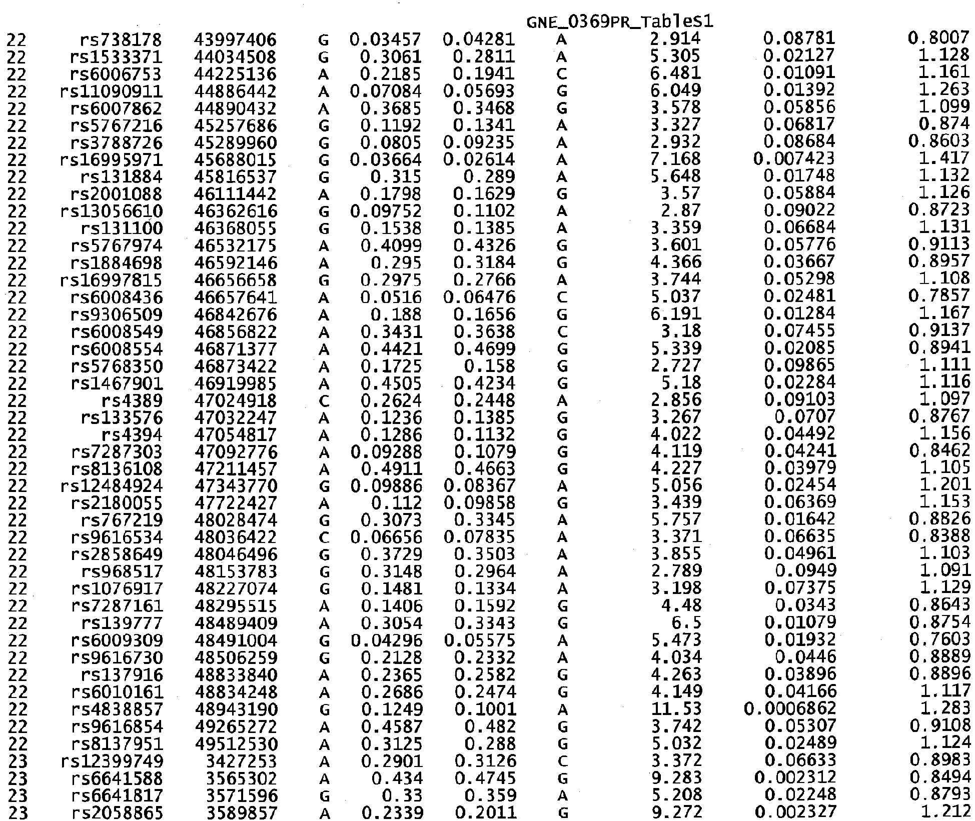

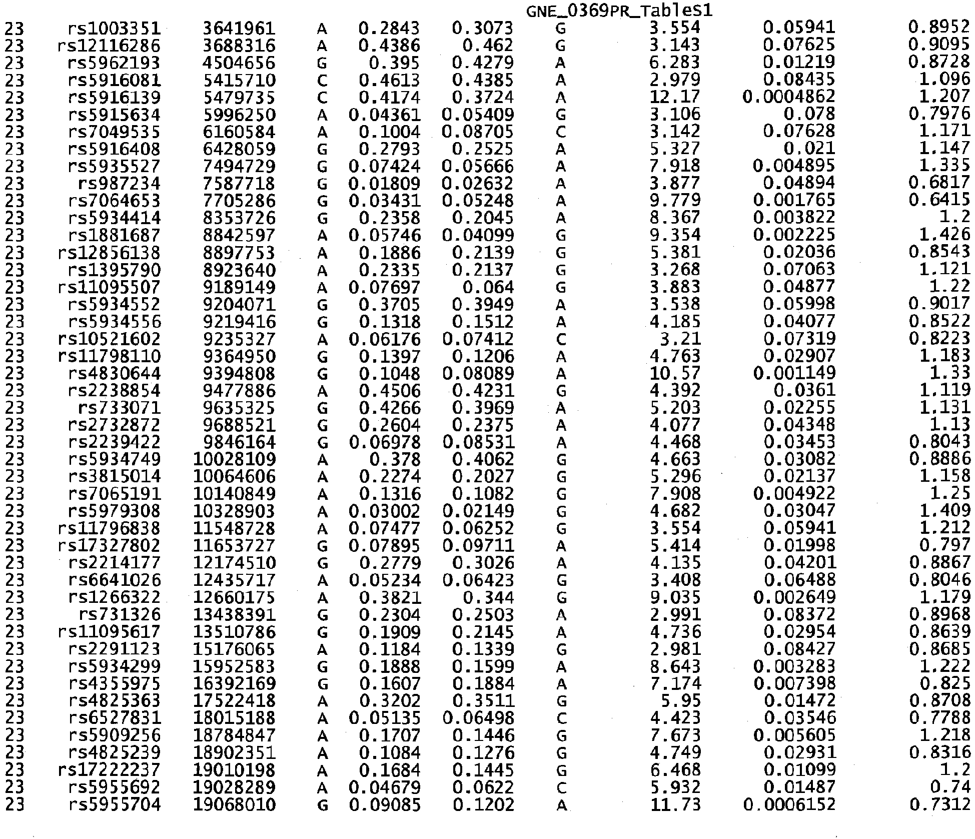

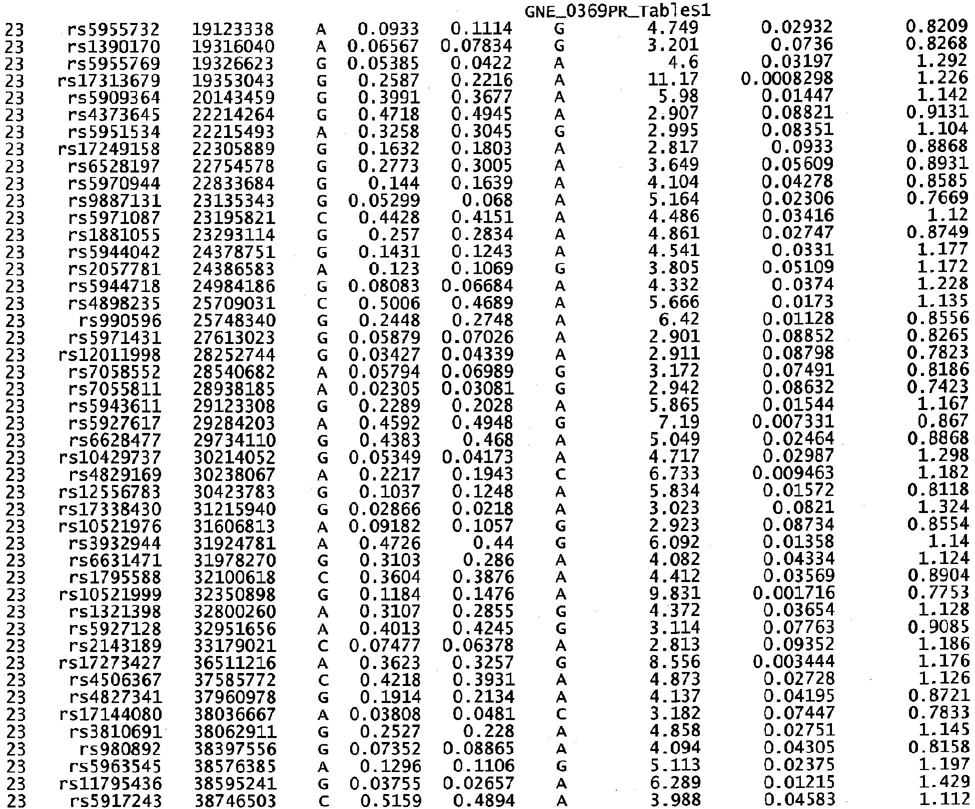

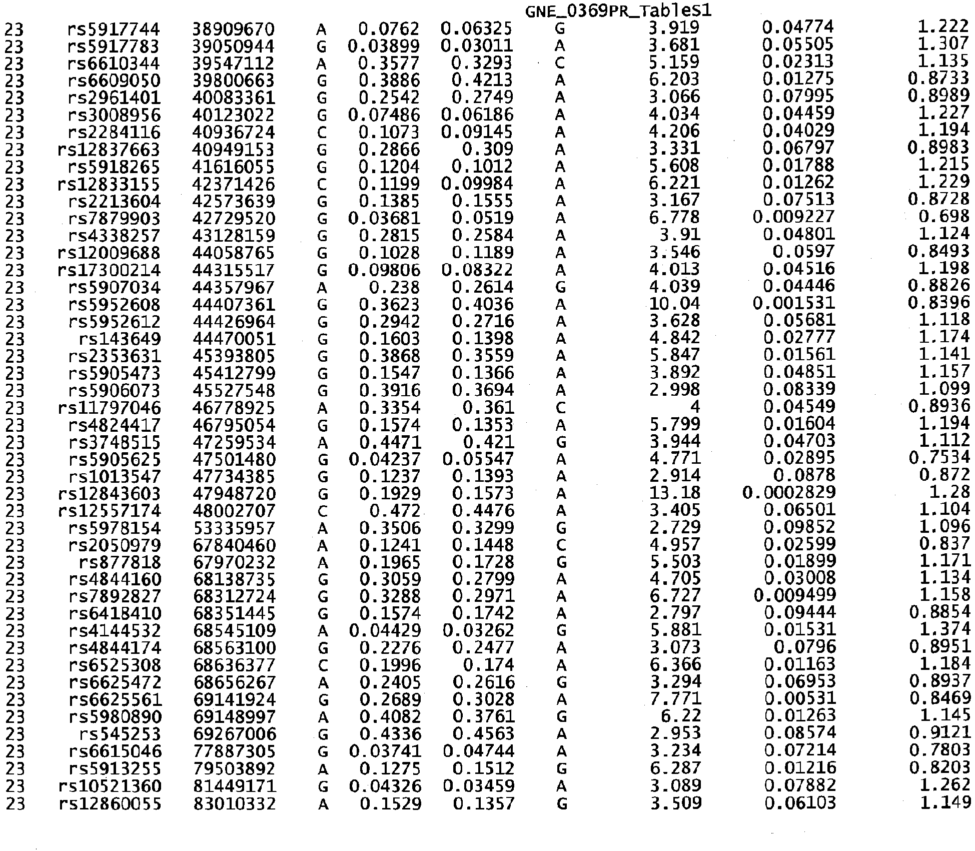

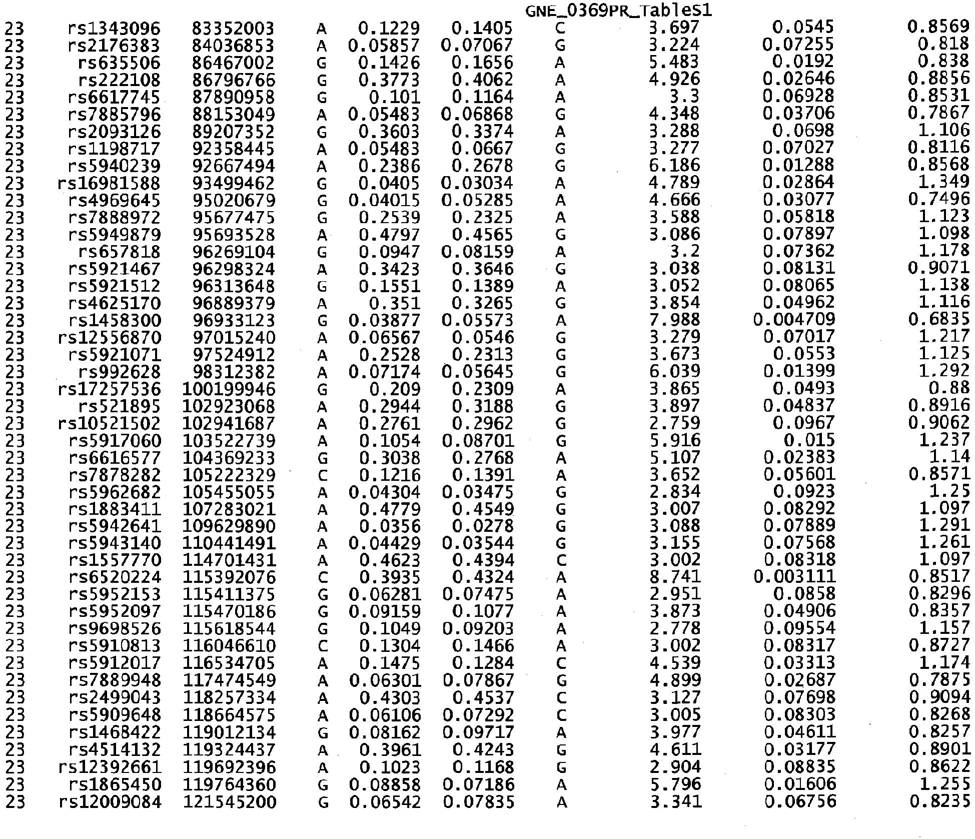

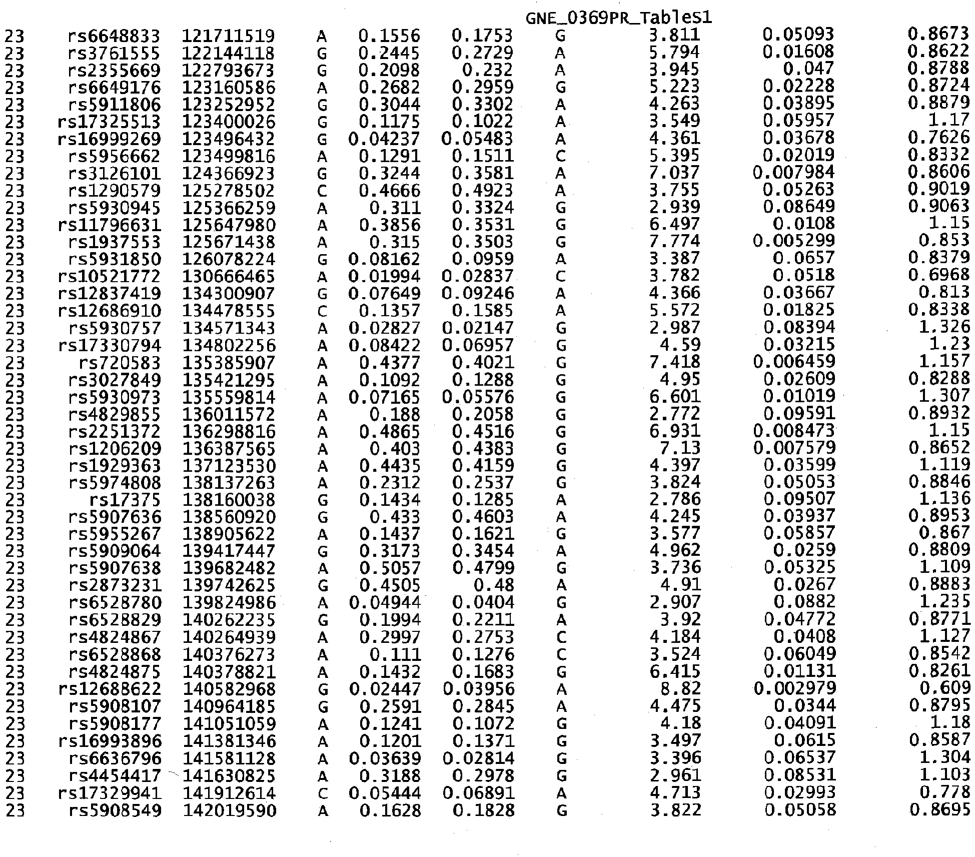

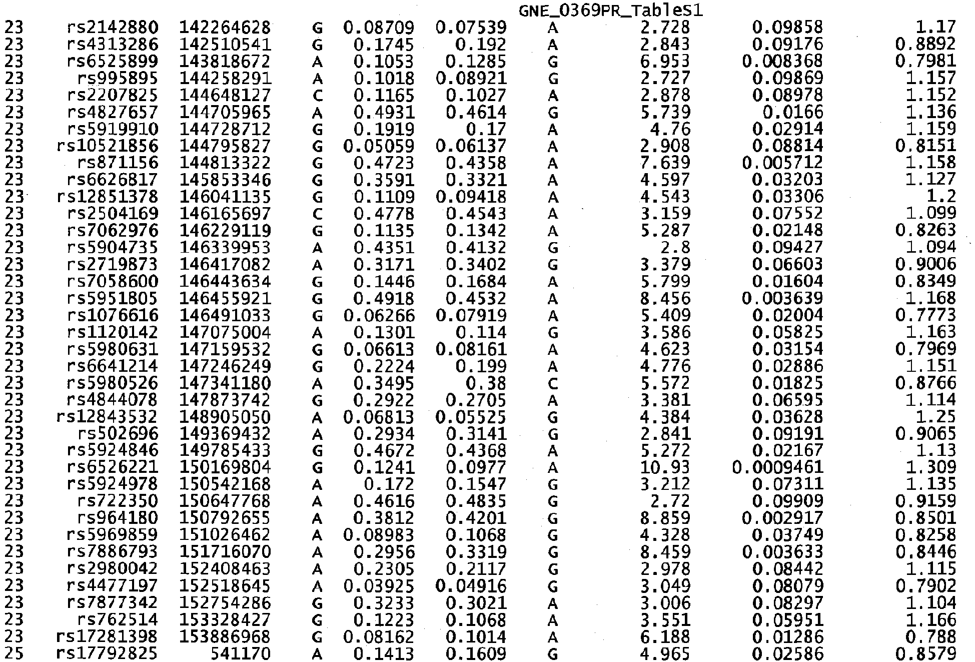

## Claims

1. A method for identifying a human subject diagnosed with early stage or intermediate stage age-related macular degeneration (AMD) as being at risk for developing advanced AMD comprising
(a) determining the allelic frequency of at least 10000 independent loci selected from the single nucleotide polymorphisms set forth in Table S1,
(b) calculating the polygenic score for said subject by multiplying the log of the Od Ratio for each of said single nucleotide polymorphisms by the number of reference alleles carried by said human subject, and dividing the resulting sum by the number of single nucleotide polymorphisms tested,
(c) identifying the human subject as being at risk for developing advanced AMD, when the polygenic score is greater than 0.0001, and
(d) selecting the human subject identified as being at risk for developing advanced AMD for AMD treatment.

2. The method of claims 1 further comprising assessing one or more aspects of the subject's personal history.

3. The method of claim 2 wherein said one or more aspects are selected from the group consisting of age, ethnicity, body mass index, alcohol consumption history, smoking history, exercise history, diet, family history of AMD or other age-related ocular condition, including the age of the relative at the time of their diagnosis, and a personal history of treatment of AMD.

4. The method of claims 1 to 3, wherein determining the presence or absence of risk alleles is achieved by amplification of nucleic acid from said sample.

5. The method of claims 1 to 4, wherein amplification comprises PCR.

6. The method of claims 1 to 4, wherein primers for amplification are located on a chip.

7. The method of claims 4 to 6 wherein the amplification comprises: (i) admixing an amplification primer or amplification primer pair with a nucleic acid template isolated from the biological sample, wherein the primer or primer pair is complementary or partially complementary to a region proximal to or including the polymorphism, and is capable of initiating nucleic acid polymerization by a polymerase on the nucleic acid template; and, b) extending the primer or primer pair in a DNA polymerization reaction comprising a polymerase and the template nucleic acid to generate the amplicon.

8. The method of claim 7, wherein the amplicon is detected by a process that includes one or more of: hybridizing the amplicon to an array, digesting the amplicon with a restriction enzyme, or real-time PCR analysis.

9. The method of claim 4, wherein the amplification comprises performing a polymerase chain reaction (PCR), reverse transcriptase PCR (RT-PCR), or ligase chain reaction (LCR) using nucleic acid isolated from the organism or biological sample as a template in the PCR, RT-PCR, or LCR.

10. The method of claims 4 to 9, wherein said sample is derived from saliva or blood.

11. A method for selecting a therapy in the treatment of AMD with anti-factor D antibodies, anti-VEGF antibodies, CRIg, and CRIg-Ig fusion, wherein a human subject has been identified according to a method of claims 1-10.

12. The method of claim 11 wherein the selected therapy is anti-factor D antibody therapy.

## Patentansprüche

1. Verfahren zum Bestimmen, ob bei einem humanen Individuum, das mit altersbedingter Makuladegeneration (AMD) im Frühstadium oder im Mittelstadium diagnostiziert wurde, das Risiko besteht, fortgeschrittene AMD zu entwickeln, wobei das Verfahren umfasst:
(a) Bestimmen der Allelhäufigkeit von mindestens 10000 unabhängigen Loci, die aus den in Tabelle S1 dargestellten Einzelnucleotid-Polymorphismen ausgewählt wurden,
(b) Errechnen des polygenischen Scores für das Individuum durch Multiplizieren des Logarithmus des Od-Verhältnisses (Od Ratio) für jeden der Einzelnucleotid-Polymorphismen mit der Anzahl der Referenz-Allele, die das humane Individuum trägt, und Dividieren des resultierenden Betrags durch die Anzahl der getesteten Einzelnucleotid-Polymorphismen,
(c) Bestimmen, ob für das humane Individuum das Risiko besteht, fortgeschrittene AMD zu entwickeln, wenn der polygenische Score größer als 0,0001 ist, und
(d) Auswählen der AMD-Behandlung für das humane Individuum, bei dem festgestellt wurde, dass ein Risiko besteht, fortgeschrittene AMD zu entwickeln.

2. Verfahren nach Anspruch 1, das des Weiteren das Evaluieren eines Aspekts oder mehrerer Aspekte der persönlichen Historie des Individuums umfasst.

3. Verfahren nach Anspruch 2, wobei der eine Aspekt oder die mehreren Aspekte ausgewählt ist/sind aus der Gruppe bestehend aus Alter, Ethnizität, Body-Mass-Index, Alkoholkonsumgewohnheiten, Rauchgewohnheiten, Gewohnheiten der körperlichen Bewegung, Ernährung, Familien-Historie bezüglich AMD oder anderer altersbedingter Augenzustände, einschließlich des Alters des Verwandten zum Zeitpunkt ihrer/seiner Diagnose und persönliche Historie bezüglich AMD-Behandlung.

4. Verfahren nach den Ansprüchen 1 bis 3, wobei das Bestimmen der Anwesenheit oder Abwesenheit von Risiko-Allelen durch Amplifikation von Nucleinsäuren aus der Probe erreicht wird.

5. Verfahren nach den Ansprüchen 1 bis 4, wobei die Amplifikation eine PCR umfasst.

6. Verfahren den Ansprüchen 1 bis 4, wobei die Primer für die Amplifikation auf einem Chip lokalisiert sind.

7. Verfahren den Ansprüchen 4 bis 6, wobei die Amplifikation umfasst: (i) Beimischen eines Amplifikationsprimers oder eines Amplifikationsprimerpaares zu einer aus der biologischen Probe isolierten Nucleinsäure-Matrize, wobei der Primer oder das Primerpaar zu einer Region komplementär oder teilweise komplementär ist, die zu dem Polymorphismus proximal ist oder diesen einschließt, und wobei der Primer oder das Primerpaar fähig ist, die Nucleinsäure-Polymerisation durch eine Polymerase auf der Nucleinsäure-Matrize zu initiieren; und b) Verlängern des Primers oder Primerpaars in einer DNA-Polymerisationsreaktion, die eine Polymerase und die Matrizen-Nucleinsäure umfasst, um das Amplicon zu erzeugen.

8. Verfahren nach Anspruch 7, wobei das Amplicon durch ein Verfahren nachgewiesen wird, das eines oder mehrere einschließt von: Hybridisieren des Amplicons mit einem Array, Verdauen des Amplicons mit einem Restriktionsenzym oder Real-Time-PCR-Analyse.

9. Verfahren nach Anspruch 4, wobei die Amplifikation das Durchführen einer Polymerase-Kettenreaktion (PCR), Reverse-Transkriptase-PCR (RT-PCR) oder Ligase-Kettenreaktion (LCR) unter Verwendung einer Nucleinsäure umfasst, die aus dem Organismus oder aus der biologischen Probe isoliert wurde, als Matrize in der PCR, RT-PCR oder LCR.

10. Verfahren nach den Ansprüchen 4 bis 9, wobei die Probe aus Speichel oder Blut stammt.

11. Verfahren zum Auswählen einer Therapie bei der Behandlung von AMD mit Anti-Faktor-D-Antikörpern, Anti-VEGF-Antikörpern, CRIg und CRIg-Ig-Fusion, wobei das humane Individuum gemäß einem Verfahren nach den Ansprüchen 1 bis 10 bestimmt wurde.

12. Verfahren nach Anspruch 11, wobei die ausgewählte Therapie eine Anti-Faktor-D-Antikörper-Therapie ist.

## Revendications

1. Procédé pour identifier un sujet humain chez lequel a été diagnostiquée une dégénérescence maculaire liée à l'âge (DMLA) à un stade précoce ou à un stade intermédiaire comme sujet à risque de développer une DMLA avancée, comprenant :
(a) la détermination de la fréquence allélique d'au moins 10 000 locus indépendants choisis parmi les polymorphismes de nucléotides uniques indiqués sur le tableau S1,
(b) le calcul du score polygénique dudit sujet en multipliant la valeur logarithmique du rapport Od pour chacun desdits polymorphismes de nucléotides uniques par le nombre d'allèles de référence portés par ledit sujet humain, et la division de la somme résultante par le nombre de polymorphismes de nucléotides uniques testés,
(c) l'identification du sujet humain comme sujet à risque de développer une DMLA avancée, lorsque le score polygénique est supérieur à 0,0001, et
(d) la sélection du sujet humain identifié comme sujet à risque de développer une DMLA avancée pour un traitement de la DMLA.

2. Procédé suivant la revendication 1, comprenant en outre l'évaluation d'un ou plusieurs aspects des antécédents personnels du sujet.

3. Procédé suivant la revendication 2, dans lequel ledit un ou plusieurs aspects sont choisis dans le groupe consistant en l'âge, l'origine ethnique, l'indice de masse corporelle, les antécédents de consommation d'alcool, les antécédents de tabagisme, les antécédents d'exercice physique, le régime alimentaire, les antécédents familiaux de DMLA ou d'une autre affection oculaire due à l'âge, comprenant l'âge du sujet apparenté au moment de son diagnostic, et des antécédents personnels de traitement de DMLA.

4. Procédé suivant les revendications 1 à 3, dans lequel la détermination de la présence ou de l'absence d'allèles à risque est réalisée par amplification d'un acide nucléique provenant dudit échantillon.

5. Procédé suivant les revendications 1 à 4, dans lequel l'amplification comprend une réaction en chaîne avec une polymérase (PCR).

6. Procédé suivant les revendications 1 à 4, dans lequel les amorces pour l'amplification sont situées sur une puce.

7. Procédé suivant les revendications 4 à 6, dans lequel l'amplification comprend : (i) le mélange d'une amorce d'amplification ou d'une paire d'amorces d'amplification avec une matrice d'acide nucléique isolée de l'échantillon biologique, l'amorce ou la paire d'amorces étant complémentaire ou partiellement complémentaire d'une région proximale au, ou comprenant le, polymorphisme, et étant capable de déclencher la polymérisation d'acide nucléique par une polymérase sur la matrice d'acide nucléique ; et b) l'extension de l'amorce ou de la paire d'amorces dans une réaction de polymérisation d'ADN comprenant une polymérase et l'acide nucléique de matrice pour produire l'amplicon.

8. Procédé suivant la revendication 7, dans lequel l'amplicon est détecté par un processus qui comprend une ou plusieurs : de l'hybridation de l'amplicon avec un réseau, de la digestion de l'amplicon avec une enzyme de restriction, et de l'analyse par PCR en temps réel.

9. Procédé suivant la revendication 4, dans lequel l'amplification comprend la conduite d'une réaction en chaîne avec une polymérase (PCR), d'une PCR avec transcriptase inverse (RT-PCR) ou d'une réaction en chaîne avec une ligase (LCR) en utilisant un acide nucléique isolé de l'organisme ou de l'échantillon biologique comme matrice dans la PCR, la RT-PCR ou la LCR.

10. Procédé suivant les revendications 4 à 9, dans lequel ledit échantillon est obtenu à partir de salive ou de sang.

11. Procédé pour sélectionner une thérapie dans le traitement de la DMLA avec des anticorps anti-facteur D, des anticorps anti-VEGF, une CRIg, et une fusion CRIg-Ig, dans lequel un sujet humain a été identifié suivant un procédé des revendications 1 à 10.

12. Procédé suivant la revendication 11, dans lequel la thérapie choisie est une thérapie par anticorps anti-facteur D.
